# EUROPEAN PATENT APPLICATION

(11) **EP 4 772 513 A1**
(43) Date of publication of application: **08.07.2026**
(21) Application number: 24858639.8
(22) Date of filing: 28.08.2024
(51) Int. Cl.: C07D 491/052, C07D 491/147, A61K 31/4353, A61P 35/00

(54) **PI3K-ALPHA INHIBITOR AND USE THEREOF**

(30) Priority: 28.08.2023 CN 202311088252
(71) Applicant: TYK Medicines (Zhengzhou), Inc., Zhengzhou, Henan 451162 (CN); TYK Medicines, Inc., Huzhou, Zhejiang 313100 (CN)
(72) Inventor: NIU, Chengshan, Zhengzhou, Henan 451162 (CN); ZHENG, Maolin, Zhengzhou, Henan 451162 (CN); CHEN, Mingtao, Zhengzhou, Henan 451162 (CN); LI, Yuanjie, Zhengzhou, Henan 451162 (CN); GUO, Zhongwei, Zhengzhou, Henan 451162 (CN); DONG, Shengli, Huzhou, Zhejiang 313100 (CN); JI, Kaige, Zhengzhou, Henan 451162 (CN); YIN, Zhou, Huzhou, Zhejiang 313100 (CN); CHEN, Shaoqing, Huzhou, Zhejiang 313100 (CN); LI, Jun, Huzhou, Zhejiang 313100 (CN); WU, Yusheng, Huzhou, Zhejiang 313100 (CN)
(74) Representative: dompatent
(86) International application number: PCT/CN2024/115255
(87) International publication number: WO 2025/045106

(57) **Abstract**

A PI3Kα inhibitor and the use thereof. The inhibitor has a structure as shown in formula (I), has a good selective inhibitory activity on mutant PI3Kα, and can be used for preparing antitumor drugs.

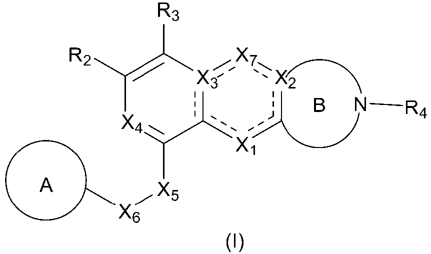

## Description

### FIELD OF THE INVENTION

The present invention relates to the field of pharmaceutical technology, specifically, it relates to a class of compounds used as PI3Kα inhibitors, and the stereoisomers, deuterated compounds, pharmaceutically acceptable salts, solvates or prodrugs thereof, to the pharmaceutical compositions comprising such compounds and salts, and to the uses thereof, as well as their uses in regulating PI3Kα activity or treating PI3Kα-related diseases.

### BACKGROUND OF THE INVENTION

Phosphatidylinositol 3-kinases (PI3Ks) belong to a unique and conserved family of intracellular lipid kinases that phosphorylate the 3'-hydroxyl group of phosphatidylinositol or phosphoinositide. They play critical roles in many fundamental biological processes, including proliferation, survival, differentiation, and metabolism. PI3Ks are currently classified into class IA, class IB, class II, and class III. Class I PI3Ks (p110α, p110β, p110δ, and p110γ) are typically activated by tyrosine kinases or G protein-coupled receptors to generate PIP3, which binds to downstream effectors, such as those in the Akt/PDK1, mTOR, Tec family kinases, and Rho family GTPase pathways. Class II and class III PI3Ks play key roles in intracellular trafficking by synthesizing PI(3)P and PI(3,4)P2. Among them, the class IA family consists of three isoforms: α, β, and δ. The class IB family includes only the γ isoform. Among the various PI3K subfamily proteins, PI3Kα is a heterodimer comprising the p110α catalytic subunit and the p85α regulatory subunit. The PIK3CA gene encoding the p110α subunit is mutated at a rate of approximately 30% in human cancers, including colorectal cancer, glioblastoma, and gastric cancer etc.. Importantly, hyperactivation of PI3Kα is directly associated with resistance to current therapeutic agents and poor prognosis.

Mutations in the gene encoding PI3Kα mainly occur as point mutations in several hotspots of the helical and kinase domains, such as E542K, E545K, and H1047R. These mutations have been identified as oncogenic gain-of-function mutations. Due to the high incidence of PI3Kα mutations, targeting this pathway may offer valuable therapeutic opportunities. Other PI3K isoforms, such as PI3Kδ or PI3Kγ, are predominantly expressed in hematopoietic cells, whereas PI3Kα and PI3Kβ are constitutively expressed.

Owing to the central role of PI3Kα in regulating systemic glucose homeostasis, PI3K inhibition commonly leads to hyperglycemia and/or hyperinsulinemia in patients. Elevated circulating insulin levels may promote mitosis and/or anti-apoptosis of cancer cells, thereby attenuating the antiproliferative effects of PI3K inhibitors.

In the PI3Kα-mutant cancers environment, one approach to overcome the compensatory production of insulin and/or glucose following systemic inhibition of PI3Kα is to develop inhibitors with higher selectivity for mutant PI3Kα over wild-type PI3Kα. This would create a wider window for dose-dependent selective inhibition of pathological signaling of mutant PI3Kα in cancer cells without affecting wild-type PI3Kα, which controls systemic metabolism in host tissues, thereby limiting toxicity and allowing higher doses and more complete target inhibition.

Current PI3Kα inhibitors exhibit nearly equivalent potency against wild-type and mutant type. Mutant-selective inhibitors have been challenging to develop because the PI3Kα mutation sites are distal to the active site. Therefore, inhibitors targeting allosteric binding pockets near known mutations (e.g., H1047R, E542K, and E545K) may offer novel avenues for the selective inhibition of PI3Kα. Thus, targeting allosteric binding pockets of mutant PI3Kα may, in turn, provide valuable therapeutic targets for drug development.

### SUMMARY OF THE INVENTION

The object of the present invention is to provide a compound of Formula (I) and the preparation method therefor, as well as the use thereof as the selective PI3Kα inhibitor drugs.

In the first aspect of the present invention, provided is a compound of Formula (I), or a pharmaceutically acceptable salt, a stereoisomer, a deuterated compound, a solvate or a prodrug thereof, 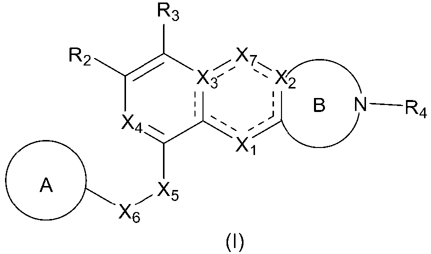 wherein,
X₁ is selected from the group consisting of: N, O, S, NR₁₁, and C(R₁₁)_{q};
X₂ and X₃ are each independently selected from the group consisting of: N and C;
X₄ is N or CR;
X₅ is selected from the group consisting of: absent, O, NR₅₁, C₁₋₆ alkylene, C₃₋₆ cycloalkylene, and 3-6 membered heterocycloalkylene containing 1-4 heteroatoms selected from N, O and S, wherein the C₁₋₆ alkylene, C₃₋₆ cycloalkylene, and 3-6 membered heterocycloalkylene are optionally substituted with 1, 2, 3, 4, 5 or 6 R₅₂ groups;
X₆ is selected from the group consisting of: absent, NR₆₁, and C₁₋₆ alkylene, wherein the C₁₋₆ alkylene is optionally substituted with 1, 2, 3, 4, 5 or 6 R₆₂ groups;
X₇ is selected from the group consisting of: C=O, C=S, and CR₇₁;
ring B is a 5-7 membered heterocycloalkyl containing one N atom and 0-3 additional heteroatoms selected from N, O and S, which is optionally substituted with one or more R₅ groups;
ring A is selected from the group consisting of: C₆₋₁₀ aryl, 5-10 membered heteroaryl containing 1-4 heteroatoms selected from N, O and S, and 5-10 membered heterocycloalkyl containing 1-4 heteroatoms selected from N, O and S; wherein the C₆₋₁₀ aryl and 5-10 membered heteroaryl are optionally substituted with m R₆ groups;
R, R₂, R₃, R₁₁, R₅₂ and R₆₂ are each independently selected from the group consisting of: H, deuterium, halogen, oxo (=O), -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, halogenated C₃₋₆ cycloalkyl, and deuterated C₃₋₆ cycloalkyl;
alternatively, R and R₂ together with the atoms to which they are attached form a C₄₋₆ cycloalkyl or a 4-6 membered heterocycloalkyl containing 1-4 heteroatoms selected from N, O and S; wherein the C₄₋₆ cycloalkyl and 4-6 membered heterocycloalkyl are optionally substituted with 1, 2, 3, 4, 5 or 6 R₆ groups;
alternatively, R₂ and R₃ together with the atoms to which they are attached form a C₄₋₆ cycloalkyl or a 4-6 membered heterocycloalkyl containing 1-4 heteroatoms selected from N, O and S; wherein the C₄₋₆ cycloalkyl and 4-6 membered heterocycloalkyl are optionally substituted with 1, 2, 3, 4, 5 or 6 R₆ groups;
R₅₁ and R₆₁ are each independently selected from the group consisting of: H, -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl;
R₇₁ is selected from the group consisting of: H, halogen, -CN, -NO₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, and -C(O)R₈;
R₄ is selected from the group consisting of: -L-(C₁₋₆ alkyl), -L-C₆₋₁₀ aryl, -L-(5-10 membered heteroaryl containing 1-4 heteroatoms selected from N, O and S), -L-(saturated or partially saturated C₃₋₈ cycloalkyl), and -L-(saturated or partially saturated 4-8 membered heterocycloalkyl containing 1-4 heteroatoms selected from N, O and S); wherein the C₁₋₆ alkyl, C₆₋₁₀ aryl, 5-10 membered heteroaryl, C₃₋₈ cycloalkyl, and 4-8 membered heterocycloalkyl are optionally substituted with n R₇ groups;
each R₅ is independently selected from the group consisting of: H, deuterium, halogen, -CN, oxo (=O), C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, halogenated C₃₋₆ cycloalkyl, and 3-8 membered heterocycloalkyl containing 1-3 heteroatoms selected from N, O and S;
each R₆ is independently selected from the group consisting of: H, deuterium, halogen, -CN, - NO₂, C₁₋₆ alkyl, halogenated C₁₋₆ alkyl, deuterated C₁₋₆ alkyl, C₁₋₆ alkoxy, halogenated C₁₋₆ alkoxy, - C(O)R₈, -C(O)OR₈, -C(O)NR₈R₉, -SO₂R₈, -S(O)(NR₈)R₉, -SO₂NR₈R₉, and -P(O)R₈R₉;
each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -NR₈R₉, =NR₈, - C(O)R₈, -C(O)OR₈, -C(O)NR₈R₉, -NR₈C(O)R₉, -SR₈, -SO₂R₈, -NR₈SO₂R₉, -SO₂NR₈R₉, - NR₈SO₂NR₉R₁₀, -P(O)R₈R₉, -L₁-(C₁₋₆ alkyl), -L₁-(saturated or partially saturated C₃₋₁₀ cycloalkyl), - L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-12 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, 3-6 membered heterocycloalkyl containing 1-4 heteroatoms selected from N, O and S, - SO₂R₈, -C(O)R₈, -C(O)OR₈, -C(O)NR₈R₉, and -NR₈R₉;
alternatively, two R₇ groups together with the atoms to which they are attached form C₃₋₆ cycloalkyl, C₆₋₁₀ aryl, 3-6 membered heterocycloalkyl containing 1-4 heteroatoms selected from N, O and S, or 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NR₈R₉, =NR₈, -C(O)R₈, -C(O)OR₈, -C(O)NR₈R₉, -NR₈C(O)R₉, -SR₈, -SO₂R₈, - NR₈SO₂R₉, -SO₂NR₈R₉, -NR₈SO₂NR₉R₁₀, -P(O)R₈R₉, C₁₋₆ alkyl, C₁₋₆ alkoxy, C₁₋₆ heteroalkyl containing 1-4 heteroatoms selected from N, O and S, halogenated C₁₋₆ alkyl, halogenated C₁₋₆ alkoxy, halogenated C₁₋₆ heteroalkyl containing 1-4 heteroatoms selected from N, O and S, C₃₋₆ cycloalkyl, and 3-6 membered heterocycloalkyl containing 1-4 heteroatoms selected from N, O and S;
L and L₁ are each independently a bond or selected from the group consisting of: C₁₋₆ alkylene, C₂₋₆ alkenylene, C₂₋₆ alkynylene, 1-6 membered heteroalkylene containing 1-4 heteroatoms selected from N, O and S, -NR₈-, -O-, -C(O)-, -C(O)NR₈-, -C(O)O-, -NR₈C(O)-, -(SO₂)-, -SO₂NR₈-, -NR₈SO₂-, -NR₈SO₂NR₉-, and -S(O)(NR₈)-, wherein the C₁₋₆ alkylene, C₂₋₆ alkenylene, C₂₋₆ alkynylene, and 1-6 membered heteroalkylene are optionally substituted with 0-6 substituents selected from the group consisting of: deuterium, halogen, =O, -CN, -OH, -NR₈R₉, and =NR₈;
each of R₈, R₉ and R₁₀ is independently selected from the group consisting of: H, C₁₋₆ alkyl, halogenated C₁₋₆ alkyl, C₃₋₆ cycloalkyl, C₆₋₁₀ aryl, 3-6 membered heterocycloalkyl containing 1-4 heteroatoms selected from N, O and S, and 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ alkylamino, halogenated C₁₋₆ alkylamino, C₁₋₆ alkylthio, halogenated C₁₋₆ alkylthio, C₁₋₆ heteroalkyl containing 1-4 heteroatoms selected from N, O and S, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S, halogenated C₃₋₆ cycloalkyl, halogenated 3-6 membered heterocycloalkyl containing 1-4 heteroatoms selected from N, O and S;
alternatively, R₈ and R₉ together with the heteroatom to which they are attached form a 3-6 membered heterocycloalkyl containing 1-4 heteroatoms selected from N, O and S, wherein the 3-6 membered heterocycloalkyl is optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ alkylamino, halogenated C₁₋₆ alkylamino, C₁₋₆ alkylthio, halogenated C₁₋₆ alkylthio, C₁₋₆ heteroalkyl containing 1-4 heteroatoms selected from N, O and S, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S, halogenated C₃₋₆ cycloalkyl, and halogenated 3-6 membered heterocycloalkyl containing 1-4 heteroatoms selected from N, O and S;
alternatively, R₉ and R₁₀ together with the heteroatom to which they are attached form a 3-6 membered heterocycloalkyl containing 1-4 heteroatoms selected from N, O and S, wherein the 3-6 membered heterocycloalkyl is optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ alkylamino, halogenated C₁₋₆ alkylamino, C₁₋₆ alkylthio, halogenated C₁₋₆ alkylthio, C₁₋₆ heteroalkyl containing 1-4 heteroatoms selected from N, O and S, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S, halogenated C₃₋₆ cycloalkyl, and halogenated 3-6 membered heterocycloalkyl containing 1-4 heteroatoms selected from N, O and S;
each "-̅ -̅ -̅" is independently a single bond or a double bond;
each of m, n and q is independently selected from the group consisting of: 0, 1, 2, 3, 4, and 5.

In another preferred embodiment, the compound has a structure selected from the group consisting of: 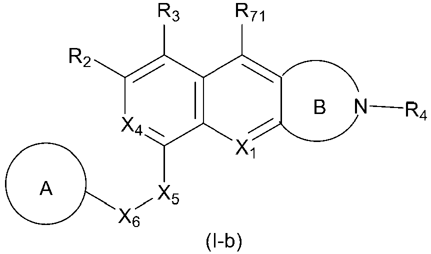
wherein, X₄, X₅, X₆, R₂, R₃, R₄, ring A, and ring B are as defined above;
X₁ is selected from the group consisting of: N and CR₁₁;
R₁₁ is selected from the group consisting of: H, halogen, -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl;
R₇₁ is selected from the group consisting of: H, halogen, -CN, -NO₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, and -C(O)R₈;
R₈ is selected from the group consisting of: H, C₁₋₆ alkyl, halogenated C₁₋₆ alkyl, C₃₋₆ cycloalkyl, and 3-6 membered heterocycloalkyl containing 1-4 heteroatoms selected from N, O and S, wherein the C₃₋₆ cycloalkyl and 3-6 membered heterocycloalkyl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ heteroalkyl containing 1-4 heteroatoms selected from N, O and S, and halogenated C₁₋₆ heteroalkyl containing 1-4 heteroatoms selected from N, O and S.

In another preferred embodiment, the compound has a structure selected from the group consisting of: 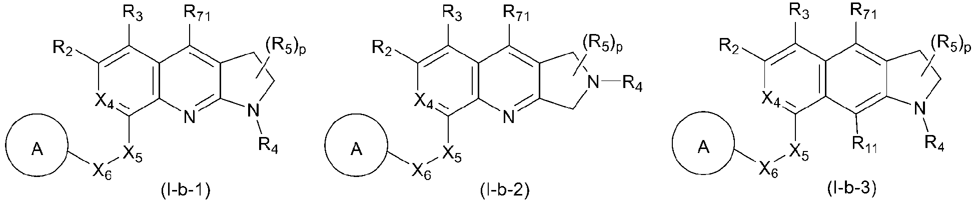 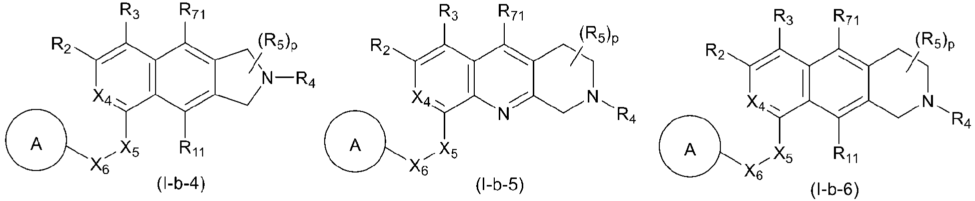 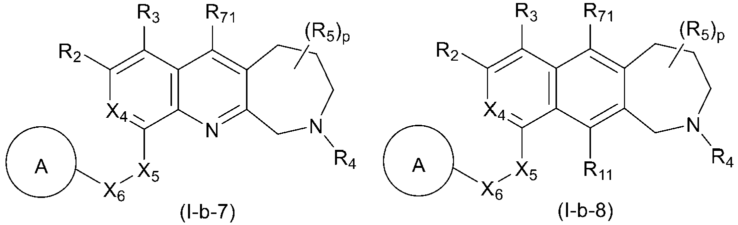
the structure 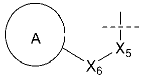 is selected from the following configurations: 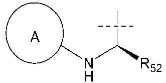 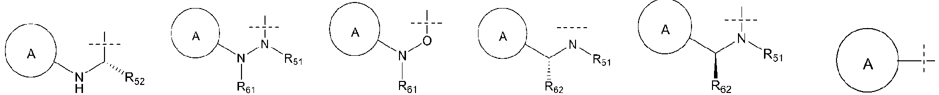 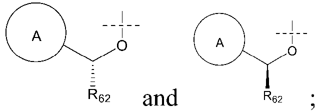
wherein, X₄, X₅, X₆, R₂, R₃, R₄, R₅, R₇₁, ring A, and p are as defined above;
R₅₂ and R₆₂ are each independently selected from the group consisting of: H, halogen, oxo (=O), -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl;
R₅₁ and R₆₁ are each independently selected from the group consisting of: H, -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl.

In another preferred embodiment, the compound has a structure selected from the group consisting of: 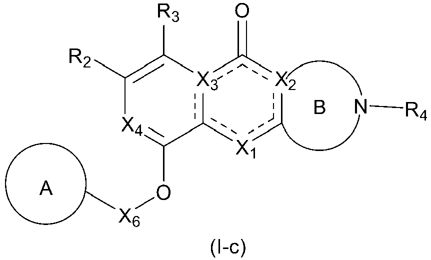 wherein, X₁, X₂, X₃, X₄, X₆, R₂, R₃, R₄, ring A, and ring B are as defined above.

In another preferred embodiment, the compound has a structure selected from the group consisting of: 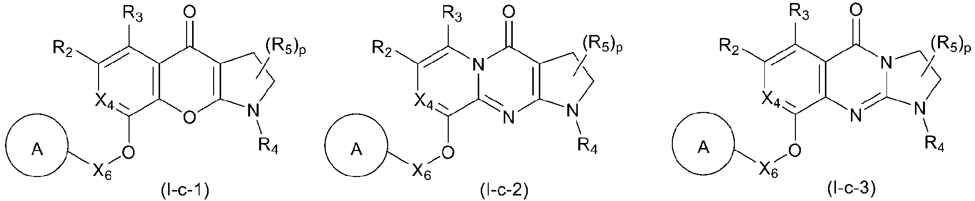 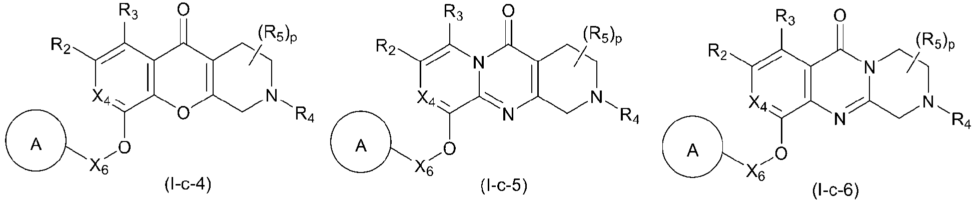 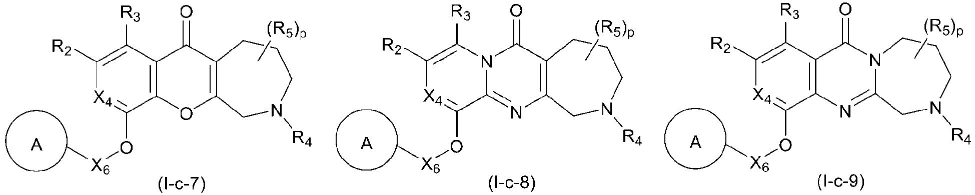
the structure 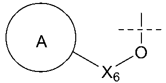 is selected from the following configurations: 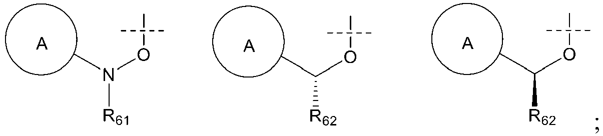
wherein, X₄, X₆, R₂, R₃, R₄, R₅, ring A, and p are as defined above;
R₆₂ is selected from the group consisting of: H, halogen, oxo (=O), -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl;
R₆₁ is selected from the group consisting of: H, -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl.

In another preferred embodiment, the compound has a structure selected from the group consisting of: 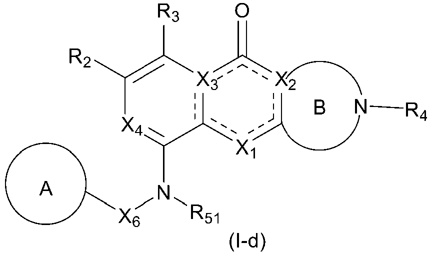 wherein, X₁, X₂, X₃, X₄, X₆, R₂, R₃, R₄, R₅₁, ring A, and ring B are as defined above.

In another preferred embodiment, the compound has a structure selected from the group consisting of: 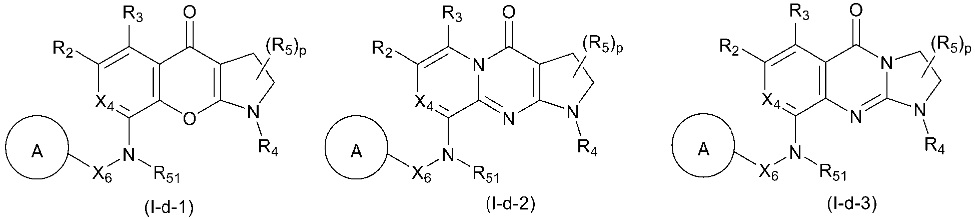 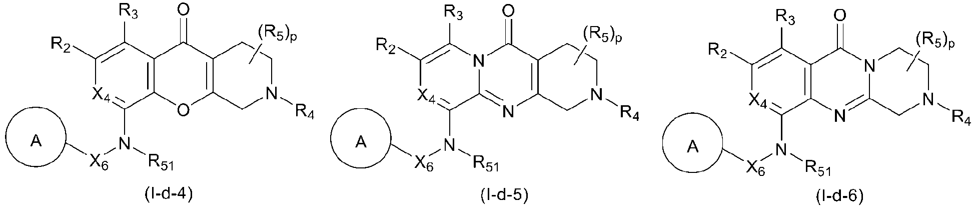 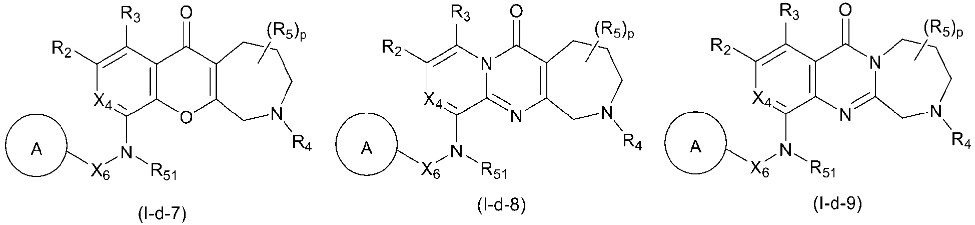
the structure 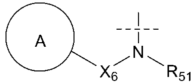 is selected from the following configurations: 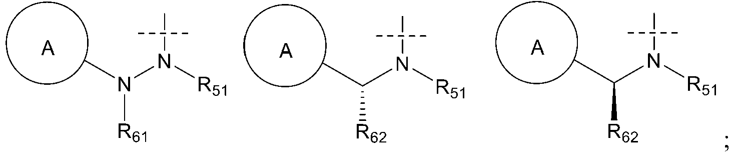
wherein, X₄, X₆, R₂, R₃, R₄, R₅, ring A, and p are as defined above;
R₅₁ and R₆₁ are each independently selected from the group consisting of: H, -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl;
R₆₂ is selected from the group consisting of: H, halogen, oxo (=O), -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl.

In another preferred embodiment, the compound is a compound of Formula (I-a), or a pharmaceutically acceptable salt, a stereoisomer, a deuterated compound, a solvate or a prodrug thereof, 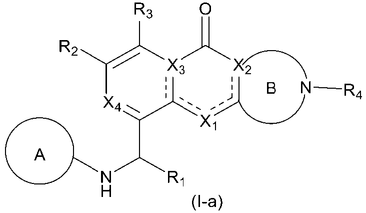 wherein,
X₁ is selected from the group consisting of: N, O, and C(R₁₁)_{q};
X₂ and X₃ are each independently selected from the group consisting of: N and C;
X₄ is N or CR;
ring B is a 5-7 membered heterocycloalkyl containing one N atom and 0-3 additional heteroatoms selected from N, O and S, which is optionally substituted with one or more R₅ groups;
ring A is selected from the group consisting of: C₆₋₁₀ aryl and 5-8 membered heteroaryl containing 1-4 heteroatoms selected from N, O and S; wherein the C₆₋₁₀ aryl and 5-8 membered heteroaryl are optionally substituted with m R₆ groups;
R₁ is selected from the group consisting of: H, deuterium, halogen, -CN, C₁₋₆ alkyl, halogenated C₁₋₆ alkyl, deuterated C₁₋₆ alkyl, C₁₋₆ alkoxy, and halogenated C₁₋₆ alkoxy;
R, R₂, R₃ and R₁₁ are each independently selected from the group consisting of: H, halogen, -CN, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl;
R₄ is selected from the group consisting of: -L-(C₁₋₆ alkyl), -L-C₆₋₁₀ aryl, -L-(5-8 membered heteroaryl containing 1-4 heteroatoms selected from N, O and S), -L-(saturated or partially saturated C₃₋₈ cycloalkyl), and -L-(saturated or partially saturated 4-8 membered heterocycloalkyl containing 1-4 heteroatoms selected from N, O and S); wherein the C₁₋₆ alkyl, C₆₋₁₀ aryl, 5-8 membered heteroaryl, C₃₋₈ cycloalkyl, and 4-8 membered heterocycloalkyl are optionally substituted with n R₇ groups;
each R₅ is independently selected from the group consisting of: H, deuterium, halogen, -CN, oxo (=O), C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, halogenated C₃₋₆ cycloalkyl, and 3-8 membered heterocycloalkyl containing 1-3 heteroatoms selected from N, O and S;
each R₆ is independently selected from the group consisting of: H, deuterium, halogen, -CN, - NO₂, C₁₋₆ alkyl, halogenated C₁₋₆ alkyl, deuterated C₁₋₆ alkyl, C₁₋₆ alkoxy, halogenated C₁₋₆ alkoxy, - C(O)R₈, -C(O)OR₈, -C(O)NR₈R₉, -SO₂R₈, -S(O)(NR₈)R₉, -SO₂NR₈R₉, and -P(O)R₈R₉;
each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -NR₈R₉, =NR₈, - C(O)R₈, -C(O)OR₈, -C(O)NR₈R₉, -NR₈C(O)R₉, -SR₈, -SO₂R₈, -NR₈SO₂R₉, -SO₂NR₈R₉, - NR₈SO₂NR₉R₁₀, -P(O)R₈R₉, -L₁-(C₁₋₆ alkyl), -L₁-(saturated or partially saturated C₃₋₁₀ cycloalkyl), - L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-10 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, 3-6 membered heterocycloalkyl containing 1-4 heteroatoms selected from N, O and S, - SO₂R₈, -C(O)R₈, -C(O)OR₈, -C(O)NR₈R₉, and -NR₈R₉;
alternatively, two R₇ groups together with the atoms to which they are attached form C₃₋₆ cycloalkyl, C₆₋₁₀ aryl, 3-6 membered heterocycloalkyl containing 1-4 heteroatoms selected from N, O and S, or 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NR₈R₉, =NR₈, -C(O)R₈, -C(O)OR₈, -C(O)NR₈R₉, -NR₈C(O)R₉, -SR₈, -SO₂R₈, - NR₈SO₂R₉, -SO₂NR₈R₉, -NR₈SO₂NR₉R₁₉, -P(O)R₈R₉, C₁₋₆ alkyl, C₁₋₆ alkoxy, C₁₋₆ heteroalkyl containing 1-4 heteroatoms selected from N, O and S, halogenated C₁₋₆ alkyl, halogenated C₁₋₆ alkoxy, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S;
L and L₁ are each independently a bond or selected from the group consisting of: C₁₋₆ alkylene, 1-6 membered heteroalkylene containing 1-4 heteroatoms selected from N, O and S, -NR₈-, -O-, - C(O)-, -C(O)NR₈-, -C(O)O-, -NR₈C(O)-, -(SO₂)-, -SO₂NR₈-, -NR₈SO₂-, -NR₈SO₂NR₉-, and - S(O)(NR₈)-;
each of R₈, R₉ and R₁₀ is independently selected from the group consisting of: H, C₁₋₆ alkyl, halogenated C₁₋₆ alkyl, C₃₋₆ cycloalkyl, C₆₋₁₀ aryl, 3-6 membered heterocycloalkyl containing 1-4 heteroatoms selected from N, O and S, and 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ heteroalkyl containing 1-4 heteroatoms selected from N, O and S, and halogenated C₁₋₆ heteroalkyl containing 1-4 heteroatoms selected from N, O and S;
alternatively, R₈ and R₉ together with the heteroatom to which they are attached form a 3-6 membered heterocycloalkyl containing 1-4 heteroatoms selected from N, O and S;
alternatively, R₉ and R₁₀ together with the heteroatom to which they are attached form a 3-6 membered heterocycloalkyl containing 1-4 heteroatoms selected from N, O and S;
" -̅ -̅ -̅ " is a single bond or a double bond;
each of m, n and q is independently selected from the group consisting of: 0, 1, 2, 3, 4, and 5.

In another preferred embodiment, ring A is selected from the group consisting of: C₆₋₁₀ aryl and 5-10 membered heteroaryl containing 1-4 heteroatoms selected from N, O and S; wherein the C₆₋₁₀ aryl and 5-10 membered heteroaryl are optionally substituted with m R₆ groups.

In another preferred embodiment, each R₆ is independently selected from the group consisting of: H, deuterium, halogen, C₁₋₆ alkyl, halogenated C₁₋₆ alkyl, deuterated C₁₋₆ alkyl, -C(O)R₈, -C(O)OR₈, and -SO₂R₈.

In another preferred embodiment, each of R₈, R₉ and R₁₀ is each independently selected from the group consisting of: H, C₁₋₆ alkyl, and halogenated C₁₋₆ alkyl.

In another preferred embodiment, X₆ is selected from the group consisting of: NR₆₁, and C₁₋₆ alkylene.

In another preferred embodiment, R₅₁ and R₆₁ are each independently selected from the group consisting of: H, C₁₋₆ alkyl, halogenated C₁₋₆ alkyl, and deuterated C₁₋₆ alkyl.

In another preferred embodiment, X₅ is C₁₋₆ alkylene, wherein the C₁₋₆ alkylene is optionally substituted with 1, 2, 3, 4, 5 or 6 R₅₂ groups.

In another preferred embodiment, each of R, R₂, R₃, R₁₁, R₅₂ and R₆₂ are each independently selected from the group consisting of: H, deuterium, halogen, C₁₋₆ alkyl, halogenated C₁₋₆ alkyl, and deuterated C₁₋₆ alkyl.

In another preferred embodiment, X₄ is CR.

In another preferred embodiment, R is selected from the group consisting of: H, C₁₋₃ alkyl, halogenated C₁₋₃ alkyl, deuterated C₁₋₃ alkyl, C₁₋₃ alkoxy, halogenated C₁₋₃ alkoxy, deuterated C₁₋₃ alkoxy, C₃₋₅ cycloalkyl, and halogenated C₃₋₅ cycloalkyl.

In another preferred embodiment, R is selected from the group consisting of: H, C₁₋₃ alkyl, halogenated C₁₋₃ alkyl, and deuterated C₁₋₃ alkyl.

In another preferred embodiment, R is H.

In another preferred embodiment, X₄ is CH.

In another preferred embodiment, X₁ is selected from the group consisting of: N, O, and C(R₁₁)_{q}.

In another preferred embodiment, X₁ is selected from the group consisting of: N and O.

In another preferred embodiment, X₂ and X₃ are each independently selected from the group consisting of: N and C.

In another preferred embodiment, X₇ is selected from the group consisting of: C=O and C=S.

In another preferred embodiment, ring B is a 5-7 membered heterocycloalkyl containing one N atom and 0-3 additional heteroatoms selected from N, O and S, which is optionally substituted with one or more R₅ groups.

In another preferred embodiment, each R₅ is independently selected from the group consisting of: H, deuterium, halogen, oxo (=O), C₁₋₆ alkyl, halogenated C₁₋₆ alkyl, and deuterated C₁₋₆ alkyl.

In another preferred embodiment, R₄ is selected from the group consisting of: -L-(C₁₋₆ alkyl), - L-C₆₋₁₀ aryl, -L-(5-10 membered heteroaryl containing 1-4 heteroatoms selected from N, O and S), - L-(saturated or partially saturated C₃₋₈ cycloalkyl), and -L-(saturated or partially saturated 4-8 membered heterocycloalkyl containing 1-4 heteroatoms selected from N, O and S); wherein the C₁₋₆ alkyl, C₆₋₁₀ aryl, 5-10 membered heteroaryl, C₃₋₈ cycloalkyl, and 4-8 membered heterocycloalkyl are optionally substituted with n R₇ groups.

In another preferred embodiment, L and L₁ are each independently a bond or selected from the group consisting of: C₁₋₆ alkylene, -NR₈-, -O-, -C(O)-, -C(O)NR₈-, -C(O)O-, -NR₈C(O)-, -(SO₂)-, - SO₂NR₈-, -NR₈SO₂-, -NR₈SO₂NR₉-, and -S(O)(NR₈)-.

In another preferred embodiment, L and L₁ are each independently a bond or selected from the group consisting of: C₁₋₆ alkylene, -C(O)-, -C(O)NR₈-, -C(O)O-, -NR₈C(O)-, -(SO₂)-, -SO₂NR₈-, - NR₈SO₂-, -NR₈SO₂NR₉-, and -S(O)(NR₈)-.

In another preferred embodiment, each R₇ is independently selected from the group consisting of: hydrogen, deuterium, halogen, oxo (=O), -CN, -OH, C₁₋₆ alkyl, C₁₋₆ alkoxy, halogenated C₁₋₆ alkyl, halogenated C₁₋₆ alkoxy, deuterated C₁₋₆ alkyl, -C(O)NR₈R₉, -NR₈C(O)R₉, -SO₂R₈, -NR₈SO₂R₉, and - SO₂NR₈R₉, wherein the C₁₋₆ alkyl is optionally substituted with 0-6 substituents selected from the group consisting of: -OH, C₁₋₆ alkyl, and halogenated C₁₋₆ alkyl;
alternatively, two R₇ groups together with the atoms to which they are attached form C₃₋₆ cycloalkyl, or 3-6 membered heterocycloalkyl containing 1-4 heteroatoms selected from N, O and S.

In another preferred embodiment, the compound has a structure selected from the group consisting of: 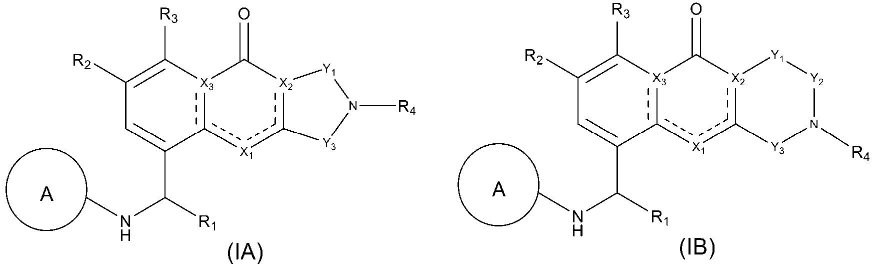 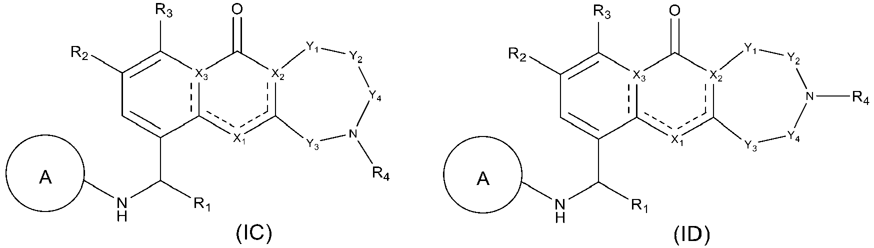 wherein,
X₁ is selected from the group consisting of: N, O, and C(R₁₁)_{q};
X₂ and X₃ are each independently selected from the group consisting of: N and C;
Y₁, Y₂, Y₃ and Y₄ are each independently selected from the group consisting of: O, C(R₅)₂, CHR₅, C(=O), S(=O), S(=O)₂, and NR₅;
ring A is selected from the group consisting of: phenyl and 5-6 membered heteroaryl containing 1-4 heteroatoms selected from N, O and S; wherein the phenyl and 5-6 membered heteroaryl are optionally substituted with m R₆ groups;
R₁ is selected from the group consisting of: H, deuterium, halogen, -CN, C₁₋₃ alkyl, halogenated C₁₋₃ alkyl, deuterated C₁₋₃ alkyl, C₁₋₃ alkoxy, and halogenated C₁₋₃ alkoxy;
R₂, R₃ and R₁₁ are each independently selected from the group consisting of: H, halogen, -CN, C₁₋₃ alkyl, halogenated C₁₋₃ alkyl, deuterated C₁₋₃ alkyl, C₁₋₃ alkoxy, halogenated C₁₋₃ alkoxy, deuterated C₁₋₃ alkoxy, C₃₋₅ cycloalkyl, and halogenated C₃₋₅ cycloalkyl;
R₄ is selected from the group consisting of: -L-(C₁₋₆ alkyl), -L-phenyl, -L-(5-6 membered heteroaryl containing 1-4 heteroatoms selected from N, O and S), -L-(saturated or partially saturated C₃₋₆ cycloalkyl), and -L-(saturated or partially saturated 4-6 membered heterocycloalkyl containing 1-4 heteroatoms selected from N, O and S); wherein the C₁₋₆ alkyl, phenyl, 5-6 membered heteroaryl, C₃₋₆ cycloalkyl, and 4-6 membered heterocycloalkyl are optionally substituted with n R₇ groups;
each R₅ is independently selected from the group consisting of: H, deuterium, halogen, -CN, oxo (=O), C₁₋₃ alkyl, halogenated C₁₋₃ alkyl, deuterated C₁₋₃ alkyl, C₁₋₃ alkoxy, halogenated C₁₋₃ alkoxy, deuterated C₁₋₃ alkoxy, C₃₋₅ cycloalkyl, halogenated C₃₋₅ cycloalkyl, and 3-5 membered heterocycloalkyl containing 1-3 heteroatoms selected from N, O and S;
each R₆ is independently selected from the group consisting of: H, deuterium, halogen, -CN, - NO₂, C₁₋₆ alkyl, halogenated C₁₋₆ alkyl, deuterated C₁₋₃ alkyl, C₁₋₆ alkoxy, halogenated C₁₋₆ alkoxy, - C(O)R₈, -C(O)OR₈, -C(O)NR₈R₉, -SO₂R₈, -S(O)(NR₈)R₉, -SO₂NR₈R₉, and -P(O)R₈R₉;
each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -NR₈R₉, =NR₈, - C(O)R₈, -C(O)OR₈, -C(O)NR₈R₉, -NR₈C(O)R₉, -SR₈, -SO₂R₈, -NR₈SO₂R₉, -SO₂NR₈R₉, - NR₈SO₂NR₉Riₒ, -P(O)R₈R₉, -L₁-(C₁₋₆ alkyl), -L₁-(saturated or partially saturated C₃₋₁₀ cycloalkyl), - L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-10 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, 3-6 membered heterocycloalkyl containing 1-4 heteroatoms selected from N, O and S, - SO₂R₈, -C(O)R₈, -C(O)OR₈, -C(O)NR₈R₉, and -NR₈R₉;
alternatively, two R₇ groups together with the atoms to which they are attached form C₃₋₆ cycloalkyl, C₆₋₁₀ aryl, 3-6 membered heterocycloalkyl containing 1-4 heteroatoms selected from N, O and S, or 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NR₈R₉, =NR₈, -C(O)R₈, -C(O)OR₈, -C(O)NR₈R₉, -NR₈C(O)R₉, -SR₈, -SO₂R₈, - NR₈SO₂R₉, -SO₂NR₈R₉, -NR₈SO₂NR₉R₁₀, -P(O)R₈R₉, C₁₋₆ alkyl, C₁₋₆ alkoxy, C₁₋₆ heteroalkyl containing 1-4 heteroatoms selected from N, O and S, halogenated C₁₋₆ alkyl, halogenated C₁₋₆ alkoxy, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S;
L and L₁ are each independently a bond or selected from the group consisting of: C₁₋₆ alkylene, 1-6 membered heteroalkylene containing 1-4 heteroatoms selected from N, O and S, -NR₈-, -O-, - C(O)-, -C(O)NR₈, -C(O)O-, -NR₈C(O)-, -(SO₂)-, -SO₂NR₈-, -NR₈SO₂-, and -NR₈SO₂NR₉-;
each of R₈, R₉ and R₁₀ is independently selected from the group consisting of: H, C₁₋₆ alkyl, halogenated C₁₋₆ alkyl, C₃₋₆ cycloalkyl, C₆₋₁₀ aryl, 3-6 membered heterocycloalkyl containing 1-4 heteroatoms selected from N, O and S, and 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ heteroalkyl containing 1-4 heteroatoms selected from N, O and S, and halogenated C₁₋₆ heteroalkyl containing 1-4 heteroatoms selected from N, O and S;
alternatively, R₈ and R₉ together with the heteroatom to which they are attached form a 3-6 membered heterocycloalkyl containing 1-4 heteroatoms selected from N, O and S;
alternatively, R₉ and R₁₀ together with the heteroatom to which they are attached form a 3-6 membered heterocycloalkyl containing 1-4 heteroatoms selected from N, O and S;
" -̅ -̅ -̅ " is a single bond or a double bond;
each of m, n and q is independently selected from the group consisting of: 0, 1, 2, 3, 4, and 5.

In another preferred embodiment, X₂ and X₃ are both C, or one of X₂ and X₃ is N, the other is C.

In another preferred embodiment, X₂ is C, X₃ is N.

In another preferred embodiment, Y₁, Y₂, Y₃ and Y₄ are each independently selected from the group consisting of: O, C(R₅)₂, CHR₅, and C(=O).

In another preferred embodiment, Y₁, Y₂, Y₃ and Y₄ are each independently selected from the group consisting of: O, CH₂, CHR₅, and C(=O).

In another preferred embodiment, Y₁, Y₂, Y₃ and Y₄ are each independently selected from the group consisting of: CH₂, and CHR₅.

In another preferred embodiment, one of Y₁, Y₂, Y₃ and Y₄ is O, CHR₅, or C(=O), the others are all CH₂.

In another preferred embodiment, each R₅ is independently selected from the group consisting of: H, deuterium, halogen, -CN, oxo (=O), C₁₋₃ alkyl, halogenated C₁₋₃ alkyl, deuterated C₁₋₃ alkyl, C₁₋₃ alkoxy, halogenated C₁₋₃ alkoxy, deuterated C₁₋₃ alkoxy, C₃₋₅ cycloalkyl, halogenated C₃₋₅ cycloalkyl, and 3-5 membered heterocycloalkyl containing 1-3 heteroatoms selected from N, O and S.

In another preferred embodiment, each R₅ is independently selected from the group consisting of: H, deuterium, halogen, oxo (=O), C₁₋₃ alkyl, halogenated C₁₋₃ alkyl, deuterated C₁₋₃ alkyl, C₁₋₃ alkoxy, halogenated C₁₋₃ alkoxy, and deuterated C₁₋₃ alkoxy.

In another preferred embodiment, each R₅ is independently selected from the group consisting of: H, deuterium, halogen, oxo (=O), C₁₋₃ alkyl, halogenated C₁₋₃ alkyl, and deuterated C₁₋₃ alkyl.

In another preferred embodiment, the compound has a structure selected from the group consisting of: 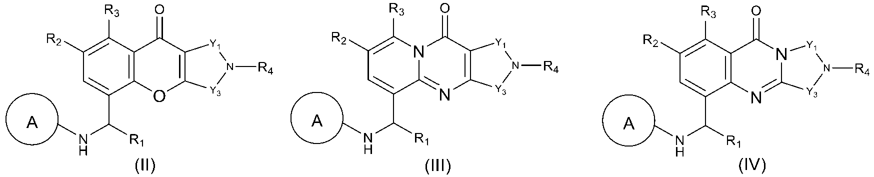 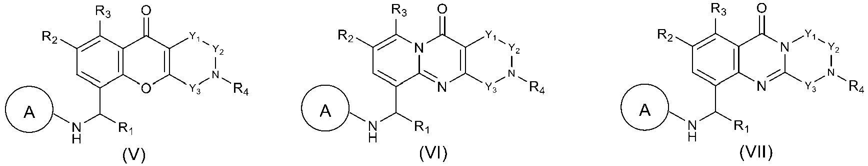 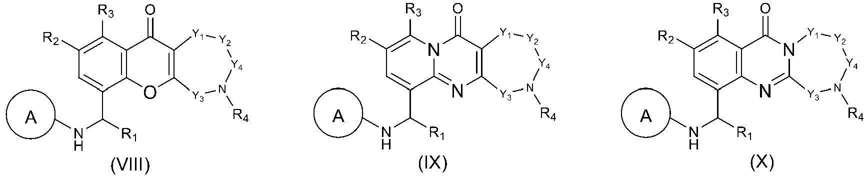 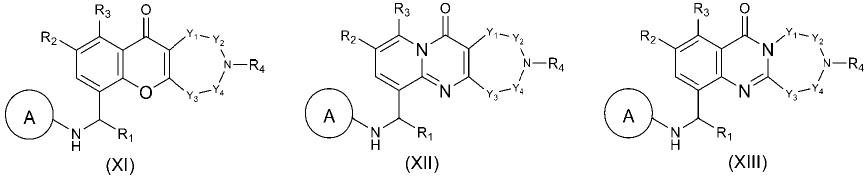 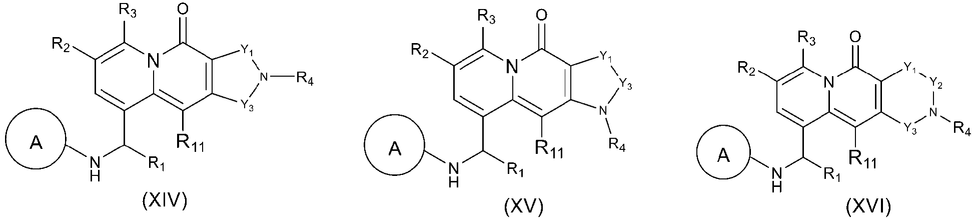 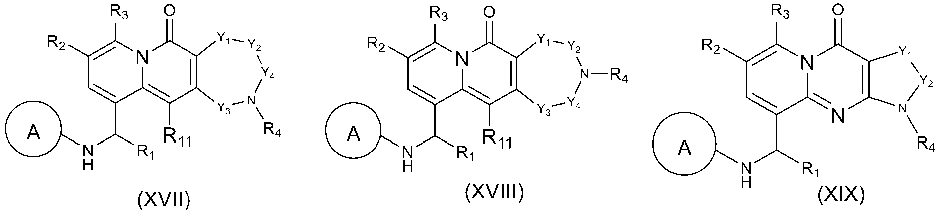
wherein, R₁, R₂, R₃, R₄, R₁₁, Y₁, Y₂, Y₃, Y₄, and ring A are as defined above;
the structure 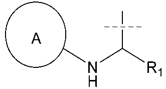 includes the following configurations: 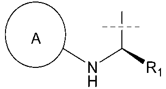 and

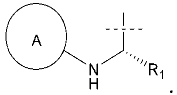

In another preferred embodiment, the structure 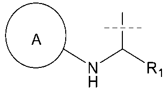 includes the following configurations:

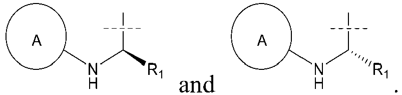

In another preferred embodiment, the compound has a structure selected from the group consisting of: 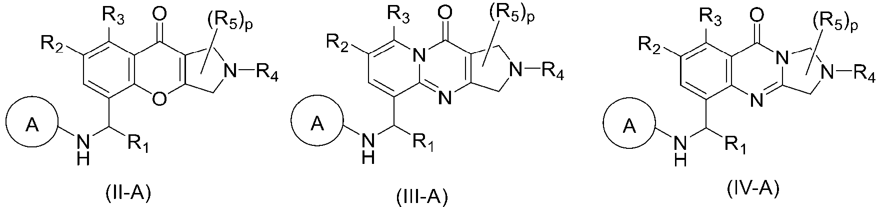 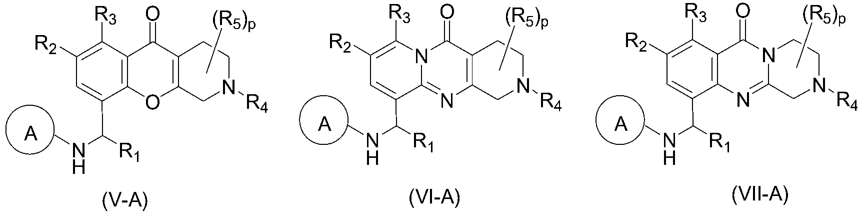 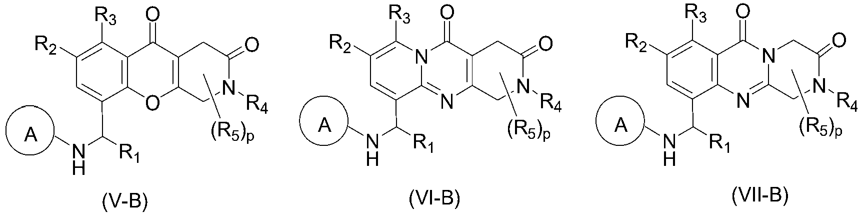 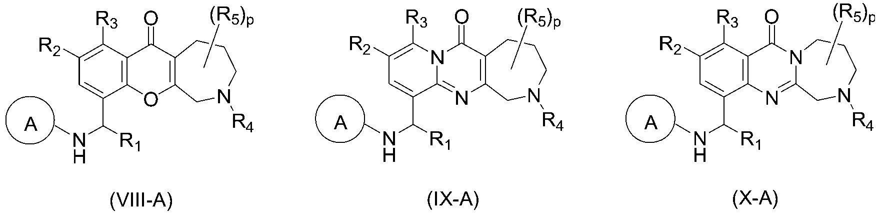 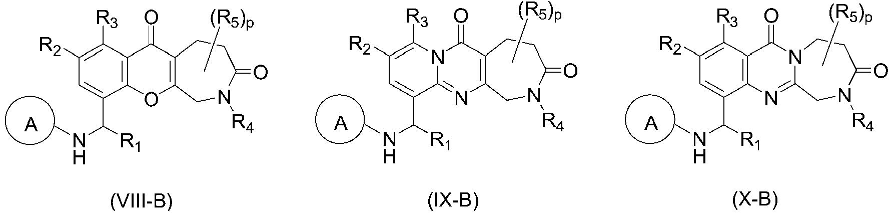 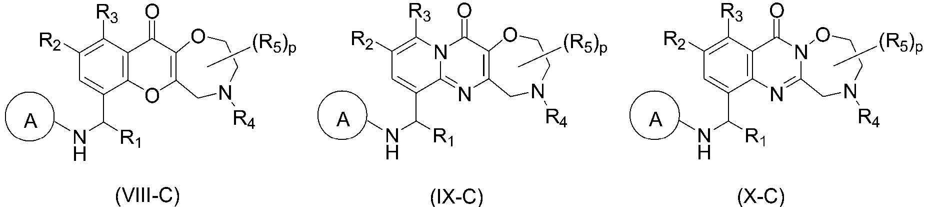 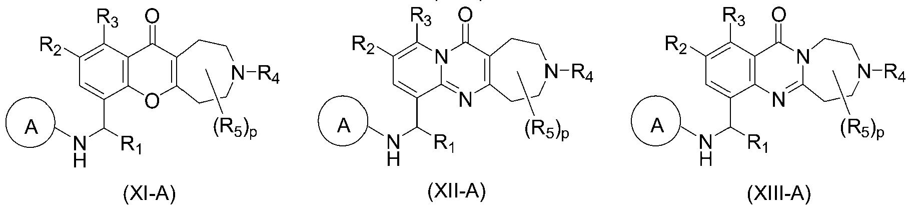 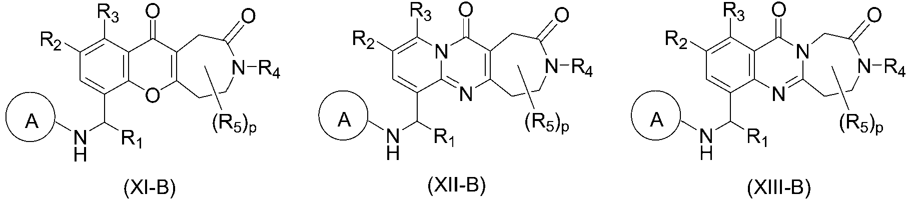 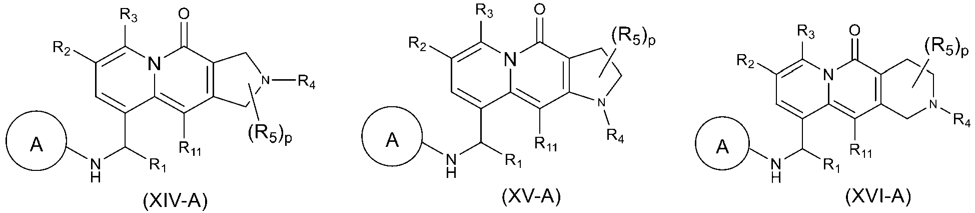 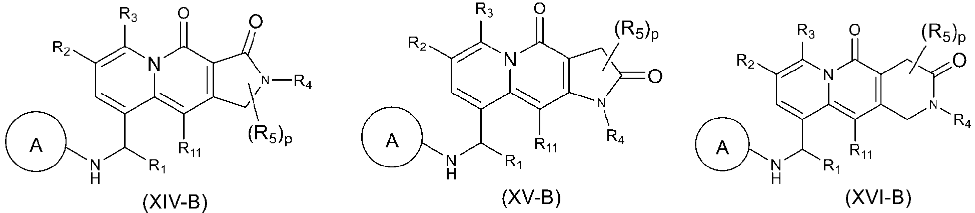 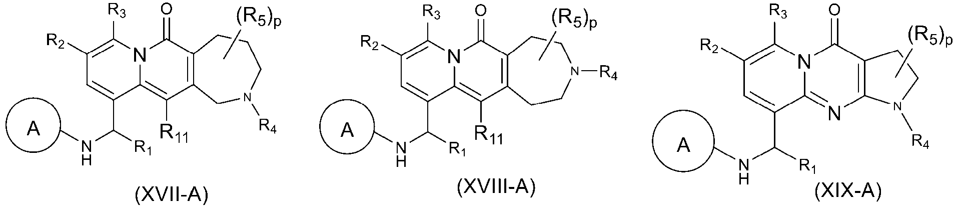 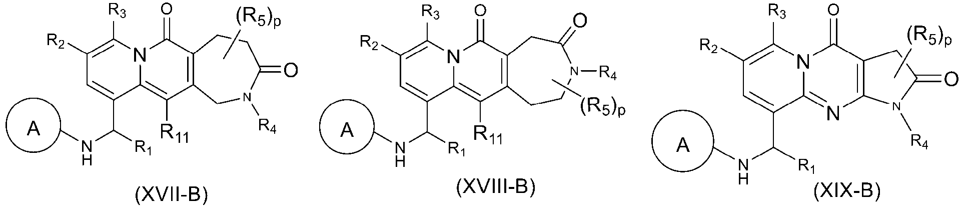
wherein, R₁, R₂, R₃, R₄, R₅, R₁₁, and ring A are as defined herein, p is 0, 1, 2, 3, or 4;
the structure 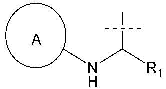 includes the following configurations: 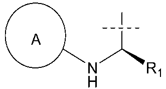 and

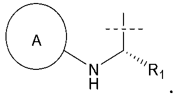

In another preferred embodiment, ring A is selected from the group consisting of: C₆₋₁₀ aryl and 5-6 membered heteroaryl containing 1 N heteroatom and 0-3 additional heteroatoms selected from N, O and S; wherein the phenyl and 5-6 membered heteroaryl are optionally substituted with m R₆ groups.

In another preferred embodiment, ring A is selected from the group consisting of: phenyl and 5-6 membered heteroaryl containing 1-4 N heteroatoms; wherein the phenyl and 5-6 membered heteroaryl are optionally substituted with m R₆ groups.

In another preferred embodiment, ring A is selected from the group consisting of: phenyl, pyridyl, thienyl, furyl, pyrazolyl, pyridazinyl, pyrimidinyl, pyrazinyl, and thiazolyl, where the groups are optionally substituted with m R₆ groups.

In another preferred embodiment, ring A is selected from the group consisting of: phenyl, and pyridyl, where the phenyl and pyridyl are optionally substituted with m R₆ groups.

In another preferred embodiment, each R₆ is independently selected from the group consisting of: H, deuterium, halogen, C₁₋₆ alkyl, halogenated C₁₋₆ alkyl, deuterated C₁₋₆ alkyl, C₁₋₆ alkoxy, halogenated C₁₋₆ alkoxy, -C(O)R₈, -C(O)OR₈, and -C(O)NR₈R₉.

In another preferred embodiment, each R₆ is independently selected from the group consisting of: H, deuterium, halogen, -C(O)R₈, -C(O)OR₈, -C(O)NR₈R₉, and -SO₂R₈.

In another preferred embodiment, each R₆ is independently selected from the group consisting of: H, deuterium, halogen, -C(O)OR₈, and -SO₂R₈.

In another preferred embodiment, each R₆ is independently selected from the group consisting of: H, deuterium, halogen, and -C(O)OH.

In another preferred embodiment, R₁ is selected from the group consisting of: H, deuterium, halogen, -CN, C₁₋₃ alkyl, halogenated C₁₋₃ alkyl, deuterated C₁₋₃ alkyl, C₁₋₃ alkoxy, and halogenated C₁₋₃ alkoxy.

In another preferred embodiment, R₁ is selected from the group consisting of: H, deuterium, C₁₋₆ alkyl, halogenated C₁₋₆ alkyl, deuterated C₁₋₆ alkyl, C₁₋₆ alkoxy, and halogenated C₁₋₆ alkoxy.

In another preferred embodiment, R₁ is selected from the group consisting of: C₁₋₆ alkyl, halogenated C₁₋₆ alkyl, and deuterated C₁₋₆ alkyl.

In another preferred embodiment, R₁ is C₁₋₃ alkyl, for example, methyl, ethyl, n-propyl or isopropyl.

In another preferred embodiment, R₁ is in the R or S configuration.

In another preferred embodiment, R₁ is a methyl in the R configuration.

In another preferred embodiment, R, R₂, and R₃ are each independently selected from the group consisting of: H, halogen, -CN, C₁₋₃ alkyl, halogenated C₁₋₃ alkyl, deuterated C₁₋₃ alkyl, C₁₋₃ alkoxy, halogenated C₁₋₃ alkoxy, deuterated C₁₋₃ alkoxy, C₃₋₅ cycloalkyl, and halogenated C₃₋₅ cycloalkyl.

In another preferred embodiment, R₂ and R₃ are each independently selected from the group consisting of: H, halogen, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃ cycloalkyl, and halogenated C₃ cycloalkyl.

In another preferred embodiment, R₂ is selected from the group consisting of: H, halogen, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃ cycloalkyl, and halogenated C₃ cycloalkyl, R₃ is H.

In another preferred embodiment, R₂ is selected from the group consisting of: H, halogen, C₁₋₃ alkyl, halogenated C₁₋₃ alkyl, deuterated C₁₋₃ alkyl, C₁₋₃ alkoxy, halogenated C₁₋₃ alkoxy, and deuterated C₁₋₃ alkoxy, R₃ is H.

In another preferred embodiment, R₂ is selected from the group consisting of: H, halogen, C₁₋₃ alkyl, halogenated C₁₋₃ alkyl, and deuterated C₁₋₃ alkyl, R₃ is H.

In another preferred embodiment, ring A has a structure selected from the group consisting of: 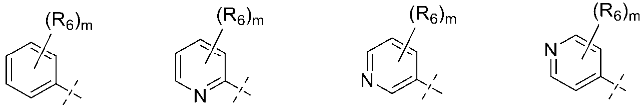 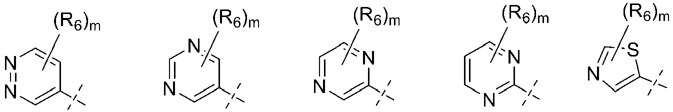
wherein, each R₆ is independently selected from the group consisting of: H, deuterium, halogen, -CN, -NO₂, C₁₋₆ alkyl, halogenated C₁₋₆ alkyl, deuterated C₁₋₃ alkyl, C₁₋₆ alkoxy, halogenated C₁₋₆ alkoxy, -C(O)R₈, -C(O)OR₈, -C(O)NR₈R₉, -SO₂R₈, -S(O)(NR₈)R₉, -SO₂NR₈R₉, and -P(O)R₈R₉;
each R₈ and R₉ are as defined above; m is selected from the group consisting of: 0, 1, 2, 3, 4, and 5.

In another preferred embodiment, ring A has a structure selected from the group consisting of:

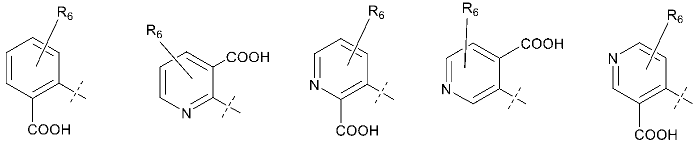

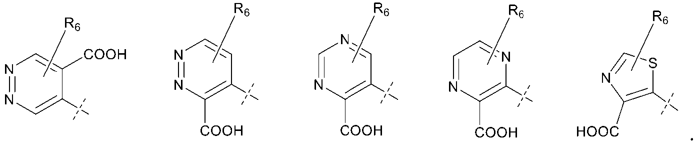

In another preferred embodiment, ring A has a structure selected from the group consisting of:

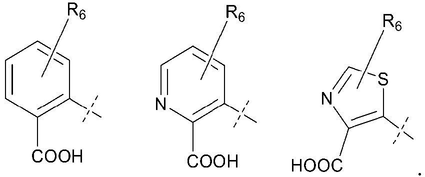

In another preferred embodiment, R₄ has a structure selected from the group consisting of: 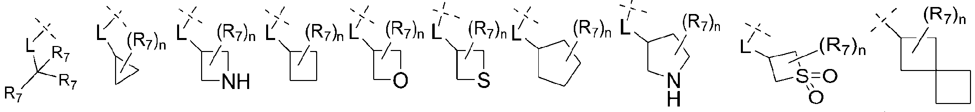 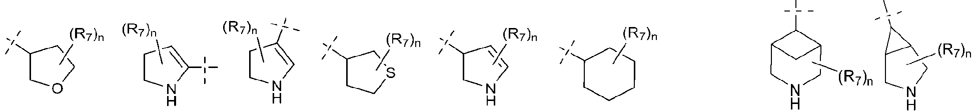 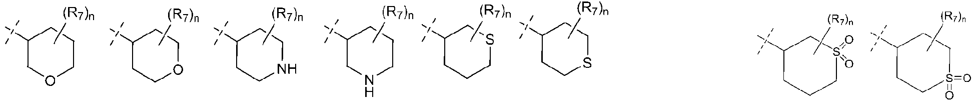 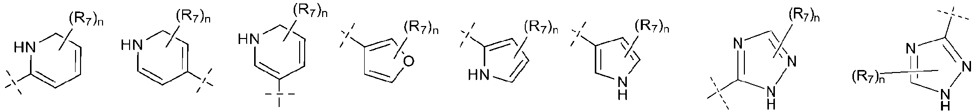 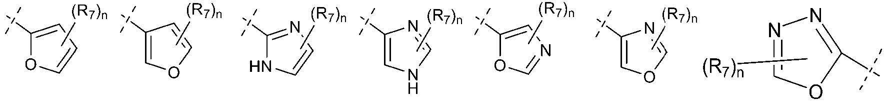 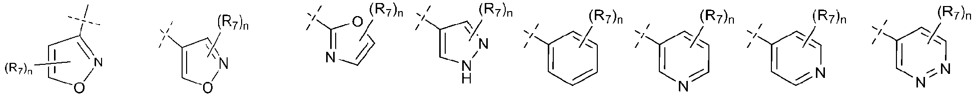 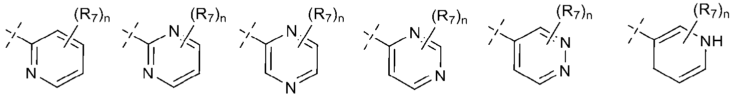 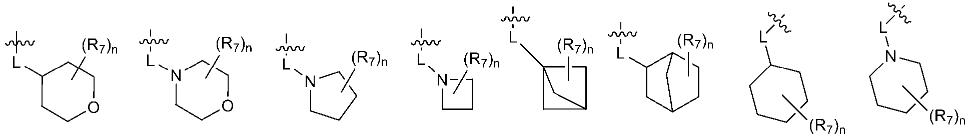 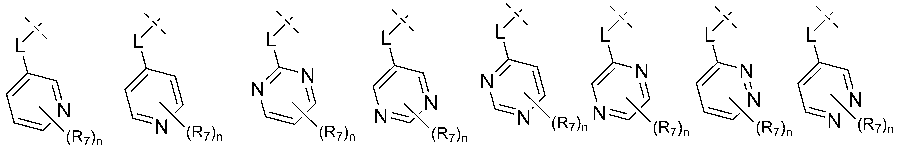 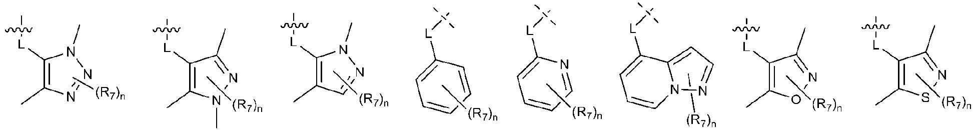
wherein, L and L₁ are each independently a bond or selected from the group consisting of: C₁₋₆ alkylene, -C(O)-, -C(O)NH-, -C(O)O-, -(SO₂)-, and -SO₂NH-;
each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -NR₈R₉, =NR₈, - C(O)R₈, -C(O)OR₈, -C(O)NR₈R₉, -NR₈C(O)R₉, -SR₈, -SO₂R₈, -NR₈SO₂R₉, -SO₂NR₈R₉, - NR₈SO₂NR₉R₁₀, -P(O)R₈R₉, -L₁-(C₁₋₆ alkyl), -L₁-(saturated or partially saturated C₃₋₁₀ cycloalkyl), - L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-12 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, 3-6 membered heterocycloalkyl containing 1-4 heteroatoms selected from N, O and S, - SO₂R₈, -C(O)R₈, -C(O)OR₈, -C(O)NR₈R₉, and -NR₈R₉;
alternatively, two R₇ groups together with the atoms to which they are attached form C₃₋₆ cycloalkyl, C₆₋₁₀ aryl, 3-6 membered heterocycloalkyl containing 1-4 heteroatoms selected from N, O and S, or 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NR₈R₉, =NR₈, -C(O)R₈, -C(O)OR₈, -C(O)NR₈R₉, -NR₈C(O)R₉, -SR₈, -SO₂R₈, - NR₈SO₂R₉, -SO₂NR₈R₉, -NR₈SO₂NR₉R₁₀, -P(O)R₈R₉, C₁₋₆ alkyl, C₁₋₆ alkoxy, C₁₋₆ heteroalkyl containing 1-4 heteroatoms selected from N, O and S, halogenated C₁₋₆ alkyl, halogenated C₁₋₆ alkoxy, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S;
each of R₈, R₉ and R₁₀ is as defined above; n is selected from the group consisting of: 0, 1, 2, 3, 4, and 5.

In another preferred embodiment, each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -L₁-(C₁₋₆ alkyl), -L₁-(saturated or partially saturated C₃₋₁₀ cycloalkyl), -L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-12 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, and halogenated C₃₋₆ cycloalkyl.

In another preferred embodiment, each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, and -L₁-(C₁₋₆ alkyl), wherein the C₁₋₆ alkyl is optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, and halogenated C₃₋₆ cycloalkyl.

In another preferred embodiment, each R₇ is independently selected from the group consisting of: hydrogen, deuterium, halogen, oxo (=O), -CN, -OH, -NO₂, C₁₋₆ alkyl, halogenated C₁₋₆ alkyl, deuterated C₁₋₆ alkyl, and -L₁-(C₁₋₆ alkyl), wherein the C₁₋₆ alkyl is optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, and halogenated C₁₋₆ alkoxy.

In another preferred embodiment, each of R₈, R₉ and R₁₀ is each independently selected from the group consisting of: H, C₁₋₆ alkyl, halogenated C₁₋₆ alkyl, C₃₋₆ cycloalkyl, and 3-6 membered heterocycloalkyl containing 1-4 heteroatoms selected from N, O and S, wherein the C₃₋₆ cycloalkyl, and 3-6 membered heterocycloalkyl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, and halogenated C₁₋₆ alkoxy.

In another preferred embodiment, each of R₈, R₉ and R₁₀ is each independently selected from the group consisting of: H, C₁₋₆ alkyl, halogenated C₁₋₆ alkyl, C₃₋₄ cycloalkyl, and 3-4 membered heterocycloalkyl containing 1-4 heteroatoms selected from N, O and S, wherein the C₃₋₆ cycloalkyl, and 3-6 membered heterocycloalkyl are optionally substituted with 0-6 substituents selected from the group consisting of: deuterium, halogen, =O, -CN, -OH, -NH₂, C₁₋₃ alkyl, halogenated C₁₋₃ alkyl, C₁₋₃ alkoxy, and halogenated C₁₋₃ alkoxy.

In another preferred embodiment, each of R₈, R₉ and R₁₀ is each independently selected from the group consisting of: H, C₁₋₆ alkyl, and halogenated C₁₋₆ alkyl,.

In another preferred embodiment, the compound has a structure selected from the group consisting of: 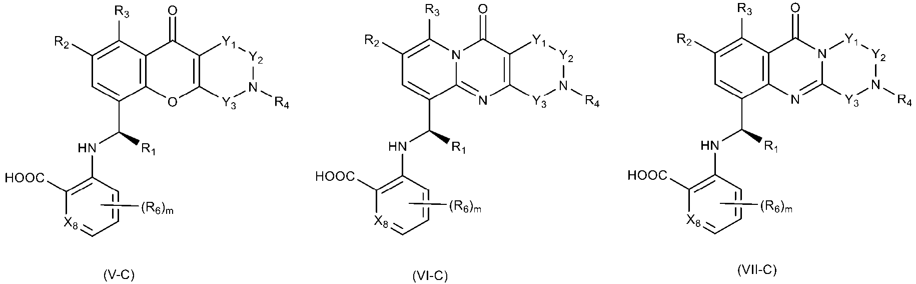 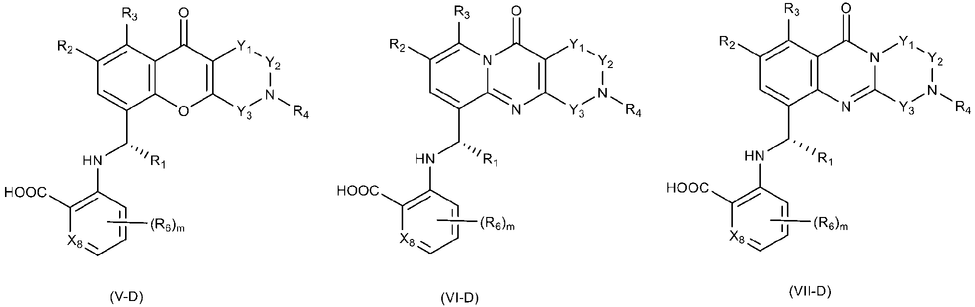
wherein, R₁, R₂, R₃, R₄, Y₁, Y₂ and Y₃ are as defined herein;
X₈ is CR₆ or N;
each R₆ is independently selected from the group consisting of: H, deuterium, halogen, -CN, - NO₂, C₁₋₆ alkyl, halogenated C₁₋₆ alkyl, deuterated C₁₋₃ alkyl, C₁₋₆ alkoxy, halogenated C₁₋₆ alkoxy, - C(O)R₈, -C(O)OR₈, -C(O)NR₈R₉, -SO₂R₈, -S(O)(NR₈)R₉, -SO₂NR₈R₉, and -P(O)R₈R₉;
m is selected from the group consisting of: 0, 1, 2, 3 and 4.

In another preferred embodiment, X₁, X₂, X₃, X₄, R₁, R₂, R₃, R₄, R₅, R₆, R₇, R₈, R₉, R₁₀, L, L₁, ring A, and ring B are each independently the corresponding groups in the Example compounds C1-C2.

In another preferred embodiment, the compound is selected from the group consisting of:

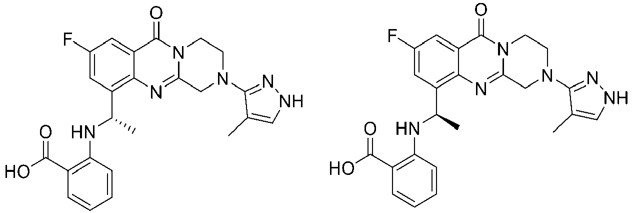

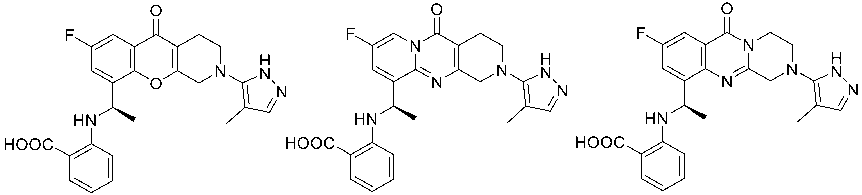

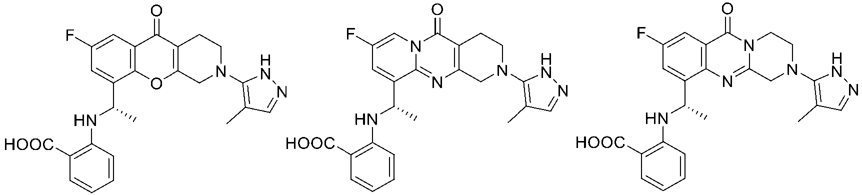

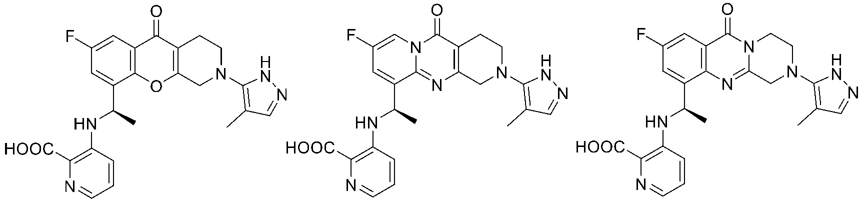

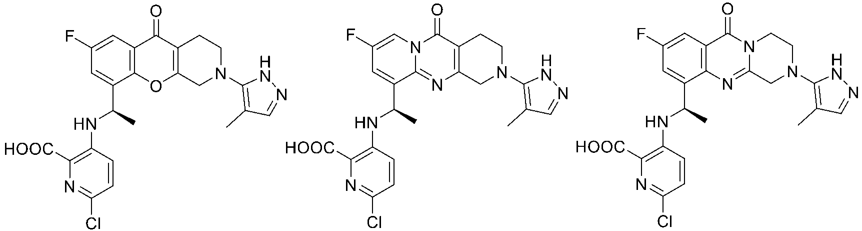

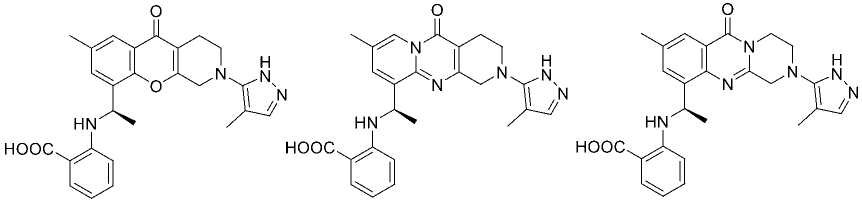

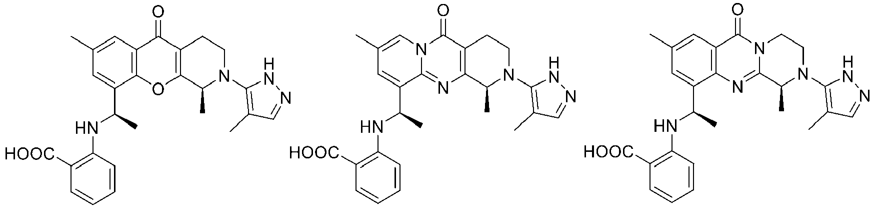

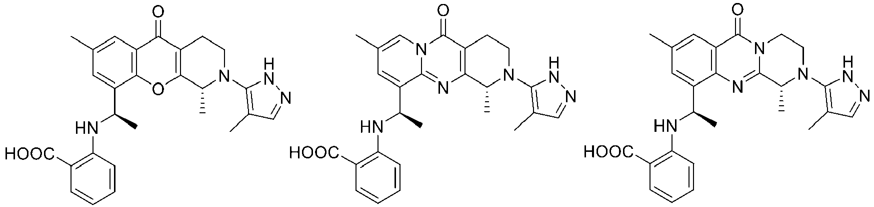

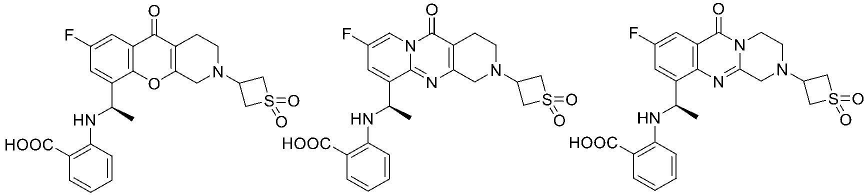

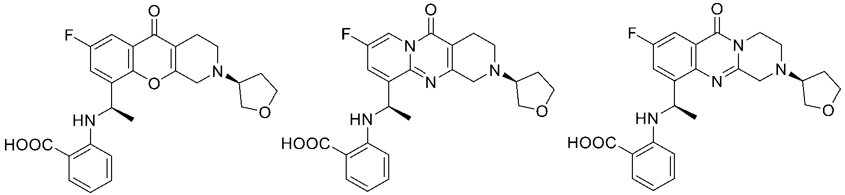

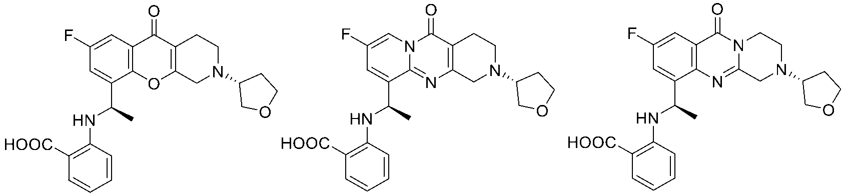

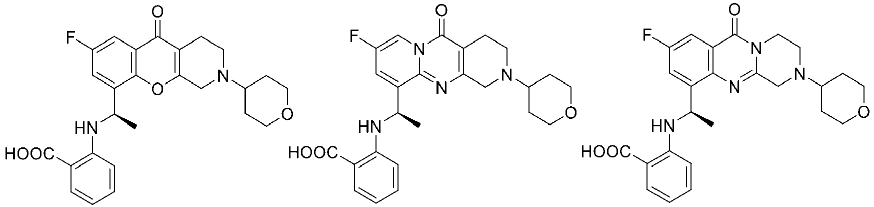

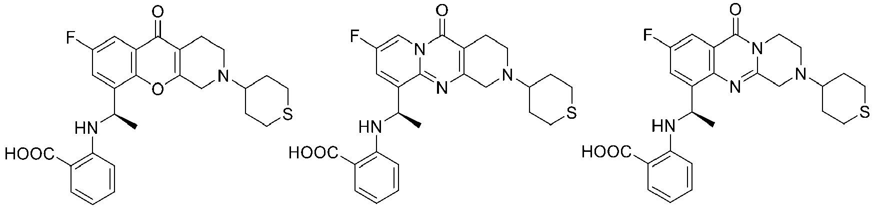

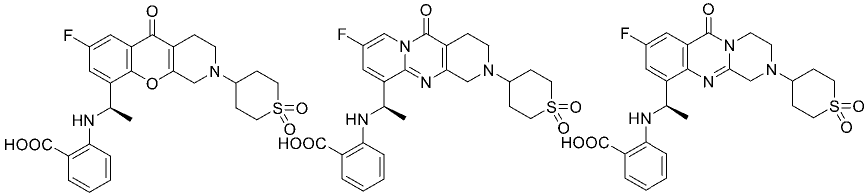

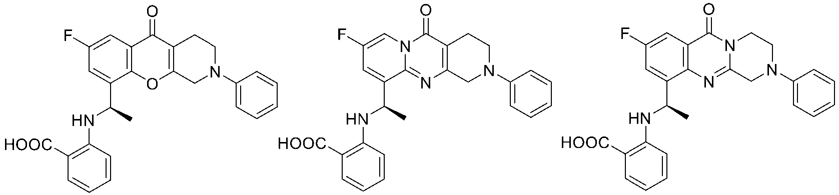

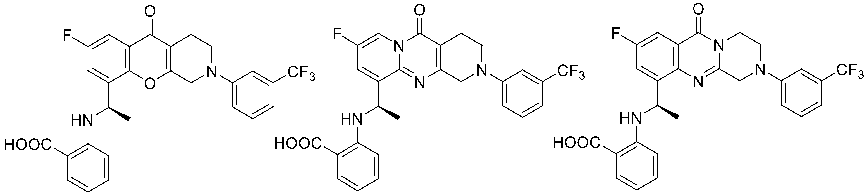

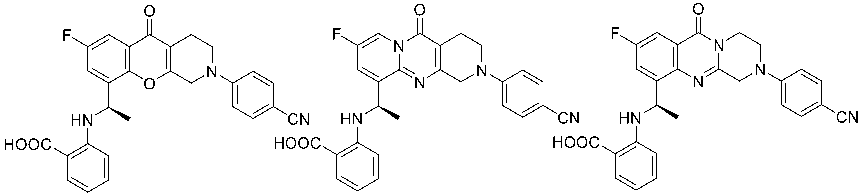

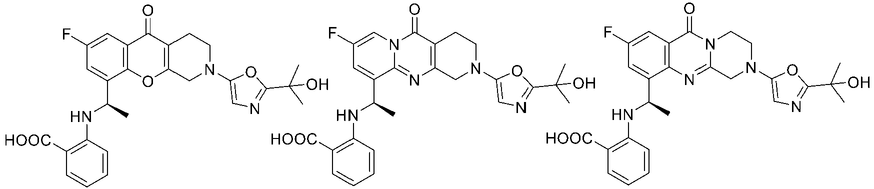

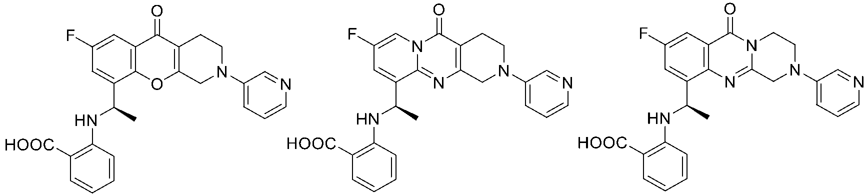

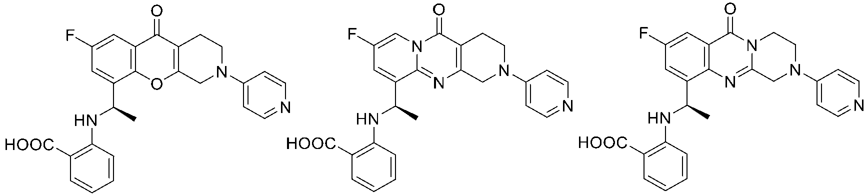

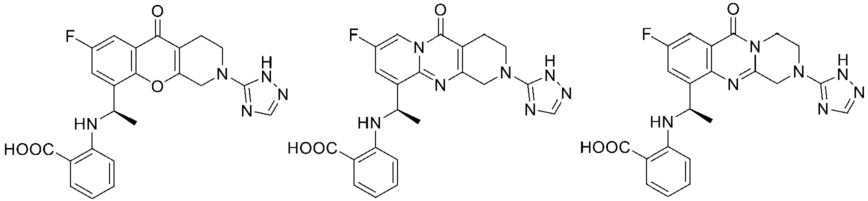

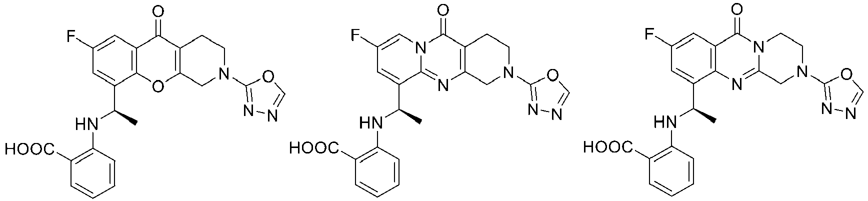

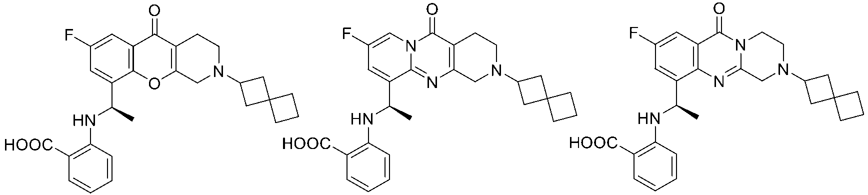

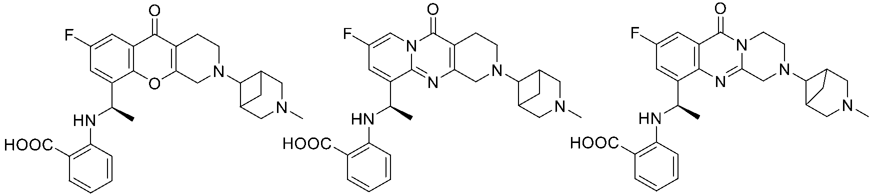

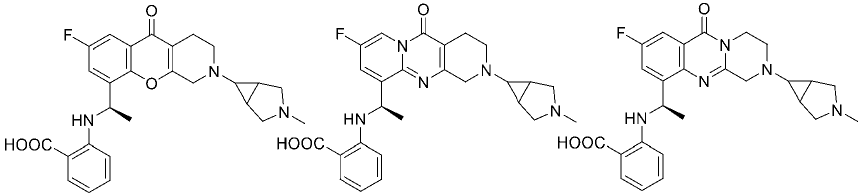

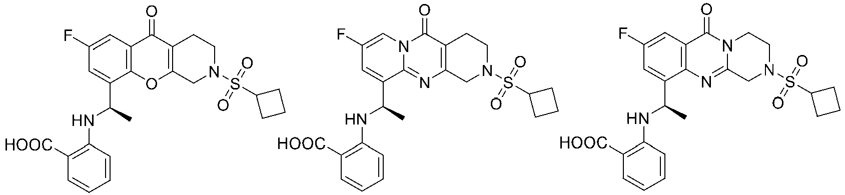

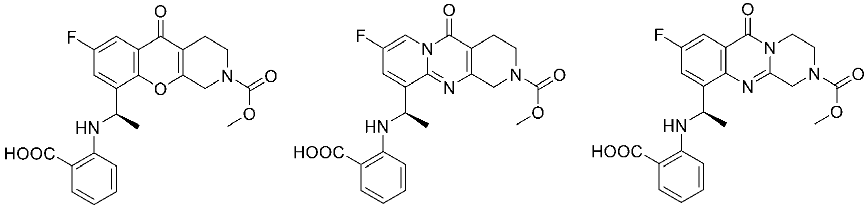

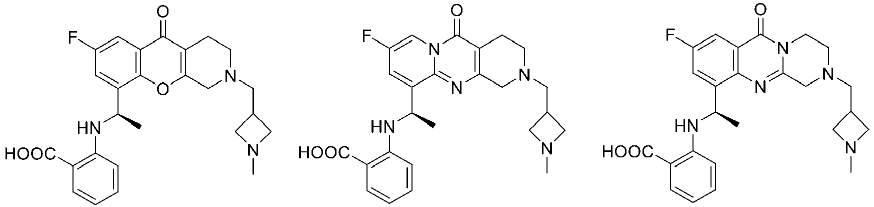

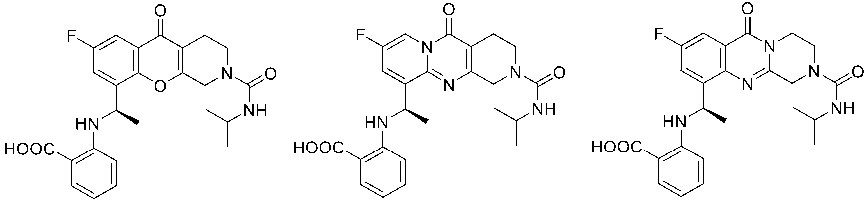

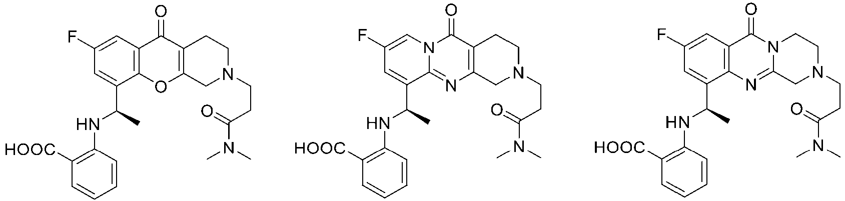

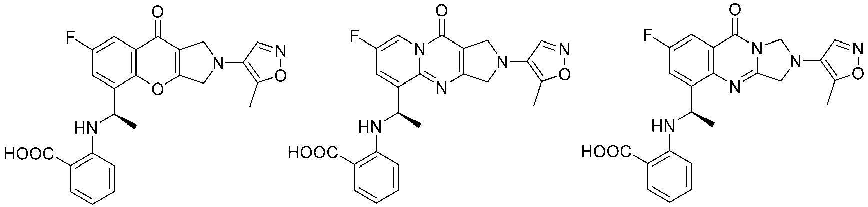

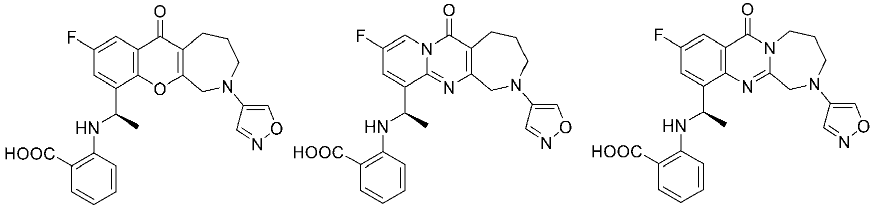

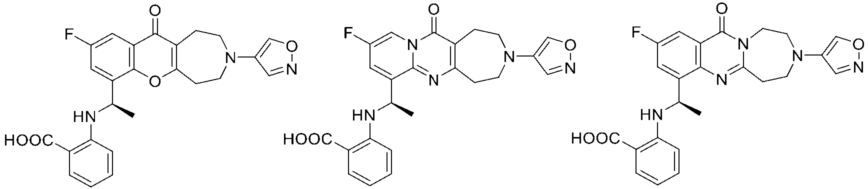

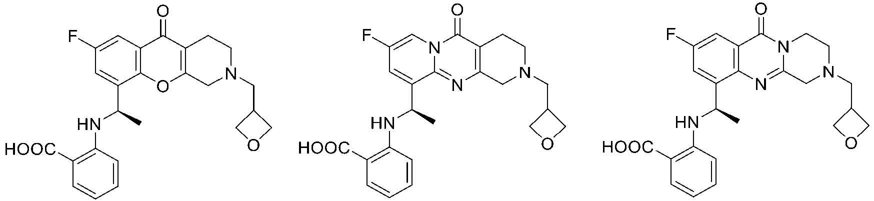

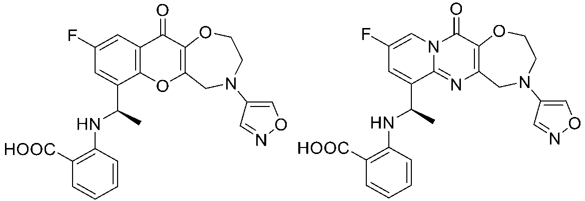

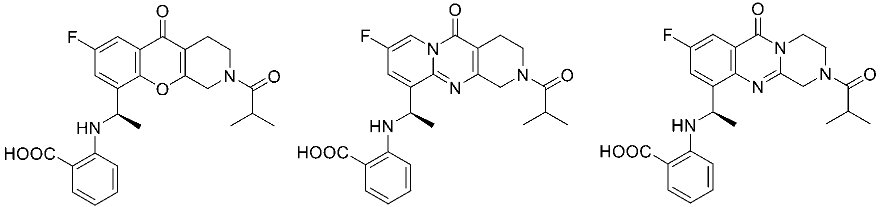

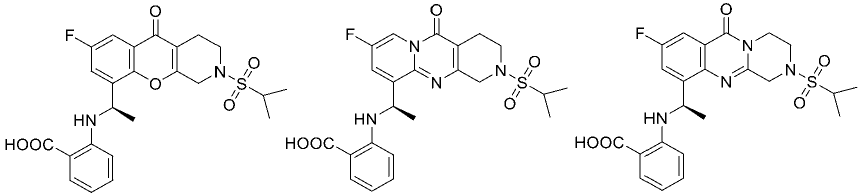

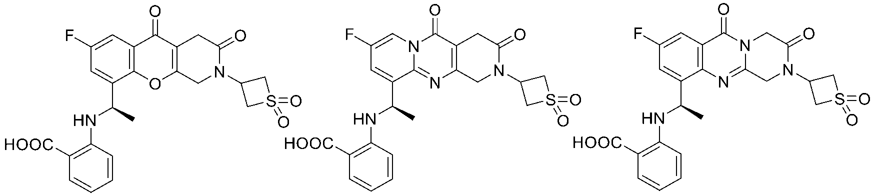

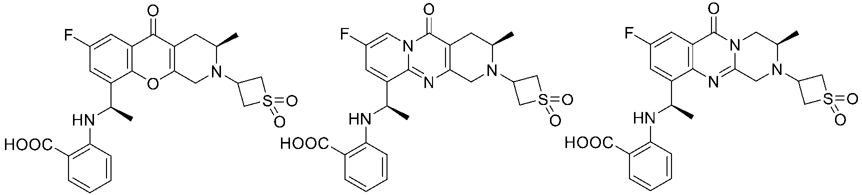

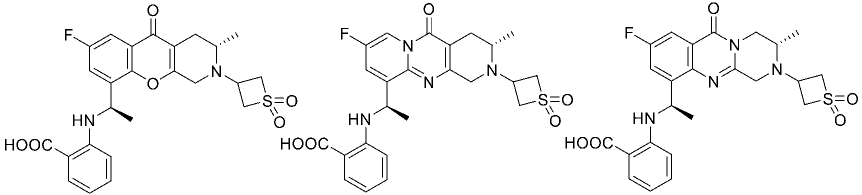

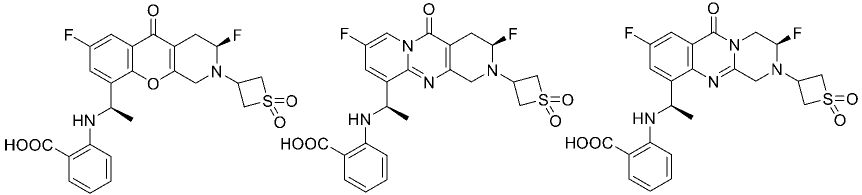

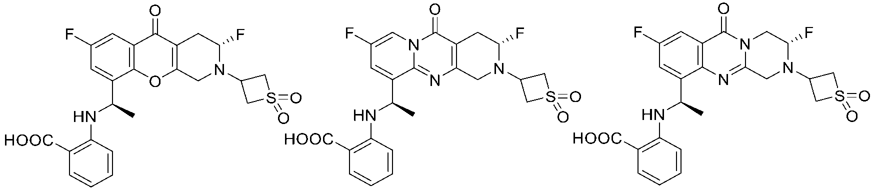

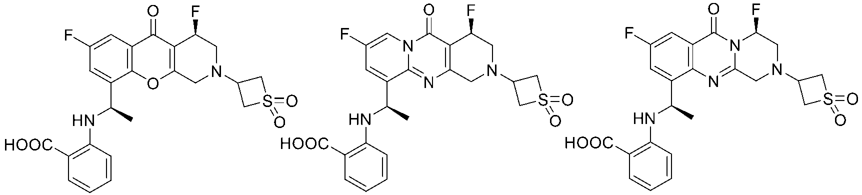

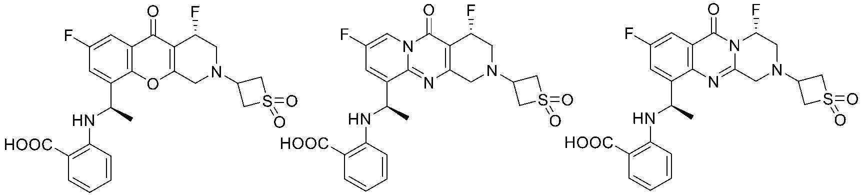

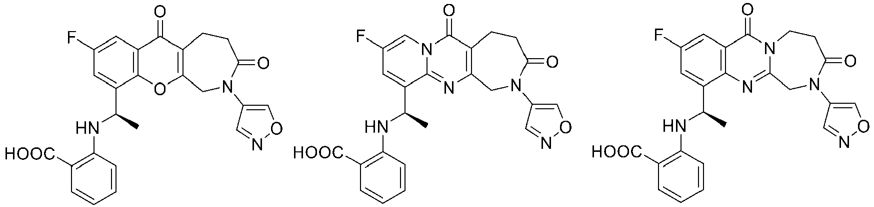

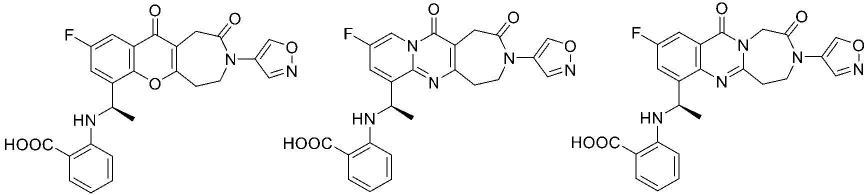

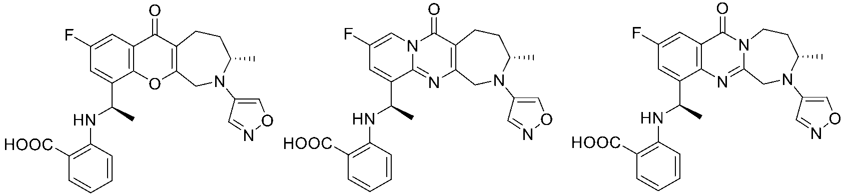

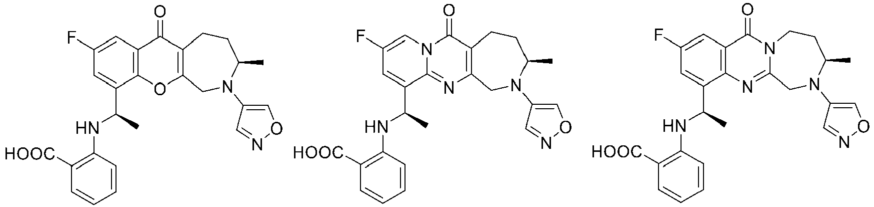

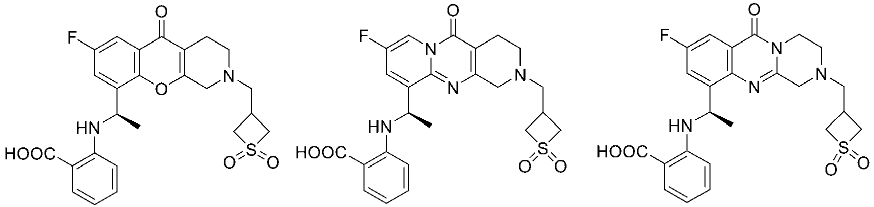

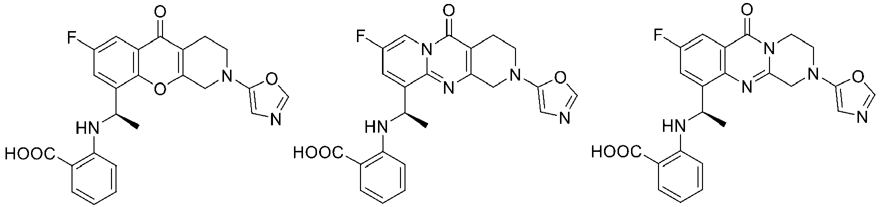

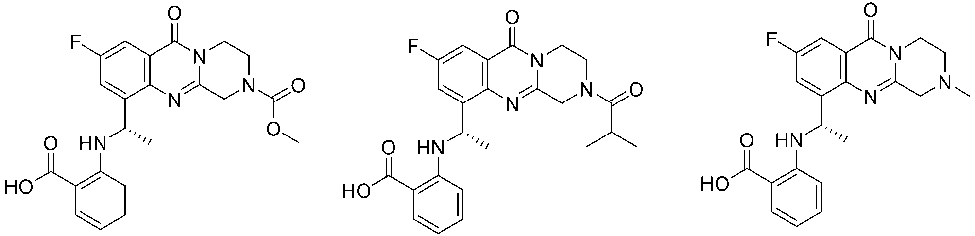

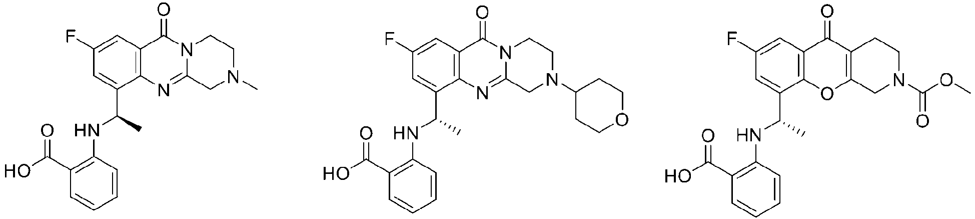

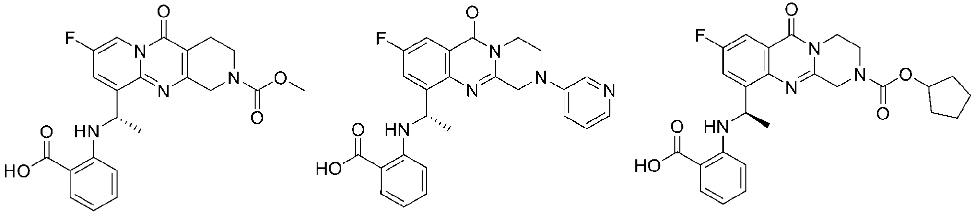

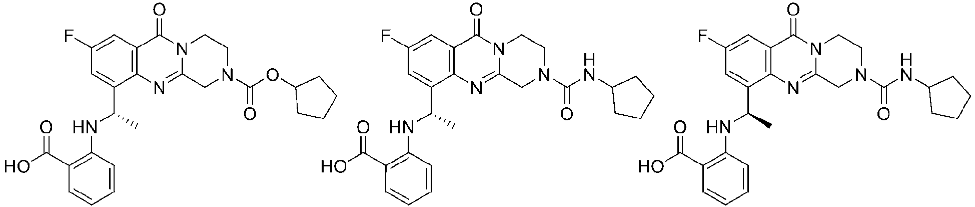

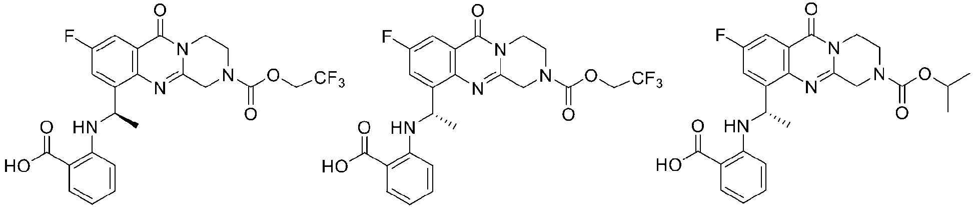

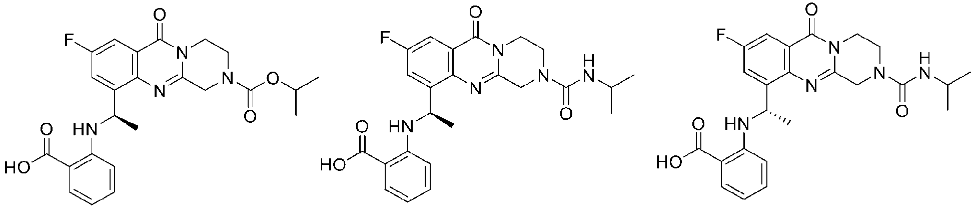

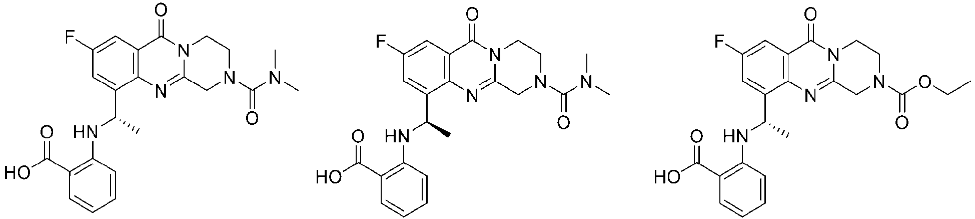

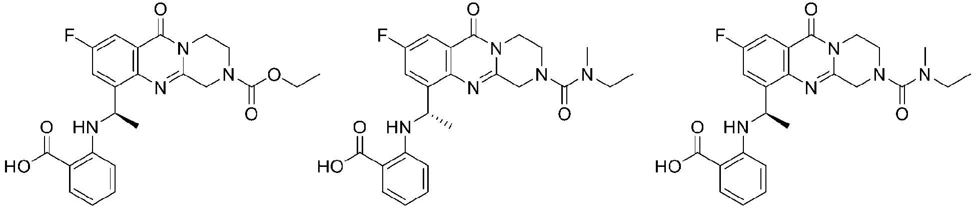

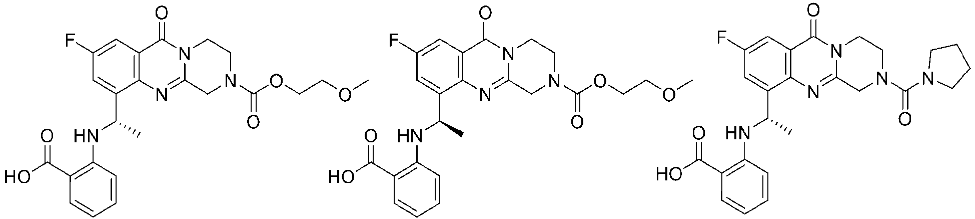

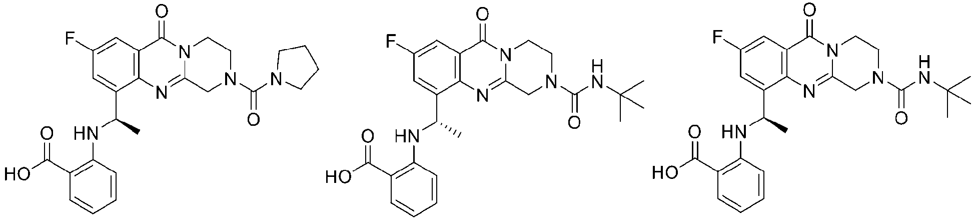

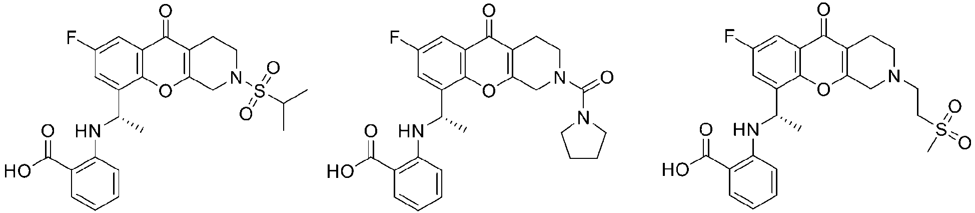

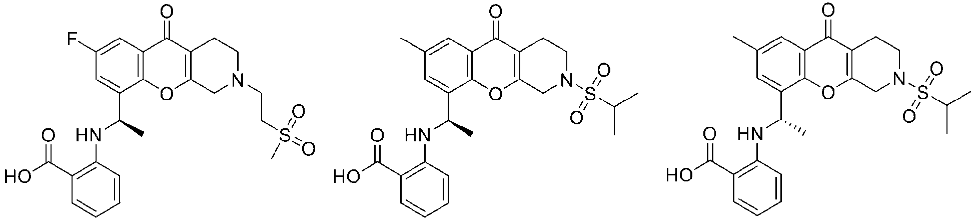

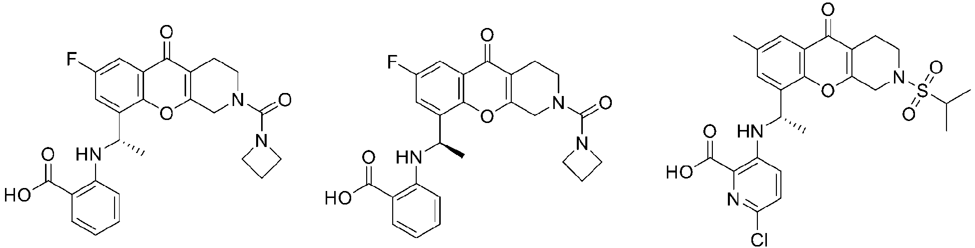

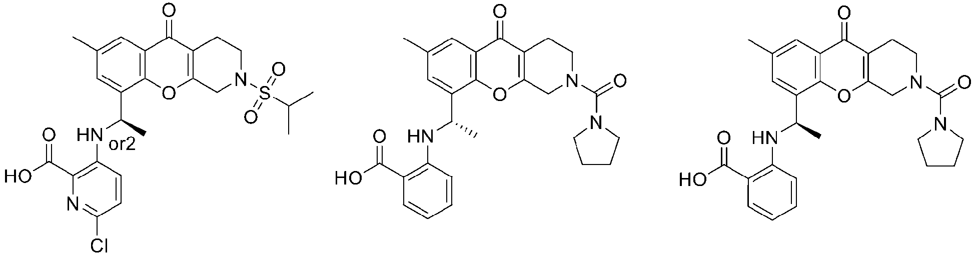

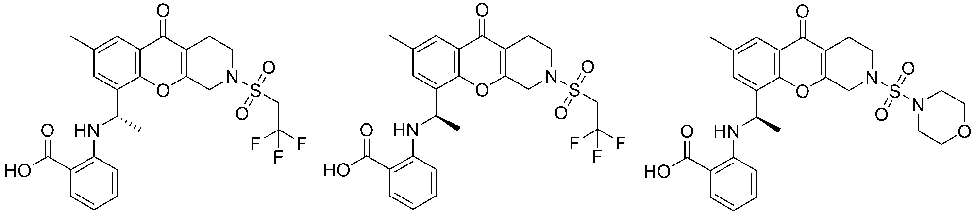

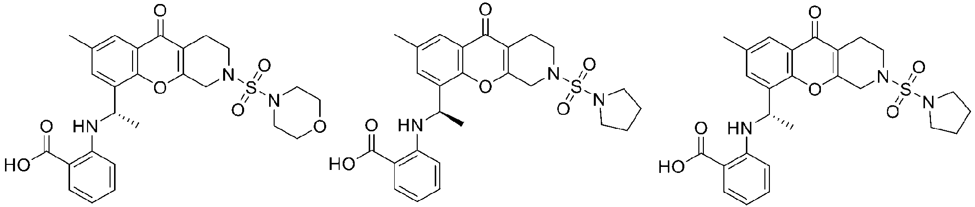

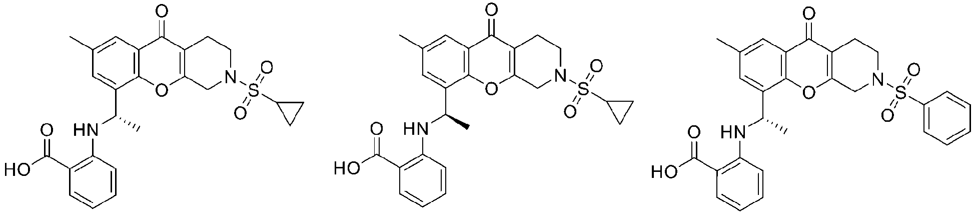

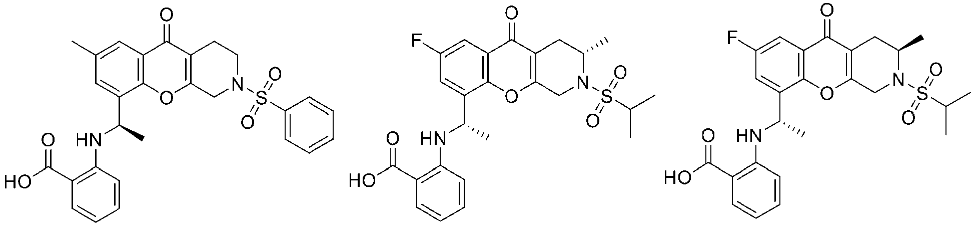

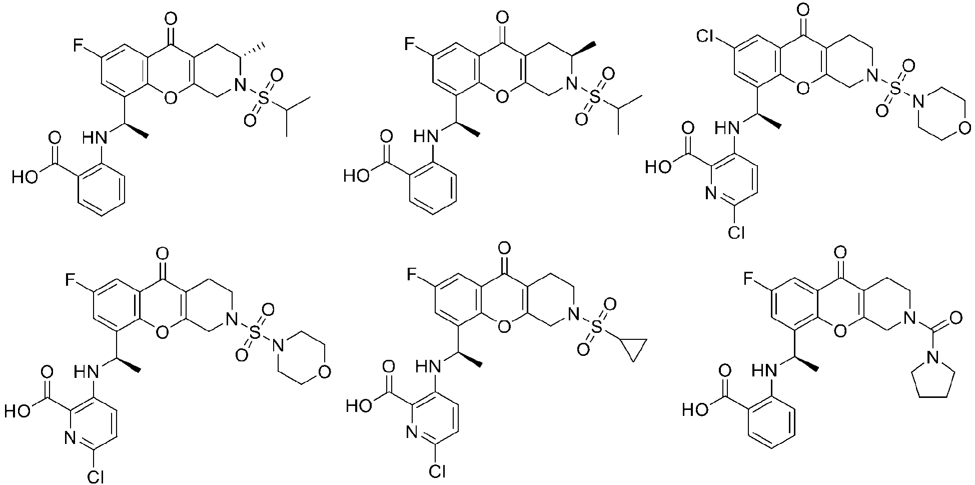

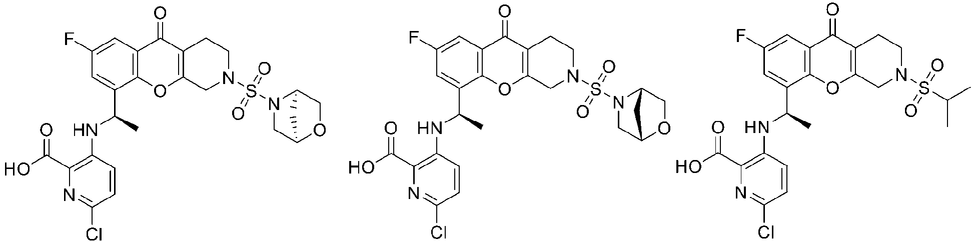

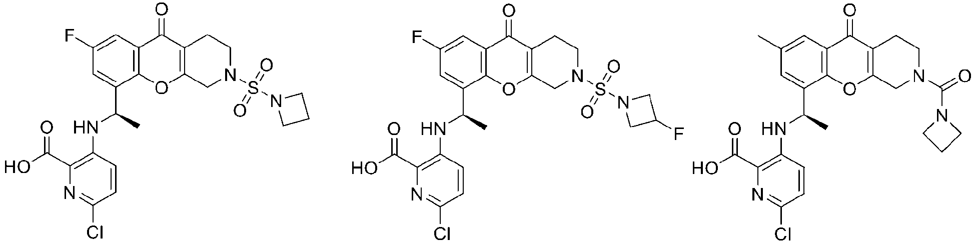

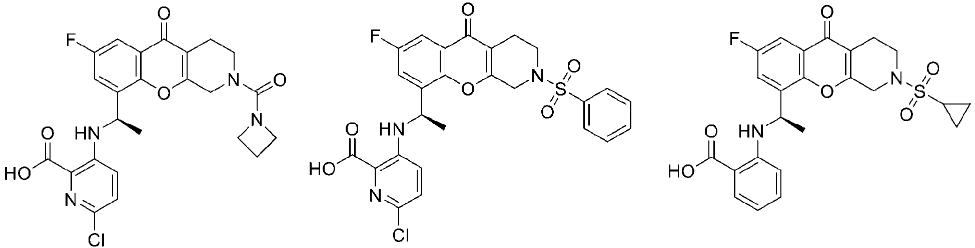

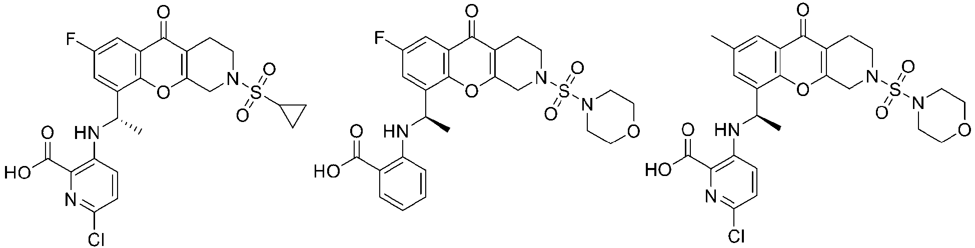

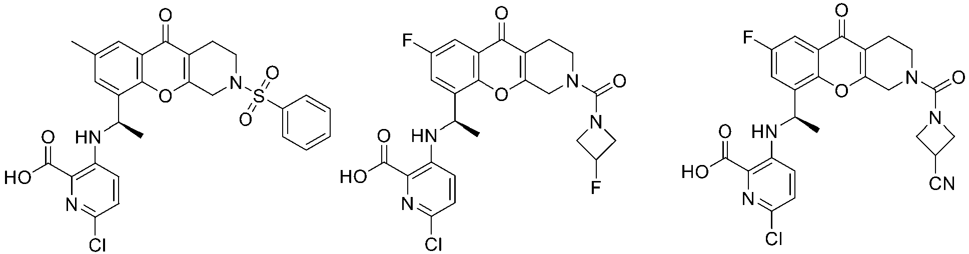

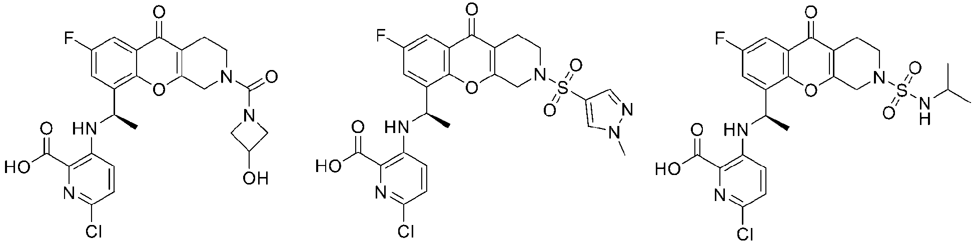

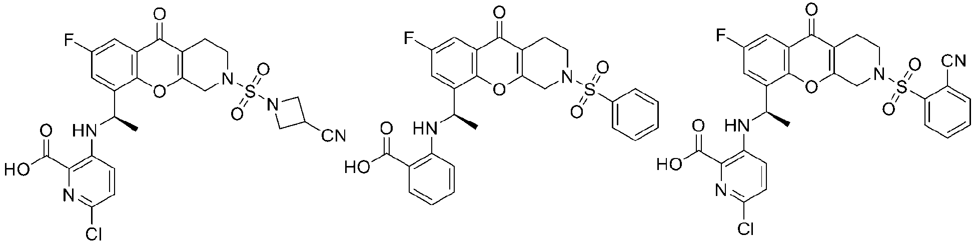

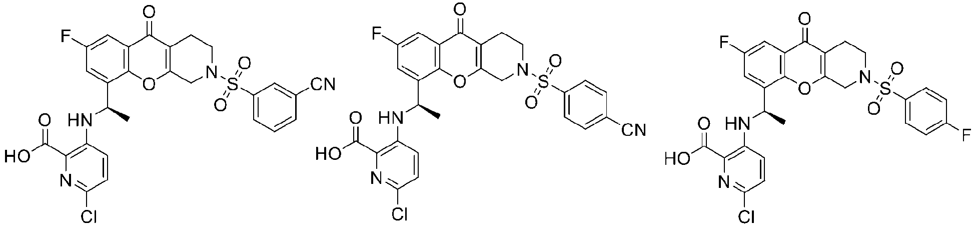

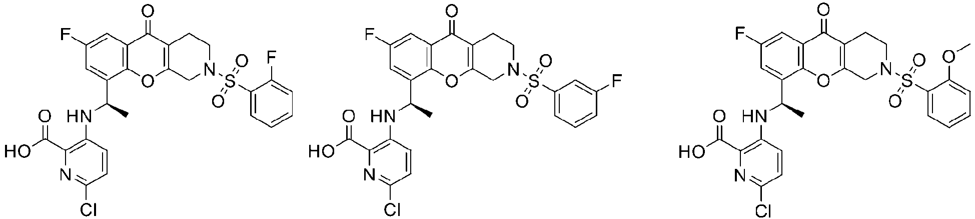

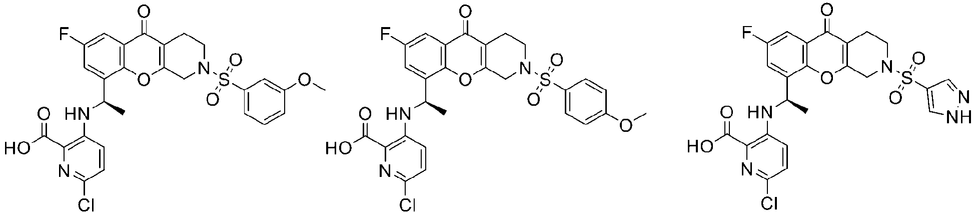

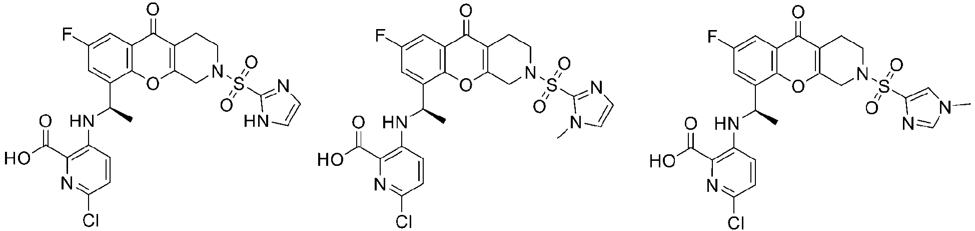

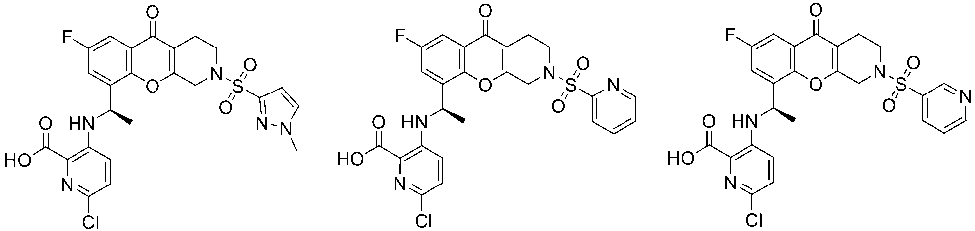

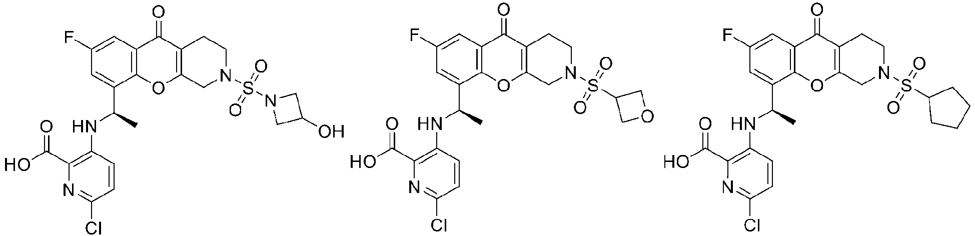

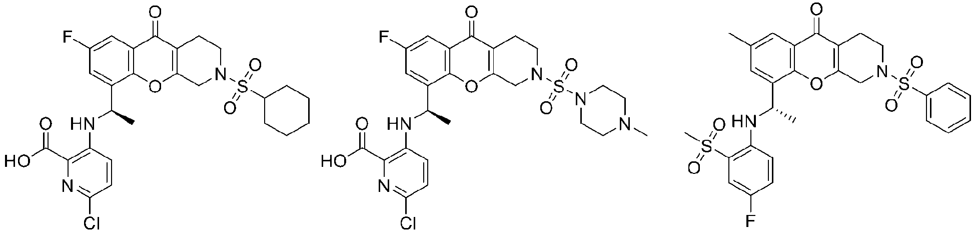

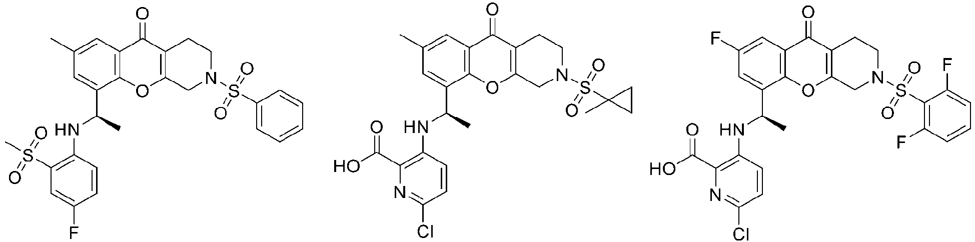

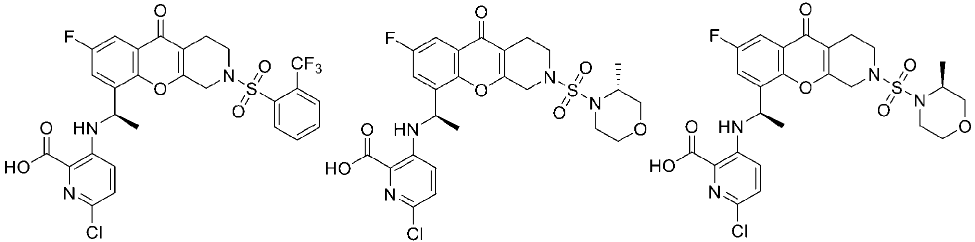

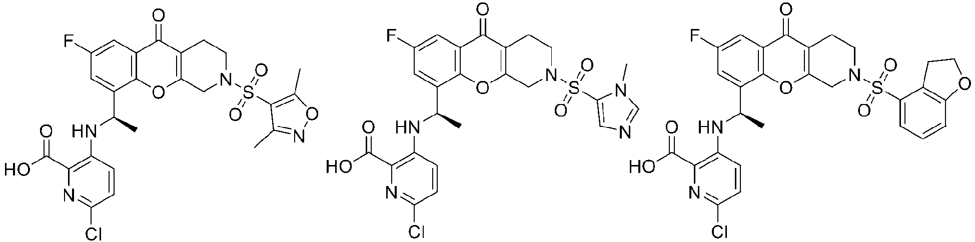

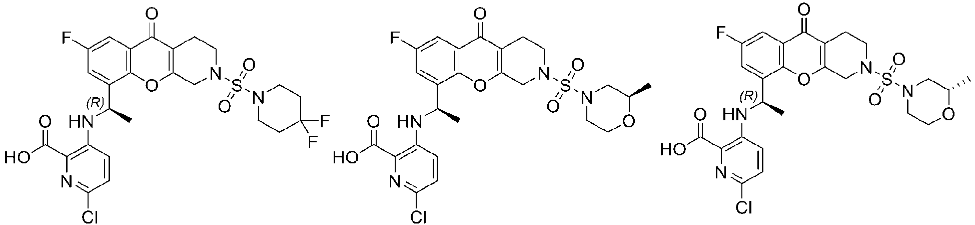

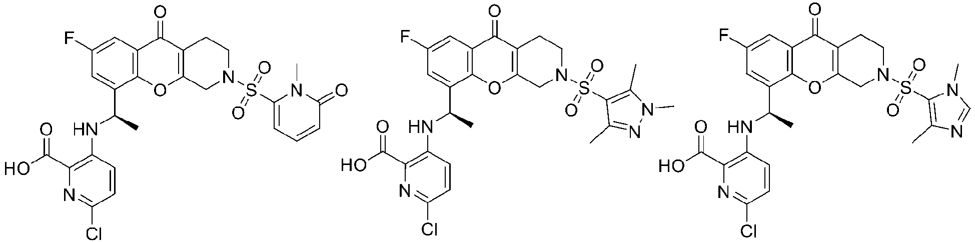

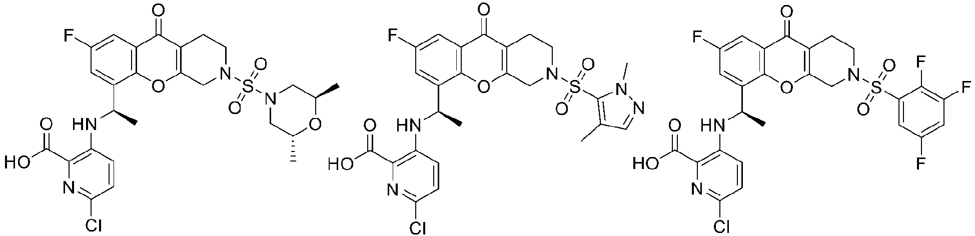

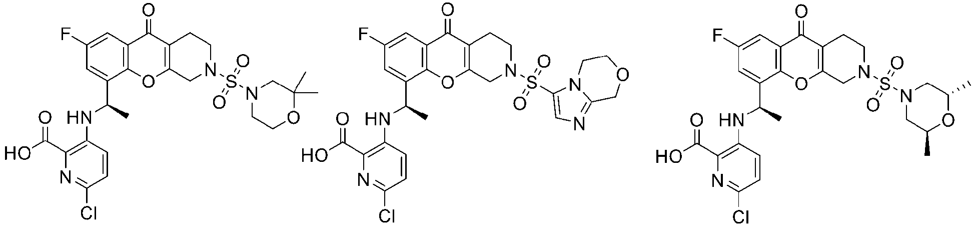

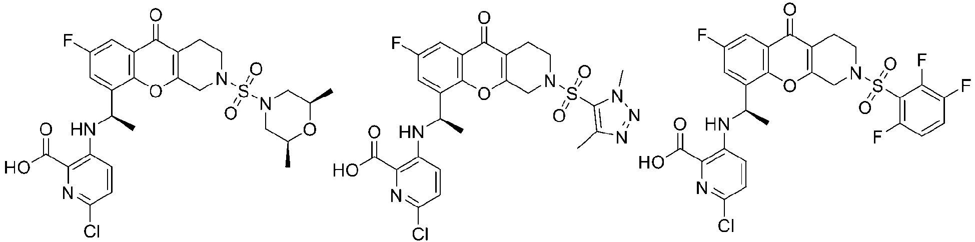

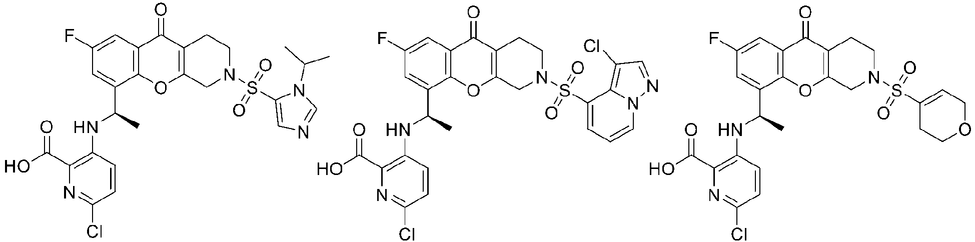

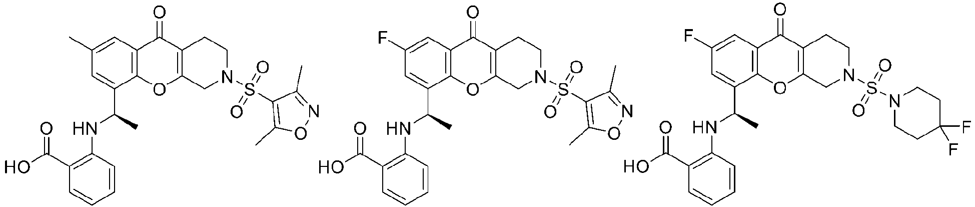

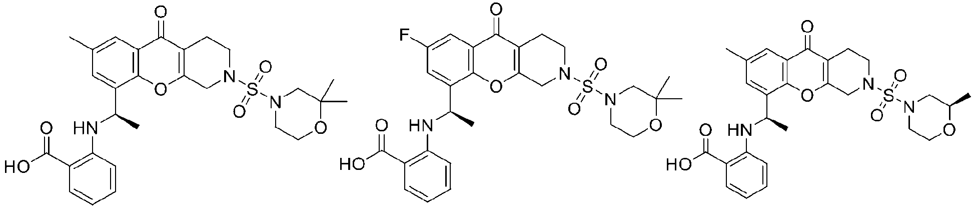

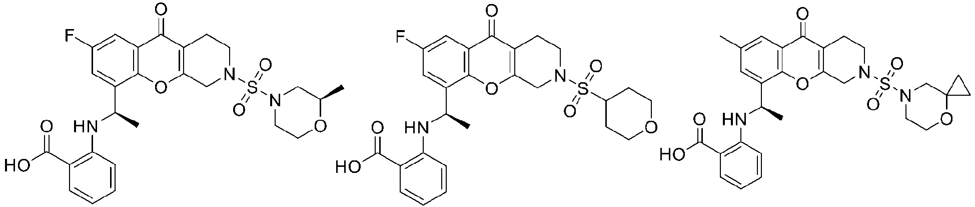

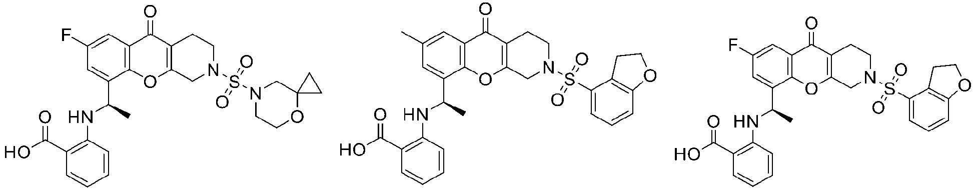

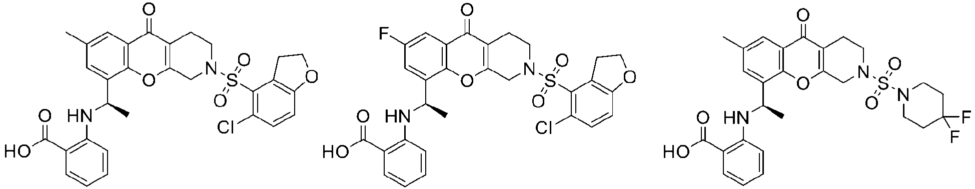

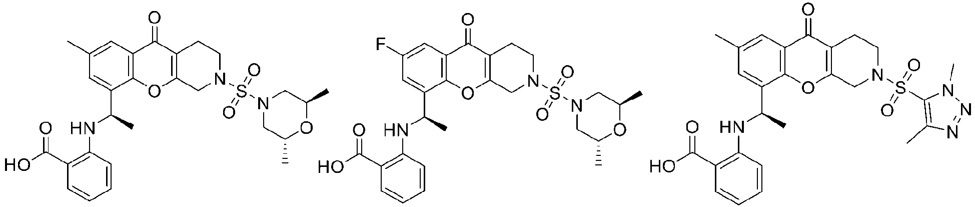

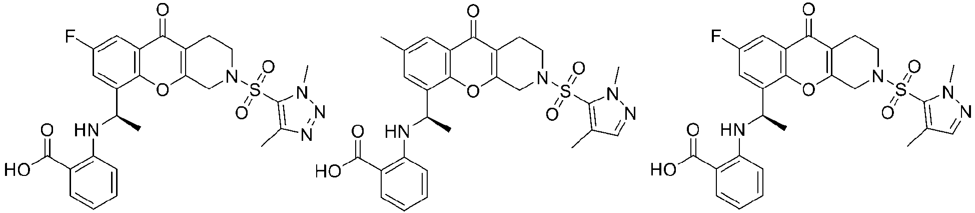

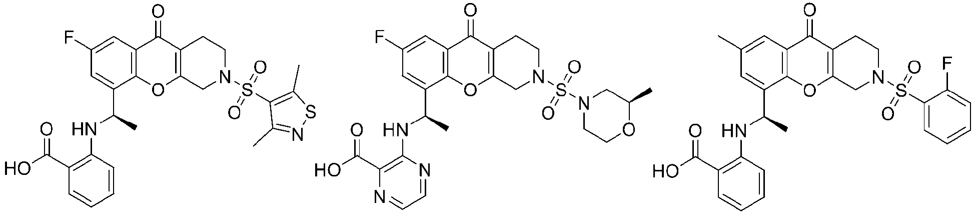

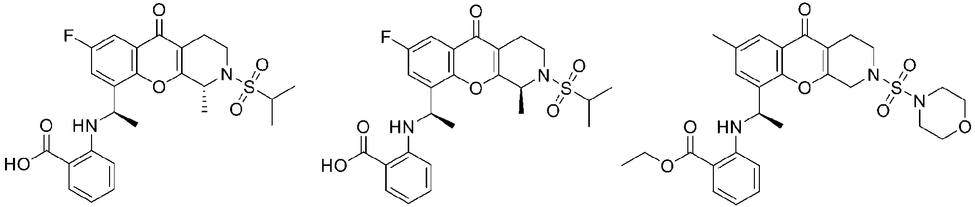

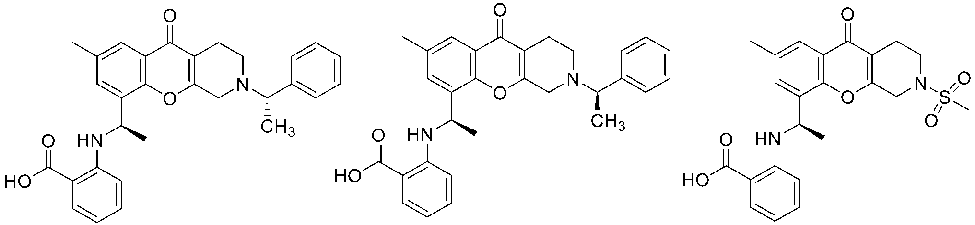

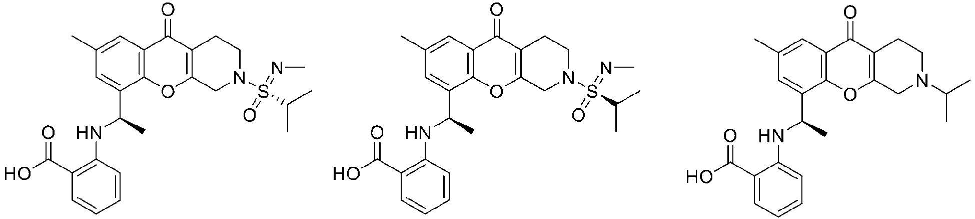

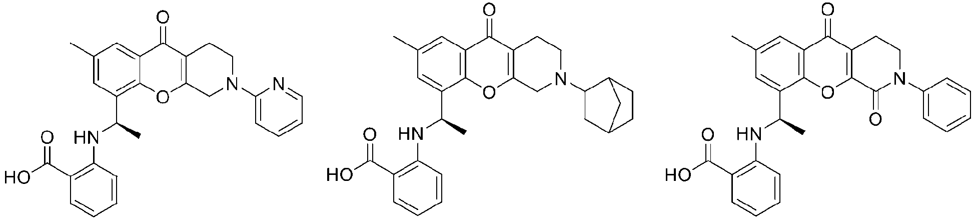

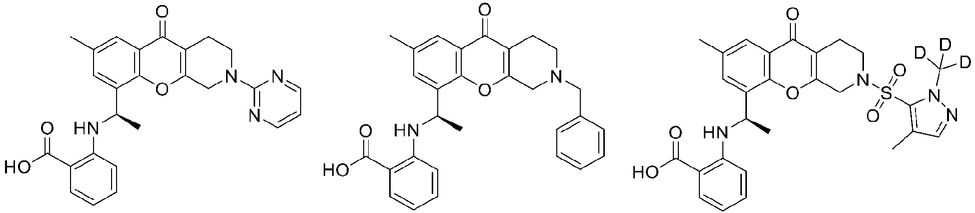

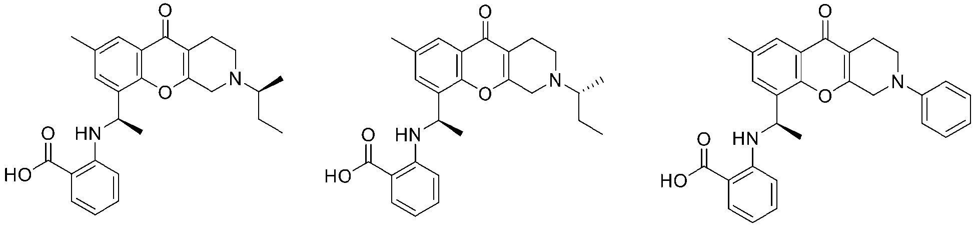

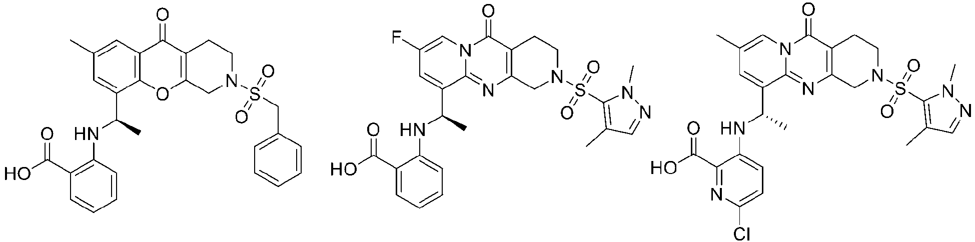

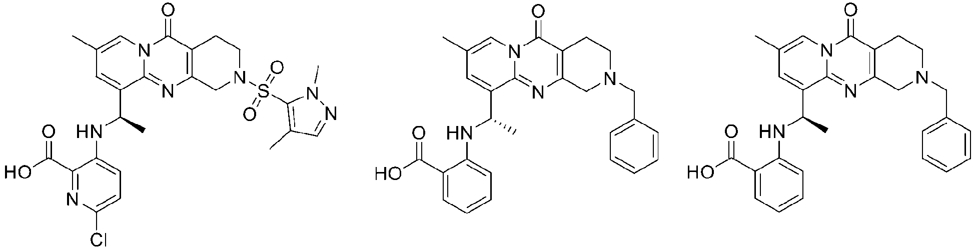

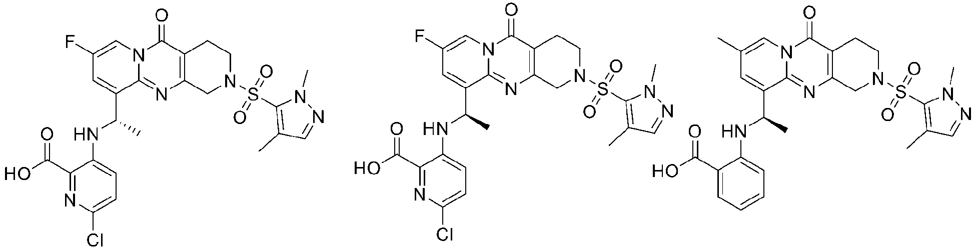

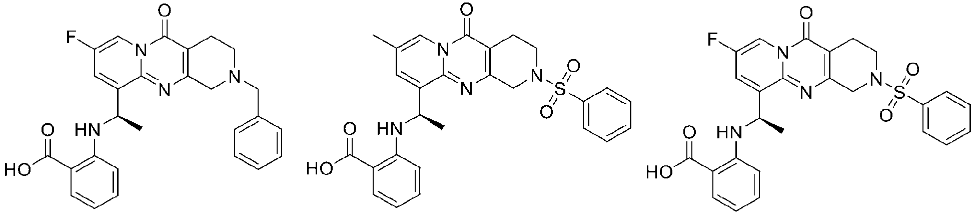

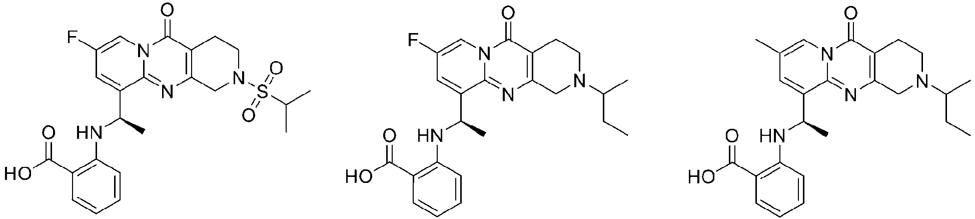

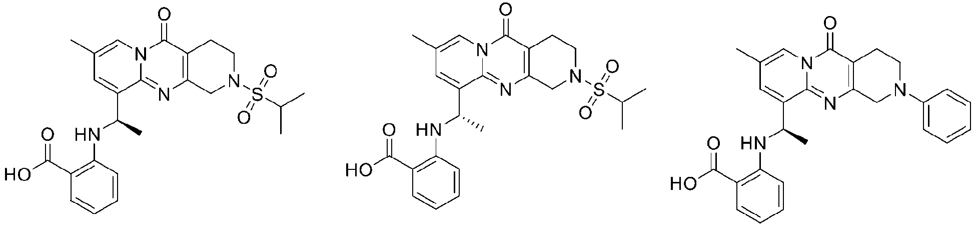

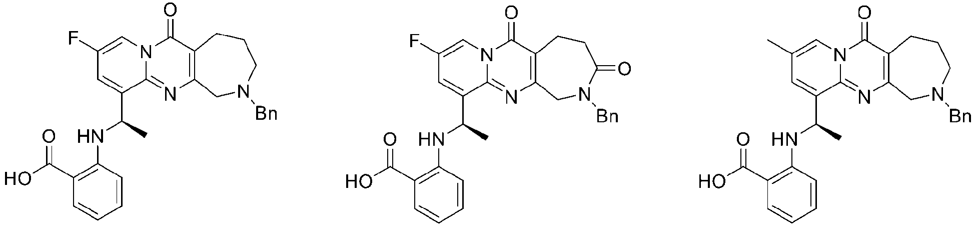

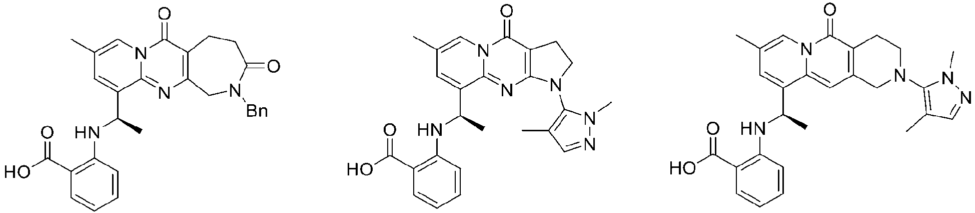

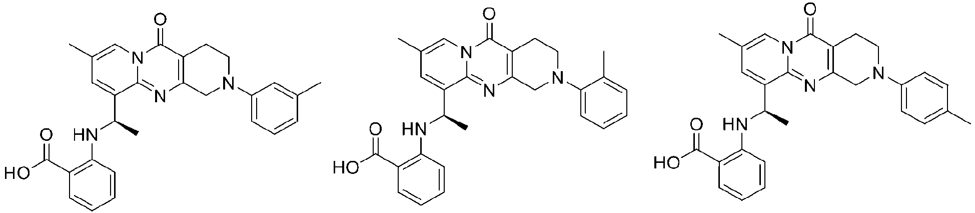

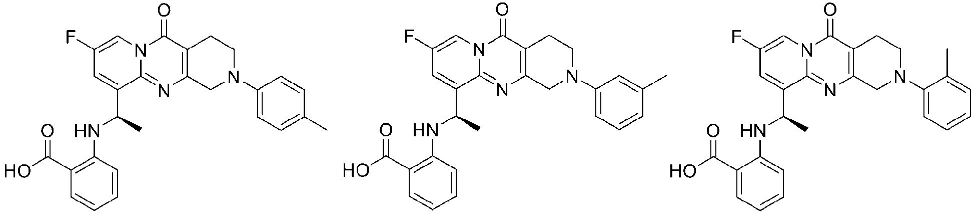

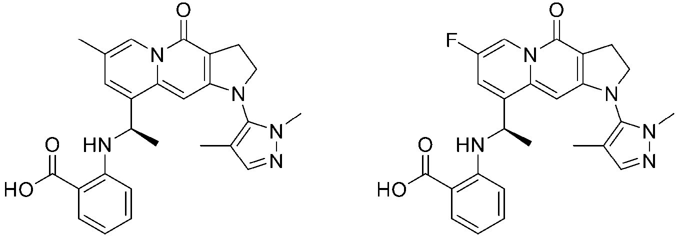

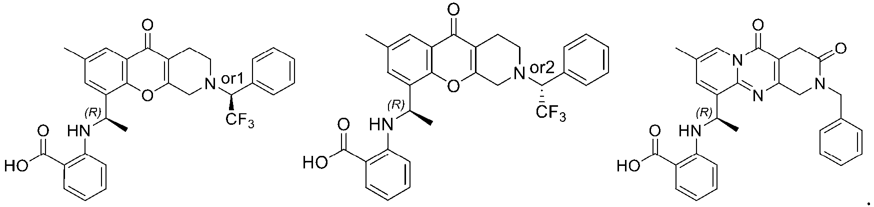

In another preferred embodiment, the compound is selected from the group consisting of:

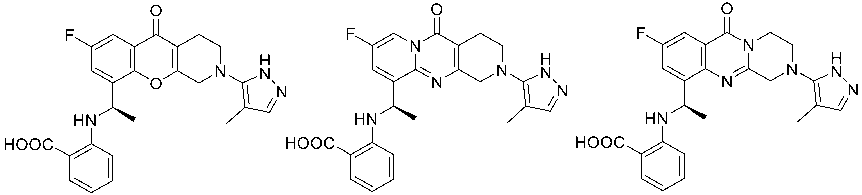

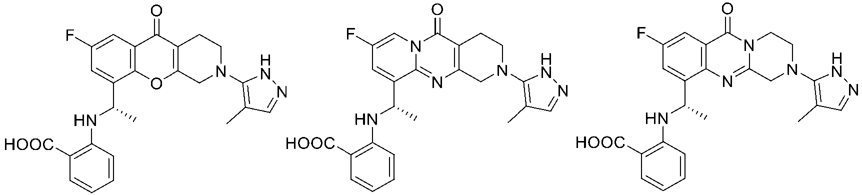

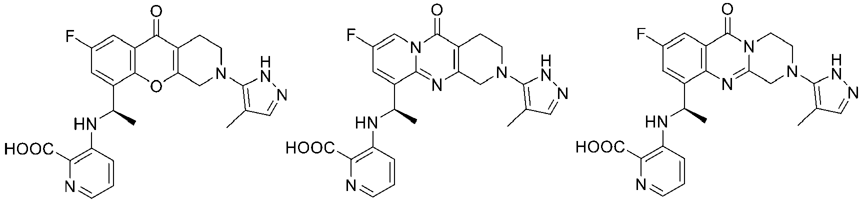

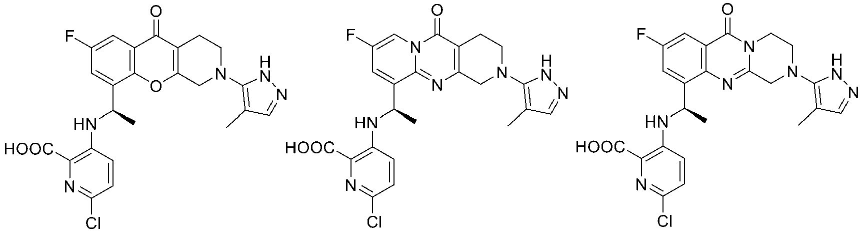

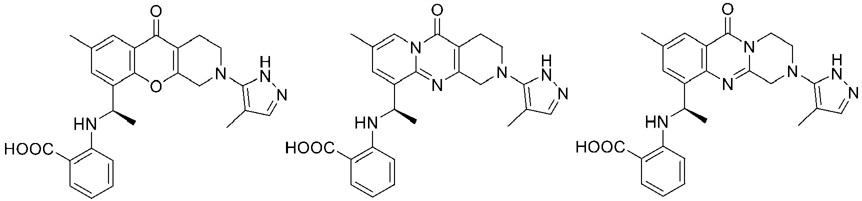

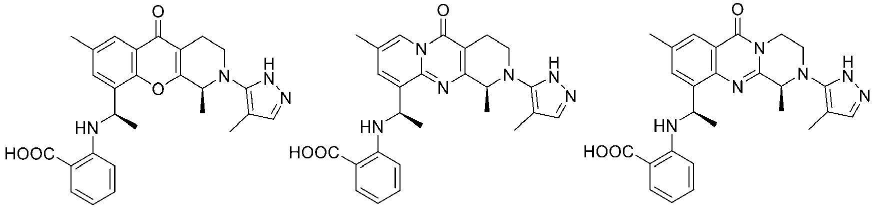

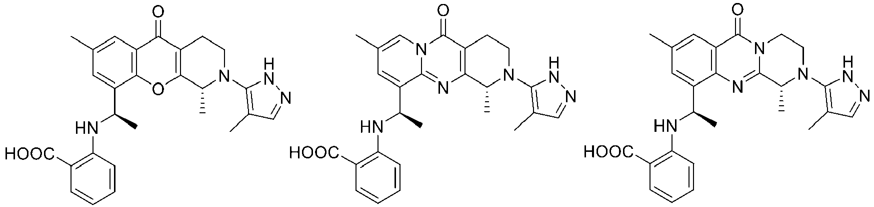

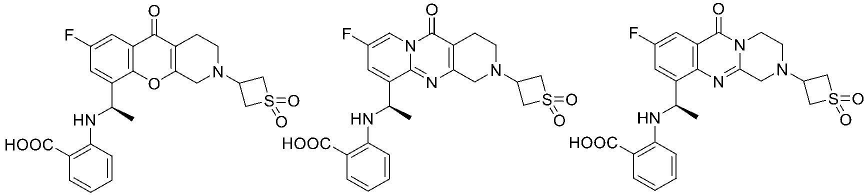

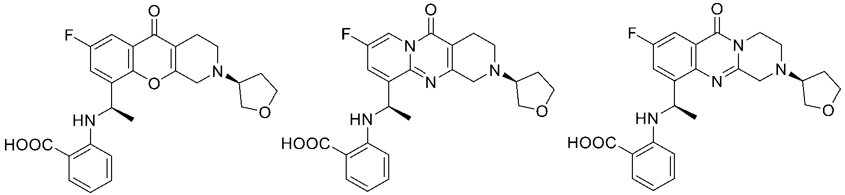

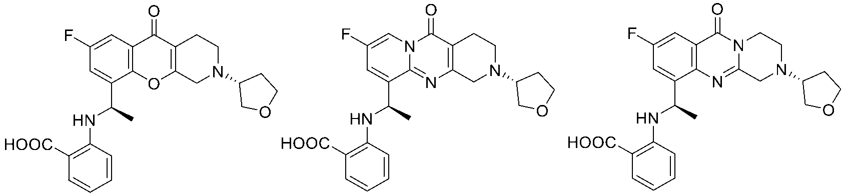

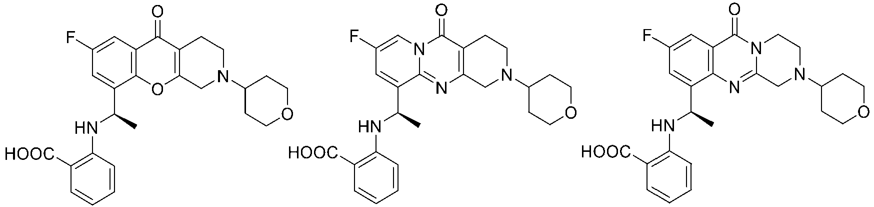

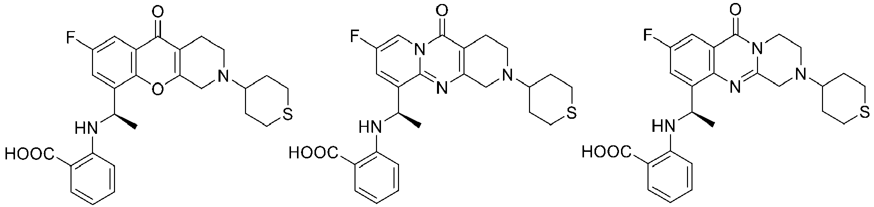

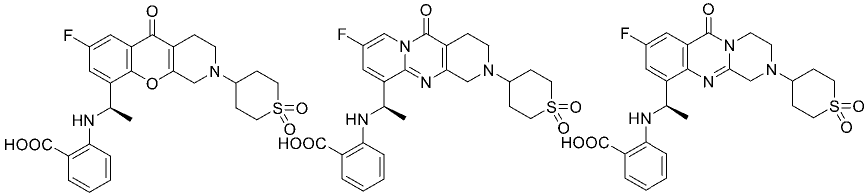

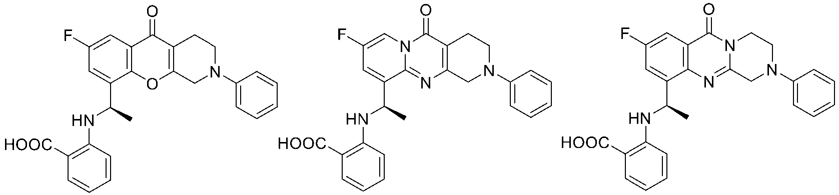

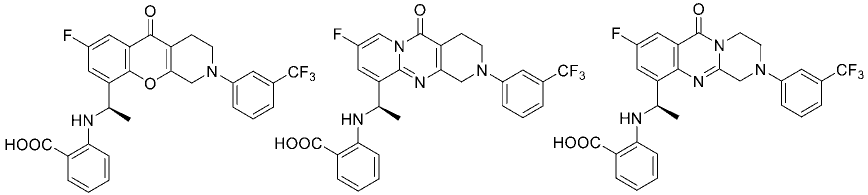

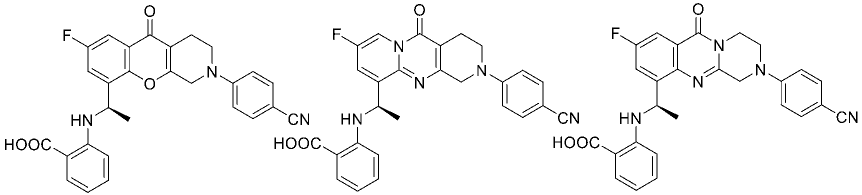

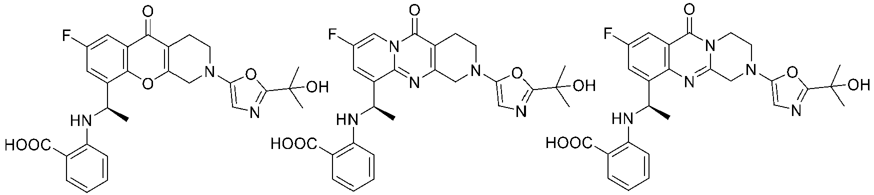

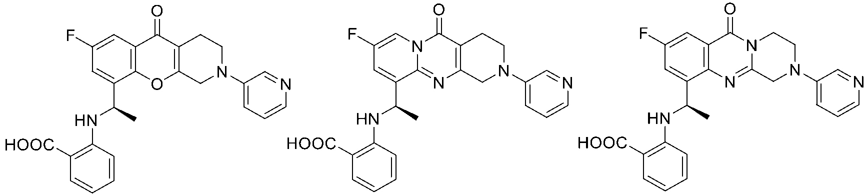

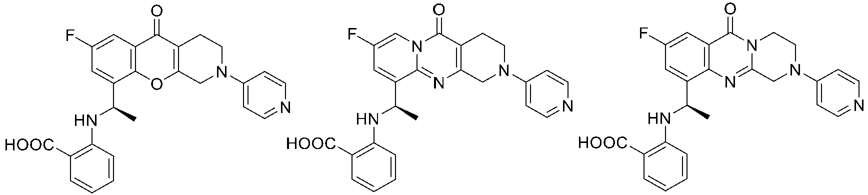

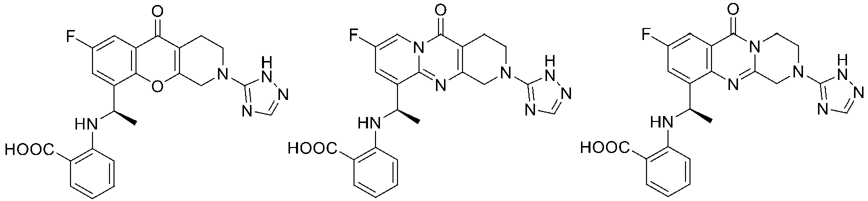

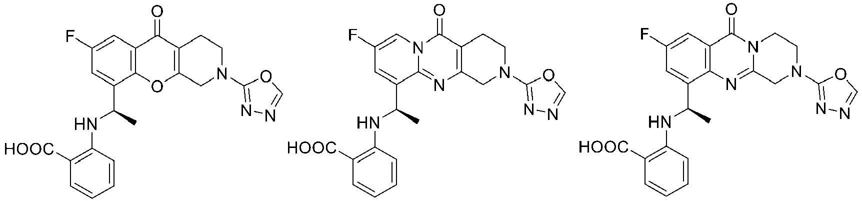

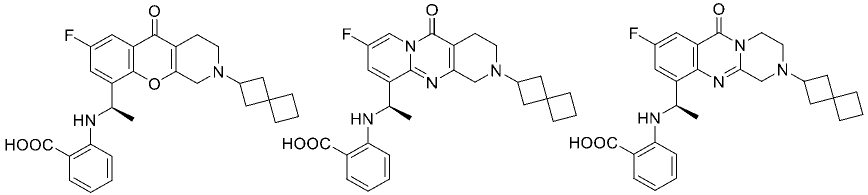

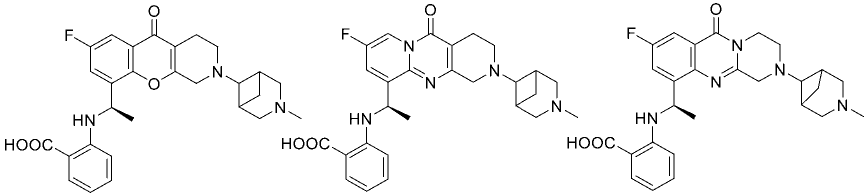

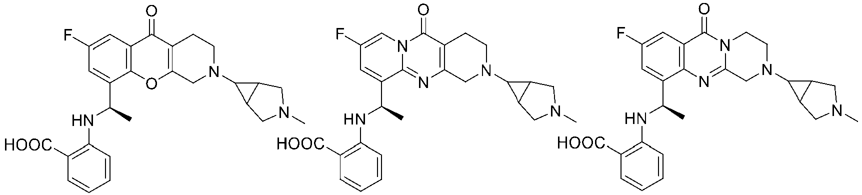

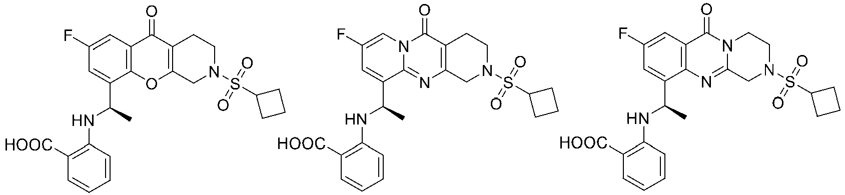

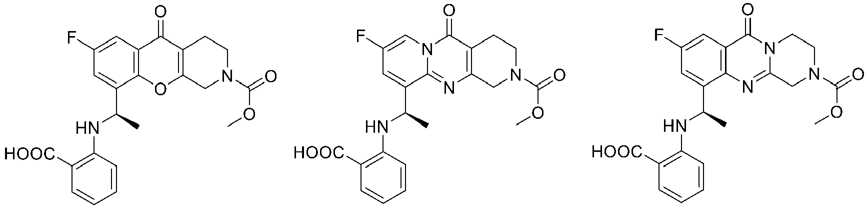

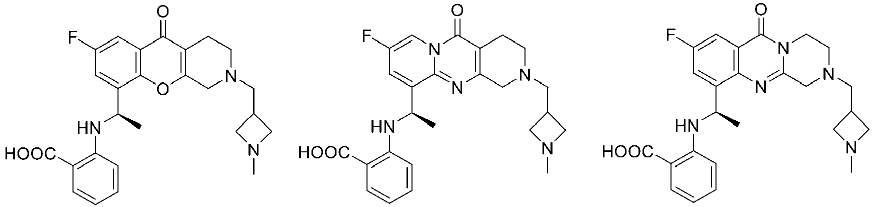

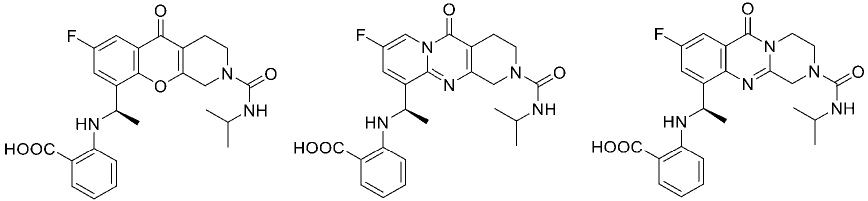

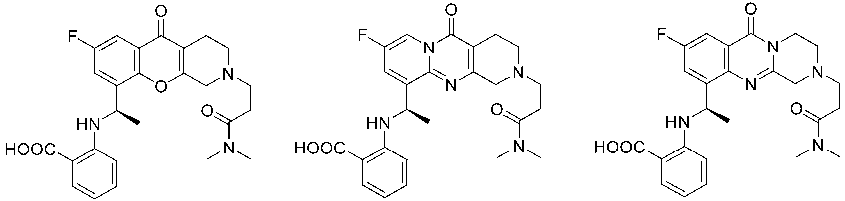

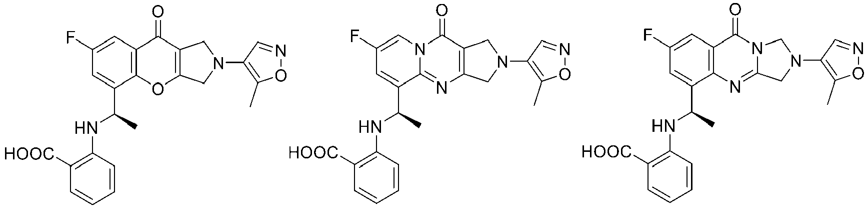

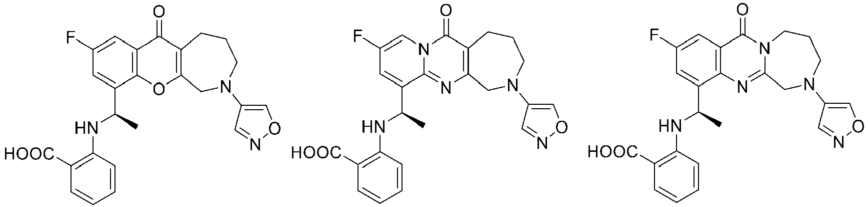

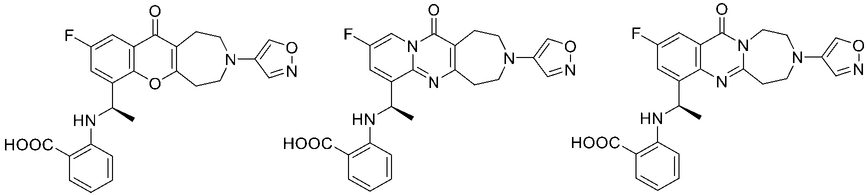

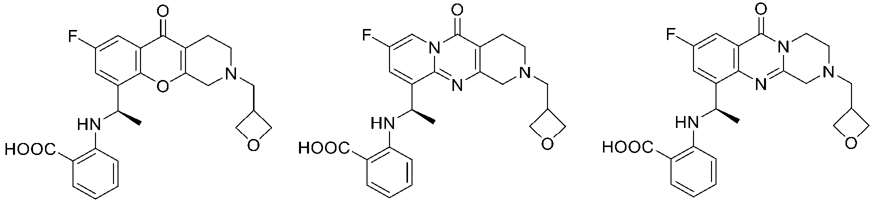

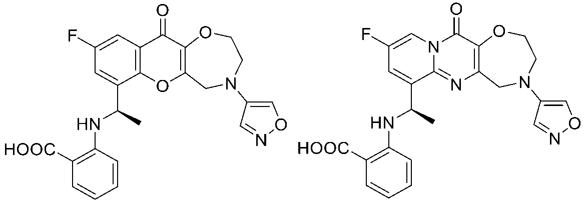

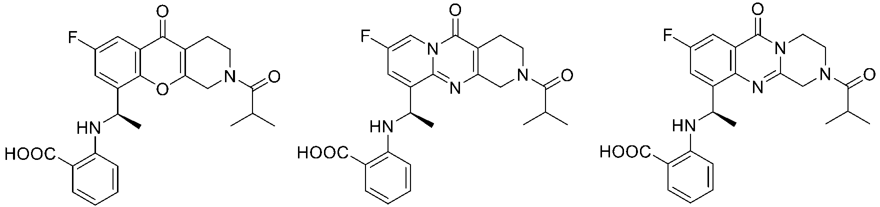

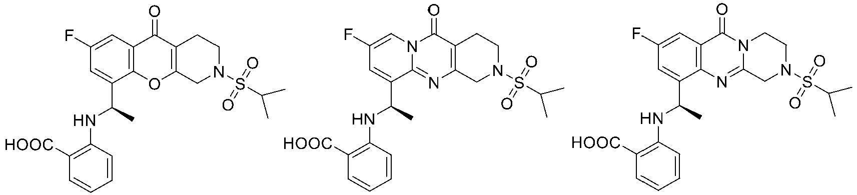

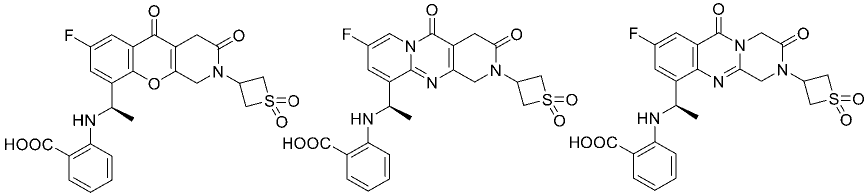

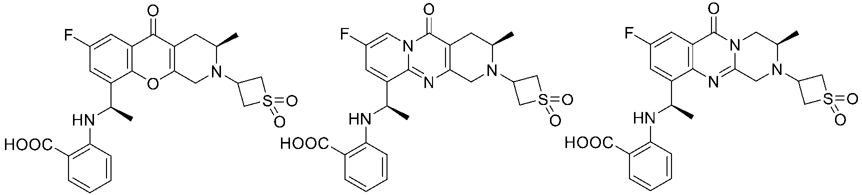

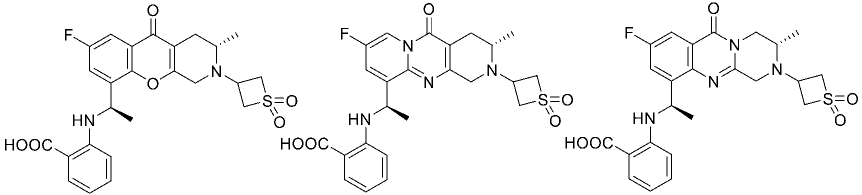

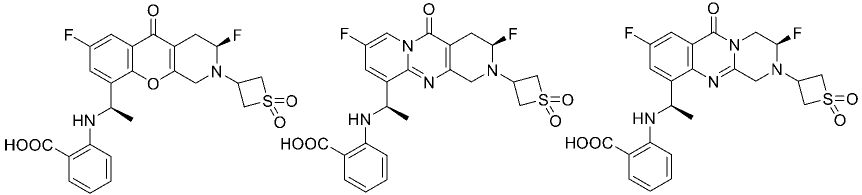

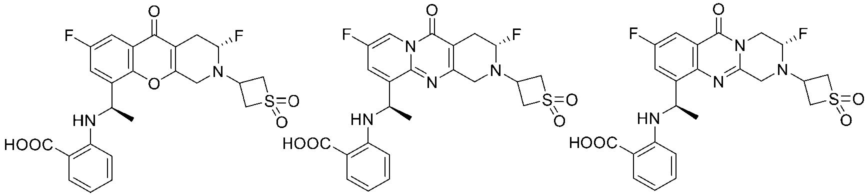

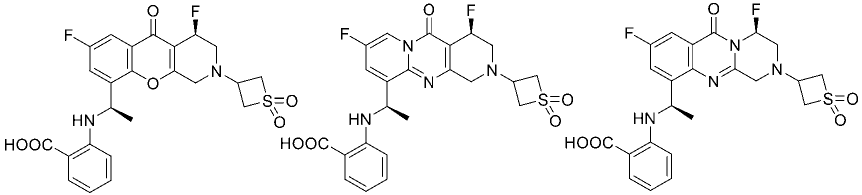

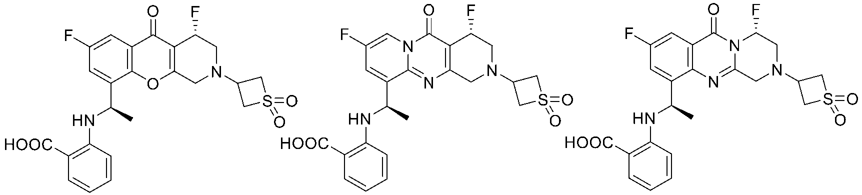

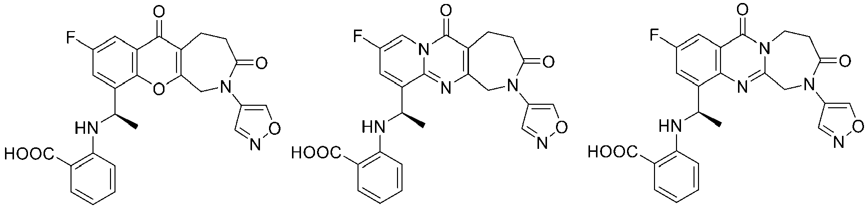

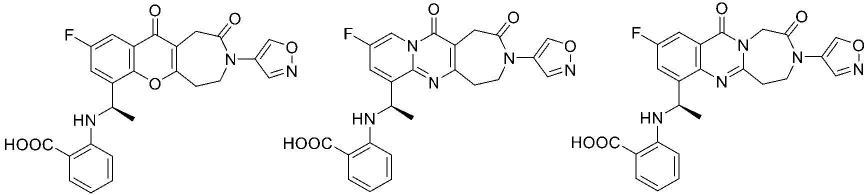

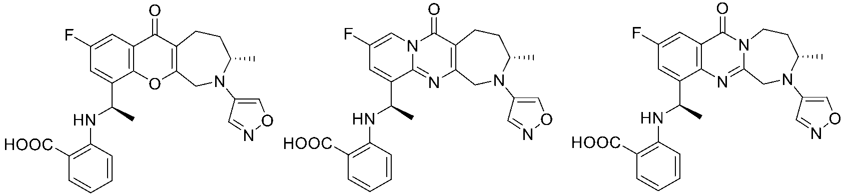

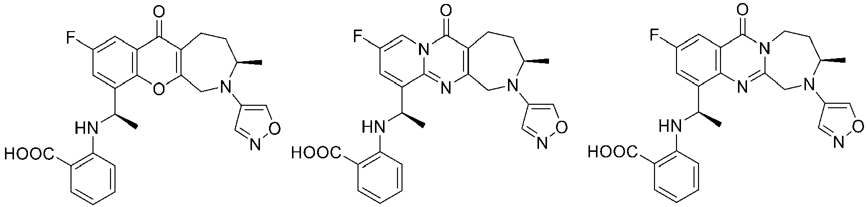

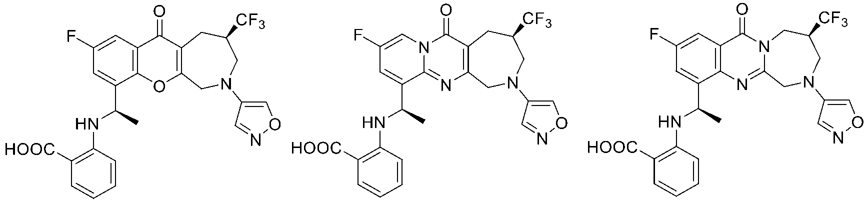

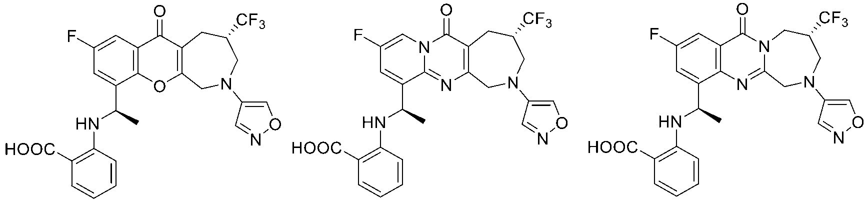

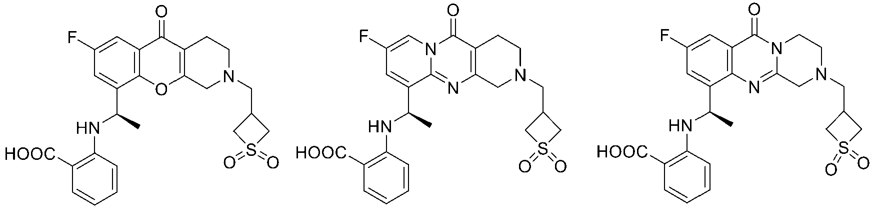

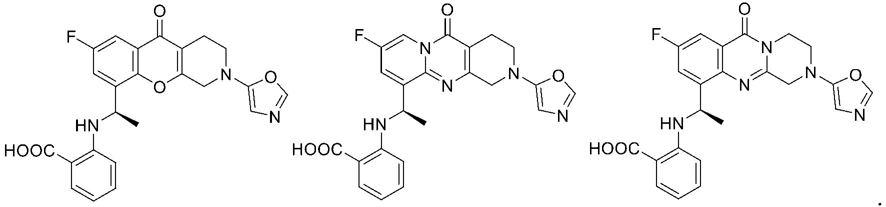

In the second aspect of the present invention, provided is a pharmaceutical composition, comprising a safe and effective amount of the compound according to the first aspect of the present invention, or the pharmaceutically acceptable salt, the stereoisomer, the deuterated compound, the solvate, or the prodrug thereof, and pharmaceutically acceptable carriers.

In the third aspect of the present invention, provided is use of the compound according to the first aspect of the present invention, or the pharmaceutically acceptable salt, the stereoisomer, the deuterated compound, the solvate, or the prodrug thereof, for a use selected from the group consisting of:
1) preparation of selective PI3Kα inhibitors;
2) preparation of drugs for regulating PI3Kα activity or treating PI3Kα-related diseases.

In another preferred embodiment, the PI3Kα has one or more mutations selected from the group consisting of: H1047R, E542K, and E545K.

In another preferred embodiment, the PI3Kα-related disease is a cancer or tumor.

In another preferred embodiment, the cancer or tumor is selected from the group consisting of: acute lymphoblastic leukemia (ALL), acute myeloid leukemia (AML), adrenocortical carcinoma, AIDS-related cancer, AIDS-related lymphoma, anal cancer, astrocytoma, basal cell carcinoma, bile duct cancer, bladder cancer, bone cancer, osteosarcoma, malignant fibrous histiocytoma, brain tumor, breast cancer, bronchial tumor, Burkitt lymphoma, carcinoid tumor, cancer of unknown primary site, cardiac (heart) tumor, atypical teratoid/rhabdoid tumor, primary central nervous system lymphoma, cervical cancer, cholangiocarcinoma, chordoma, chronic lymphocytic leukemia (CLL), chronic myelogenous leukemia (CML), colorectal cancer, craniopharyngioma, cutaneous T-cell lymphoma, mycosis, S cell lymphoma, syndrome, ductal carcinoma in situ (DCIS), embryonal tumor, medulloblastoma, endometrial cancer, ependymoma, esophageal cancer, esthesioneuroblastoma, Ewing sarcoma, extracranial germ cell tumor, extragonadal germ cell tumor, fallopian tube cancer, gallbladder cancer, gastric cancer, gastrointestinal carcinoid tumor, malignant gastrointestinal stromal tumor (GIST), germ cell tumor, gestational trophoblastic disease, hairy cell leukemia, head and neck cancer, hepatocellular carcinoma, Langerhans cell histiocytosis, Hodgkin lymphoma, islet cell tumor, pancreatic neuroendocrine tumor, Kaposi sarcoma, kidney cancer, laryngeal cancer, leukemia, liver cancer, lung cancer, lymphoma, male breast cancer, intraocular melanoma, Merkel cell carcinoma, malignant mesothelioma, metastatic cancer, metastatic squamous neck cancer, NUT gene-altered midline carcinoma, oral cancer, multiple endocrine neoplasia syndromes, multiple myeloma/plasma cell neoplasm, myelodysplastic syndrome, myelodysplastic neoplasms, myeloproliferative neoplasm, chronic myeloproliferative neoplasm, nasal cavity and paranasal sinus cancer, nasopharyngeal cancer, neuroblastoma, non-Hodgkin lymphoma, non-small cell lung cancer, oral cavity cancer, lip and oral cavity cancer, oropharyngeal cancer, malignant fibrous histiocytoma of bone, ovarian cancer, pancreatic cancer, pancreatic neuroendocrine tumor (islet cell tumor), papillomatosis, paraganglioma, nasal cavity and paranasal sinus carcinomas parathyroid cancer, penile cancer, pharyngeal cancer, pheochromocytoma, pituitary tumor, plasmacytoma, multiple myeloma, pleuropulmonary blastoma, primary central nervous system lymphoma, primary peritoneal cancer, prostate cancer, rectal cancer, recurrent cancer, renal cell (kidney) cancer, retinoblastoma, rhabdomyosarcoma, salivary gland cancer, sarcoma, childhood vascular tumor, skin cancer, small cell lung cancer, small intestine cancer, soft tissue sarcoma, squamous cell carcinoma of the skin, testicular cancer, oropharyngeal cancer, hypopharyngeal cancer, thymoma, thymic carcinoma, thyroid cancer, tracheobronchial tumor, transitional cell carcinoma of the renal pelvis and ureter, urethral cancer, uterine sarcoma, vaginal cancer, vascular tumor, vulvar cancer, and nephroblastoma.

In another preferred embodiment, the cancer is endometrial cancer, breast cancer, esophageal squamous cell carcinoma, cervical squamous cell carcinoma, cervical adenocarcinoma, colorectal adenocarcinoma, bladder urothelial carcinoma, glioblastoma, ovarian cancer, non-small cell lung cancer, esophagogastric cancer, schwannoma, head and neck squamous cell carcinoma, melanoma, esophagogastric adenocarcinoma, soft tissue sarcoma, prostate cancer, fibrolamellar carcinoma, hepatocellular carcinoma, diffuse glioma, colorectal cancer, pancreatic cancer, cholangiocarcinoma, B-cell lymphoma, mesothelioma, adrenocortical carcinoma, renal non-clear cell carcinoma, renal clear cell carcinoma, germ cell cancer, thymoma, pheochromocytoma, other neuroepithelial tumor, thyroid cancer, leukemia, or capsular glioma.

In another preferred embodiment, the cancer is breast cancer, prostate cancer, or brain cancer.

In the fourth aspect of the present invention, provided is a method for preventing and/or treating a PI3Kα activity-modulating disorder or treating a PI3Kα-related disease, comprising the step of: administering to a subject in need thereof the compound according to the first aspect of the present invention, or the pharmaceutically acceptable salt, the stereoisomer, the deuterated compound, the solvate, or the prodrug thereof.

In another preferred embodiment, the subject is a human or a non-human mammal, e.g., a rat, a mouse, a rabbit, a pig, a dog, a sheep, etc.

It should be understood that within the scope of the present invention, the above-described technical features of the present invention and the technical features described in detail below (e.g., examples) may be combined with each other to constitute a new or preferred technical solution. Limited by space, it will not be repeated here.

### DETAILED DESCRIPTION OF THE INVENTION

After long-term and in-depth research, the inventors have unexpectedly prepared a class of compounds having excellent PI3Kα mutant-selective inhibitory activity and the preparation method therefor. Specifically, through structural optimization, the inventors have obtained a class of compounds having excellent PI3Kα mutant-selective inhibitory activity. On this basis, the inventors have completed the present invention.

### Terms

In the present invention, unless otherwise specified, the terms used have the ordinary meanings well known to those skilled in the art.

In the present invention, the term "halogen" refers to F, Cl, Br or I.

In the present invention, "C₁₋₆ alkyl" refers to a linear or branched alkyl group comprising 1-6 carbon atoms, such as methyl, ethyl, propyl, isopropyl, butyl, isobutyl, tert-butyl, neopentyl, tepentyl, or similar groups. Preferably, it is C₁₋₃ alkyl. Other similar terms have similar meanings.

In the present invention, the term "C₁₋₆ alkoxy" refers to a linear or branched alkoxy group having 1-6 carbon atoms, including, without limitation, methoxy, ethoxy, propoxy, isopropoxy, butoxy and the like. Preferably, it is C₁₋₃ alkoxy. Other similar terms have similar meanings.

In the present invention, the term "deuterated C₁₋₆ alkyl" refers to a linear or branched alkyl group comprising 1-6 carbon atoms wherein one, more than one, or all hydrogen atoms on carbon atoms are optionally substituted with deuterium, such as -CD₃, -CH₂CD₃, -CH(CH₃)CD₃ and the like. The term "deuterated C₁₋₃ alkyl" has the same meaning.

In the present invention, the term "deuterated C₁₋₆ alkoxy" refers to a linear or branched alkoxy group comprising 1-6 carbon atoms wherein one, more than one, or all hydrogen atoms on carbon atoms are optionally substituted with deuterium, such as -OCD₃, -OCH₂CD₃, and - OCH(CH₃)CD₃ and the like. The term "deuterated C₁₋₃ alkoxy" has the same meaning.

In the present invention, the term "C₁₋₆ heteroalkyl" refers to a linear or branched alkyl group having 1-6 carbon atoms and containing 1-4 heteroatoms optionally selected from N, O and S, including, without limitation, methoxymethyl, ethoxymethyl, propoxymethyl, isopropoxymethyl, butoxymethyl, -CH₂OH, -CH₂NH₂, -CH₂NHCH₃, -CH₂N(CH₃)₂, - CH₂CH₂OCH₃, -CH₂CH₂NHCH₃, and -CH₂CH₂N(CH₃)₂, and the like.

In the present invention, the term "C₁₋₆ alkylene" refers to a linear or branched alkylene structure comprising 1-6 carbon atoms, including, without limitation: -CH₂-, -CH₂CH₂-, - CH₂CH₂CH₂-, -CH(CH₃)CH₂-, -CH(CH₃)CH₂CH₂-, -CH₂CH(CH₃)CH₂-, -CH₂CH₂CH₂CH₂-, - CH₂CH₂CH₂CH₂CH₂-, and -CH₂CH₂CH₂CH₂CH₂CH₂-. Other similar terms have similar meanings.

In the present invention, the term "saturated or partially saturated C₃₋₁₀ cycloalkyl" refers to a cyclic alkyl group having 3-10 carbon atoms on the ring, including C₅₋₁₀ spirocycloalkyl, C₅₋₁₀ bridged cycloalkyl, C₃₋₈ monocycloalkyl, C₄₋₁₂ fused cycloalkyl, wherein some carbon atoms on the ring may form an unsaturated structure with adjacent carbon atoms in the form of double bonds, including, without limitation: 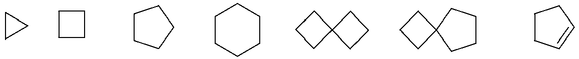 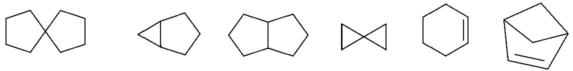 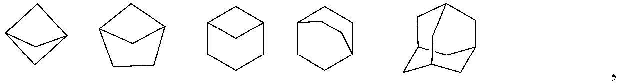 etc. The term "saturated or partially saturated C₃₋₆ cycloalkyl" has similar meanings.

In the present invention, the term "saturated or partially saturated 3-12 membered heterocycloalkyl" refers to a cyclic alkyl group containing 1-4 heteroatoms optionally selected from N, O and S, the ring may be a saturated ring or a partially unsaturated ring, which includes 5-12 membered spirocycloalkyl containing 1-4 heteroatoms optionally selected from N, O and S, 5-12 membered bridged cycloalkyl containing 1-4 heteroatoms optionally selected from N, O and S, 3-8 membered monocycloalkyl containing 1-4 heteroatoms optionally selected from N, O and S, and 4-12 membered fused cycloalkyl containing 1-4 heteroatoms optionally selected from N, O and S, and includes, without limitation:

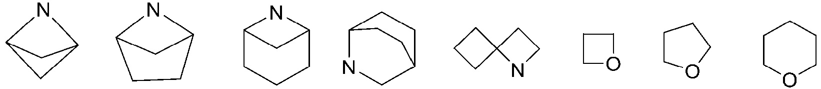

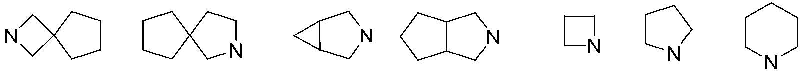

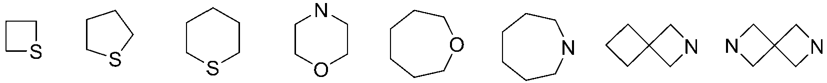

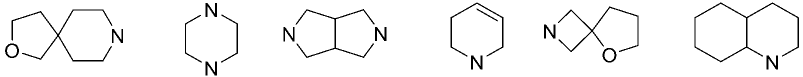

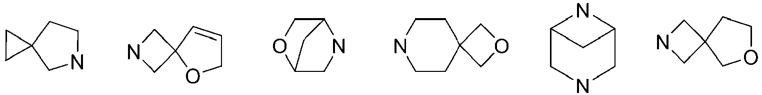

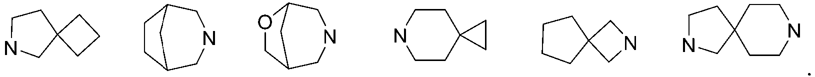

The terms "4-6 membered heterocycloalkyl", "5-7 membered heterocycloalkyl" have the same meanings.

In the present invention, the terms "aromatic ring" and "aryl" have the same meaning. The term "C₆₋₁₀ aryl" refers to an aryl group having an aromatic structure and containing 6-10 carbon atoms without heteroatoms, which includes monocyclic aryl, bicyclic C₈₋₁₀ aryl, and includes, without limitation: 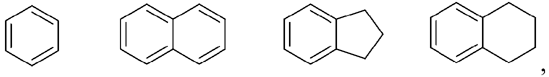 etc. The aryl group may be optionally substituted or unsubstituted. The moiety connected to the parent is an aromatic ring.

In the present invention, the terms "heteroaromatic ring" and "heteroaryl" have the same meaning, referring to a monocyclic or polycyclic heteroaromatic group containing 1-4 heteroatoms optionally selected from N, O and S. For example, "5-10 membered heteroaryl" refers to an aromatic heterocyclic ring containing 1-4 heteroatoms selected from N, O and S and 1-9 carbon atoms, including: 5-6 membered monocyclic heteroaryl, 8-10 membered polycyclic heteroaryl. Wherein the "5-6 membered monocyclic heteroaryl" includes, without limitation: furyl, thienyl, pyridyl, pyrazolyl, pyrrolyl, N-alkylpyrrolyl, pyrimidinyl, pyrazinyl, imidazolyl, tetrazolyl and the like; the "8-10 membered polycyclic heteroaryl" includes, without limitation:

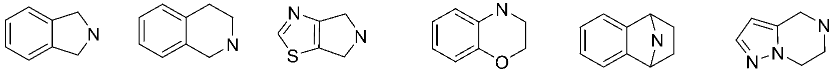

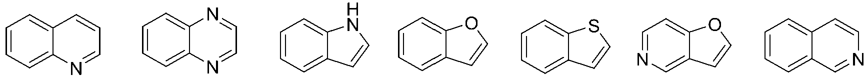

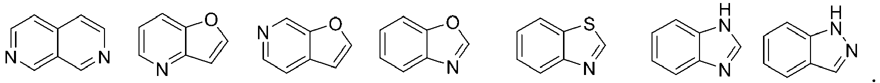

The heteroaryl may be optionally substituted or unsubstituted. Wherein, the moiety connected to the parent is an aromatic ring.

In the present invention, the term "halogenated" refers to being substituted by halogen.

In the present invention, the term "substituted" means that one or more hydrogen atoms on a specific group are replaced by specific substituents. The specific substituents are those correspondingly described in the preceding text, or those appearing in each examples. Unless otherwise specified, a substituted group may have a substituent selected from a specific group at any substitutable position of the group, and the substituents may be the same or different at each position. Those skilled in the art should understand that the combinations of substituents contemplated by the present invention are those that are stable or chemically achievable. Such substituents are for example (but not limited to), deuterium, halogen, hydroxyl, cyano, amino, alkylamino, =O, C₁₋₆ alkyl, C₁₋₆ alkoxy, C₃₋₁₀ cycloalkyl, halogenated C₁₋₆ alkyl, halogenated C₁₋₆ alkoxy, halogenated C₃₋₁₀ cycloalkyl, halogenated alkylamino, 3-10 membered heterocyclyl, aryl, heteroaryl, C₂₋₁₀ acyl, C₂₋₁₀ ester group, C₂₋₆ alkynyl, C₂₋₆ alkenyl and the like.

In the present invention, the term "0-6" refers to 0, 1, 2, 3, 4, 5 or 6. Other similar terms have similar meanings.

In the present invention, the term "one or more" refers to 1, 2, 3, 4, 5 or 6. Other similar terms have similar meanings.

It should be understood that when a certain group exists at multiple different positions of the compound, its definition at each position is independent of each other and may be the same or different. That is, the terms "selected from the group consisting of" have the same meaning as "each independently selected from the group consisting of" and "independently selected from the group consisting of".

### Compounds

The present invention provides a compound, wherein the compound is a compound of formula (I), or a pharmaceutically acceptable salt, a stereoisomer, a deuterated compound, a solvate or a prodrug thereof, 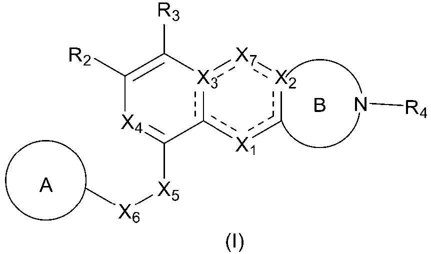 wherein R₂, R₃, R₄, X₁, X₂, X₃, X₄, X₅, X₆, X₇, ring A, and ring B are as defined above.

In another preferred embodiment, in the compounds, any one of R₂, R₃, R₄, X₁, X₂, X₃, X₄, X₅, X₆, X₇, ring A, and ring B is independently the corresponding specific group in the specific compounds.

As used herein, the term "pharmaceutically acceptable salt" refers to a salt formed by the compound of the present invention with an acid or a base, which is suitable for use as a medicine. Pharmaceutically acceptable salts include inorganic salts and organic salts. One preferred class of salts are salts formed of the compounds of the present invention with acids. Suitable acids for forming salts include, but are not limited to inorganic acids, such as hydrochloric acid, hydrobromic acid, hydrofluoric acid, sulfuric acid, nitric acid, phosphoric acid; organic acids, such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethanesulfonic acid, p-toluenesulfonic acid, benzenesulfonic acid, naphthalenesulfonic acid; and amino acids, such as proline, phenylalanine, aspartic acid, glutamic acid.

Another preferred class of salts are salts formed of the compounds of the present invention with bases, such as alkali metal salts (e.g., sodium or potassium salts), alkaline earth metal salts (e.g., magnesium or calcium salts), ammonium salts (e.g., lower alkanolammonium salts and other pharmaceutically acceptable amine salts), such as methylamine salt, ethylamine salt, propylamine salt, dimethylamine salt, trimethylamine salt, diethylamine salt, triethylamine salt, tert-butylamine salt, ethylenediamine salt, hydroxyethylamine salt, dihydroxyethylamine salt, trihydroxyethylamine salt, and amine salts formed from morpholine, piperazine, lysine, respectively.

The compounds of the present invention also include prodrugs of the compound represented by formula (I). The term "prodrug" includes a class of compounds which may themselves be biologically active or inactive, and which, upon administration by a suitable method, undergo metabolism or chemical reaction in the human body to convert into a compound of formula (I), or a salt or solution composed of a compound of formula (I). Such prodrugs include (but are not limited to) carboxylate, carbonate, phosphate, nitrate, sulfate, sulfone ester, sulfoxide ester, amino compound, carbamate, azo compound, phosphoramide, glucoside, ether, acetal and other forms of the compound.

The term "solvate" refers to a complex formed by coordination of the compound of the present invention with solvent molecules in a specific ratio.

It should be understood that the specific preparation methods of the compound of formula (I) of the present invention do not limit the present invention in any way. The compounds of the present invention can also be conveniently prepared by optionally combining various synthetic methods described in the present specification or known in the art, and such combinations can be readily carried out by those skilled in the art to which the present invention belongs.

Typically, the compounds of the present invention can be prepared via the synthetic processes shown in the examples, wherein the raw materials and reagents used are commercially available unless otherwise specified.

### Pharmaceutical compositions and administration method

The present invention further provides a pharmaceutical composition, comprising safe and effective amounts of one or more of the compound and pharmaceutically acceptable carriers.

Because the compound of the present invention has excellent anti-tumor activity, the compound of the present invention and its various crystal forms, pharmaceutically acceptable inorganic or organic salts, hydrates or solvates, and pharmaceutical compositions containing the compound of the present invention as the main active ingredient can be used to treat, prevent, and alleviate tumor-related diseases.

The pharmaceutical composition of the invention comprises the compound of the present invention or the pharmaceutically acceptable salts thereof in a safe and effective dosage range, and pharmaceutically acceptable excipients or carriers. Wherein, "safe and effective amount" means that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with each other and with the compounds of the present invention without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, powders, or granules.

There is no special limitations on the administration mode of the compounds or pharmaceutical compositions of the present invention, and representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrants such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or a mixture thereof. In capsules, tablets, and pills, the dosage form may also include a buffer.

Solid dosage forms such as tablets, sugar pills, capsules, pills, and granules can be prepared using coatings and shell materials, such as casings and other well-known materials in the art. They can contain opaque agents. The active compounds or compounds in the compositions can be released in a delayed manner in a given part of the digestive tract. Examples of embedding components that can be used are polymeric substances and waxes. If necessary, the active compound can also form microcapsules with one or more of the aforementioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition can also include additives such as wetting agents, emulsifiers and suspending agents, sweeteners, flavoring agents, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols, and their suitable mixtures.

The dosage forms of the compounds of the present invention used for topical administration include ointments, powders, patches, sprays, and inhalants. The active ingredients are mixed under sterile conditions with physiologically acceptable carriers and any preservatives, buffers, or propellants if necessary.

The compounds of the present invention can be administered alone or in combination with other pharmaceutically acceptable compounds (such as anti-tumor drugs).

The treatment method of the present invention can be administered alone or in combination with other treatment means or therapeutic drugs.

When the pharmaceutical composition is used, a safe and effective amount of the compound of the present invention is administered to a mammal (such as a human) in need of treatment, wherein the dosage at the time of administration is the pharmaceutically effective dosage, for people having a body weight of 60kg, the daily dose is usually 1~2000mg, preferably 50~ 1000mg. Of course, the specific dosage should also consider factors such as the route of administration and the patient's health status, which are within the skill range of a skilled physician.

**Compared with the prior art, the present invention has the following main advantages:**
(1) The compounds exhibit excellent inhibitory activity with selectivity against PI3Kα mutations;
(2) The compounds exhibit excellent anti-tumor effects;
(3) The compounds exhibit excellent safety;
(4) The compounds exhibit excellent pharmacokinetic properties.

The present invention will be further explained below in combination with specific examples. It should be understood that these examples are only used to illustrate the present invention and not to limit the scope of the present invention. In the following examples, the test methods without specific conditions are usually in accordance with conventional conditions or the conditions recommended by the manufacturer. Unless otherwise specified, percentages and parts are calculated by weight.

Unless otherwise defined, all professional and scientific terminology used herein have the same meanings as known to those skilled in the art. In addition, any methods and materials similar or equal to the recorded content can apply to the methods of the invention. The preferred embodiments and materials described herein are only for demonstration purposes.

### Synthetic Routes of the Examples

Unless otherwise specified, the absolute stereochemistry of all chiral atoms is defined as follows. A compound labeled with "or1" is a single-configurational compound with unknown absolute configuration. A compound labeled with "or2" is a single-configurational compound, which is the enantiomer of the structure labeled "or1", with unknown absolute configuration. A compound labeled with "abs" have the absolute configuration as shown in the structure. A compound labeled with "rac" is a racemate. A compound labeled with "S" or "R" have a known chiral configuration at the corresponding carbon atom as indicated.

### Synthesis of Intermediates

### Synthesis of Intermediate int1:

The synthetic route and experimental procedure were as follows:

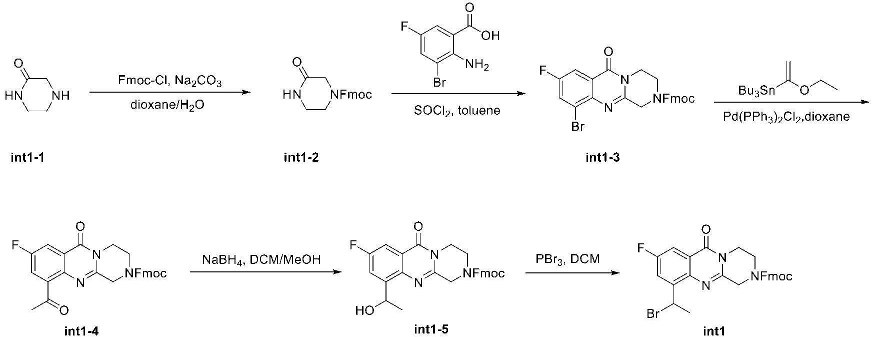

### 1. Synthesis of Compound int1-2:

Int1-1 (2 g, 1 eq), sodium carbonate (4.2 g, 2 eq), water (20 mL) and 1,4-dioxane (60 mL) were added to a 100 mL single-necked flask. The mixture was cooled to 0 °C, and Fmoc-Cl (5.7 g, 1.1 eq) was added. The mixture was allowed to warm to room temperature naturally and stirred for 4 h, then diluted with saturated brine (200 mL). The mixture was extracted with ethyl acetate. The organic phases were combined, dried and concentrated to afford 6.7 g of the product.

### 2. Synthesis of Compound int1-3

Int1-2 (5 g, 1 eq), toluene (100 mL), 2-amino-3-bromo-5-fluorobenzoic acid (4.7 g, 1.3 eq), and thionyl chloride (2.58 g, 1.4 eq) were added to a 100 mL three-necked flask. The mixture was stirred at 80 °C for 1 h, then heated to 120 °C and stirred for 3 h. After the reaction was completed, the mixture was directly purified by column chromatography to afford 2.2 g of the product.

¹H NMR (DMSO-d6,400 MHz) δ 8.22 (s, 1H), 7.94 - 7.86 (m, 2H), 7.75 (s, 1H), 7.69 - 7.62 (m, 2H), 7.34 (qd, J = 7.9, 7.3, 1.2 Hz, 4H), 4.68 - 4.38 (m, 4H), 4.33 (t, J = 6.2 Hz, 1H), 4.14 - 3.96 (m, 2H), 3.69 (s, 2H).

### 3. Synthesis of Compound int1-4

Int1-3 (2.2 g, 1 eq), 1,4-dioxane (30 mL), tributyl(1-ethoxyvinyl)tin (1.82 g, 1.2 eq), and Pd(PPh₃)₂Cl₂ (0.59 g, 0.2 eq) were added to a reaction flask. The reaction was carried out at 80 °C for 3 h under nitrogen protection. After the reaction was completed, the reaction system was cooled to room temperature. 100 mL of 2 M hydrochloric acid was added, and the mixture was stirred for 30 min. Then 100 mL of saturated potassium fluoride solution was added, and the mixture was stirred for another 30 min. The pH was adjusted to nearly neutral with the addition of saturated sodium bicarbonate solution. The mixture was extracted with ethyl acetate, dried, concentrated, and purified by column chromatography to afford 1.7 g of the product.

### 4. Synthesis of Compound int1-5

The above int1-4 (1.7 g, 1 eq), methanol (3 mL) and dichloromethane (30 mL) were added to a 500 mL single-necked flask. The mixture was cooled to 0 °C, and NaBH₄ (0.2 g, 1.5 eq) was added. After addition, the mixture was stirred at 0 °C for 1 h. The reaction was completed as monitored by LC-MS. Saturated ammonium chloride solution (10 mL) was added, and the mixture was stirred for 30 min. The mixture was extracted with dichloromethane (20 mL × 3), dried, concentrated, and purified by column chromatography to afford 1.22 g of the product.

¹H NMR (400 MHz, Methanol-d4) δ 7.74 (d, J = 9.3 Hz, 3H), 7.62 (t, J = 7.1 Hz, 3H), 7.38 - 7.23 (m, 4H), 5.67 (s, 1H), 4.62 (s, 2H), 4.29 (d, J = 23.7 Hz, 2H), 4.02 (s, 1H), 3.79 (d, J = 44.0 Hz, 1H), 3.60 (s, 1H), 1.50 (d, J = 6.3 Hz, 3H).

### 5. Synthesis of Compound int1

Int1-5 (1.22 g, 1 eq) and dichloromethane (60 mL) were added to a 25 mL three-necked flask. Under nitrogen protection, the mixture was cooled to 0 °C, and phosphorus tribromide (1 mL) was added dropwise. The mixture was stirred for 16 h. After the reaction was completed, the mixture was concentrated to give 2.2 g of crude product, which was used directly in the next reaction step.

¹H NMR (400 MHz, Chloroform-d) δ 7.88 (d, J = 7.9 Hz, 1H), 7.77 (s, 3H), 7.58 (s, 2H), 7.45 - 7.29 (m, 4H), 6.30 (d, J = 7.3 Hz, 1H), 4.60 (q, J = 21.5, 21.0 Hz, 4H), 4.27 (t, J = 6.0 Hz, 1H), 4.17 - 4.02 (m, 2H), 3.68 (s, 2H), 2.06 (d, J = 13.4 Hz, 3H).

### Synthesis of Intermediate int2

The synthetic route and experimental procedure were as follows:

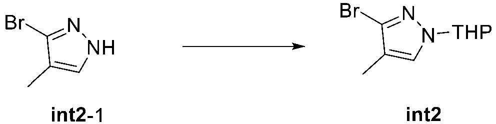

Int2-1 (300 mg, 1.0 eq), DHP (234 mg, 2 eq) and dichloromethane (50 mL) were added to a 100 mL three-necked flask, followed by the addition of p-toluenesulfonic acid (64 mg, 0.2 eq). The mixture was reacted at room temperature for 1 h under nitrogen protection. After the reaction was completed, the mixture was washed with saturated aqueous sodium chloride solution, extracted with dichloromethane, concentrated and purified to afford 300 mg of the product.

### Synthesis of Intermediate int3

The synthetic route and experimental procedure were as follows:

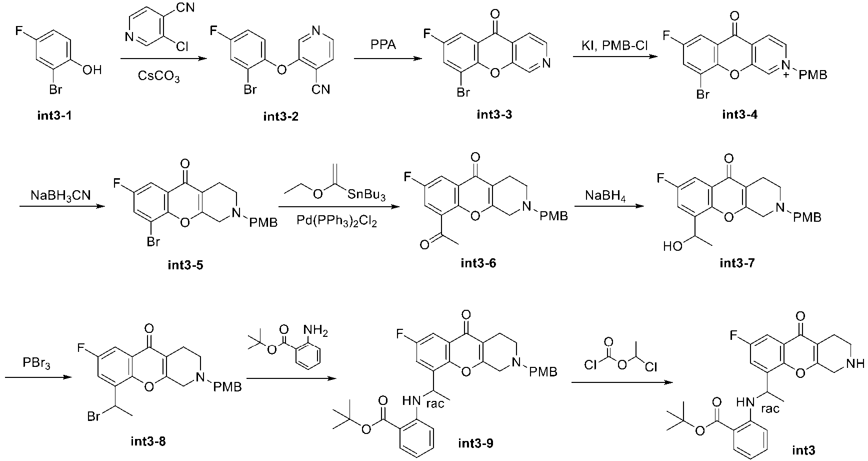

### 1. Synthesis of Compound int3-2

Compound int3-1 (8.25 g, 43.2 mmol) was dissolved in 150 mL of N,N-dimethylformamide. 3-chloro-4-cyanopyridine (5 g, 36 mmol) and cesium carbonate (23.5 g, 72 mmol) were added. After the addition, the mixture was heated to 110 °C and reacted for 2 h until the starting materials were reacted completely. The reaction was quenched by slow addition of water. The mixture was extracted with dichloromethane. The organic phase was separated, dried over anhydrous sodium sulfate, and the filtrate was concentrated under reduced pressure and purified to afford 12 g of the product.

¹H NMR (400 MHz, Chloroform-*d*) δ 8.48 (d, *J* = 4.8 Hz, 1H), 8.11 (s, 1H), 7.56 (d, *J =* 4.8 Hz, 1H), 7.45 (dd, *J =* 7.6, 2.8 Hz, 1H), 7.24 - 7.09 (m, 2H).

### 2. Synthesis of Compound int3-3

Int3-2 (12 g, 41 mmol) was dissolved in 120 g of polyphosphoric acid. The mixture was slowly heated to 220 °C and stirred for 3 h. After the starting materials were reacted completely, the reaction solution was cooled to room temperature, dissolved in water, and basified with saturated aqueous sodium bicarbonate solution. The mixture was then extracted with ethyl acetate for three times. The organic phases were combined, dried over anhydrous sodium sulfate, concentrated under reduced pressure, and purified to afford 9.69 g of the product.

¹H NMR (400 MHz, Chloroform-d) δ 9.17 (s, 1H), 8.71 (d, *J =* 5.1 Hz, 1H), 8.09 (d, *J =* 5.1 Hz, 1H), 7.97 (dd, *J =* 7.6, 3.2 Hz, 1H), 7.83 (dd, *J =* 7.2, 3.2 Hz, 1H).

### 3. Synthesis of Compound int3-4

Int3-3 (9 g, 30.6 mmol), potassium iodide (11.2 g, 67.32 mmol), and benzyl chloride (9.6 g, 61.2 mmol) were added to 360 mL of acetonitrile. The reaction solution was slowly heated to 80 °C and stirred overnight. After the starting materials were reacted completely, the reaction solution was concentrated under reduced pressure to give 17 g of crude product, which was used directly in the next reaction step.

### 4. Synthesis of Compound int3-5

Compound int3-4 (17 g, 30.9 mmol) was dissolved in 200 mL of anhydrous ethanol, followed by the addition of sodium cyanoborohydride (15.5 g, 247.2 mmol) and acetic acid (1.8 g, 30.6 mmol). The reaction mixture was stirred at room temperature for 30 minutes. After the starting materials were reacted completely, the reaction solution was diluted with water and then extracted for three times with dichloromethane. The organic phases were combined, dried over anhydrous sodium sulfate, concentrated under reduced pressure and purified to afford 6 g of the product.

¹H NMR (400 MHz, Chloroform-*d*) δ 7.81 (dd, *J =* 8.0, 3.2 Hz, 1H), 7.61 (dd, *J =* 7.2, 3.2 Hz, 1H), 7.32 - 7.27 (m, 2H), 6.95 - 6.86 (m, 2H), 3.82 (s, 3H), 3.69 (s, 2H), 3.53 (t, *J =* 1.6 Hz, 2H), 2.77 (t, *J* = 5.8 Hz, 2H), 2.71 - 2.62 (m, 2H).

### 5. Synthesis of Compound int3-6

Compound int3-5 (6 g, 14.4 mmol), tributyl(1-ethoxyvinyl)tin (6.2 g, 17.2 mmol) and Pd(PPh₃)₂Cl₂ (1 g, 1.44 mmol) were dissolved in 250 mL of dry 1,4-dioxane, and the reaction mixture was stirred at 80 °C for 12 hours. Then potassium fluoride solution was added at room temperature, and the mixture was stirred for 0.5 hours and filtered. The mother liquor was extracted for three times with ethyl acetate, and the organic phase was separated and dried by rotary evaporation. The residue was dissolved in dichloromethane. Then 2 mL of concentrated hydrochloric acid was added, and the mixture was stirred at 25 °C for 10 minutes. After the reaction was completed, the mixture was purified by column chromatography to afford the product (4 g).

¹H NMR (400 MHz, Chloroform-d) δ 8.03 (dd, *J =* 7.6, 3.2 Hz, 1H), 7.80 (dd, *J =* 8.0, 3.2 Hz, 1H), 7.31 - 7.27 (m, 2H), 6.93 - 6.87 (m, 2H), 3.82 (s, 3H), 3.69 (s, 2H), 3.53 (d, *J =* 1.6 Hz, 2H), 2.78 (t, *J* = 5.8 Hz, 2H), 2.71 (s, 3H), 2.68 (d, *J =* 6.8 Hz, 2H).

### 6. Synthesis of Compound int3-7

Compound int3-6 (1 g, 2.62 mmol) was dissolved in a mixed solvent of 10 mL dichloromethane and 10 mL methanol. Under nitrogen protection, sodium borohydride (50 mg, 1.31 mmol) was added slowly, and the reaction solution was stirred at room temperature for 10 min. After the starting materials were reacted completely, the reaction mixture was diluted with water and then extracted three times with dichloromethane. The organic phases were combined, dried over anhydrous sodium sulfate, concentrated under reduced pressure and purified to afford 600 mg of the product.

¹H NMR (400 MHz, Chloroform-d) δ 7.67 (dd, *J =* 7.9, 3.2 Hz, 1H), 7.52 (dd, *J =* 8.8, 3.2 Hz, 1H), 7.36 - 7.29 (m, 2H), 6.97 - 6.86 (m, 2H), 5.28 (q, *J =* 6.1 Hz, 1H), 3.83 (s, 3H), 3.76 - 3.63 (m, 2H), 3.40 (s, 2H), 2.86 - 2.70 (m, 2H), 2.66 (d, *J =* 5.8 Hz, 2H), 1.47 (d, *J =* 6.4 Hz, 3H).

### 7. Synthesis of Compound int3-8

Compound int3-7 (600 mg, 1.56 mmol) was dissolved in 50 mL of anhydrous dichloromethane. Under nitrogen protection, phosphorus tribromide (1.3 g, 4.68 mmol) was added slowly, and the reaction mixture was stirred at room temperature for 12 h. After the starting materials were reacted completely, the reaction mixture was concentrated under reduced pressure to afford 600 mg of the product.

### 8. Synthesis of Compound int3-9

Compound int3-8 (600 mg, 1.35 mmol) was dissolved in 20 mL of anhydrous N,N-dimethylformamide. At room temperature, *tert*-butyl 2-aminobenzoate (782 mg, 4.05 mmol) was added slowly, the temperature was raised slowly to 60 °C, and the mixture was stirred and reacted for 12 h. After the starting materials were reacted completely, the reaction mixture was diluted with water and then extracted three times with ethyl acetate. The organic phases were combined, dried over anhydrous sodium sulfate, concentrated under reduced pressure and purified to afford 400 mg of the product.

¹H NMR (400 MHz, Chloroform-d) δ 8.27 (d, *J =* 6.0 Hz, 1H), 7.88 (dd, *J =* 8.0, 1.6 Hz, 1H), 7.70 (dd, *J=* 8.0, 3.2 Hz, 1H), 7.38 (dd, *J* = 8.8, 3.2 Hz, 1H), 7.31 (d, *J =* 8.4 Hz, 2H), 7.19 - 7.08 (m, 1H), 6.94 - 6.87 (m, 2H), 6.56 (t, *J =* 7.6 Hz, 1H), 6.21 (d, *J =* 8.4 Hz, 1H), 5.05 (t, *J =* 6.4 Hz, 1H), 3.82 (s, 3H), 3.74 - 3.64 (m, 2H), 3.55 (d, *J =* 6.9 Hz, 2H), 2.78 (dt, *J* = 9.6, 5.6 Hz, 2H), 2.71 (d, *J =* 6.0 Hz, 2H), 1.62 (s, 9H).

### 9. Synthesis of Compound int3

Compound int3-9 (180 mg, 0.32 mmol) was dissolved in 20 mL of 1,2-dichloroethane. At room temperature, 1-chloroethyl chloroformate (92 mg, 0.64 mmol) was added slowly, the temperature was raised slowly to 83 °C, and the mixture was stirred and reacted for 3 h. After the starting materials were reacted completely, the reaction mixture was concentrated to dryness. Methanol was added thereto, and the mixture was heated to 65 °C and stirred and reacted for 1 h. The reaction mixture was then concentrated, and the residue was triturated with methyl *tert-butyl* ether to afford 140 mg of the product.

¹H NMR (400 MHz, Methanol-*d*₄) δ 7.87 (dd, *J =* 8.0, 1.6 Hz, 1H), 7.70 (dd, *J =* 8.0, 3.2 Hz, 1H), 7.55 (dd, *J =* 8.8, 3.2 Hz, 1H), 7.19 (m, 1H), 6.59 (ddd, *J* = 8.0, 7.2, 1.2 Hz, 1H), 6.46 (d, *J =* 8.4 Hz, 1H), 5.23 (q, *J =* 6.8 Hz, 1H), 4.54 - 4.39 (m, 2H), 3.58 (t, *J =* 6.0 Hz, 2H), 2.96 - 2.86 (m, 2H), 1.71 (d, *J* = 6.8 Hz, 3H), 1.66 (s, 9H).

### Synthesis of Intermediate int4

The synthetic route and experimental procedure were as follows:

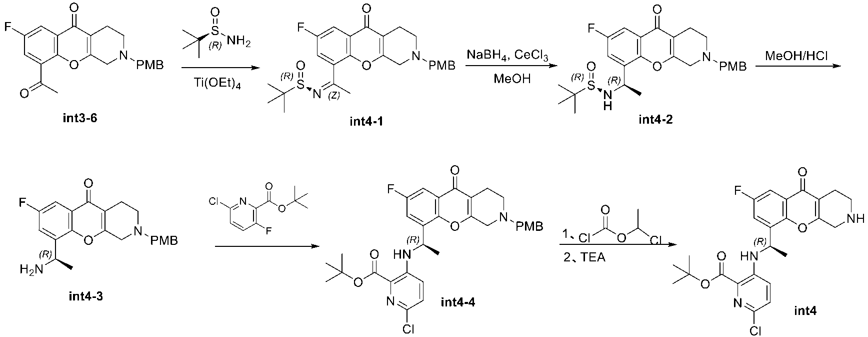

### 1. Synthesis of int4-1

Compound int3-6 (9 g, 23.6 mmol) was dissolved in 200 mL of tetrahydrofuran, followed by the addition of R-*tert*-butyl sulfinamide (5.7 g, 47.2 mmol) and tetraethyl titanate (21.5 g, 94.4 mmol). The mixture was stirred at 70 °C overnight. After the reaction was completed, water was added and the mixture was filtered. The filter cake was washed with ethyl acetate, and the aqueous phase was extracted with ethyl acetate. The organic phases were combined, concentrated and subjected to column chromatography to afford 10 g of the product.

### 2. Synthesis of int4-2

Compound int4-1 (10 g, 20.6 mmol) was dissolved in 400 mL of methanol, and cerium chloride heptahydrate (3.8 g, 10.3 mmol) was then added. Upon completion of addition, sodium borohydride (1.17 g, 30.9 mmol) was added to the reaction system in batches, and the mixture was reacted at room temperature for 10 min. After the reaction was completed, the mixture was concentrated and subjected to column chromatography to afford 8.87 g of the product.

### 3. Synthesis of int4-3

Compound int4-2 (12 g, 24.6 mmol) was dissolved in 120 mL of methanol/hydrogen chloride solution, and the reaction was carried out at room temperature for 10 min. After the reaction was completed, the reaction mixture was directly rotary evaporated to dryness. The resulting residue was basified with saturated aqueous sodium bicarbonate solution, extracted with ethyl acetate, dried, concentrated and subjected to column chromatography to afford 10 g of the product.

### 4. Synthesis of int4-4

Compound int4-3 (5 g, 13.1 mmol) was dissolved in 100 mL of dimethyl sulfoxide, and N,N-diisopropylethylamine (8.5 g, 65.5 mmol) was added. Upon completion of addition, *tert*-butyl 6-chloro-3-fluoropyridin-2-carboxylate (18.1 g, 78.6 mmol) was added to the reaction system, and the reaction was carried out at 70 °C for 4 h under nitrogen protection. After the reaction was completed, water was added and the mixture was extracted with ethyl acetate, dried, concentrated and subjected to column chromatography to afford 3.4 g of the product.

### 5. Synthesis of int4

Compound int4-4 (3.4 g, 5.72 mmol) was dissolved in 300 mL of 1,2-dichloroethane, and 1-chloroethyl chloroformate (1.63 g, 11.44 mmol) was added. Upon completion of addition, the reaction was carried out at 83 °C for 0.5 h. After the reaction was completed, the mixture was directly rotary evaporated to dryness. 300 mL of methanol was added to dissolve the residue after rotary evaporation, and triethylamine (2.3 g, 22.88 mmol) was immediately added. Upon completion of addition, the reaction was carried out at 65 °C for 2 h. After the reaction was completed, the mixture was concentrated and subjected to column chromatography to afford 2.3 g of the product.

### Synthesis of Intermediate int5

The synthetic route and experimental procedure were as follows:

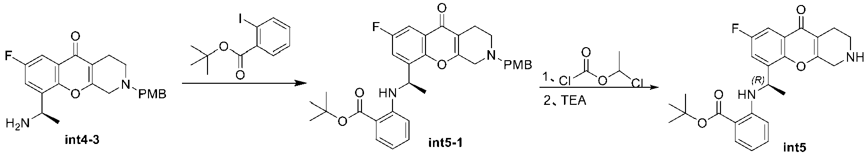

### 1. Synthesis of int5-1

Compound int4-3 (930 mg, 2.43 mmol) was dissolved in 40 mL of dry toluene, followed by the sequential addition of *tert*-butyl 2-iodobenzoate (888.1 mg, 2.92 mmol), cesium carbonate (2.14 g, 6.56 mmol), palladium acetate (54.5 mg, 0.25 mmol) and Xantphos (281 mg, 0.5 mmol). Upon completion of the addition, the reaction was carried out at 60 °C overnight under nitrogen protection. The reaction mixture was then filtered, and the filter cake was washed with ethyl acetate, mixed with silica gel, subjected to column chromatography, and the resulting mixture was dried, concentrated and subjected to column chromatography to afford 710 mg of the product.

### 2. Synthesis of int5

Compound **int5-1** (710 mg, 1.27 mmol) was dissolved in 50 mL of 1,2-dichloroethane, and 1-chloroethyl chloroformate (365 mg, 2.54 mmol) was added. Upon completion of the addition, the reaction was carried out at 83 °C for 1 h. After the reaction was completed, the reaction mixture was directly rotary evaporated to dryness. 50 mL of methanol was added to dissolve the residue after evaporation, and triethylamine (600 mg, 5.08 mmol) was immediately added. Upon completion of the addition, the reaction was carried out at 65 °C for 3 h. After the reaction was completed, the mixture was dried by rotary evaporation, concentrated and subjected to column chromatography to afford 710 mg of the product.

### Synthesis of Intermediate int6

The synthetic route and experimental procedure were as follows:

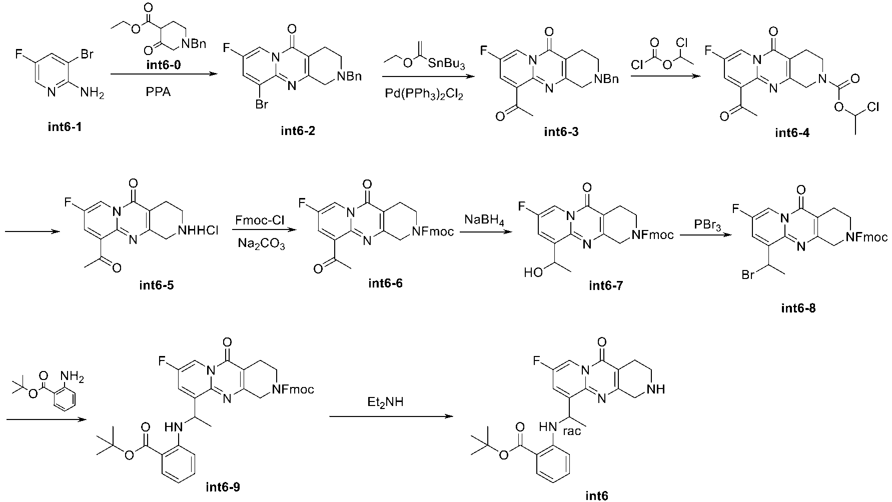

### 1. Synthesis of Compound int6-2

Compound int6-1 (3.8 g, 20 mmol) and int6-0 (6 g, 20 mmol) were dissolved in 1,2-dichloroethane (20 mL). The above solution was then added to a sealed tube containing 18 g of polyphosphoric acid, and the mixture was stirred at 85 °C for 12 h before being cooled to room temperature. After the reaction was completed, saturated aqueous sodium bicarbonate solution was added to the reaction solution above to adjust pH to alkaline. The mixture was extracted three times with ethyl acetate, dried by rotary evaporation, and purified by column chromatography to afford 1.7 g of the product.

¹HNMR: (CDCl₃,400Hz),*δ* 8.90~8.88 (m,1H), 7.94~7.92(m,1H), 7.39~7.28 (m,5H), 3.75(s,2H), 3.68 (s, 2H), 2.84~2.80(m,4H).

### 2. Synthesis of Compound int6-3

Compound int6-2 (1.2 g, 3.1 mmol), tributyl(1-ethoxyvinyl)tin (1.57 g, 4.3 mmol) and Pd(PPh₃)₂Cl₂ (200 mg, 0.31 mmol) were dissolved in dry 1,4-dioxane (50 mL), and the mixture was stirred at 80 °C for 12 h. Potassium fluoride solution was then added at room temperature, and the reaction mixture was stirred for 0.5 h, and filtered. The mother liquor was extracted three times with ethyl acetate, and the organic phase was separated and dried by rotary evaporation. 10 mL of 1,4-dioxane and 10 mL of concentrated hydrochloric acid were added to the residue, and the mixture was stirred at 25 °C for 1 h. After the reaction was completed, saturated aqueous sodium bicarbonate solution was added. The mixture was extracted with ethyl acetate, dried by rotary evaporation, and purified by column chromatography to afford 0.88 g of the product.

¹HNMR: (CDCl₃, 400Hz), *δ* 8.99~8.97 (m,1H), 7.87~7.85(m,1H), 7.38~7.28 (m,5H), 3.75(s,2H), 3.63 (s, 2H), 2.84~2.80(m,7H).

### 3. Synthesis of Compound int6-4

Compound int6-3 (465 mg, 13.3 mmol) was dissolved in 1,2-dichloroethane (10 mL). Under nitrogen protection at room temperature, 1-chloroethyl chloroformate (0.29 g, 26.5 mmol) was added, and the mixture was stirred at 83 °C for 2 h. After the reaction was completed, the reaction mixture was concentrated and directly used for the next reaction step.

### 4. Synthesis of Compound int6-5

The compound int6-4 (650 mg, crude) was dissolved in methanol (10 mL), and the reaction mixture was reacted at 65 °C for 1 h under nitrogen protection. After the reaction was completed, the mixture was dried by rotary evaporation, and the residue was triturated with methyl *tert*-butyl ether to afford 360 mg of the product.

¹HNMR: (DMSO-d6, 400Hz)*δ*9.75(s, 2H), 9.03~9.01(m, 1H), 8.23~8.21 (m,1H) , 4.22(s,2H), 3.38~3.35(m,2H), 2.87~2.84(m,2H), 2.71 (s, 2H ),.

### 5. Synthesis of Compound int6-6

Compound int6-5 (150 mg, 0.5 mmol) was dissolved in dioxane/water (3 mL/1 mL). Under nitrogen protection at 0 °C, sodium carbonate (160 mg, 1.5 mmol) and Fmoc-Cl (142 mg, 0.55 mmol) were added, and the mixture was slowly warmed to room temperature and stirred for 12 h. After the reaction was completed, saturated brine was added, and the mixture was extracted three times with dichloromethane, dried by rotary evaporation, and triturated to afford 120 mg of the product.

¹HNMR: (CDCl₃, 400Hz),*δ* 9.00~8.97(m, 1H), 7.95~7.94(m, 1H), 7.78~7.58(m, 4H), 7.40~7.36(m, 4H), 4.62~4.54(m, 2H), 4.42~4.28 (m, 2H), 3.71~3.70(m, 3H), 2.86(s, 3H), 2.77(s,2H).

### 6. Synthesis of Compound int6-7

Compound int6-6 (120 mg, 0.25 mmol) was dissolved in methanol/dichloromethane (3 mL/3 mL). Under nitrogen protection at room temperature, sodium borohydride (9.4 mg, 0.25 mmol) was added, and the mixture was stirred at 25 °C for 10 min. After the reaction was completed, water was added to quench the reaction, and the mixture was dried by rotary evaporation, and separated by column chromatography to afford 80 mg of the product.

### 7. Synthesis of Compound int6-8

Compound int6-7 (80 mg, 0.165 mmol) was dissolved in dry dichloromethane (10 mL). Under nitrogen protection at room temperature, phosphorus tribromide (134 mg, 0.494 mmol) was added, and the mixture was stirred at 25 °C for 12 h. The reaction mixture was concentrated and directly used for the next reaction step.

### 8. Synthesis of Compound int6-9

The compound int6-8 (180 mg, crude) was dissolved in dry N,N-dimethylformamide (1 mL), and the mixture was added to a solution of *tert*-butyl 2-aminobenzoate (191 mg, 0.989 mmol) in N,N-dimethylformamide (2 mL) at 25 °C. The reaction was carried out at 60 °C and stirred for 12 h. After the reaction was completed, saturated brine was added, and the mixture was extracted three times with ethyl acetate, concentrated, and separated by column chromatography to afford 15 mg of the product.

### 9. Synthesis of Compound int6

Compound int6-9 (15 mg, 0.223 mmol) was dissolved in a mixture of dry dichloromethane (5 mL) and diethylamine (0.5 mL), and the mixture was stirred at room temperature for 12 h under nitrogen protection. After the reaction was completed, the mixture was separated by column chromatography to afford 6 mg of the product.

¹HNMR:(CDCl₃,400Hz) *δ* 8.82~8.80(m,1H), 8.33~8.30(m,1H), 7.92~7.90(m,1H), 7.65~7.63(m,1H), 7.16~7.12(m,1H), 6.61~6.57(m,1H), 6.21~6.19(m,1H), 5.44~5.32(m,2H), 3.38~3.36 (m, 2H), 3.00~2.91(m,4H), 1.65(s,9H).

### Synthesis of Intermediate int7

The synthetic route and experimental procedure were as follows:

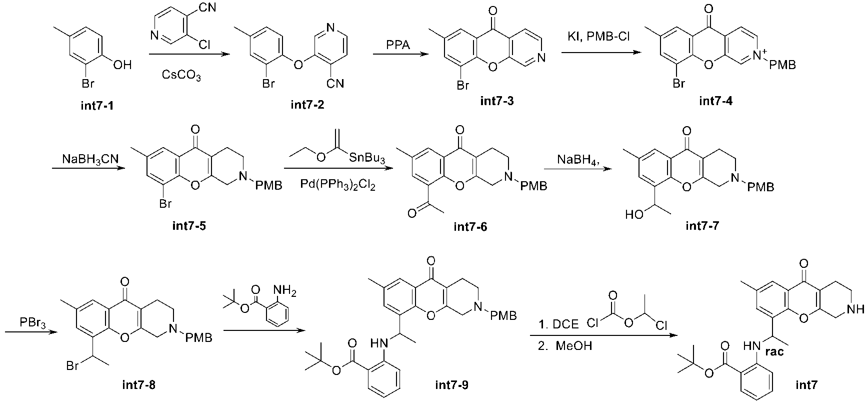

### 1. Synthesis of Compound int7-2

Compound int7-1 (4.48 g, 24 mmol) was dissolved in 60 mL of N,N-dimethylformamide, followed by the addition of 3-chloro-4-cyanopyridine (2.78 g, 20 mmol) and cesium carbonate (13 g, 40 mmol). Upon completion of the addition, the temperature was raised to 110 °C and the reaction was carried out for 3 h until the starting materials were reacted completely. Water was slowly added to quench the reaction, and the mixture was then extracted with dichloromethane. The organic phase was separated, and dried over anhydrous sodium sulfate, and the filtrate was concentrated under reduced pressure, and purified by column chromatography to afford 4.5 g of the product.

¹H NMR (400 MHz, Chloroform-*d*) δ 8.47 (d, *J =* 4.8 Hz, 1H), 8.12(s, 1H), 7.56 ~ 7.52 (m, 2H), 7.23~7.20(m, 1H), 7.11 ~ 7.09(m, 1H), 2.41 (s, 3H).

### 2. Synthesis of Compound int7-3

Compound **int7-2** (4.5 g, 15.5 mmol) was dissolved in 40 g of polyphosphoric acid, the temperature was slowly raised to 220 °C and the mixture was stirred for 3 h. After the starting materials were reacted completely, the reaction solution was cooled to room temperature and dissolved in water. The solution was basified with saturated aqueous sodium bicarbonate solution and then extracted three times with ethyl acetate. The organic phases were combined, dried over anhydrous sodium sulfate, concentrated under reduced pressure, and purified by trituration to afford 3.5 g of the product.

¹H NMR (400 MHz, Chloroform-d) δ 9.17 (s, 1H), 8.71 (d, *J* = 5.1 Hz, 1H), 8.12 (s, 1H), 8.03 (d, *J =* 5.2 Hz, 1H), 7.99 (s, 1H).

### 3. Synthesis of Compound int7-4

Int7-3 (2.5 g, 8.6 mmol), potassium iodide (3.15 g, 19 mmol) and benzyl chloride (2.7 g, 17.2 mmol) were added to 40 mL of acetonitrile, and the reaction solution was slowly heated to 80 °C and stirred for 2 h. After the starting materials were reacted completely, the reaction mixture was concentrated under reduced pressure to afford 8.2 g of a crude product, which was directly used in the next step.

### 4. Synthesis of Compound int7-5

Compound int7-4 (8.2 g, 20 mmol) was dissolved in 200 mL of anhydrous ethanol, followed by the addition of sodium cyanoborohydride (10.8 g, 160 mmol) and acetic acid (1.2 g, 20 mmol). The mixture was maintained at room temperature and stirred for 30 min. After the starting materials were reacted completely, the reaction solution was diluted with water and then extracted three times with dichloromethane. The organic phases were combined, dried over anhydrous sodium sulfate, concentrated under reduced pressure, and purified to afford 3.8 g of the product.

### 5. Synthesis of Compound int7-6

Compound int7-5 (3 g, 7.24 mmol), tributyl(1-ethoxyvinyl)tin (3.14 g, 8.7 mmol) and Pd(PPh₃)₂Cl₂ (510 mg, 0.724 mmol) were dissolved in 50 mL of dry 1,4-dioxane, and the mixture was stirred at 80 °C for 12 h. Potassium fluoride solution was then added at room temperature, and the reaction mixture was stirred for 0.5 h, and filtered. The mother liquor was extracted three times with ethyl acetate, and the organic phase was separated and dried by rotary evaporation. The residue was dissolved in dichloromethane. 2 mL of concentrated hydrochloric acid was then added, and the mixture was stirred at 25 °C for 10 min. After the reaction was completed, the mixture was subjected to column chromatography to obtain the product (2.5 g).

¹H NMR (400 MHz, Chloroform-*d*) δ 8.20 (s, 1H), 7.91(s, 1H), 7.32 (d, *J =* 8.4 Hz, 2H), 6.93(d, *J =* 8.4 Hz, 2H), 3.84(s, 3H), 3.71(s, 2H), 3.54(s, 2H), 2.79~2.77(m, 2H), 2.71~2.68(m,5H), 2.48 (s, 3H).

### 6. Synthesis of Compound int7-7

Compound int7-6 (2.5 g, 6.63 mmol) was dissolved in a mixed solvent of 30 mL dichloromethane and 30 mL methanol. Under nitrogen protection, sodium borohydride (251 mg, 6.63 mmol) was slowly added, and the reaction solution was stirred at room temperature for 10 min. After the starting materials were reacted completely, the reaction solution was diluted with water and then extracted three times with dichloromethane. The organic phases were combined, dried over anhydrous sodium sulfate, concentrated under reduced pressure, and purified to afford 1.5 g of the product.

¹H NMR (400 MHz, Chloroform-d) δ 7.90 (s, 1H), 7.61(s, 1H), 7.34 (d, *J =* 8.4 Hz, 2H), 6.94(d, *J* = 8.4 Hz, 2H), 5.35~5.32(m,1H), 3.85(s, 3H), 3.71~3.68(m,2H), 3.43(s, 2H), 2.78~2.68(m,4H), 2.44(s,3H), 1.27 (d, *J =* 6.4 Hz, 2H).

### 7. Synthesis of Compound int7-8

Compound int7-7 (1.5 g, 3.95 mmol) was dissolved in 50 mL of anhydrous dichloromethane. Under nitrogen protection, phosphorus tribromide (3.21 g, 11.9 mmol) was slowly added, and the mixture was stirred at room temperature for 12 h. After the starting materials were reacted completely, the reaction mixture was then concentrated under reduced pressure to afford 3.5 g of the product.

### 8. Synthesis of Compound int7-9

Compound int7-8 (3.5 g, 3.95 mmol) was dissolved in 100 mL of anhydrous N,N-dimethylformamide. At room temperature, *tert*-butyl 2-aminobenzoate (4.57 g, 23.7 mmol) was slowly added, and the mixture was heated slowly to 60 °C and stirred for 12 h. After the starting materials were reacted completely, the reaction solution was diluted with water and then extracted three times with ethyl acetate. The organic phases were combined, dried over anhydrous sodium sulfate, concentrated under reduced pressure, and the purified to afford 1.1 g of the product.

### 9. Synthesis of Compound int7

Compound int7-9 (90 mg, 0.165 mmol) was dissolved in 5 mL of 1,2-dichloroethane. At room temperature, 1-chloroethyl chloroformate (47.1 mg, 0.33 mmol) was slowly added, and the mixture was heated slowly to 83 °C and stirred for 3 h. After the starting materials were reacted completely, the reaction mixture was concentrated to dryness. Then methanol was added, and the mixture was heated to 65 °C, stirred and reacted for 1 h. The reaction mixture was then concentrated, and triturated with methyl *tert*-butyl ether to afford 51 mg of the product.

### Synthesis of Intermediate int8

The synthetic route and experimental procedure were as follows:

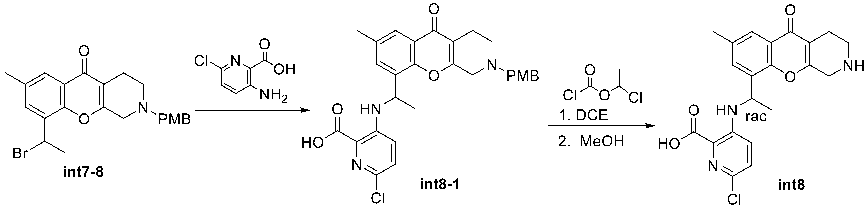

### 1. Synthesis of int8-1

Int7-8 (260 mg, 0.59 mmol) was dissolved in N,N-dimethylformamide (5 mL), followed by the addition of 3-amino-6-chloropyridin-2-carboxylic acid (507.0 mg, 2.95 mmol). The reaction was carried out at 80 °C for 16 h. After the reaction was completed, the reaction solution was diluted with ethyl acetate, basified with aqueous sodium bicarbonate solution, and washed three times with saturated ammonium chloride solution. The organic phase was dried, concentrated, and purified by column chromatography to afford 90 mg of the product.

### 2. Synthesis of int8

Int8-1 (90 mg, 0.17 mmol) was dissolved in 1,2-dichloroethane (4 mL), and 1-chloroethyl chloroformate (72.5 mg, 0.51 mmol) was added. The reaction was carried out at 83 °C for 3 h. After the reaction was completed, the reaction solution was dried by rotary evaporation. Then methanol was added, and the reaction was carried out at 65 °C for 1 h. The obtained reaction solution was concentrated to afford 70 mg of the product.

### Synthesis of Intermediate int9

The synthetic route and experimental procedure were as follows:

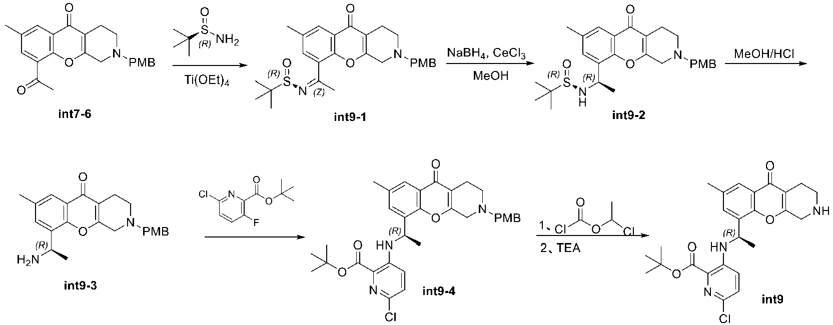

### 1. Synthesis of int9-1

Compound int7-6 (10 g) was dissolved in 210 mL of tetrahydrofuran, followed by the addition of R-tert-butyl sulfinamide (6.0 g) and tetraethyl titanate (22.3 g). The mixture was stirred at 70 °C overnight. After the reaction was completed, water was added and the mixture was filtered. The filter cake was washed with ethyl acetate, and the aqueous phase was extracted with ethyl acetate. The organic phases were combined, concentrated and subjected to column chromatography to afford 11.2 g of the product.

### 2. Synthesis of int9-2

Compound int9-1 (11.2 g) was dissolved in 400 mL of methanol, and cerium chloride heptahydrate (3.9 g) was then added. Upon completion of the addition, sodium borohydride (1.3 g) was added to the reaction system in batches, and the reaction was carried out at room temperature for 10 min. After the reaction was completed, the mixture was concentrated and subjected to column chromatography to afford 9.1 g of the product.

### 3. Synthesis of int9-3

Compound int9-2 (9.1 g) was dissolved in 120 mL of methanol/hydrogen chloride solution, and the mixture was reacted at room temperature for 10 min. After the reaction was completed, the mixture was directly dried by rotary evaporation. The residue was basified with saturated aqueous sodium bicarbonate solution and extracted with ethyl acetate, dried, concentrated and subjected to column chromatography to afford 7.2 g of the product.

### 4. Synthesis of int9-4

Compound int9-3 (7.0 g) was dissolved in 100 mL of dimethyl sulfoxide, and N,N-diisopropylethylamine (9.3 g) was added. Upon completion of the addition, *tert*-butyl 6-chloro-3-fluoropyridin-2-carboxylate (20.1 g) was added to the reaction system, and the reaction was carried out at 70 °C for 4 h under nitrogen protection. After the reaction was completed, water was added, and the mixture was extracted with ethyl acetate, dried, concentrated and subjected to column chromatography to afford 4.3 g of the product.

### 5. Synthesis of int9

Compound int9-4 (4.3 g) was dissolved in 300 mL of 1,2-dichloroethane, and 1-chloroethyl chloroformate (2.33 g) was added. Upon completion of the addition, the reaction mixture reacted at 85 °C for 0.5 h. After the reaction was completed, the mixture was directly dried by rotary evaporation. After that, the residue was dissolved with 300 mL of methanol, triethylamine (3.5 g) was immediately added. Upon completion of the addition, the reaction was carried out at 65 °C for 2 h. After the reaction was completed, the mixture was concentrated and subjected to column chromatography to afford 3.1 g of the product.

### Synthesis of Intermediate int10

The synthetic route and experimental procedure were as follows:

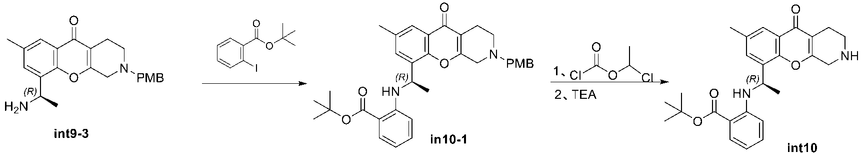

### 1. Synthesis of int10-1

Compound int9-3 (3.78 g) was dissolved in 150 mL of dry toluene, followed by the sequential addition of *tert*-butyl 2-iodobenzoate (3.34 g), cesium carbonate (8.14 g), palladium acetate (216.5 mg) and Xantphos (964 mg). Upon completion of the addition, the reaction was carried out overnight at 60 °C under nitrogen protection. The reaction mixture was filtered, and the filter cake was washed with ethyl acetate, mixed with silica gel and subjected to column chromatography, and the resulting mixture was dried, concentrated and subjected to column chromatography to afford 2.84 g of the product.

### 2. Synthesis of int10

Compound **int10-1** (2.84 g) was dissolved in 150 mL of 1,2-dichloroethane, and 1-chloroethyl chloroformate (1.46 g) was added. Upon completion of the addition, the reaction was carried out at 85 °C for 1 h. After the reaction was completed, the mixture was directly dried by rotary evaporation. After that, the residue was dissolved in 150 mL of methanol, triethylamine (2.43 g) was immediately added. Upon completion of the addition, the reaction was carried out at 65 °C for 3 h. After the reaction was completed, the mixture was dried by rotary evaporation, concentrated and subjected to column chromatography to afford 1.78 g of the product.

### Synthesis of Intermediate int11

The synthetic route and experimental procedure were as follows:

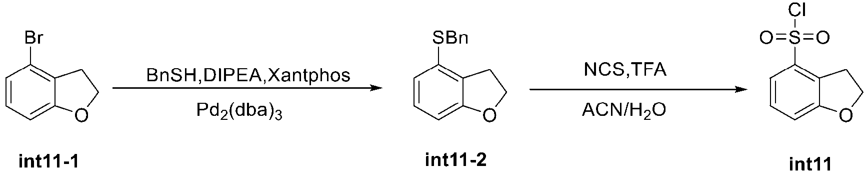

### 1. Synthesis of int11-2

Int11-1 (800 mg) was dissolved in 1,4-dioxane (25 mL). Benzyl mercaptan (500 mg), N,N-diisopropylethylamine (2.1 mL), Xantphos (465 mg) and Pd₂(dba)₃ (368 mg) were added sequentially at room temperature. The reaction solution was then stirred and reacted at 100 °C for 18 h under nitrogen atmosphere. After the starting materials were reacted completely, a small amount of ethyl acetate was added to the reaction solution, which was then directly concentrated under reduced pressure and purified by column chromatography to afford 603 mg of the product.

### 2. Synthesis of int11

Int11-2 (603 mg) was dissolved in a mixed solvent of acetonitrile (9 mL) and water (1 mL). Trifluoroacetic acid (7.79 g) and N-chlorosuccinimide (667 mg) were added under ice-water bath. The mixture was stirred and reacted at room temperature for 18 h. N-chlorosuccinimide (667 mg) was supplemented the next day, then the mixture was stirred and reacted at room temperature for an additional 4 h until the starting materials were reacted completely. The reaction solution was concentrated under reduced pressure, and saturated aqueous sodium bicarbonate solution was added thereto so as to adjust the pH to alkaline. The aqueous phase was extracted with dichloromethane. The organic phase was dried over anhydrous sodium sulfate and filtered. The filtrate was concentrated under reduced pressure to afford 588 mg of a crude product, which was directly used in the next reaction step.

### Synthesis of Intermediate int12

The synthetic route and experimental procedure were as follows:

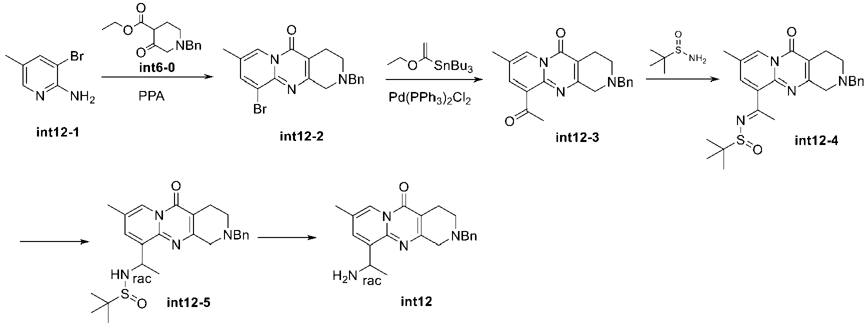

### 1. Synthesis of int12-2

Int6-0 (10 g, 53.47 mmol), int12-1 (10 g, 53.47 mmol), polyphosphoric acid (25 mL) and 1,2-dichloroethane (50 mL) were added to a 250 mL single-necked flask, and the mixture was stirred at 85 °C for 16 h, then cooled to room temperature. Saturated sodium carbonate solution was added to adjust the pH to 8, and the mixture was extracted with ethyl acetate (2×100 mL). The combined organic phases were washed with saturated sodium chloride solution, dried over anhydrous sodium sulfate, filtered, concentrated and purified by column chromatography to afford 3.5 g of the product.

### 2. Synthesis of int12-3

Compound int12-2 (7 g, 18.3 mmol), tributyl(1-ethoxyvinyl)tin (7.9 g, 21.9 mmol) and Pd(PPh₃)₂Cl₂ (1.26 g, 1.83 mmol) were dissolved in dry 1,4-dioxane (70 mL), and the mixture was stirred at 80 °C for 12 h. Potassium fluoride solution (70 mL) was then added at room temperature, and the mixture was stirred for 0.5 h and filtered. The mother liquor was extracted with ethyl acetate (3×50 mL), and the organic phase was separated and dried by rotary evaporation. The residue was dissolved in ethyl acetate (70 mL), followed by the addition of 6 M hydrochloric acid (130 mL). The mixture was stirred at 25 °C for 10 min, with a large amount of solid precipitated out. The solid was filtered and the filter cake was washed with ethyl acetate (2×100 mL). Saturated sodium bicarbonate solution was added to adjust the pH to neutral, the mixture was extracted with dichloromethane (2×70 mL), dried, concentrated and purified by column chromatography to afford 3.9 g of the product.

### 3. Synthesis of int12-4

Compound int12-3 (3.8 g, 10.95 mmol) was dissolved in tetrahydrofuran (38 mL). Under nitrogen protection, R-*tert*-butyl sulfinamide (2.65 g, 21.9 mmol) and tetraethyl titanate (9.98 g, 43.8 mmol) were added, and the reaction was carried out at 70 °C for 16 h under nitrogen protection. After the starting materials were reacted completely, water (38 mL) was added to the reaction solution, which was then extracted with ethyl acetate (4×60 mL). The combined organic phases were washed with saturated sodium chloride solution (60 mL), dried over anhydrous sodium sulfate and concentrated under reduced pressure to afford 4.64 g of a crude product.

### 4. Synthesis of int12-5

Compound int12-4 (4.64 g, 10.3 mmol) was dissolved in methanol (70 mL). Cerium chloride heptahydrate (1.92 g, 5.16 mmol) was added under nitrogen protection. The mixture was heated to 50 °C and stirred for 30 min under nitrogen protection, then cooled to 15 °C. Sodium borohydride (0.58 g, 15.5 mmol) was added in batches, and after the completion of addition, the mixture was incubated at 15 °C and reacted for 30 min. After the starting materials were reacted completely, dichloromethane (140 mL) and water (70 mL) were added to the reaction solution. After the mixture was separated into layers, the aqueous phase was extracted with dichloromethane (60 mL), and the organic phases were combined, dried over anhydrous sodium sulfate, concentrated under reduced pressure and purified by column chromatography to afford 3 g of the product.

### 5. Synthesis of int12

Compound int12-5 (3 g) was dissolved in ethanol (15 mL). Under nitrogen protection, 2 M HCl (30 mL) was added, and the mixture was reacted at 30 °C for 30 min. After the starting materials were reacted completely, the reaction solution was concentrated. Dichloromethane (500 mL) was added, and the resulting mixture was adjusted to neutral with saturated sodium bicarbonate solution. The aqueous phase was extracted with dichloromethane (20 mL) once, and the organic phases were combined, dried over anhydrous sodium sulfate, concentrated under reduced pressure and purified by column chromatography to afford 2.1 g of the product.

### Synthesis of Intermediate int13

The synthetic route and experimental procedure were as follows:

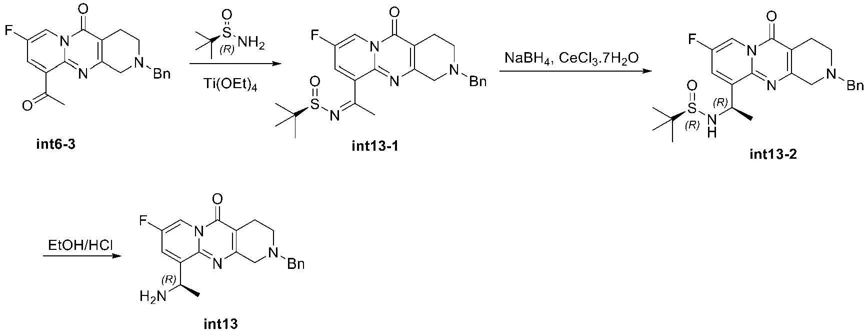

### 1. Synthesis of int13-1

Compound int6-3 (4.2 g, 11.93 mmol) was dissolved in 50 mL of tetrahydrofuran, followed by the addition of R-*tert*-butyl sulfinamide (2.88 g, 23.80 mmol) and tetraethyl titanate (10.8 g, 47.37 mmol). The reaction was carried out at 70 °C for 4 h. After the starting materials were reacted completely, 100 mL of water was added to the reaction solution. Then the mixture was extracted with ethyl acetate (100 mL × 4). The organic phases were combined, washed with saturated sodium chloride solution (300 mL), dried over anhydrous sodium sulfate, concentrated under reduced pressure, and purified by column chromatography to afford the product (4 g).

### 2. Synthesis of int13-2

Compound int13-1 (4 g, 8.79 mmol) was dissolved in 60 mL of methanol. Then cerium chloride heptahydrate (1.64 g, 4.4 mmol) was added under nitrogen protection. The mixture was heated to 50 °C and stirred for 30 min under nitrogen protection, then cooled to 15 °C, and sodium borohydride (0.50 g, 13.2 mmol) was added to reaction system in batches. After the addition, the reaction mixture was maintained at 15 °C for 30 min. After the starting materials were reacted completely, the mixture was concentrated under reduced pressure and purified by column chromatography to afford the product (2.8 g).

### 3. Synthesis of int13

Compound int13-2 (2.8 g, 6.13 mmol) was added to 2 M hydrochloric acid (30 mL), and the reaction was carried out at room temperature for 10 min. After the starting materials were reacted completely, the mixture was concentrated, and 50 mL of ethyl acetate was added to the reaction solution. The pH of the mixture was adjusted to alkaline with saturated sodium bicarbonate solution, and the layers were separated. The aqueous phase was extracted with ethyl acetate (20 mL × 1), and the organic phases were combined, dried over anhydrous sodium sulfate and concentrated under reduced pressure to afford the product (2 g).

### Synthesis of Intermediate int14

The synthetic route and experimental procedure were as follows:

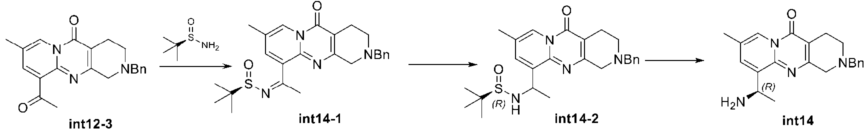

### 1. Synthesis of int14-1

Compound int12-3 (3.47 g, 10 mmol) was dissolved in 50 mL of tetrahydrofuran, followed by the addition of R-*tert*-butyl sulfinamide (2.42 g, 20 mmol) and tetraethyl titanate (9.12 g, 40 mmol). The reaction was carried out at 70 ° C for 4 h. After the starting materials were reacted completely, 100 mL of water was added to the reaction solution. Then the mixture was extracted with ethyl acetate (100 mL × 4). The organic phases were combined, washed with saturated sodium chloride solution, dried over anhydrous sodium sulfate, concentrated under reduced pressure and purified by column chromatography to afford the product (4.1 g).

### 2. Synthesis of int14-2

Compound int14-1 (4.1 g, 9.1 mmol) was dissolved in 80 mL of methanol. Cerium chloride heptahydrate (1.65 g, 4.4 mmol) was added under nitrogen protection. The mixture was heated to 50 °C and stirred for 30 min under nitrogen protection, then cooled to 15 °C, and sodium borohydride (0.55 g) was added to reaction system in batches. After the addition, the reaction mixture was maintained at 15 °C for 30 min. After the starting materials were reacted completely, the mixture was concentrated under reduced pressure and purified by column chromatography to afford the product (2.73 g).

### 3. Synthesis of int14

Compound int14-2 (2.73 g, 6.0 mmol) was added to 2 M hydrochloric acid (30 mL), and the reaction was carried out at room temperature for 10 min. After the starting materials were reacted completely, the mixture was concentrated, and 50 mL of ethyl acetate was added to the reaction solution. The pH of the mixture was adjusted to alkaline with saturated sodium bicarbonate solution, and the layers were separated. The aqueous phase was extracted with ethyl acetate (20 mL × 1), and the organic phases were combined, dried over anhydrous sodium sulfate and concentrated under reduced pressure to afford the product (2.08 g).

### Synthesis of Intermediate int15

The synthetic route and experimental procedure were as follows:

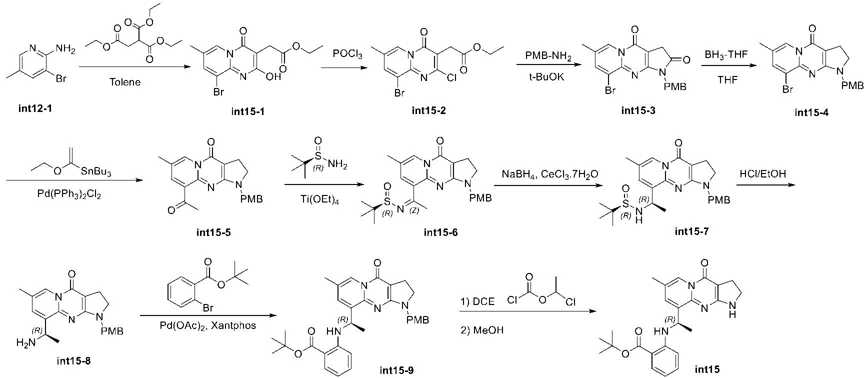

### 1. Synthesis of int15-1

Int12-1 (5.0 g, 26.88 mmol) and triethyl 1,1,2-ethanetricarboxylate (6.61 g, 26.88 mmol) were dissolved in toluene (100 mL), and the mixture was heated to 110 °C and stirred for 24 h until the starting materials were reacted completely. The reaction solution was cooled to room temperature, and the solid was washed with cold ethanol to afford the crude product (3.6 g), Ms [M+H]⁺: 341.0.

### 2. Synthesis of int15-2

Int15-1 (3.5 g, 10.29 mmol) was dissolved in phosphorus oxychloride (50 mL). The mixture was heated to 120 °C and reacted overnight. The reaction solution was slowly poured into water to quench the reaction, then extracted with ethyl acetate, dried over anhydrous sodium sulfate, concentrated under reduced pressure, and purified by column chromatography to afford the product (1.81 g), Ms [M+H]⁺: 359.0.

### 3. Synthesis of int15-3

Int15-2 (1.7 g, 4.74 mmol) was dissolved in tetrahydrofuran (35 mL), followed by the addition of 4-methoxybenzylamine (0.58 g, 5.69 mmol). The mixture was cooled to 0 °C, and potassium *tert-*butoxide (0.80 g, 7.12 mmol) was added in batches. After the completion of addition, the mixture was warmed to room temperature and stirred for 3 h until the starting materials were reacted completely. Water was added to the reaction system at low temperature, and the reaction solution was extracted with ethyl acetate, dried over anhydrous sodium sulfate, concentrated under reduced pressure, and purified by column chromatography to afford the product (1.3 g), Ms [M+H]⁺: 414.0.

### 4. Synthesis of int15-4

Compound int15-3 (1.3 g, 3.14 mmol) was dissolved in tetrahydrofuran (25 mL), and boranetetrahydrofuran complex (3.77 mL, 3.77 mmol) was added dropwise. The mixture was heated to 60 °C and reacted for 1 h until the starting materials were reacted completely. The reaction solution was diluted with ethyl acetate, and the organic phase was washed with saturated brine, dried over anhydrous sodium sulfate, concentrated under reduced pressure, and purified by column chromatography to afford the product (0.74 g), Ms [M+H]⁺: 400.1.

### 5. Synthesis of int15-5

Compound int15-4 (0.74 g, 1.85 mmol), tributyl(1-ethoxyvinyl)tin (1.08 g, 2.23 mmol) and Pd(PPh₃)₂Cl₂ (0.14 g, 0.19 mmol) were dissolved in dry dioxane (70 mL), and the mixture was heated to 80 °C and stirred for 12 h. Then at room temperature, 30 mL of potassium fluoride solution was added and the mixture was stirred for 0.5 h, followed by filtration. The mother liquor was extracted with ethyl acetate, and the organic phase was dried over anhydrous sodium sulfate and concentrated under reduced pressure. The residue was dissolved in ethyl acetate (50 mL), 6 M hydrochloric acid (80 mL) was added, and the mixture was stirred at 25 °C for 10 min, then filtered. The filter cake was washed with ethyl acetate, and the filtrate was adjusted to neutral pH with saturated sodium bicarbonate solution, then extracted with dichloromethane, dried over anhydrous sodium sulfate, concentrated, and purified by column chromatography to afford the product (0.55 g), Ms [M+H]⁺: 364.2.

### 6. Synthesis of int15-6

Compound int15-5 (0.55 g, 1.51 mmol) was dissolved in tetrahydrofuran (20 mL). R-tert-butyl sulfinamide (0.37 g, 3.02 mmol) and tetraethyl titanate (1.38 g, 6.04 mmol) were added under nitrogen protection. The mixture was heated to 70 °C and reacted for 16 h under nitrogen protection. After the starting materials were reacted completely, 20 mL of water was added to the reaction solution, then the mixture was extracted with ethyl acetate. The organic phases were combined, washed with saturated aqueous sodium chloride solution, dried over anhydrous sodium sulfate and concentrated under reduced pressure to afford the crude product (0.47 g), Ms [M+H]⁺: 467.2.

### 7. Synthesis of int15-7

Compound int15-64 (0.47 g, 1.18 mmol) was dissolved in methanol (20 mL). Cerium chloride heptahydrate (0.192 g, 0.59 mmol) was added under nitrogen protection. The mixture was heated to 50 °C and stirred for 30 min under nitrogen protection, then cooled to 15 °C, and sodium borohydride (0.06 g, 1.77 mmol) was added in batches. After the completion of addition, the mixture was maintained at 15 °C and reacted for 30 min. After the starting materials were reacted completely, dichloromethane and water were added to the reaction solution, and the mixture was extracted and separated. The aqueous phase was extracted with dichloromethane, and the organic phases were combined, dried over anhydrous sodium sulfate, concentrated under reduced pressure and purified by column chromatography to afford the product (0.28 g), Ms [M+H]⁺: 469.2.

### 8. Synthesis of int15-8

Compound int15-7 (0.28 g, 0.60 mmol) was dissolved in dichloromethane (5 mL). 2 M hydrogen chloride/ethanol solution (2 mL) was added under nitrogen protection. The reaction was carried out at room temperature for 30 min. After the starting materials were reacted completely, the reaction solution was concentrated. Dichloromethane was added to the residue, and the pH was adjusted to neutral with saturated sodium bicarbonate solution. The aqueous phase was extracted with dichloromethane, and the organic phases were combined, dried over anhydrous sodium sulfate and concentrated under reduced pressure to afford the product (0.2 g), Ms [M+H]⁺: 365.2.

### 9. Synthesis of int15-9

Compound int15-8 (0.2 g, 0.55 mmol), *tert*-butyl 2-bromobenzoate (0.17 g, 0.66 mmol), palladium acetate (12 mg, 0.055 mmol), 4,5-bis(diphenylphosphino)-9,9-dimethylxanthene (63 mg, 0.11 mmol) and cesium carbonate (0.48 g, 1.48 mmol) were dissolved in dry toluene (10 mL), and the mixture was heated to 100 °C and stirred for 2 h. After the reaction was completed, the mixture was filtered, and the filter cake was washed with ethyl acetate. The organic phase was dried by rotary evaporation and purified by column chromatography to afford the product (0.26 g), Ms [M+H]⁺: 541.3.

### 10. Synthesis of int15

Compound int15-9 (0.26 g, 0.48 mmol) was dissolved in 1,2-dichloroethane (10 mL), followed by the addition of 1-chloroethyl chloroformate (0.14 g, 0.96 mmol). After the completion of addition, the mixture was heated to 80 °C and reacted for 1 h. After the reaction was completed, the mixture was concentrated, and 20 mL of methanol was added to dissolve the residue, then triethylamine (0.19 g, 1.92 mmol) was added. After the completion of addition, the mixture was heated to 65 °C and reacted for 3 h. After the reaction was completed, the mixture was concentrated and purified by column chromatography to afford the product (0.15 g), Ms [M+H]⁺: 421.2.

### Synthesis of Intermediate int16

The synthetic route and experimental procedure were as follows:

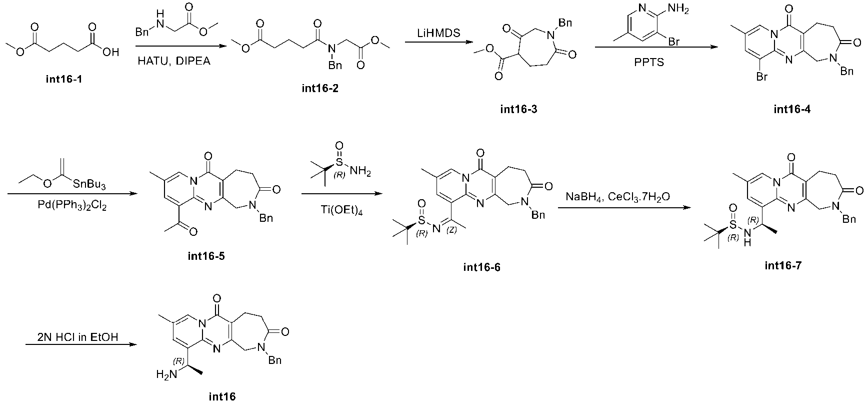

### 1. Synthesis of int16-2

Int16-1 (5.0 g, 34.2 mmol), methyl 2-(benzylamino)acetate (6.74 g, 37.7 mmol) and N,N-diisopropylethylamine (17.9 mL, 102.6 mmol) were dissolved in 60 mL of anhydrous N,N-dimethylformamide. Under an ice bath, O-(7-azabenzotriazol-1-yl)-N,N,N',N'- tetramethyluronium hexafluorophosphate (19.5 g, 51.3 mmol) was added in batches, and the mixture was warmed to room temperature and reacted for 30 minutes. After the starting materials were reacted completely, water was added under an ice bath, and the aqueous phase was extracted with ethyl acetate. The organic phases were combined, washed with saturated sodium chloride solution for several times, dried over anhydrous sodium sulfate, filtered, concentrated and purified by column chromatography to afford the product (11.5 g), [M+H]⁺: 308.

### 2. Synthesis of int16-3

Int16-2 (5.0 g, 16.29 mmol) was dissolved in 60 mL of anhydrous tetrahydrofuran. Under a nitrogen atmosphere and an ice bath, lithium bis(trimethylsilyl)amide (33 mL, 33 mmol) was added dropwise slowly, and the mixture was allowed to warm to room temperature naturally and reacted for 4 hours. After the starting materials were reacted completely, saturated aqueous ammonium chloride solution was added under an ice bath, and the aqueous phase was extracted with ethyl acetate. The organic phase was dried over anhydrous sodium sulfate, filtered, concentrated and purified by column chromatography to afford the product (2.18 g), [M+H]⁺: 276.

### 3. Synthesis of int16-4

Int16-3 (1.0 g, 3.64 mmol), 2-amino-3-bromo-5-methylpyridine (680 mg, 3.64 mmol) and pyridinium 4-toluenesulfonate (1.10 g, 4.37 mmol) were added to a 50 mL single-neck glass sealed vessel, the mixture was heated to 120 °C and stirred for 16 hours, then the reaction mixture was cooled to room temperature. The pH was adjusted to alkaline with saturated sodium bicarbonate solution, and the mixture was extracted with ethyl acetate. The combined organic phases were washed with saturated sodium chloride solution, dried over anhydrous sodium sulfate, filtered, concentrated and purified by column chromatography to afford the product (878 mg), [M+H]⁺: 412/414.

### 4. Synthesis of int16-5

Compound int16-4 (878 mg, 2.14 mmol), tributyl(1-ethoxyvinyl)tin(1.16 g, 3.20 mmol) and Pd(PPh₃)₂Cl₂ (150 mg, 0.21 mmol) were dissolved in 15 mL of dry dioxane, the mixture was heated to 85 °C and stirred for 2 hours. Then at room temperature, 15 mL of potassium fluoride solution was added and the mixture was stirred for 0.5 hours, followed by filtration. The reaction solution was extracted with ethyl acetate, and the organic phase was separated and dried by rotary evaporation. The residue was redissolved in 5 mL of ethyl acetate. 1 mL of 6 M hydrochloric acid was added, and the mixture was stirred at 25 °C for 10 minutes. After the reaction was completed, the aqueous phase was extracted with ethyl acetate, then the resulting aqueous phase was adjusted to neutral with saturated sodium bicarbonate solution and extracted with ethyl acetate again; the organic phases were dried, then concentrated and purified by column chromatography to afford the product (500 mg), [M+H]⁺: 376.

### 5. Synthesis of int16-6

Compound int16-5 (500 mg, 1.33 mmol) was dissolved in 20 mL of tetrahydrofuran, followed by the addition of R-*tert*-butyl sulfinamide (323 mg, 2.67 mmol) and tetraethyl titanate (1.21 g, 5.32 mmol). The reaction solution was heated to 70 °C and reacted for 18 hours. After the starting materials were reacted completely, 5 mL of water was added to the reaction solution, followed by filtration. The filter cake was washed with ethyl acetate for several times, and the combined organic phases were washed with saturated sodium chloride solution, dried over anhydrous sodium sulfate, concentrated under reduced pressure and purified by column chromatography to afford the product (460 mg), [M+H]⁺: 479.

### 6. Synthesis of int16-7

Compound int16-6 (460 mg, 0.96 mmol) was dissolved in 10 mL of methanol. Cerium chloride heptahydrate (179 mg, 0.48 mmol) was added under nitrogen protection. Then sodium borohydride (54 mg, 1.44 mmol) was added in batches under an ice bath and nitrogen protection. The reaction was carried out for 30 minutes. After the starting materials were reacted completely, the mixture was directly concentrated under reduced pressure and purified by column chromatography to afford the product (286 mg), [M+H]⁺: 481.

### 7. Synthesis of int16

Compound int16-7 (286 mg, 0.59 mmol) was dissolved in dichloromethane, followed by the addition of 2 M hydrogen chloride/ethanol solution (2 mL). The reaction was carried out at room temperature for 10 minutes. After the starting materials were reacted completely, ethanol was removed by rotary evaporation. The reaction solution was redissolved in ethyl acetate, adjusted to alkaline with saturated sodium bicarbonate solution, and the aqueous phase was separated. The organic phases were combined, dried over anhydrous sodium sulfate and concentrated under reduced pressure to afford the product (220 mg), [M+H]⁺: 377.

### Synthesis of Intermediate int17

The synthetic route and experimental procedure were as follows:

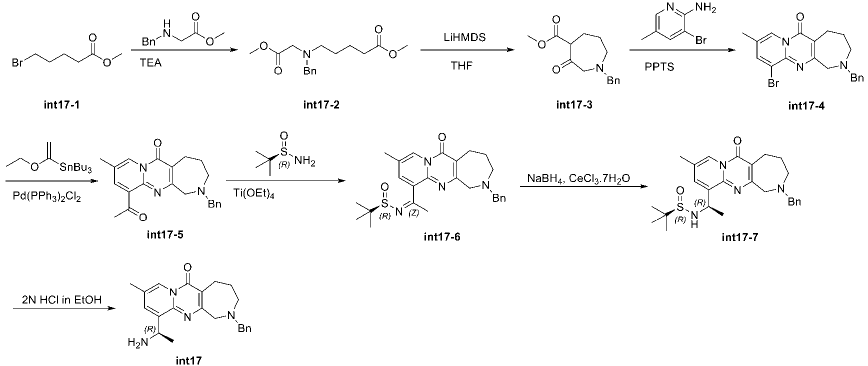

### 1. Synthesis of int17-2

Int17-1 (16.34 g, 83.80 mmol), *tert*-butyl benzylglycinate (10 g, 55.87 mmol) and triethylamine (8.46 g, 83.80 mmol) were dissolved in acetonitrile (200 mL) and the reaction was carried out at 80 °C overnight. The reaction solution was cooled to room temperature, concentrated and purified by column chromatography to afford the product (11.4 g). [M+H]⁺: 294.

### 2. Synthesis of int17-3

Int17-2 (11.4 g, 38.91 mmol) was dissolved in ultra-dry tetrahydrofuran (300 mL), cooled to - 30 °C, and lithium bis(trimethylsilyl)amide (97.28 mL, 97.28 mmol) was added dropwise slowly. The reaction solution was warmed up to 5 °C slowly and stirred until the starting materials were consumed completely. The reaction was quenched by the addition of aqueous ammonium chloride solution in an ice bath. The aqueous phase was extracted with ethyl acetate, and the organic phase was dried over anhydrous sodium sulfate, concentrated and purified by column chromatography to afford the product (950 mg). [M+H]⁺: 262.

¹H NMR (400 MHz, DMSO) *δ* ppm 7.39 - 7.30 (m, 4H), 7.30 - 7.24 (m, 1H), 3.90 (d, J=6.0 Hz, 1H), 3.67 (s, 2H), 3.63 (s, 3H), 3.31 - 3.24 (m, 2H), 2.63 - 2.55 (m, 1H), 2.50 - 2.43 (m, 1H), 1.97 - 1.81 (m, 2H), 1.73 - 1.51 (m, 2H).

### 3. Synthesis of int17-4

Int17-3 (950 mg, 3.64 mmol), 2-amino-3-bromo-5-methylpyridine (680 mg, 3.64 mmol) and pyridinium 4-toluenesulfonate (1.10 g, 4.37 mmol) were stirred at 120 °C for 16 h, and the reaction solution was then cooled to room temperature, adjusted to alkaline pH with saturated sodium bicarbonate solution, and extracted with ethyl acetate. The combined organic phases were washed with saturated sodium chloride solution, dried over anhydrous sodium sulfate, filtered, concentrated and purified by column chromatography to afford the product (380 mg). [M+H]⁺: 398/400.

¹H NMR (400 MHz, DMSO-*d*₆) *δ* ppm 8.27 - 8.23 (m, 1H), 8.01 - 7.96 (m, 1H), 7.43 - 7.36 (m, 2H), 7.33 - 7.26 (m, 3H), 4.52 (s, 2H), 4.41 (s, 2H), 3.21 (t, *J =* 5.6 Hz, 2H), 2.30 (s, 3H), 2.14 (t, *J =* 6.0 Hz, 2H), 1.88 - 1.72 (m, 2H).

### 4. Synthesis of int17-5

Compound int17-4 (380 mg, 0.96 mmol), tributyl(1-ethoxyvinyl)tin (415 mg, 1.15 mmol) and Pd(PPh₃)₂Cl₂ (150 mg, 0.09 mmol) were dissolved in dry 1,4-dioxane (15 mL) and stirred at 90 °C for 16 h. Then at room temperature, 15 mL of potassium fluoride solution was added and stirred for 0.5 h, followed by filtration. The reaction solution was extracted with ethyl acetate, and the organic phase was separated and concentrated. The residue was redissolved in 5 mL of ethyl acetate. 1 mL of 6 M hydrochloric acid was added, and the mixture was stirred at 25 °C for 10 min. After the reaction was completed, the aqueous phase was extracted with ethyl acetate, and then the resulting aqueous phase was adjusted to neutral with saturated sodium bicarbonate solution, extracted with ethyl acetate again, dried, concentrated and purified by column chromatography to afford the product (250 mg). [M+H]⁺: 362.

### 5. Synthesis of int17-6

Compound int17-5 (250 mg, 0.69 mmol) was dissolved in 20 mL of tetrahydrofuran, followed by the addition of R-*tert*-butyl sulfinamide (167 mg, 1.38 mmol) and tetraethyl titanate (629 mg, 2.76 mmol). The reaction solution was heated to 75 °C and reacted for 18 h. After the starting materials were reacted completely, 5 mL of water was added to the reaction solution, followed by filtration. The filter cake was washed with ethyl acetate for several times, and the combined organic phases were washed with saturated sodium chloride solution, dried over anhydrous sodium sulfate, concentrated under reduced pressure and subjected to column chromatography to afford the product (200 mg). [M+H]⁺: 465.

### 6. Synthesis of int17-7

Compound int17-6 (200 mg, 0.43 mmol) was dissolved in 10 mL of methanol. Cerium chloride heptahydrate (82 mg, 0.22 mmol) was added under nitrogen protection. Sodium borohydride (25 mg, 0.65 mmol) was added in batches in an ice bath under nitrogen protection. The reaction was carried out for 30 min. After the starting materials were reacted completely, the mixture was concentrated under reduced pressure and purified by column chromatography to afford the product (120 mg). [M+H]⁺: 467.

### 7. Synthesis of int17

Compound intl7-7 (120 mg, 0.26 mmol) was dissolved in dichloromethane, followed by the addition of 2 M HCl/EtOH (2 mL). The reaction was carried out at room temperature for 10 min. After the starting materials were reacted completely, ethanol was removed by rotary evaporation. The reaction solution was redissolved in ethyl acetate, adjusted to alkaline with saturated sodium bicarbonate solution, and the aqueous phase was separated. The organic phases were combined, dried over anhydrous sodium sulfate, concentrated under reduced pressure and purified by column chromatography to afford the product (80 mg). [M+H]⁺: 363.

### Example 1

Compounds of the present invention

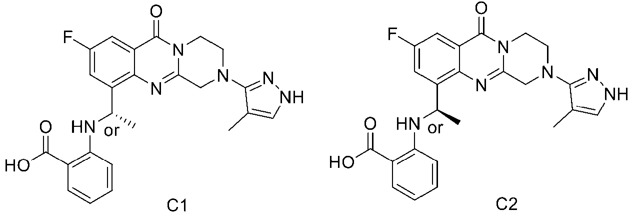

The synthetic route and experimental procedure were as follows:

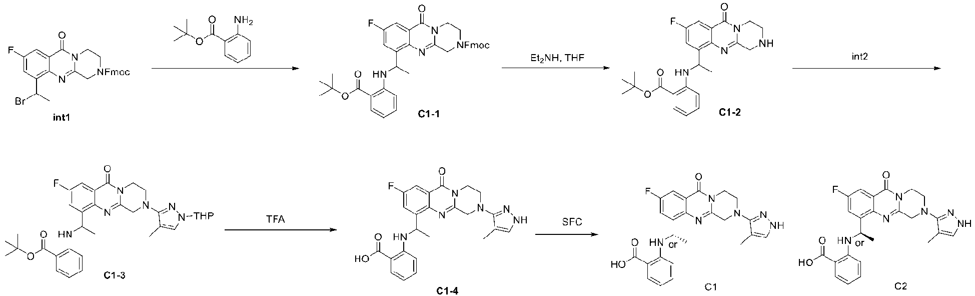

### 1. Synthesis of Compound C1-1

Compound int1 (2.2 g) above, dry N,N-dimethylformamide (60 mL) and *tert*-butyl 2-aminobenzoate (2.9 g, 6 eq) were added to a 100 mL three-necked flask. The reaction was carried out at 80 °C for 8 h under nitrogen protection. After the reaction was completed, saturated ammonium chloride solution was added, and the mixture was extracted with ethyl acetate, dried and concentrated to afford a crude product (1.7 g).

### 2. Synthesis of Compound C1-2

C1-1 (1.7 g, 1.0 eq), diethylamine (5 mL) and dichloromethane (50 mL) were added to a 100 mL three-necked flask, and the mixture was reacted at room temperature for 8 h under nitrogen protection. After the reaction was completed, the mixture was washed with saturated aqueous ammonium chloride solution, extracted with dichloromethane, concentrated, and purified by column chromatography to afford the product (258 mg).

¹H NMR (400 MHz, Chloroform-d) δ 8.30 (d, J = 6.2 Hz, 1H), 7.86 (dd, J = 8.0, 1.7 Hz, 1H), 7.75 (dd, J = 8.2, 3.0 Hz, 1H), 7.48 (dd, J = 9.2, 3.0 Hz, 1H), 7.08 (ddd, J = 8.7, 7.1, 1.7 Hz, 1H), 6.51 (ddd, J *=* 8.1, 7.0, 1.1 Hz, 1H), 6.28 (d, J *=* 8.4 Hz, 1H), 5.50 (t, J *=* 6.5 Hz, 1H), 4.17 (s, 2H), 4.04 (td, J = 5.6, 1.4 Hz, 2H), 3.34 (q, J = 7.6, 6.7 Hz, 2H), 1.63 (s, 9H). 1.60 (d, J = 6.7 Hz, 3H).

### 3. Synthesis of Compound C1-3

C1-2 (100 mg, 1.0 eq), Pd₂(dba)₃ (41.73 mg, 0.2 eq), RuPhos (42.75 mg, 0.4 eq), potassium *tert-*butoxide (77.4 mg, 3 eq), int2 (112 mg, 2 eq) and toluene (5 mL) were added to a 20 mL sealed vial, and the mixture was reacted at 100 °C for 0.5 h. After the reaction was completed, the mixture was filtered, concentrated and purified by column chromatography to afford the product (60 mg).

### 4. Synthesis of Compound C1-4

C1-3 (110 mg, 1.0 eq), dichloromethane (5 mL) and trifluoroacetic acid (2.5 mL) were added to a 50 mL three-necked flask, and the mixture was reacted at 40 °C for 6 h. After the reaction was completed, the mixture was concentrated and purified by preparative thin-layer chromatography to afford the product (72 mg).

### 5. Synthesis of Compounds C1 and C2

Compound C1-4 was subjected to separation by preparative chiral chromatography under the following chiral resolution conditions: Instrument: Agilent 1260 InfinityII, chromatographic column: phenomenex Lux^{®} 5 µm Amylose-1 (250 × 21.2 mm), mobile phase: *n*-hexane : isopropanol = 7 : 3, column temperature: 25 °C. The separated products were obtained as follows:
C1 (Retention time: 8.2 min, 9.54 mg) ¹H NMR (400 MHz, DMSO-d₆) δ 12.71 (s, 1H), 11.86 (s, 1H), 8.60 (s, 1H), 7.88 - 7.78 (m, 1H), 7.68 (dd, J = 8.4, 3.0 Hz, 1H), 7.56 (d, J = 8.6 Hz, 1H), 7.42 (s, 1H), 7.18 (d, J = 8.1 Hz, 1H), 6.51 (t, J = 7.5 Hz, 1H), 6.42 (d, J = 8.6 Hz, 1H), 5.44 (d, J = 7.1 Hz, 1H), 4.43 (s, 2H), 3.94 (q, J = 5.4 Hz, 2H), 3.58 (t, J = 5.7 Hz, 2H), 2.03 (s, 3H), 1.59 (d, J = 6.6 Hz, 3H).
C2 (Retention time: 16.2 min, 2 mg) ¹H NMR (400 MHz, DMSO-d₆) δ 12.73 (s, 1H), 11.88 (s, 1H), 8.61 (s, 1H), 7.86 - 7.77 (m, 1H), 7.66 (dd, J = 8.4, 3.0 Hz, 1H), 7.54 (d, J = 8.6 Hz, 1H), 7.42 (s, 1H), 7.18 (d, J = 8.1 Hz, 1H), 6.51 (t, J = 7.5 Hz, 1H), 6.40 (d, J = 8.6 Hz, 1H), 5.41 (d, J = 7.1 Hz, 1H), 4.43 (s, 2H), 3.94 (q, J = 5.4 Hz, 2H), 3.58 (t, J = 5.7 Hz, 2H), 2.03 (s, 3H), 1.59 (d, J = 6.6 Hz, 3H).

### Example 2

Compounds of the present invention

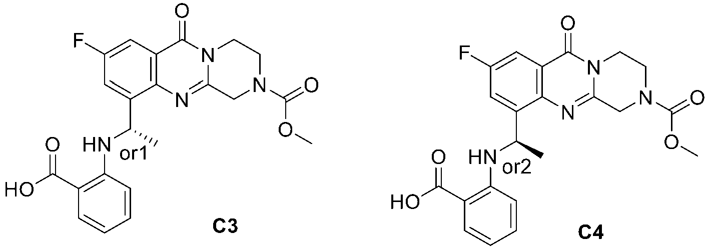

The synthetic route and experimental procedure were as follows:

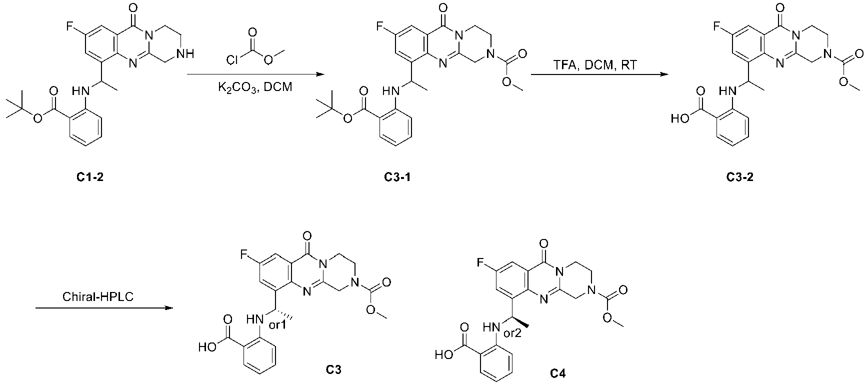

### 1. Synthesis of Compound C3-1

Compound C1-2 (100 mg, 0.23 mmol) was dissolved in 5 mL of dichloromethane. Under an ice-water bath, potassium carbonate (95 mg, 0.69 mmol) and methyl chloroformate (28 mg, 0.3 mmol) were added, and the mixture was stirred and reacted at room temperature for 2 h before the reaction was completed. Saturated brine was added, and the mixture was extracted with ethyl acetate for three times. The organic phase was washed with saturated brine for three times, dried, dried by rotary evaporation and purified by column chromatography to afford the product (46 mg).

### 2. Synthesis of Compound C3-2

Compound C3-1 (46 mg, 0.09 mmol) was dissolved in a mixed solvent composed of dichloromethane (4 mL)/trifluoroacetic acid (2 mL). The mixture was stirred at 23 °C for 12 h under nitrogen protection, and the reaction was completed. The reaction mixture was dried by rotary evaporation to afford the product (35 mg).

### 3. Synthesis of Compounds C3 and C4

Compound C3-2 (35 mg, 0.08 mmol) was separated by the following chiral resolution method:
Instrument: Agilent 1260 InfinityII, chromatographic column: phenomenex Lux^{®} 5 µm Amylose-1 (250 × 21.2 mm), mobile phase: *n*-hexane : isopropanol = 7:3, column temperature: 25 °C. The separated products were obtained as follows:
C3 (6.5 min, 15 mg) [M+H]⁺: 441.2
C4 (8.8 min, 10 mg) [M+H]⁺: 441.2

### Example 3

Compounds of the present invention

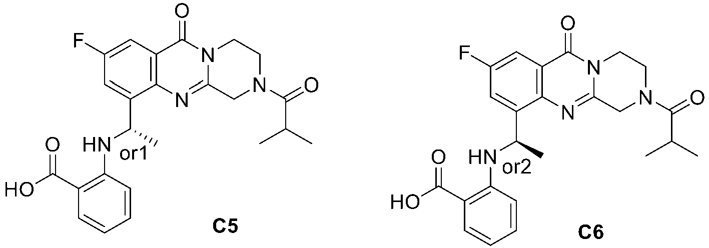

The synthetic route and experimental procedure were as follows:

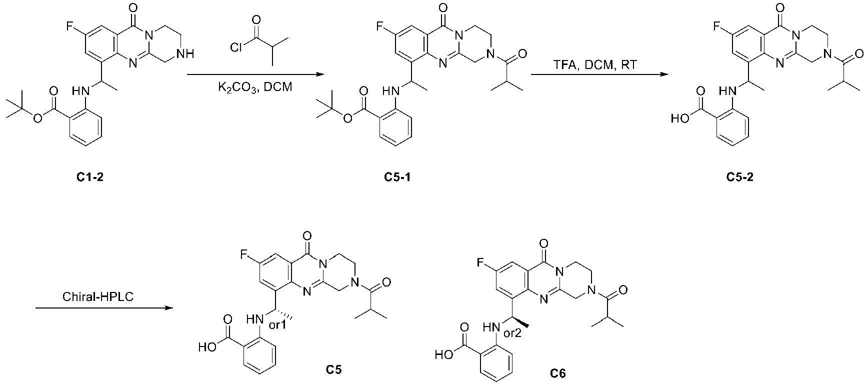

### 1. Synthesis of Compound C5-1

Compound C1-2 (100 mg, 0.23 mmol) was dissolved in 5 mL of dichloromethane. Potassium carbonate (96 mg, 0.69 mmol) was added and the mixture was stirred for 10 min, then isobutyryl chloride (30 mg, 0.27 mmol) was added. The mixture was stirred and reacted at room temperature for 3 h, and the reaction was completed. The mixture was filtered, dried by rotary evaporation and purified by column chromatography to afford the product (40 mg). [M+H]⁺: 509.0.

### 2. Synthesis of Compound C5-2

Compound C5-1 (40 mg, 0.08 mmol) was dissolved in a mixed solvent composed of dichloromethane (3 mL)/trifluoroacetic acid (1.5 mL). The mixture was stirred at 25 °C for 16 h, and the reaction was completed. The reaction mixture was dried by rotary evaporation to afford the product (25 mg).

### 3. Synthesis of Compounds C5 and C6

Compound C5-2 (25 mg, 0.06 mmol) was separated by the following chiral resolution method:
Instrument: Agilent 1260 InfinityII, chromatographic column: phenomenex Lux^{®} 5 µm Amylose-1 (250 × 21.2 mm), mobile phase: n-hexane : ethanol = 4:6, column temperature: 25 °C. The separated products were obtained as follows:
C5 (5.8 min, 8.7 mg) [M+H]⁺: 453.3 ¹H NMR (400 MHz, DMSO-*d*₆) δ 12.73 (s, 1H), 8.47 (d, *J* = 6.3 Hz, 1H), 7.80 (dd, *J =* 7.9, 1.7 Hz, 1H), 7.70 (dd, *J =* 8.3, 3.1 Hz, 1H), 7.61 - 7.51 (m, 1H), 7.17 (t, *J* = 7.9 Hz, 1H), 6.53 (t, *J =* 7.5 Hz, 1H), 6.38 (d, *J =* 8.5 Hz, 1H), 5.47 (s, 1H), 4.95 - 4.67 (m, 2H), 4.16 (s, 2H), 3.97 (s, 1H), 3.81 (s, 1H), 3.06 - 2.88 (m, 1H), 1.58 (d, *J =* 6.6 Hz, 3H), 1.06 (d, *J* = 6.7 Hz, 6H).
C6 (9.8 min, 6.6 mg) [M+H]⁺: 453.3 ¹H NMR (400 MHz, DMSO-*d*₆) δ 12.74 (s, 1H), 8.54 - 8.41 (m, 1H), 7.80 (dd, *J* = 7.9, 1.7 Hz, 1H), 7.70 (dd, *J =* 8.4, 3.0 Hz, 1H), 7.55 (dd, *J =* 9.3, 3.3 Hz, 1H), 7.17 (t, *J =* 7.9 Hz, 1H), 6.53 (t, *J =* 7.5 Hz, 1H), 6.38 (d, *J =* 8.5 Hz, 1H), 5.47 (s, 1H), 4.89 (s, 1H), 4.82 - 4.67 (m, 1H), 4.17 (s, 2H), 3.97 (s, 1H), 3.81 (s, 1H), 3.05 - 2.87 (m, 1H), 1.58 (d, *J =* 6.6 Hz, 3H), 1.06 (d, *J =* 6.6 Hz, 6H).

### Example 4

Compounds of the present invention

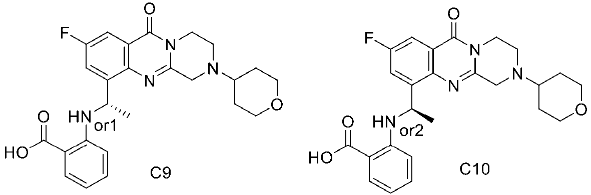

The synthetic route and experimental procedure were as follows:

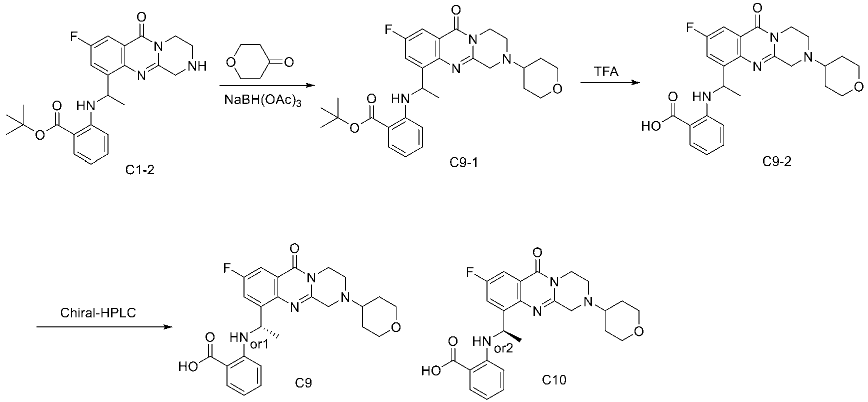

### 1. Synthesis of C9-1

C1-2 (200 mg, 0.46 mmol), tetrahydropyranone (60 mg, 0.59 mmol), sodium triacetoxyborohydride (155 mg, 0.73 mmol) and 20 mL of dichloromethane were added to a 10 mL single-necked flask. After all starting materials were added, the mixture was stirred at room temperature overnight under nitrogen protection. The reaction mixture was post-reaction treated, and purified by column chromatography to afford 170 mg of the product.

### 2. Synthesis of C9-2

Compound C9-1 (170 mg) was dissolved in 4 mL of anhydrous dichloromethane and 2 mL of trifluoroacetic acid. The mixture was stirred at room temperature overnight under nitrogen protection. The reaction solution was added dropwise to saturated sodium bicarbonate solution, then extracted, dried, concentrated, and subjected to column chromatography to afford 100 mg of the product.

### 3. Synthesis of C9 and C10

Firstly, compound C9-2 (100 mg) was separated by the following chiral resolution method:
Instrument: Agilent 1260 InfinityII, chromatographic column: CHIRALPAK IA 5 µm (250 × 20 mm), mobile phase: n-hexane : ethanol = 95:5, column temperature: 25 °C. The separated products were obtained as follows:
C9 (14.1 min, 9.33 mg) [M+H]⁺: 467.3; ¹H NMR (400 MHz, DMSO-d₆) δ 8.52 (m, 1H), 7.81 (d, J = 7.8 Hz, 1H), 7.74 (dd, J = 8.2, 2.9 Hz, 1H), 7.62 (dd, J = 9.4, 3.1 Hz, 1H), 7.18 (dd, J = 8.6, 6.9 Hz, 1H), 6.54 (t, J = 7.5 Hz, 1H), 6.37 (d, J = 8.5 Hz, 1H), 5.45 (m, 1H), 4.47 (m, 2H), 4.23 -3.31(m, 9H), 2.03 (d, J = 12.2 Hz, 2H), 1.81 - 1.64 (m, 2H), 1.59 (d, J = 6.6 Hz, 2H).
C10 (16.2 min, 10.53 mg) [M+H]⁺: 467.3; ¹H NMR (400 MHz, DMSO-d₆) δ 8.52 (m, 1H), 7.81 (d, J = 7.8 Hz, 1H), 7.74 (dd, J = 8.2, 2.9 Hz, 1H), 7.62 (dd, J = 9.4, 3.1 Hz, 1H), 7.18 (dd, J = 8.6, 6.9 Hz, 1H), 6.54 (t, J = 7.5 Hz, 1H), 6.37 (d, J = 8.5 Hz, 1H), 5.45 (m, 1H), 4.47 (m, 2H), 4.23 -3.31(m, 9H), 2.03 (d, J = 12.2 Hz, 2H), 1.81 - 1.64 (m, 2H), 1.59 (d, J = 6.6 Hz, 2H).

### Example 5

Compounds of the present invention

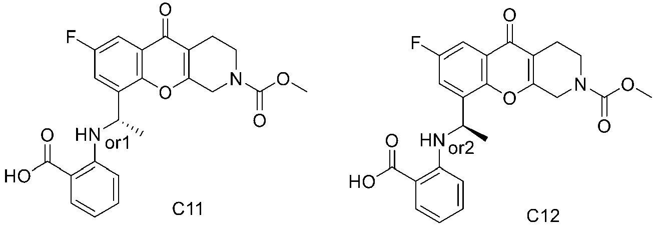

The synthetic route and experimental procedure were as follows:

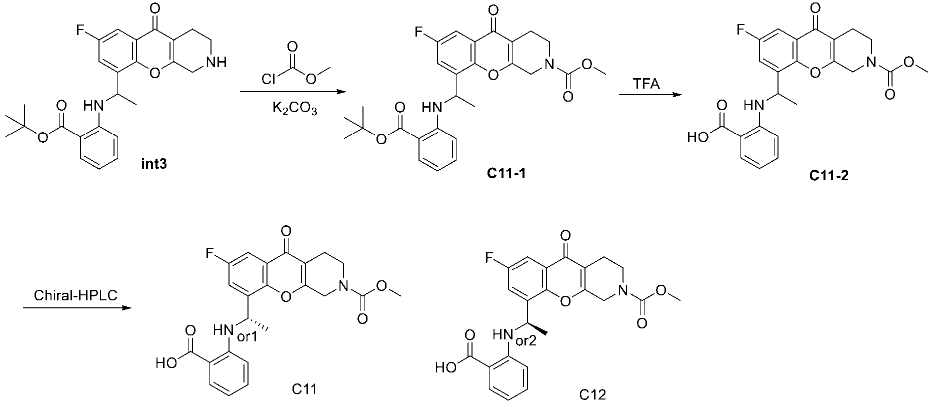

### 1. Synthesis of Compound C11-1

Compound int3 (110 mg, 0.25 mmol) was dissolved in 5 mL of N,N-dimethylformamide. Potassium carbonate (103 mg, 0.75 mmol) was added at room temperature, followed by slow dropwise addition of methyl chloroformate (28.5 mg, 0.3 mmol). After completion of the addition, the mixture was stirred at room temperature for 3 h. After the starting materials were reacted completely, the reaction was quenched with water, and the mixture was extracted with dichloromethane three times. The organic phases were combined, concentrated, dried and purified by column chromatography to afford the product (80 mg).

### 2. Synthesis of Compound C11-2

Compound C11-1 (80 mg, 0.16 mmol) was dissolved in a mixed solvent composed of dichloromethane (5 mL) and trifluoroacetic acid (5 mL). The mixture was stirred and reacted at 25 °C for 12 h. After the reaction was completed, the mixture was dried by rotary evaporation and separated by preparative plates to afford the product (40 mg).

### 3. Synthesis of Compounds C11 and C12

Compound C11-2 (40 mg) was separated by the following chiral resolution method:
Instrument: Agilent 1260 InfinityII, chromatographic column: phenomenex Lux^{®} 5 µm cellulose-4 (250 × 21.2 mm), mobile phase: *n*-hexane : ethanol = 9:1, column temperature: 25 °C. The separated products were obtained as follows:
C11 (5.5 min, 14 mg) [M+H]⁺: 441.2
¹H NMR (400 MHz, DMSO-*d*₆) δ 12.81 (s, 1H), 8.45 (d, *J =* 7.2 Hz, 1H), 7.81 (dd, *J* = 7.8*,* 1.6 Hz, 1H), 7.59 (m, 2H), 7.24 (m, 1H), 6.60 - 6.51 (m, 2H), 5.21 (t, *J =* 6.8 Hz, 1H), 4.68 - 4.52 (m, 2H), 3.70 (s, 5H), 2.54 (d, *J =* 5.6 Hz, 2H), 1.64 (d, *J =* 6.8 Hz, 3H).
C12 (8.2 min, 13 mg) [M+H]⁺: 441.2
¹H NMR (400 MHz, DMSO-*d*₆) δ 12.81 (s, 1H), 8.45 (d, *J =* 7.2 Hz, 1H), 7.81 (dd, *J* = 7.8*,* 1.6 Hz, 1H), 7.59 (m, 2H), 7.24 (m, 1H), 6.60 - 6.51 (m, 2H), 5.21 (t, *J =* 6.8 Hz, 1H), 4.68 - 4.52 (m, 2H), 3.70 (s, 5H), 2.54 (d, *J =* 5.6 Hz, 2H), 1.64 (d, *J =* 6.8 Hz, 3H).

### Example 6

Compounds of the present invention

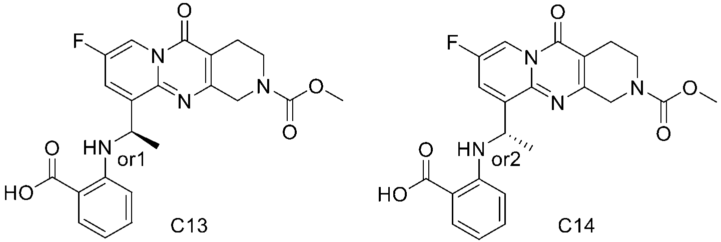

The synthetic route and experimental procedure were as follows:

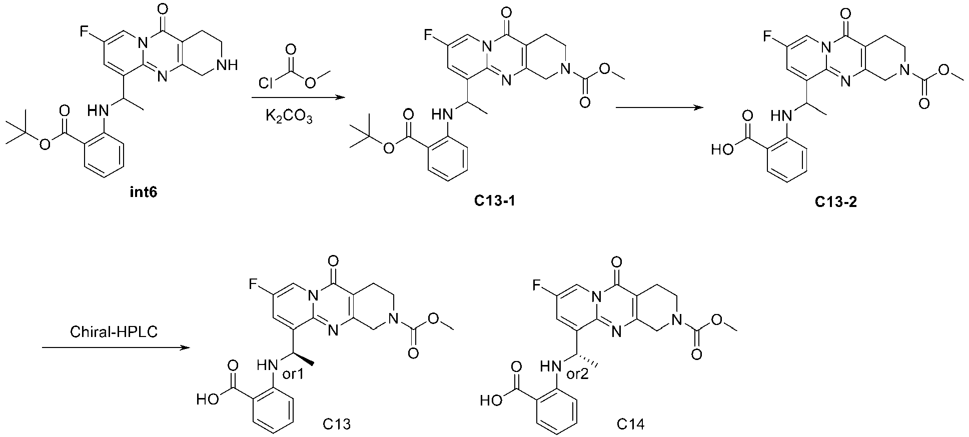

### 1. Synthesis of Compound C13-1

Compound int6 (17 mg, 0.039 mmol) and potassium carbonate (16 mg, 0.117 mmol) were dissolved in dry dichloromethane (10 mL). Methyl chloroformate (8.8 mg, 0.092 mmol) was added under nitrogen atmosphere at room temperature, and the mixture was stirred at 25 °C for 12 h. Then the mixture was concentrated, dried and purified by column chromatography to afford the product (9 mg).

### 2. Synthesis of Compound C13-2

Compound C13-1 (13 mg, 0.026 mmol) was dissolved in a mixed solvent of dichloromethane (2 mL) and trifluoroacetic acid (2 mL). The mixture was stirred at 23 °C for 12 h under nitrogen atmosphere. After the reaction was completed, the mixture was dried by rotary evaporation to afford the product (14 mg).

### 3. Synthesis of Compounds C13 and C14

Compound C13-2 (50 mg, 0.113 mmol) was separated by the following chiral resolution method:
Instrument: Agilent 1260 InfinityII, chromatographic column: phenomenex Lux^{®} 5 µm cellulose-4 (250 × 21.2 mm), mobile phase: n-hexane : ethanol = 2:8, column temperature: 25 °C. The separated products were obtained as follows:
C13 (11.2 min, 20.73 mg) [M+H]⁺: 441.2, ¹H NMR (DMSO-d6, 400 MHz) δ ppm 12.78 (br, 1H), 8.80 (dd, J = 4.8, 2.8 Hz, 1H), 8.50 (s, 1H), 7.86 (dd, J = 8.4, 2.8 Hz, 1H), 7.82 (dd, J = 8.0, 2.0 Hz, 1H), 7.24 - 7.19 (m, 1H), 6.57 (t, J = 8.0 Hz, 1H), 6.38 (d, J = 8.8 Hz, 1H), 5.32 (s, 1H), 4.59 (s, 2H), 3.74 - 3.62 (m, 5H), 2.69 (t, J = 5.6 Hz, 2H), 1.63 (d, J = 6.8 Hz, 3H).
C14 ( 13.1 min, 21.16 mg) [M+H]⁺: 441.2, ¹H NMR: (DMSO-d₆, 400Hz)*δ*12.81(s, 1H), 8.80~8.78 (m,1H), 8.50 (s, 1H), 7.87~7.80 (m, 2H ), 7.23~7.19(m, 1H), 6.58~6.55 (m,1H), 6.39~6.37(m,1H), 5.33~5.30(m,1H), 4.58(s,2H), 3.71~3.68(m, 5H), 2.70~2.67(m,2H), 1.63(d, *J*=7.8Hz, 3H)

### Example 7

Compounds of the present invention

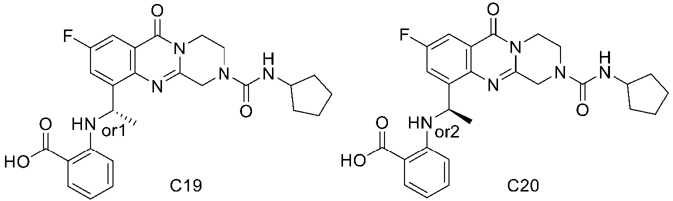

The synthetic route and experimental procedure were as follows:

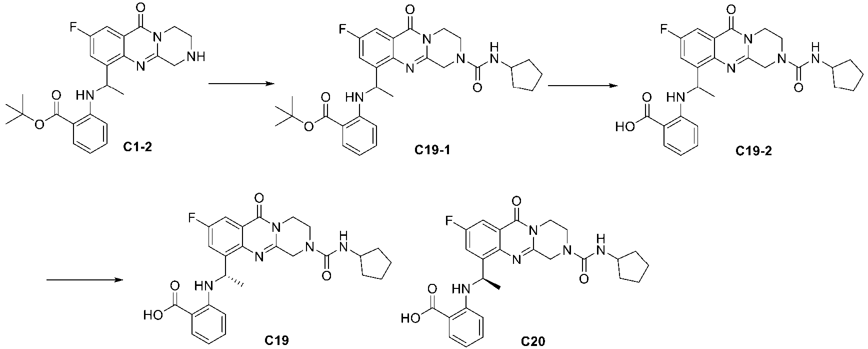

### 1. Synthesis of Compound C19-1

Compound C1-2 (100 mg, 0.23 mmol) was dissolved in 5 mL of tetrahydrofuran. 1,1'-Carbonyldiimidazole (370 mg, 2.3 mmol) was added. After the addition, the mixture was heated to 60 °C and reacted for 24 h. Then cyclopentylamine (387 mg, 4.6 mmol) was added, and the mixture was further reacted at 60 °C for 16 h. Water was added, and the mixture was extracted with ethyl acetate. The organic phase was separated, dried over anhydrous sodium sulfate, and the filtrate was concentrated under reduced pressure and purified to afford the product (50 mg).

### 2. Synthesis of Compound C19-2

Compound C19-1 (50 mg, 0.09 mmol) was dissolved in a mixed solvent of dichloromethane (5 mL) and trifluoroacetic acid (5 mL). The mixture was stirred at 25 °C for 12 h. After the reaction was completed, the mixture was dried by rotary evaporation and separated by preparative TLC to afford the product (40 mg).

### 3. Synthesis of Compounds C19 and C20

Compound C19-2 (40 mg) was separated by the following chiral resolution method:
Instrument: Agilent 1260 InfinityII, chromatographic column: Phenomenex Lux^{®} 5 µm cellulose-4 (250 × 21.2 mm), mobile phase: *n*-hexane : ethanol = 4:6, column temperature: 25 °C. The separated products were obtained as follows:
C19 (4.4 min, 15 mg) [M+H]⁺: 494.4 ¹H NMR (400 MHz, DMSO-*d*₆) δ ppm 12.79 (br, 1H), 8.46 (s, 1H), 7.81 (dd, *J =* 8.0, 2.0 Hz, 1H), 7.70 (dd, *J =* 8.0, 3.2 Hz, 1H), 7.53 (dd, *J =* 9.6, 3.2 Hz, 1H), 7.22 - 7.14 (m, 1H), 6.63 - 6.49 (m, 2H), 6.34 (d, *J =* 8.4 Hz, 1H), 5.53 - 5.41 (m, 1H), 4.77 - 4.60 (m, 2H), 4.10 (t, *J =* 5.6 Hz, 2H), 4.03 - 3.90 (m, 1H), 3.83 -3.65 (m, 2H), 1.88 - 1.76 (m, 2H), 1.72 - 1.62 (m, 2H), 1.58 (d, *J* = 7.6 Hz, 3H), 1.54 - 1.39 (m, 4H).
C20 (7.4 min, 15 mg) [M+H]⁺: 494.4 ¹H NMR (400 MHz, DMSO-*d*₆) δ ppm 12.76 (br, 1H), 8.46 (s, 1H), 7.81 (dd, *J =* 8.0, 2.0 Hz, 1H), 7.70 (dd, *J =* 8.0, 3.2 Hz, 1H), 7.53 (dd, *J =* 9.6, 3.2 Hz, 1H), 7.21 - 7.11 (m, 1H), 6.63 - 6.48 (m, 2H), 6.34 (d, *J =* 8.0 Hz, 1H), 5.53 - 5.40 (m, 1H), 4.77 - 4.60 (m, 2H), 4.10 (t, *J =* 5.6 Hz, 2H), 4.03 - 3.91 (m, 1H), 3.83 -3.66 (m, 2H), 1.88 - 1.76 (m, 2H), 1.72 - 1.62 (m, 2H), 1.58 (d, *J* = 7.2 Hz, 3H), 1.54 - 1.38 (m, 4H).

### Example 8

Compounds of the present invention

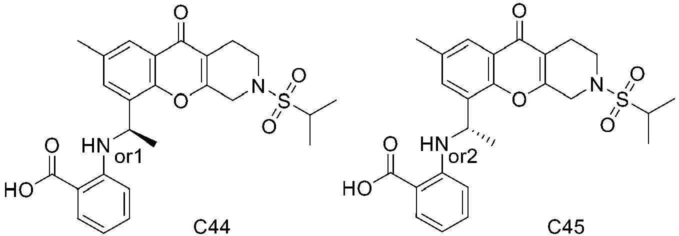

The synthetic route and experimental procedure were as follows:

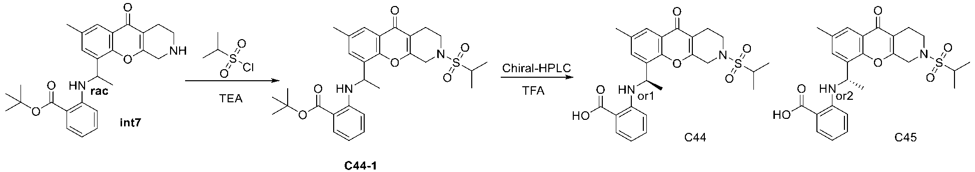

### 1. Synthesis of Compound C44-1

Compound int7 (51 mg, 0.11 mmol) was dissolved in 5 mL of dichloromethane. At 0 °C, triethylamine (56 mg, 0.55 mmol) was added, followed by slow dropwise addition of isopropylsulfonyl chloride (37 mg, 0.26 mmol). After completion of the addition, the mixture was allowed to warm to room temperature naturally, stirred and reacted for 12 h. After the starting materials were reacted completely, the reaction was quenched with water, and the mixture was extracted with dichloromethane for three times. The organic phases were combined, concentrated and purified by column chromatography to afford the product (20 mg).

### 2. Synthesis of Compounds C44 and C45

Compound C44-1 (20 mg) was separated by the following chiral resolution method:
Instrument: Agilent 1260 InfinityII, chromatographic column: Phenomenex Lux^{®} 5 µm cellulose-4 (250 × 21.2 mm), mobile phase: *n*-hexane : ethanol = 9:1, column temperature: 25 °C. After that, the mixture was concentrated. The residue was dissolved in a mixed solvent of dichloromethane (4 mL)/trifluoroacetic acid (2 mL), and stirred at 23 °C for 12 h under nitrogen protection. After the reaction was completed, the mixture was concentrated and purified by preparative thin-layer chromatography to afford the products:
C45 (17.4 min, 2.18 mg) [M+H]⁺: 485.3 ¹HNMR: (DMSO-d₆, 400Hz),*δ*8.59(s,1H), 7.81~7.79(m, 1H),7.73(s,1H), 7.54(s,1H), 7.18~7.16(m,1H), 6.54~6.47(m,2H) , 5.21~.5.13 (m,1H), 4.50~4.46(m,2H), 3.59~3.54(m,3H), 2.59~2.57(m,2H), 2.35(s,3H), 1.60(d, *J*=6.4Hz, 3H), 1.28(d,*J*=6.5Hz,6H).
C44 (20.3 min, 2.74 mg) [M+H]⁺: 485.3 ¹HNMR: (DMSO-d₆, 400Hz),*δ*8.59(s,1H), 7.81~7.79(m, 1H), 7.73(s,1H), 7.54(s,1H), 7.18~7.16(m,1H), 6.54~6.47(m,2H), 5.21~.5.13 (m,1H), 4.50~4.46(m,2H), 3.59~3.54(m,3H), 2.59~2.57(m,2H), 2.35(s,3H) ,1.60(d, *J*=6.4Hz, 3H), 1.28(d,*J*=6.5Hz,6H).

### Example 9

Compounds of the present invention

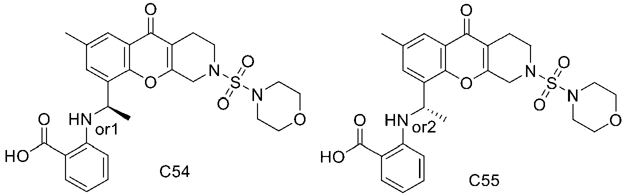

The synthetic route and experimental procedure were as follows:

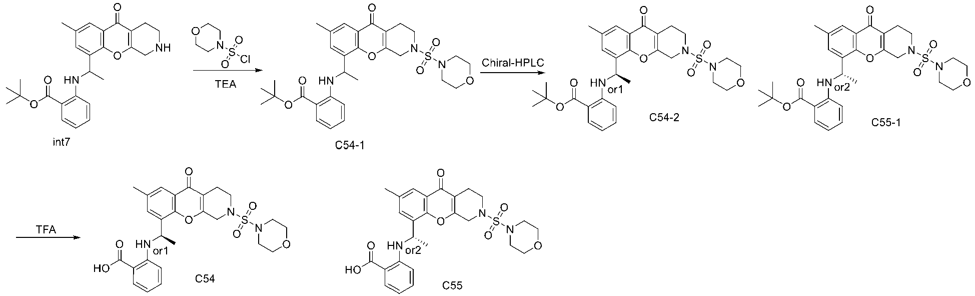

### 1. Synthesis of Compound C54-1

Int7 (80 mg, 0.18 mmol) was dissolved in dichloromethane (10 mL). Triethylamine (60 mg, 0.54 mmol) was added, and morpholinesulfonyl chloride (103 mg, 0.54 mmol) was added dropwise in an ice bath. The mixture was reacted at 30 °C for 12 h. After the reaction was completed, the reaction solution was concentrated, diluted with ethyl acetate, washed with saturated brine twice, dried and concentrated. The residue was separated by the following chiral resolution method:
Instrument: Agilent 1260 InfinityII, chromatographic column: Phenomenex Lux^{®} 5 µm cellulose-4 (250 × 21.2 mm), mobile phase: *n*-hexane : ethanol = 8:2, column temperature: 25 °C. Compound C54-2 (21.0 min, 25 mg) and C55-1 (26.7 min, 25 mg) were obtained.

### 2. Synthesis of Compounds C54 and C55

C54-2 and C55-1 were dissolved in dichloromethane (5 mL), respectively. Trifluoroacetic acid (5 mL) was added, and the mixture was reacted at 30 °C for 24 h. After the reaction was completed, the mixture was concentrated and purified by column chromatography to afford the products.

C54 (16.5 min, 10 mg) ¹H NMR (400 MHz, DMSO-*d*₆), 12.79 (m, 1H)8.47 (d, *J* = 7.2 Hz, 1H), 7.80 (dd, *J* = 8.0, 1.6 Hz, 1H), 7.73 (d, *J =* 2.4 Hz, 1H), 7.55 (d, *J =* 2.4 Hz, 1H), 7.22 (m, 1H), 6.57 - 6.49 (m, 2H), 5.17 (t, *J =* 6.8 Hz, 1H), 4.44 (d, *J =* 8.0 Hz, 2H), 3.64 (t, *J =* 4.8 Hz, 4H), 3.53 (q, *J =* 6.0 Hz, 2H), 3.19 (dd, *J =* 5.6, 3.6 Hz, 4H), 2.59 (t, *J =* 5.6 Hz, 2H), 2.35 (s, 3H), 1.60 (d, *J =* 6.4 Hz, 3H).

C55 (18.1 min, 10 mg) ¹H NMR (400 MHz, DMSO-*d*₆) δ 12.79 (m, 1H), 8.47 (d, *J* = 7.2 Hz, 1H), 7.80 (dd, *J =* 8.0, 1.6 Hz, 1H), 7.73 (d, *J =* 2.4 Hz, 1H), 7.55 (d, *J =* 2.4 Hz, 1H), 7.22 (m, 1H), 6.57 - 6.49 (m, 2H), 5.17 (t, *J =* 6.8 Hz, 1H), 4.44 (d, *J =* 8.0 Hz, 2H), 3.64 (t, *J =* 4.8 Hz, 4H), 3.53 (q, *J =* 6.0 Hz, 2H), 3.19 (dd, *J =* 5.6, 3.6 Hz, 4H), 2.59 (t, *J =* 5.6 Hz, 2H), 2.35 (s, 3H), 1.60 (d, *J =* 6.4 Hz, 3H).

### Example 10

Compounds of the present invention

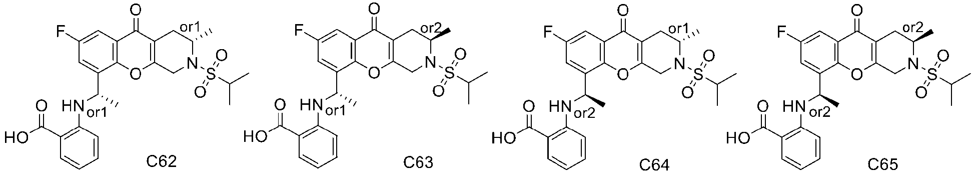

The synthetic route and experimental procedure were as follows:

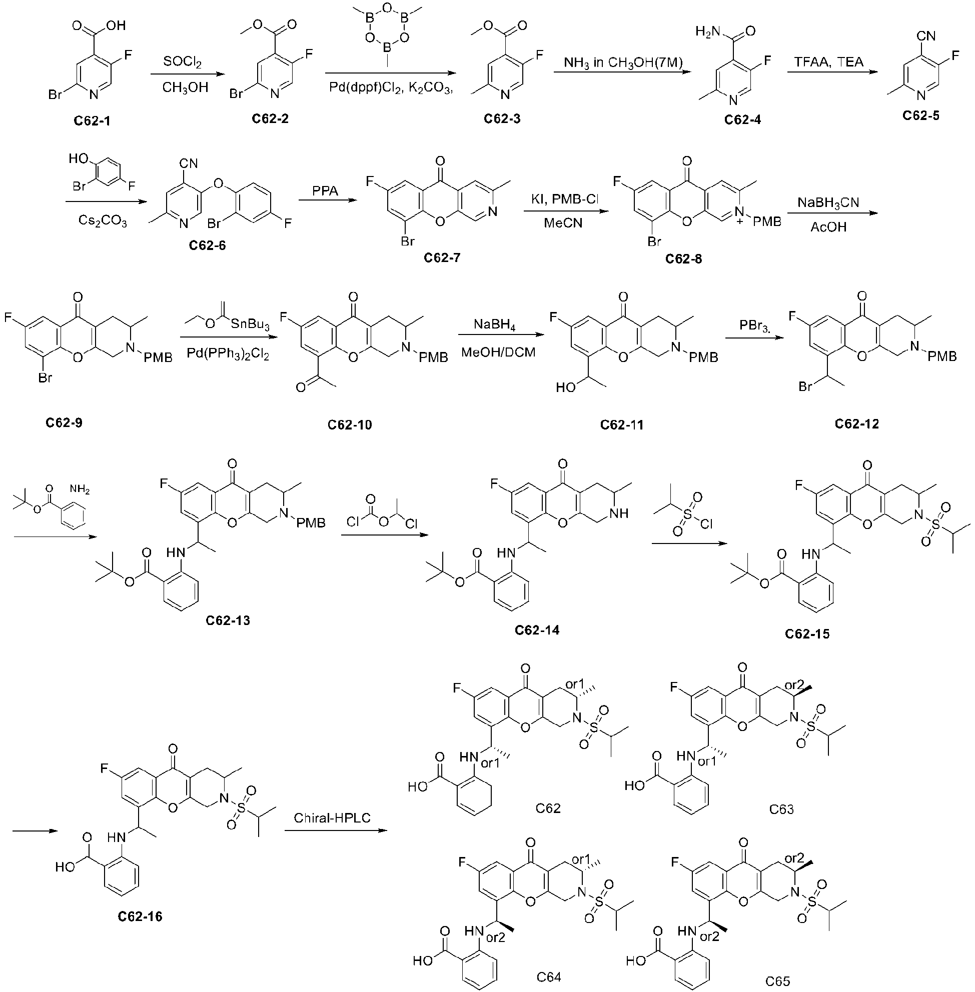

### 1. Synthesis of Compound C62-2

Compound C62-1 (25 g, 113.6 mmol) was dissolved in 400 mL of methanol. Thionyl chloride (54 g, 454.5 mmol) was added dropwise in an ice bath. After completion of the addition, the mixture was heated to 70 °C and reacted for 4 h until the starting materials were reacted completely. The mixture was dried directly by rotary evaporation, basified with saturated aqueous sodium bicarbonate solution, extracted with ethyl acetate, dried over anhydrous sodium sulfate, and filtered. The filtrate was concentrated under reduced pressure to afford the product (26 g).

¹H NMR (400 MHz, Chloroform-*d*) δ 8.41 (d, *J =* 2.0 Hz, 1H), 7.89 (dd, *J =* 56.8, 5.2 Hz, 1H), 4.00 (d, *J* = 1.2 Hz, 3H).

### 2. Synthesis of Compound C62-3

C62-2 (26 g, 111.1 mmol) was dissolved in 350 mL of dry 1,4-dioxane. Potassium carbonate (22.9 g, 166.7 mmol), Pd(dppf)Cl₂ (8.1 g, 11.1 mmol) and a solution of trimethylboroxine in THF (3.5 M, 80 mL, 277.8 mmol) were added then. After the addition, the mixture was stirred at 105 °C overnight under nitrogen protection. After the starting materials were reacted completely, the reaction solution was cooled to room temperature, diluted with water, and extracted three times with ethyl acetate. The organic phases were combined, dried over anhydrous sodium sulfate, concentrated under reduced pressure, and purified to afford the product (13.7 g).

¹H NMR (400 MHz, Chloroform-*d*) δ 8.49 (d, *J* = 2.4 Hz, 1H), 7.62 (d, *J =* 5.6 Hz, 1H), 3.98 (s, 3H), 2.60 (d, *J =* 1.2 Hz, 3H).

### 3. Synthesis of Compound C62-4

Compound C62-3 (13.7 g, 80.6 mmol) was added to 120 mL of aqueous ammonia. The mixture was stirred at 80 °C for 5 h in an iron pressure vessel. After the starting materials were reacted completely, the reaction solution was concentrated under reduced pressure, and slurried with dichloromethane and petroleum ether to afford the product (12 g).

¹H NMR (400 MHz, DMSO-*d*₆) δ 8.51 (d, *J =* 1.6 Hz, 1H), 7.94 (d, *J =* 43.2 Hz, 2H), 7.44 (d, *J* = 5.6 Hz, 1H), 2.49 (s, 3H).

### 4. Synthesis of Compound C62-5

Compound C62-4 (4 g, 25.8 mmol) was dissolved in 80 mL of dichloromethane. Triethylamine (7.82 g, 77.4 mmol) was added, followed by dropwise addition of trifluoroacetic anhydride (10.8 g, 51.6 mmol). The mixture was stirred at room temperature for 2 h until the starting materials were reacted completely. The reaction mixture was diluted with water, and extracted three times with ethyl acetate. The organic phases were combined, dried over anhydrous sodium sulfate, concentrated under reduced pressure, and purified to afford the product (3 g).

¹H NMR (400 MHz, Chloroform-*d*) δ 8.58 (s, 1H), 7.39 (d, *J =* 4.8 Hz, 1H), 2.62 (d, *J =* 1.2 Hz, 3H).

### 5. Synthesis of Compound C62-6

Compound C62-5 (3 g, 21.9 mmol) was dissolved in 60 mL of N,N-dimethylformamide. 2-Bromo-4-fluorophenol (6.3 g, 32.9 mmol) and cesium carbonate (14.4 g, 43.8 mmol) were added. After the addition, the mixture was heated to 130 °C and reacted for 1 h until the starting materials were reacted completely. The reaction was quenched by slow addition of water, and then the reaction solution was extracted with ethyl acetate. The organic phase was separated, and dried over anhydrous sodium sulfate. The filtrate was concentrated under reduced pressure, and purified to afford the product (5.7 g).

¹H NMR (400 MHz, Chloroform-*d*) δ 8.02 (s, 1H), 7.51 - 7.45 (m, 1H), 7.42 (s, 1H), 7.20 - 7.09 (m, 2H), 2.59 (s, 3H).

### 6. Synthesis of Compound C62-7

C62-6 (5.7 g, 41 mmol) was dissolved in 57 g of polyphosphoric acid. The mixture was slowly heated to 220 °C and stirred for 3 h. After the starting materials were reacted completely, the reaction solution was cooled to room temperature, dissolved in water, basified with saturated aqueous sodium bicarbonate solution, and extracted three times with ethyl acetate. The organic phases were combined, dried over anhydrous sodium sulfate, concentrated under reduced pressure, and purified to afford the product (4 g).

¹H NMR (400 MHz, Chloroform-*d*) δ 9.08 (s, 1H), 8.02 - 7.92 (m, 2H), 7.84 (dd, *J =* 7.2, 3.2 Hz, 1H), 2.74 (s, 3H).

### 7. Synthesis of Compound C62-8

C62-7 (4 g, 13 mmol), potassium iodide (9.5 g, 57.2 mmol) and benzyl chloride (8.15 g, 52 mmol) were added to 150 mL of acetonitrile. The mixture was slowly heated to 80 °C and stirred overnight. After the starting materials were reacted completely, the reaction solution was concentrated under reduced pressure to give a crude product (9 g), which was used directly in the next reaction step.

### 8. Synthesis of Compound C62-9

Compound C62-8 (9 g, 15.9 mmol) was dissolved in 100 mL of anhydrous ethanol. Sodium cyanoborohydride (8 g, 127.4 mmol) and acetic acid (0.9 g, 15.9 mmol) were added. The mixture was stirred at room temperature for 30 min until the starting materials were reacted completely. The reaction solution was diluted with water, and extracted three times with dichloromethane. The organic phases were combined, dried over anhydrous sodium sulfate, concentrated under reduced pressure, and purified to afford the product (2.5 g).

¹H NMR (400 MHz, Chloroform-*d*) δ 7.85 (dd, *J =* 8.0, 3.2 Hz, 1H), 7.64 (dd, *J =* 7.2, 3.2 Hz, 1H), 7.32 (dd, *J =* 8.8, 3.2 Hz, 2H), 6.93 - 6.91 (m, 2H), 3.84 (s, 3H), 3.79 (dd, *J =* 3.6, 1.2 Hz, 2H), 3.60 (m, 2H), 3.18 (h, *J* = 6.4 Hz, 1H), 2.85 - 2.74 (m, 1H), 2.55 - 2.44 (m, 1H), 1.22 (d, *J =* 6.4 Hz, 3H).

### 9. Synthesis of Compound C62-10

Compound C62-9 (2 g, 4.63 mmol), tributyl(1-ethoxyvinyl)tin (2 g, 5.56 mmol) and Pd(PPh₃)₂Cl₂ (0.33 g, 0.46 mmol) were dissolved in 80 mL of dry 1,4-dioxane. The mixture was stirred at 80 °C for 12 h. Then at room temperature, potassium fluoride solution was added and stirred for 0.5 h. The mixture was filtered, and the mother liquor was extracted three times with ethyl acetate. The organic phase was separated, dried by rotary evaporation, and dissolved in dichloromethane, and 2 mL of concentrated HCl was added. The mixture was stirred at 25 °C for 10 min. After the reaction was completed, the mixture was purified by column chromatography to afford the product (1.4 g).

¹H NMR (400 MHz, Chloroform-*d*) δ 8.06 (dd, *J =* 7.6, 3.2 Hz, 1H), 7.83 (dd, *J =* 8.2, 3.4 Hz, 1H), 7.31 (s, 2H), 6.94 - 6.89 (m, 2H), 3.84 (s, 3H), 3.80 (m, 2H), 3.64 - 3.55 (m, 2H), 3.21 (q, *J =* 6.0 Hz, 1H), 2.86 - 2.77 (m, 1H), 2.71 (s, 3H), 2.53 (m, 1H), 1.22 (d, *J =* 6.4 Hz, 3H).

### 10. Synthesis of Compound C62-11

Compound C62-10 (0.7 g, 1.77 mmol) was dissolved in a mixed solvent composed of 10 mL of dichloromethane and 10 mL of methanol. Sodium borohydride (40 mg, 0.88 mmol) was added slowly under nitrogen protection. The reaction solution was stirred at room temperature for 10 min until the starting materials were reacted completely. The reaction solution was diluted with water, and extracted three times with dichloromethane. The organic phases were combined, dried over anhydrous sodium sulfate, concentrated under reduced pressure, and purified to afford the product (600 mg).

### 11. Synthesis of Compound C62-12

Compound C62-11 (600 mg, 1.5 mmol) was dissolved in 30 mL of 1,2-dichloroethane. Phosphorus tribromide (1.25 g, 4.5 mmol) was added slowly under nitrogen protection, and the mixture was stirred at 50 °C for 4 h. After the starting materials were reacted completely, the mixture was concentrated under reduced pressure, and slurried with dichloromethane and petroleum ether to afford the product (600 mg).

### 12. Synthesis of Compound C62-13

Compound C62-12 (600 mg, 1.3 mmol) was dissolved in 20 mL of anhydrous N,N-dimethylformamide. Tert-butyl 2-aminobenzoate (1.6 g, 7.8 mmol) was added slowly at room temperature. The mixture was heated to 60 °C slowly, stirred and reacted for 12 h. After the starting materials were reacted completely, the reaction solution was diluted with water and extracted three times with ethyl acetate. The organic phases were combined, dried over anhydrous sodium sulfate, concentrated under reduced pressure and purified to afford the product (350 mg).

### 13. Synthesis of Compound C62-14

Compound C62-13 (350 mg, 0.61 mmol) was dissolved in 40 mL of 1,2-dichloroethane. 1-Chloroethyl chloroformate (174 mg, 1.22 mmol) was added slowly at room temperature. The mixture was slowly heated to 83 °C and stirred and reacted for 3 h. After the starting materials were reacted completely, the mixture was concentrated to dryness. Methanol was added, and the mixture was heated to 65 °C and stirred and reacted for 1 h, basified with triethylamine, then dried by rotary evaporation and purified to afford the product (200 mg).

### 14. Synthesis of Compound C62-15

Compound C62-14 (100 mg, 0.22 mmol) was dissolved in 5 mL of dichloromethane. DBU (100 mg, 0.66 mmol) was added at room temperature, followed by slow dropwise addition of a solution of isopropylsulfonyl chloride in dichloromethane (78.3 mg, 0.55 mmol). After completion of the addition, the mixture was stirred and reacted at 30 °C for 2 h. After the starting materials were reacted completely, the reaction was quenched with water and the reaction solution was extracted three times with dichloromethane. The organic phases were combined, concentrated, dried and purified by column chromatography to afford the product (80 mg).

### 15. Synthesis of Compound C62-16

Compound C62-15 (80 mg, 0.16 mmol) was dissolved in a mixed solvent of dichloromethane (5 mL) and trifluoroacetic acid (5 mL). The mixture was stirred and reacted at 25 °C for 12 h. After the reaction was completed, the mixture was dried by rotary evaporation and separated by preparative thin-layer chromatography to afford the product (70 mg).

### 16. Synthesis of Compounds C62, C63, C64 and C65

Compound C62-16 (70 mg) was separated by the following chiral resolution method: Instrument: Agilent 1260 InfinityII, chromatographic column: Phenomenex Lux^{®} 5 µm cellulose-4 (250 × 21.2 mm), mobile phase: *n*-hexane : ethanol = 9:1, column temperature: 25 °C.

C62 (19.2 min, 12.32 mg) [M+H]⁺: 503.2
¹H NMR (400 MHz, DMSO-*d*₆) δ 12.86 (m, 1H), 8.53 (m, 1H), 7.81 (dd, *J =* 8.2, 1.6 Hz, 1H), 7.58 (m, 2H), 7.23 (t, *J =* 7.6 Hz, 1H), 6.57 (dd, *J =* 8.4, 5.6 Hz, 2H), 5.23 (t, *J =* 6.8 Hz, 1H), 4.65 - 4.28 (m, 3H), 3.51 (p, *J* = 6.8 Hz, 1H), 2.72 - 2.56 (m, 2H), 1.66 (d, *J =* 6.8 Hz, 3H), 1.29 - 1.19 (m, 9H).

C63 (29.5 min, 4.42 mg) [M+H]⁺: 503.2
¹H NMR (400 MHz, DMSO-*d*₆) δ12.86 (m, 1H), 8.53 (m, 1H), 7.81 (dd, *J =* 8.2, 1.6 Hz, 1H), 7.58 (m, 2H), 7.23 (t, *J =* 7.6 Hz, 1H), 6.57 (dd, *J =* 8.4, 5.6 Hz, 2H), 5.23 (t, *J =* 6.8 Hz, 1H), 4.65 - 4.28 (m, 3H), 3.51 (p, *J* = 6.8 Hz, 1H), 2.72 - 2.56 (m, 2H), 1.66 (d, *J =* 6.8 Hz, 3H), 1.29 - 1.19 (m, 9H).

C64 (34.7 min, 1.72 mg) [M+H]⁺: 503.2
¹H NMR (400 MHz, DMSO-*d*₆) δ12.86 (m, 1H), 8.53 (m, 1H), 7.81 (dd, *J =* 8.2, 1.6 Hz, 1H), 7.58 (m, 2H), 7.23 (t, *J =* 7.6 Hz, 1H), 6.57 (dd, *J =* 8.4, 5.6 Hz, 2H), 5.23 (t, *J =* 6.8 Hz, 1H), 4.65 - 4.28 (m, 3H), 3.51 (p, *J =* 6.8 Hz, 1H), 2.72 - 2.56 (m, 2H), 1.66 (d, *J =* 6.8 Hz, 3H), 1.29 - 1.19 (m, 9H).

C65 (46.7 min, 8.72 mg) [M+H]⁺: 503.2
¹H NMR (400 MHz, DMSO-*d*₆) δ12.86 (m, 1H), 8.53 (m, 1H), 7.81 (dd, *J =* 8.2, 1.6 Hz, 1H), 7.58 (m, 2H), 7.23 (t, *J =* 7.6 Hz, 1H), 6.57 (dd, *J =* 8.4, 5.6 Hz, 2H), 5.23 (t, *J =* 6.8 Hz, 1H), 4.65 - 4.28 (m, 3H), 3.51 (p, *J =* 6.8 Hz, 1H), 2.72 - 2.56 (m, 2H), 1.66 (d, *J =* 6.8 Hz, 3H), 1.29 - 1.19 (m, 9H).

### Example 11

Compounds of the present invention

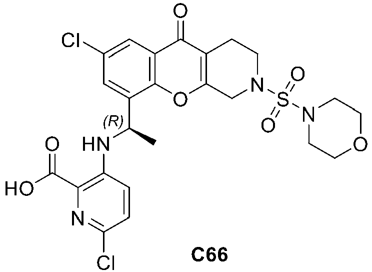

The synthetic route and experimental procedure were as follows:

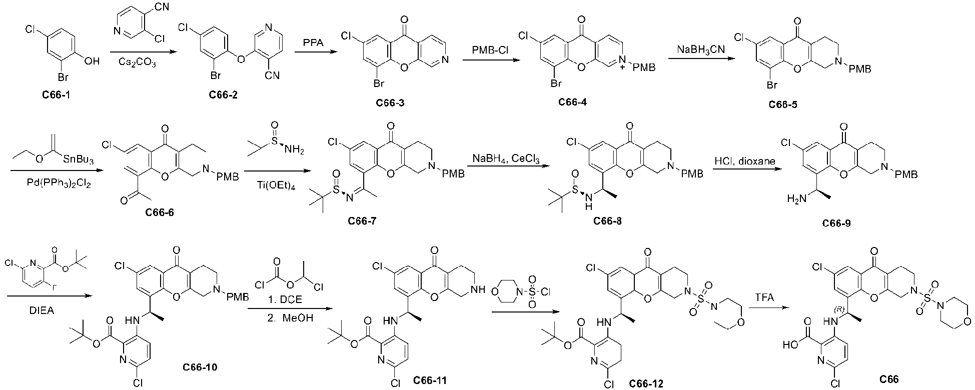

### 1. Synthesis of Compound C66-2

Compound C66-1 (4.48 g, 24 mmol) was dissolved in 60 mL of N,N-dimethylformamide. 3-Chloro-4-cyanopyridine (2.78 g, 20 mmol) and cesium carbonate (13 g, 40 mmol) were added. After the addition, the mixture was heated to 110 °C and reacted for 3 h until the starting materials were reacted completely. The reaction was quenched by slow addition of water, and then reaction solution was extracted with ethyl acetate. The organic phase was separated, dried over anhydrous sodium sulfate, concentrated under reduced pressure, and purified by column chromatography to afford the product (4.5 g).

¹H NMR (400 MHz, Chloroform-d) δ 8.53 (d, *J =* 4.8 Hz, 1H), 8.16 (s, 1H), 7.74 (d, *J =* 5.6 Hz, 1H), 7.60 (d, *J =* 4.8 Hz, 1H), 7.42~7.40(m, 1H), 7.16(d, *J =* 8.8 Hz, 1H).

### 2. Synthesis of Compound C66-3

C66-2 (4.5 g, 15.5 mmol) was dissolved in 40 g of polyphosphoric acid. The mixture was slowly heated to 220 °C and stirred for 3 h. After the starting materials were reacted completely, the reaction solution was cooled to room temperature, dissolved in water, basified with saturated aqueous sodium bicarbonate solution, and extracted three times with ethyl acetate. The organic phases were combined, dried over anhydrous sodium sulfate, concentrated under reduced pressure, triturated and purified to afford the product (3.5 g).

¹H NMR (400 MHz, Chloroform-d) δ 9.19 (s, 1H),8.74 (s,1H), 8.28 (d, *J =* 2.4 Hz, 1H), 8.12 (s, 1H), 8.05 (d, *J =* 2.8 Hz, 1H).

### 3. Synthesis of Compound C66-4

C66-3 (2.5 g, 8.6 mmol), potassium iodide (3.15 g, 19 mmol) and benzyl chloride (2.7 g, 17.2 mmol) were added to 40 mL of acetonitrile. The reaction solution was slowly heated to 80 °C and stirred for 2 h. After the starting materials were reacted completely, the reaction solution was concentrated under reduced pressure to give a crude product (8.2 g), which was used directly in the next reaction step.

### 4. Synthesis of Compound C66-5

Compound C66-4 (8.2 g, 20 mmol) was dissolved in 200 mL of anhydrous ethanol. Sodium cyanoborohydride (10.8 g, 160 mmol) and acetic acid (1.2 g, 20 mmol) were added. The mixture was stirred at room temperature for 30 min until the starting materials were reacted completely. The reaction solution was diluted with water, and extracted three times with dichloromethane. The organic phases were combined, dried over anhydrous sodium sulfate, concentrated under reduced pressure, and purified to afford the product (3.8 g).

### 5. Synthesis of Compound C66-6

Compound C66-5 (3 g, 7.24 mmol), tributyl(1-ethoxyvinyl)tin (3.14 g, 8.7 mmol) and Pd(PPh₃)₂Cl₂ (510 mg, 0.724 mmol) were dissolved in 50 mL of dry 1,4-dioxane. The mixture was stirred at 80 °C for 12 h. Then at room temperature, potassium fluoride solution was added and stirred for 0.5 h. The mixture was filtered, and the mother liquor was extracted three times with ethyl acetate. The organic phase was separated, dried by rotary evaporation, and dissolved in dichloromethane, and 2 mL of concentrated HCl was added. The mixture was stirred at 25 °C for 10 min. After the reaction was completed, the mixture was purified by column chromatography to afford the product (2.5 g).

¹H NMR (400 MHz, Chloroform-d) δ 8.35 (s, 1H), 8.03 (s, 1H), 7.31 (d, *J =* 8.4 Hz, 2H), 6.93 (d, *J* = 8.4 Hz, 2H), 3.84 (s, 3H), 3.71 (s, 2H), 3.54 (s, 2H), 2.79~2.77 (m,2H), 2.71~2.68 (m,5H), 2.48 (s, 3H).

### 6. Synthesis of Compound C66-7

Compound C66-6 (1.13 g, 3 mmol) was dissolved in 50 mL of tetrahydrofuran. (R)-(+)-*tert*-butyl sulfinamide (726 mg, 6 mmol) and Ti(OEt)₄ (2.73 g, 12 mmol) were added successively under nitrogen protection. The reaction solution was stirred at 70 °C for 12 h. After the starting materials were reacted completely, the reaction mixture was diluted with water, filtered through Celite, and washed with ethyl acetate. The filtrate was separated, and the mother liquor was extracted with ethyl acetate twice. The organic phases were combined, dried over anhydrous sodium sulfate, concentrated under reduced pressure, and purified to afford the product (1.1 g).

### 7. Synthesis of Compound C66-8

Compound C66-7 (0.4 g, 0.8 mmol) was dissolved in 20 mL of methanol. CeCl₃·7H₂O (148 mg, 0.4 mmol) and sodium borohydride (45.6 mg, 1.2 mmol) were added slowly successively under nitrogen protection. The mixture was stirred at room temperature for 20 min. After the starting materials were reacted completely, aqueous ammonium chloride solution was added, and the mixture was extracted three times with ethyl acetate. The organic phases were combined, dried over anhydrous sodium sulfate, concentrated under reduced pressure, and purified to afford the product (0.38 g).

### 8. Synthesis of Compound C66-9

Compound C66-8 (320 mg, 0.64 mmol) was dissolved in 25 mL of methanol. 4N HCl/MeOH (5 mL) was added slowly at room temperature, and the mixture was stirred and reacted for 1 h until the starting materials were reacted completely. The reaction solution was concentrated to dryness. Water (8 mL) was added, and the aqueous phase was adjusted to pH 12 with 10% aqueous ammonia, and then extracted three times with ethyl acetate. The organic phases were combined, dried over anhydrous sodium sulfate and concentrated under reduced pressure to afford the product (250 mg).

### 9. Synthesis of Compound C66-10

Compound C66-9 (80 mg, 0.2 mmol) was dissolved in dimethyl sulfoxide (5 mL). Tert-butyl 6-chloro-3-fluoropyridin-2-carboxylate (56 mg, 0.24 mmol) and N,N-diisopropylethylamine (129 mg, 1 mmol) were added at 25 °C. The mixture was stirred and reacted at 100 °C for 10 h. After the reaction was completed, saturated brine was added, and the mixture was extracted three times with ethyl acetate, dried by rotary evaporation, and purified by column chromatography to afford the product (40 mg).

¹H NMR (400 MHz, Chloroform-d) δ 8.31 (br, 1H), 8.08 (s, 1H), 7.51 (s,1H), 7.31 (d, *J =* 8.4 Hz, 2H), 7.09 (d, *J =* 8.8 Hz, 2H), 6.93 (d, *J =* 8.4 Hz, 2H), 6.63 (d, *J =* 8.8 Hz, 1H), 5.01~4.96 (m,1H), 3.73~3.67 (m, 2H), 3.54~3.50 (m, 2H), 2.82~2.72 (m, 4H), 1.69~1.65 (m,12H).

### 10. Synthesis of Compound C66-11

Compound C66-10 (240 mg, 0.4 mmol) was dissolved in 1,2-dichloroethane (24 mL). 1-Chloroethyl chloroformate (112 mg, 0.8 mmol) was added under nitrogen protection at room temperature. The mixture was stirred at 83 °C for 2 h. After the reaction was completed, the mixture was dried by rotary evaporation and used directly in the next step. The crude product in the previous step was dissolved in methanol (24 mL) and the reaction was carried out at 65 °C for 1 h under nitrogen protection. After the reaction was completed, the mixture was basified with triethylamine and dried by rotary evaporation for use in the next reaction step.

### 11. Synthesis of Compound C66-12

Compound C66-11 (19.2 mg, 0.04 mmol) was dissolved in 5 mL of 1,2-dichloroethane. Triethylamine (60 mg, 0.6 mmol) was added at room temperature, followed by slow dropwise addition of 4-morpholinosulfonyl chloride (74.4 mg, 0.4 mmol). After completion of the addition, the mixture was stirred and reacted at 30 °C for 12 h. After the starting materials were reacted completely, the reaction was quenched with aqueous sodium bicarbonate solution, and the reaction solution was extracted three times with dichloromethane. The organic phases were combined, washed with 10% aqueous citric acid solution once and saturated brine once successively, dried, concentrated, and purified by preparative TLC to afford the product (8 mg).

### 12. Synthesis of Compound C66

Compound C66-12 (40 mg, 0.06 mmol) was dissolved in dichloromethane (6 mL)/trifluoroacetic acid (3 mL). The mixture was stirred at 30 °C for 12 h under nitrogen protection. After the reaction was completed, the mixture was dried by rotary evaporation and purified by preparative thin-layer chromatography to afford the product (5.64 mg). [M+H]⁺: 583.2

¹HNMR (DMSO-d₆, 400Hz), δ 9.74 (br,1H), 7.86~7.81 (m,2H), 7.45~7.25 (m,2H), 5.21~5.19 (m,1H), 4.55~4.39 (m,2H), 3.53~3.50 (m,6H), 3.14~3.10 (m,4H), 2.57~2.50 (m,2H), 1.64 (d, J=2.4 Hz,3H).

### Example 12

Compounds of the present invention

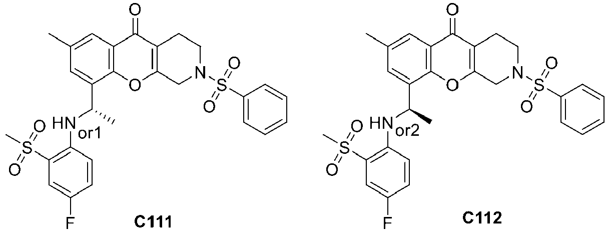

The synthetic route and experimental procedure were as follows:

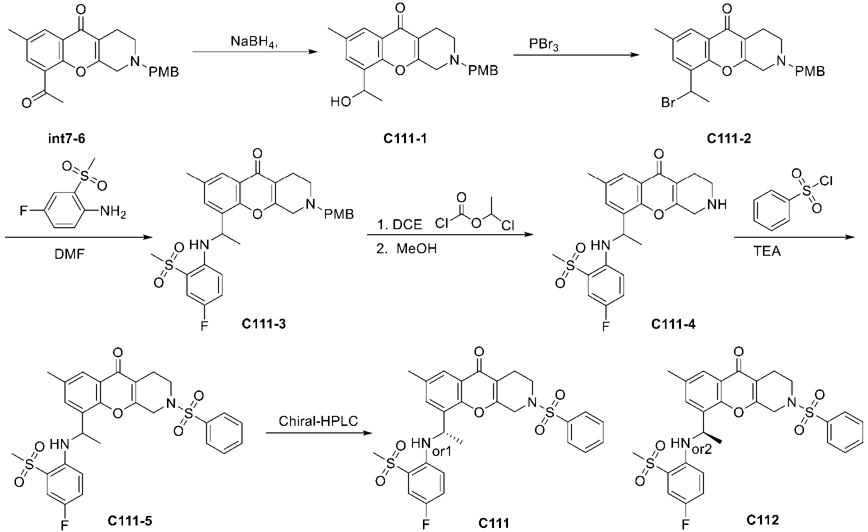

### 1. Synthesis of Compound C111-1

Compound int7-6 (0.5 g, 1.33 mmol) was dissolved in a mixed solvent of 10 mL dichloromethane and 10 mL methanol. Sodium borohydride (50 mg, 1.33 mmol) was added slowly under nitrogen protection, and the reaction solution was stirred at room temperature for 10 min. After the starting materials were reacted completely, the mixture was treated and purified to afford the product (0.3 g).

### 2. Synthesis of Compound C111-2

Compound C111-1 (0.3 g, 0.79 mmol) was dissolved in 20 mL anhydrous dichloromethane. Phosphorus tribromide (0.64 g, 2.38 mmol) was added slowly under nitrogen protection, and the mixture was stirred at room temperature for 12 h. After the starting materials were reacted completely, the mixture was treated to afford the product (0.7 g).

### 3. Synthesis of Compound C111-3

Compound C111-2 (0.7 g, 0.79 mmol) was dissolved in 20 mL anhydrous N,N-dimethylformamide. *Tert*-butyl 2-aminobenzoate (0.89 g, 4.74 mmol) was added slowly at room temperature. The mixture was slowly heated to 60 °C, stirred and reacted for 36 h. After the starting materials were reacted completely, the mixture was treated and purified to afford the product (120 mg).

### 4. Synthesis of Compound C111-4

Compound C111-3 (90 mg, 1.65 mmol) was dissolved in 5 mL 1,2-dichloroethane. 1-Chloroethyl chloroformate (47.1 mg, 3.3 mmol) was added slowly at room temperature. The mixture was slowly heated to 83 °C, stirred and reacted for 3 h. After the starting materials were reacted completely, the mixture was treated and purified to afford the product (51 mg).

### 5. Synthesis of Compound C111-5

Compound C111-4 (51 mg, 0.11 mmol) was dissolved in 5 mL dichloromethane. Triethylamine (56 mg, 0.55 mmol) was added at 0 °C, followed by slow dropwise addition of phenylsulfonyl chloride (37 mg, 0.26 mmol). After completion of the addition, the mixture was allowed to warm to room temperature naturally, stirred and reacted for 12 h. After the reaction was completed, the mixture was treated and purified to afford the product (28 mg).

### 6. Synthesis of Compounds C111 and C112

Compound C111-5 (28 mg, 0.05 mmol) was separated by the following chiral resolution method: Instrument: Agilent 1260 InfinityII, chromatographic column: Phenomenex Lux^{®} 5 µm cellulose-4 (250 × 21.2 mm), mobile phase: *n*-hexane : ethanol = 1:9, column temperature: 25 °C.

C111 (9.5 min, 10.95 mg) [M+H]⁺: 571.3 HNMR: (DMSO-d₆, 400Hz), *δ* 7.89~7.87 (m,2H), 7.71~7.68 (m,2H), 7.64~7.60 (m,2H), 7.43~7.41 (m,1H), 7.29~7.25 (m,1H) , 6.74~6.70 (m,1H), 6.60~6.58 (m,1H), 5.13~5.10 (m,1H), 4.31~4.24 (m,2H), 3.44~3.39 (m,1H), 3.28~3.21 (m,5H), 2.35 (s,3H), 1.62 (d,*J*=7.8Hz,3H).

C112 (10.2 min, 11.94 mg) [M+H]⁺: 571.3 ¹HNMR: (DMSO-d₆, 400Hz), *δ* 7.89~7.87 (m,2H), 7.71~7.68 (m,2H), 7.64~7.60 (m,2H), 7.43~7.41 (m,1H), 7.29~7.25 (m,1H), 6.74~.6.70 (m,1H), 6.60~6.58 (m,1H), 5.13~5.10 (m,1H), 4.31~4.24 (m,2H), 3.44~3.39 (m,1H), 3.28~3.21 (m,5H), 2.35 (s,3H), 1.62 (d,*J*=7.8Hz,3H).

### Example 13

Compounds of the present invention

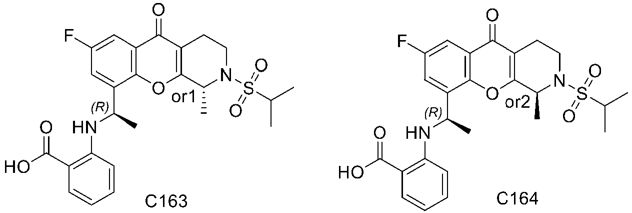

The synthetic route and experimental procedure were as follows:

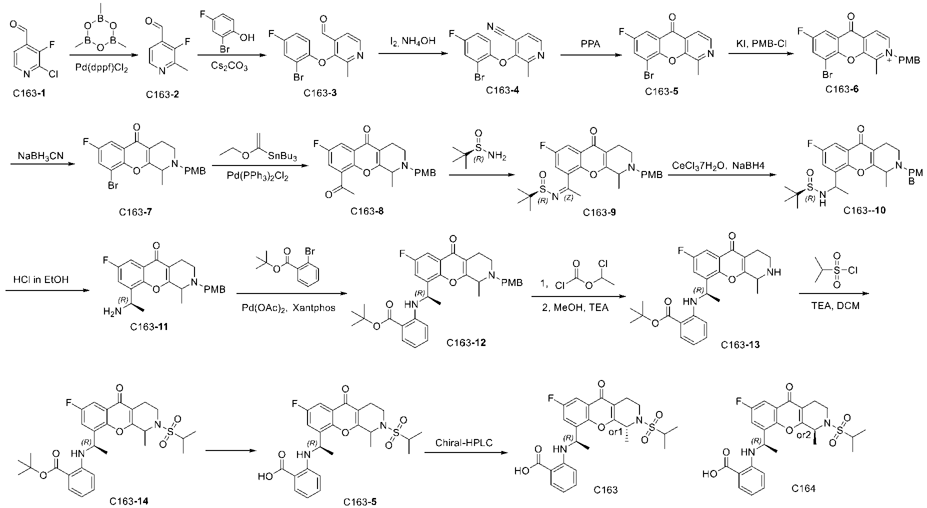

### 1. Synthesis of Compound C163-2

C163-1 (1.01 g, 2.65 mmol) was dissolved in 30 mL of 1,4-dioxane. A solution of trimethylboroxine in THF (3.5 N, 5.4 mL), potassium carbonate (2.62 g, 7.95 mmol) and DPPF-PdCl₂ (463 mg, 0.265 mmol) were added. The mixture was heated to 105 °C and reacted overnight. After the reaction was completed, the mixture was purified by column chromatography to afford the product (236 mg).

¹H NMR (400 MHz, Chloroform-d) δ 10.45 (s, 1H), 8.50 (d, J = 4.9 Hz, 1H), 7.53 (t, J = 4.9 Hz, 1H), 2.65 (d, J = 3.2 Hz, 3H).

### 2. Synthesis of Compound C163-3

Compound C163-2 (54 mg, 0.39 mmol) was dissolved in 1.5 mL of N,N-dimethylformamide. 2-Bromo-4-fluorophenol (112 mg, 0.59 mmol) and cesium carbonate (198 mg, 0.78 mmol) were added. The mixture was heated to 100 °C and reacted for 3 h. After the reaction was completed, the mixture was purified by column chromatography to afford the product (24 mg).

### 3. Synthesis of Compound C163-4

C163-3 (24 mg, 0.078 mmol) was dissolved in a mixed solvent of 0.5 mL of tetrahydrofuran and 0.5 mL of aqueous ammonia. Iodine (29.7 mg, 0.117 mmol) was added, and the reaction was carried out at room temperature overnight under nitrogen protection. After the reaction was completed, the mixture was treated to afford the product (11 mg).

¹H NMR (400 MHz, Chloroform-d) δ 8.55 (d, J = 5.0 Hz, 1H), 7.52 - 7.40 (m, 2H), 6.98 (ddd, J = 9.1, 7.5, 3.0 Hz, 1H), 6.54 (dd, J = 9.0, 4.6 Hz, 1H), 2.52 (s, 3H).

### 4. Synthesis of Compound C163-5

C163-4 (4.4 g) was added to a reaction flask, and polyphosphoric acid (50 g) was added. The mixture was heated to 220 °C. After the reaction was completed, the mixture was cooled, quenched with aqueous sodium bicarbonate solution, basified, extracted three times with dichloromethane, dried and concentrated to give a crude product (3.6 g), which was used directly in the next reaction step.

### 5. Synthesis of Compound C163-6

C163-5 (3.6 g, 11.7 mmol) was dissolved in acetonitrile. Potassium iodide (4.28 g, 25.8 mmol) and *p*-methoxybenzyl chloride (3.67 g, 23.5 mmol) were added. The reaction was carried out at 80 °C for 1 h. After the reaction was completed, the mixture was directly dried by rotary evaporation and used directly in the next reaction step.

### 6. Synthesis of Compound C163-7

C163-6 was dissolved in ethanol (150 mL). Acetic acid (211 mg, 3.5 mmol) and sodium cyanoborohydride (887 mg, 14.1 mmol) were added, and the mixture was reacted at room temperature for 10 min. After the reaction was completed, the mixture was purified by column chromatography to afford the product (1.3 g).

### 7. Synthesis of Compound C163-8

C163-7 (650 mg, 1.51 mmol) was dissolved in 1,4-dioxane (10 mL). Tributyl(1-ethoxyvinyl)tin (653.3 mg, 1.81 mmol) and Pd(PPh₃)₂Cl₂ (106 mg, 0.15 mmol) were added. The reaction was carried out at 80 °C overnight under nitrogen protection. After the reaction was completed, the mixture was purified by column chromatography to afford the product (540 mg).

### 8. Synthesis of Compound C163-9

C163-8 (540 mg, 1.37 mmol) was dissolved in tetrahydrofuran (20 mL). (R)-*tert*-butyl sulfinamide (331 mg, 2.73 mmol) and tetraethyl titanate (1.25 g, 5.47 mmol) were added. The reaction solution was reacted at 70 °C overnight under nitrogen protection. After the reaction was completed, the mixture was purified by column chromatography to afford the product (630 mg).

### 9. Synthesis of Compound C163-10

C163-9 (630 mg, 1.27 mmol) was dissolved in methanol (10 mL). Cerium chloride heptahydrate (236 mg, 0.63 mmol) was added, and the mixture was stirred for 5 min. Sodium borohydride (72 mg, 1.90 mmol) was added in batches. After the reaction was completed, the mixture was purified by column chromatography to afford the product (540 mg).

### 10. Synthesis of Compound C163-11

C163-10 (540 mg) was dissolved in ethanol (5 mL). A solution of HCl in ethanol (2 M, 5 mL) was added. The mixture was reacted at room temperature for 10 min. After the reaction was completed, the mixture was purified by column chromatography to afford the product (450 mg).

### 11. Synthesis of Compound C163-12

C163-11 (450 mg, 1.14 mmol) was dissolved in toluene. *Tert*-Butyl 2-bromobenzoate (350 mg, 1.36 mmol), Pd(OAc)₂ (25.5 mg, 0.11 mmol), Xantphos (131.6 mg, 0.23 mmol) and Cs₂CO₃ (1.00 g, 3.10 mmol) were added. The reaction was carried out at 100 °C overnight under nitrogen protection. After the reaction was completed, the mixture was purified by column chromatography to afford the product (500 mg).

### 12. Synthesis of Compound C163-13

C163-12 (500 mg, 0.87 mmol) was dissolved in 1,2-dichloroethane. 1-Chloroethyl chloroformate (250 mg, 1.75 mmol) was added, and the reaction was carried out at 80 °C for 1 h. The mixture was concentrated, and methanol and triethylamine (353 mg, 3.50 mmol) were added. The mixture was heated to 65 °C and reacted for 1 h. After the reaction was completed, the mixture was purified by column chromatography to afford the product (300 mg).

### 13. Synthesis of Compound C163-14

C163-13 (150 mg, 0.33 mmol) was dissolved in dichloromethane. DBU (252 mg, 1.66 mmol) and isopropylsulfonyl chloride (142 mg, 1.00 mmol) were added. The reaction was carried out at room temperature for 1 h. After the reaction was completed, the mixture was purified by column chromatography to afford the product (100 mg).

### 14. Synthesis of Compounds C163 and C164

C163-14 was dissolved in dichloromethane (4 mL), and trifluoroacetic acid (4 mL) was added. The reaction was carried out at room temperature overnight. After the reaction was completed, the mixture was concentrated and separated by the following chiral resolution method: Instrument: Agilent 1260 InfinityII, chromatographic column: Phenomenex Lux^{®} 5 µm cellulose-4 (250 × 21.2 mm), mobile phase: n-hexane : ethanol = 3:7, column temperature: 25 °C.

C163 (11.2 min, 23.26 mg) [M+H]⁺: 502.6 ¹H NMR (400 MHz, DMSO-d6) δ 8.41 (br, 1H), 7.84 (dd, J = 8.0, 1.6 Hz, 1H), 7.62-7.60 (m, 1H), 7.51-7.48 (m, 1H), 7.25 - 7.20 (m, 1H), 6.58 (t, J = 7.6 Hz, 1H), 6.50 (d, J = 8.4 Hz, 1H), 5.27 (br, 1H), 4.91 (q, J = 6.8 Hz, 1H), 3.87-3.82 (m, 1H), 3.49 - 3.46 (m, 1H), 2.66 - 2.54 (m, 2H), 1.62 (d, J = 6.8 Hz, 6H), 1.28-1.22 (m, 8H).

C164 (13.4 min, 23.60 mg) [M+H]⁺: 502.6 ¹H NMR (400 MHz, DMSO-d6) δ 8.48 (br, 1H), 7.81 (dd, J = 8.0, 1.6 Hz, 1H), 7.66-7.60 (m, 2H), 7.25 - 7.20 (m, 1H), 6.56 (t, J = 7.6 Hz, 1H), 6.50 (d, J = 8.4 Hz, 1H), 5.21-5.18 (m, 1H), 4.87 (q, J = 6.8 Hz, 1H), 3.87-3.82 (m, 1H), 3.47 - 3.43 (m, 1H), 2.68 - 2.52 (m, 2H), 1.66-1.63 (m, 6H), 1.23 (dd, J = 20.0, 6.8 Hz, 8H).

### Example 14

Compounds of the present invention

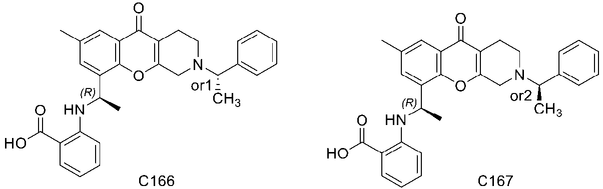

The synthetic route and experimental procedure were as follows:

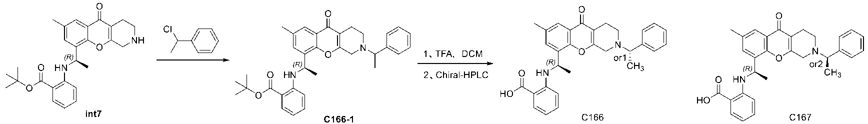

### 1. Synthesis of Compound C166-1

Intermediate int7 (200 mg, 0.46 mmol) was dissolved in 5 mL of N,N-dimethylformamide. Methylbenzyl chloride (189 mg, 1.34 mmol), triethylamine (270 mg, 2.67 mmol) and potassium iodide (120 mg, 0.72 mmol) were added. The reaction was carried out at 80 °C in a sealed tube overnight. After the reaction was completed, the mixture was purified by column chromatography to afford the product (183 mg).

### 2. Synthesis of Compounds C 166 and C 167

C166-1 (50 mg) was dissolved in dichloromethane (10 mL), and trifluoroacetic acid (10 mL) was added. The reaction was carried out at room temperature overnight. After the reaction was completed, the mixture was concentrated and separated by the following chiral resolution method: Instrument: Agilent 1260 InfinityII, chromatographic column: Phenomenex Lux^{®} 5 µm cellulose-4 (250 × 21.2 mm), mobile phase: n-hexane : ethanol = 95:5, column temperature: 25 °C.

C166 (10.4 min, 19.28 mg) [M+H]⁺: 483.3 ¹H NMR (400 MHz, DMSO-d6) δ 12.78 (br, 1H), 8.42 (d, J = 7.0 Hz, 1H), 7.82 (dd, J = 7.9, 1.7 Hz, 1H), 7.73 (d, J = 2.1 Hz, 1H), 7.66 - 7.31 (m, 6H), 7.24-7.17 (m, 1H), 6.55 (t, J = 7.5 Hz, 1H), 6.46 (d, J = 8.5 Hz, 1H), 5.15 (t, J = 6.6 Hz, 1H), 4.75-4.04(m,3H),3.28-3.16 (m, 2H), 2.76-2.57 (m, 2H), 2.35 (s, 3H), 1.68 (s, 3H), 1.59 (d, J = 6.6 Hz, 3H).

C167 (13.6 min, 19.52 mg) [M+H]⁺: 483.3 ¹H NMR (400 MHz, DMSO-d6) δ 12.79 (br, 1H), 8.42 (d, J = 6.9 Hz, 1H), 7.82 (dd, J = 8.0, 1.7 Hz, 1H), 7.73 (d, J = 2.1 Hz, 1H), 7.66 - 7.37 (m, 6H), 7.25-7.17 (m, 1H), 6.56 (t, J = 7.5 Hz, 1H), 6.47 (d, J = 8.5 Hz, 1H), 5.17 (t, J = 6.7 Hz, 1H), 4.87-4.02 (m, 3H), 3.27-2.89 (m, 2H), 2.82 - 2.56 (m, 2H), 2.35 (s, 3H), 1.69 (s, 3H), 1.59 (d, J = 6.7 Hz, 3H).

### Example 15

Compounds of the present invention

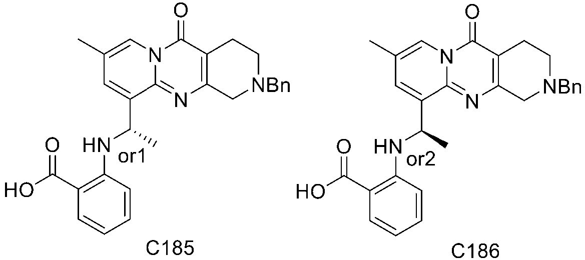

The synthetic route and experimental procedure were as follows:

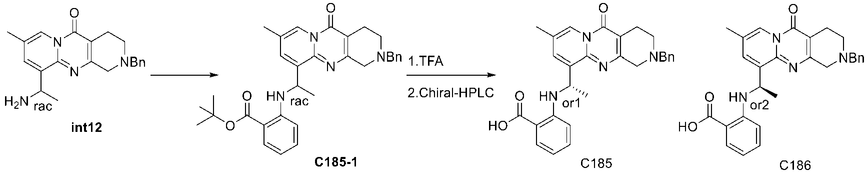

### 1. Synthesis of Compound C185-1

Compound int-12 (2.1 g, 6.03 mmol), tert-butyl 2-bromobenzoate (1.86 g, 7.24 mmol), Pd(OAc)₂ (134 mg, 0.6 mmol), Xantphos (694 mg, 1.2 mmol) and cesium carbonate (5.9 g, 18.9 mmol) were dissolved in dry toluene (21 mL). The reaction was carried out at 100 °C for 12 h. After the reaction was completed, the mixture was filtered, concentrated and purified by column chromatography to afford the product (2.4 g).

### 2. Synthesis of C185 and C186

Compound C185-1 (100 mg) was dissolved in dichloromethane (3 mL)/trifluoroacetic acid (3 mL). The mixture was stirred at 30 °C for 12 h under nitrogen protection. After the reaction was completed, the mixture was purified by column chromatography to give a crude product (60 mg), which was separated by the following chiral resolution method: Instrument: Agilent 1260 InfinityII, chromatographic column: Phenomenex Lux^{®} 5 µm Amylose-1 (250 × 21.2 mm), mobile phase: *n-*hexane : ethanol = 9:1, column temperature: 25 °C.

C185 (18.7 min, 15 mg) ¹H NMR (400 MHz, DMSO-d6) δ 8.63 (s, 1H), 7.80 (d, J = 7.8 Hz, 1H), 7.59 (d, J = 3.3 Hz, 1H), 7.43 - 7.32 (m, 4H), 7.32 - 7.23 (m, 1H), 7.23 - 7.09 (m, 1H), 6.52 (d, J = 3.5 Hz, 1H), 6.30 (d, J = 8.5 Hz, 1H), 5.30 (d, J = 6.6 Hz, 1H), 3.73 (s, 2H), 3.58 (s, 2H), 3.14 (d, J = 3.4 Hz, 3H), 2.82 - 2.73 (m, 2H), 2.68 (d, J = 5.6 Hz, 2H), 2.28 (s, 3H), 1.54 (d, J = 6.6 Hz, 3H).

C186 (22 min, 5 mg) ¹H NMR (400 MHz, DMSO-d6) δ 8.64 (s, 1H), 8.45 (d, J = 6.5 Hz, 1H), 7.80 (d, J = 7.9 Hz, 1H), 7.61 (d, J = 2.1 Hz, 1H), 7.46 - 7.32 (m, 4H), 7.29 (t, J = 6.9 Hz, 1H), 7.17 (t, J = 7.8 Hz, 1H), 6.53 (t, J = 7.5 Hz, 1H), 6.30 (d, J = 8.5 Hz, 1H), 5.30 (q, J = 6.1 Hz, 1H), 3.73 (s, 2H), 3.57 (s, 2H), 2.75 (q, J = 5.7 Hz, 2H), 2.66 (t, J = 5.8 Hz, 2H), 2.30 (s, 3H), 1.54 (d, J = 6.6 Hz, 3H).

The following compounds were synthesized by similar procedures as described above:

| No. | Compound | ¹H NMR | MS (M+1)⁺ |
|---|---|---|---|
| C7 | 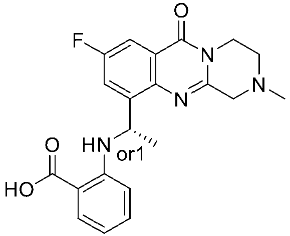 | ¹H NMR (400 MHz, DMSO-*d*₆) δ ppm 12.79 (br, 1H), 8.50 (d, *J =* 6.8 Hz, 1H), 7.81 (dd, *J* = 8.0*,* 1.6 Hz, 1H), 7.73 (dd, *J* = 8.4, 3.2 Hz, 1H), 7.60 (dd, *J* = 9.6, 3.2 Hz, 1H), 7.21 - 7.13 (m, 1H), 6.58 - 6.50 (m, 1H), 6.35 (d, *J* = 8.0 Hz, 1H), 5.47 - 5.37 (m, 1H), 4.32 (s, 2H), 4.10 (s, 2H), 3.55 - 3.49 (m, 2H), 2.83 (s, 3H), 1.58 (d, *J =* 6.8 Hz, 3H). | 397.16 |
| C8 | 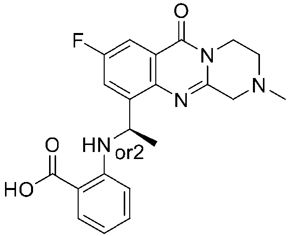 | ¹H NMR (400 MHz, DMSO-*d*₆) δ ppm 12.79 (br, 1H), 8.50 (d, *J* = 7.2 Hz, 1H), 7.81 (dd, *J* = 7.6*,* 1.6 Hz, 1H), 7.73 (dd, *J* = 8.4, 3.2 Hz, 1H), 7.60 (dd, *J* = 9.6, 3.2 Hz, 1H), 7.22 - 7.13 (m, 1H), 6.58 - 6.49 (m, 1H), 6.35 (d, *J* = 8.0 Hz, 1H), 5.49 - 5.37 (m, 1H), 4.28 (s, 2H), 4.09 (s, 2H), 3.53 - 3.46 (m, 2H), 2.79 (s, 3H), 1.58 (d, *J* = 6.8 Hz, 3H). | 397.16 |
| C15 | 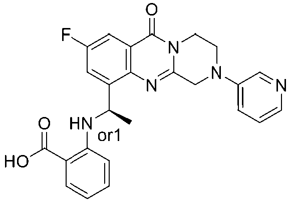 | ¹H NMR (400 MHz, DMSO-*d*₆) δ ppm 8.66 (br, 1H), 8.42 (s, 1H), 8.07 (d, *J* = 4.0 Hz, 1H), 7.82 (d, *J* = 7.6 Hz, 1H), 7.70 (d, *J* = 8.0 Hz, 1H), 7.58 (s, 1H), 7.47 (s, 1H), 7.31 (s, 1H), 7.20 - 7.06 (m, 1H), 6.45 (d, *J =* 40.0 Hz, 2H), 6.57 - 5.39 (m, 1H), 4.68 (s, 2H), 4.28 - 4.14 (m, 2H), 3.88 - 3.75 (m, 2H), 1.60 (d, *J* = 6.4 Hz, 3H). | 460.17 |
| C16 | 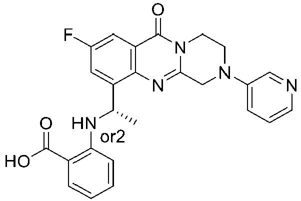 | ¹H NMR (400 MHz, DMSO-*d*₆) δ ppm 8.59 (br, 1H), 8.43 (s, 1H), 8.06 (d, *J* = 4.0 Hz, 1H), 7.82 (d, *J* = 7.6 Hz, 1H), 7.70 (d, *J* = 8.4 Hz, 1H), 7.59 (s, 1H), 7.47 (s, 1H), 7.31 (s, 1H), 7.22 - 7.10(m, 1H), 6.46 (d, *J* = 32.8 Hz, 2H), 5.48 (s, 1H), 4.68 (s, 2H), 4.30 - 4.14 (m, 2H), 3.87 - 3.76 (m, 2H), 1.61 (d, *J* = 6.8 Hz, 3H). | 460.17 |
| C17 | 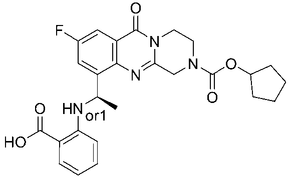 | ¹H NMR (400 MHz, DMSO-*d*₆) δ ppm 12.76 (br, 1H), 8.50 (s, 1H), 7.81 (dd, *J =* 8.0, 1.6 Hz, 1H), 7.70 (dd, *J =* 8.4, 3.2 Hz, 1H), 7.58 - 7.50 (m, 1H), 7.21 - 7.12 (m, 1H), 6.57 - 6.49 (m, 1H), 6.35 (d, *J =* 8.4 Hz, 1H), 5.54 - 5.38 (m, 1H), 5.10 - 5.04 (m, 1H), 4.74 - 4.60 (m, 2H), 4.23 - 4.08 (m, 2H), 3.83 - 3.67 (m, 2H), 1.88 - 1.76 (m, 2H), 1.75- 164 (m, 4H), 1.58 (d, *J =* 6.8 Hz, 5H). | 495.20 |
| C18 | 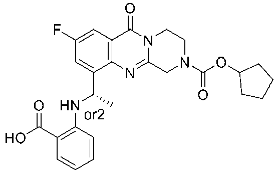 | ¹H NMR (400 MHz, DMSO-*d*₆) δ ppm 12.76 (br, 1H), 8.51 (s, 1H), 7.81 (dd, *J =* 8.0, 1.6 Hz, 1H), 7.70 (dd, *J =* 8.4, 3.2 Hz, 1H), 7.63 - 7.46 (m, 1H), 7.25 - 7.10 (m, 1H), 6.53 (t, *J =* 8.0 Hz, 1H), 6.35 (d, *J =* 8.4 Hz, 1H), 5.55 - 5.39 (m, 1H), 5.15 - 5.00 (m, 1H), 4.77 - 4.57 (m, 2H), 4.27 - 4.07 (m, 2H), 3.88 - 3.67 (m, 2H), 1.88 - 1.76 (m, 2H), 1.75- 164 (m, 4H),1.58 (d, *J =* 6.8 Hz, 5H). | 495.20 |
| C21 | 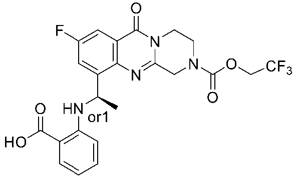 | ¹H NMR (400 MHz, DMSO-*d*₆) δ ppm 12.77 (br, 1H), 8.51 (s, 1H), 7.81 (dd, *J* = 8.0, 2.0 Hz, 1H), 7.72 (dd, *J* = 8.4, 3.2 Hz, 1H), 7.62 - 7.49 (m, 1H), 7.17 (t, *J* = 8.0 Hz, 1H), 6.53 (t, *J* = 7.6 Hz, 1H), 6.43 - 6.31 (m, 1H), 5.53 - 5.40 (m, 1H), 4.88 - 4.66 (m, 4H), 4.33 - 4.10 (m, 2H), 3.90 - 3.72 (m, 2H), 1.58 (d, *J =* 6.8 Hz, 3H). | 509.14 |
| C22 | 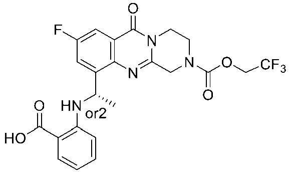 | ¹H NMR (400 MHz, DMSO-*d*₆) δ ppm 12.77 (br, 1H), 8.51 (s, 1H), 7.86 - 7.78 (m, 1H), 7.71 (dd, *J* = 8.0, 3.2 Hz, 1H), 7.62 - 7.49 (m, 1H), 7.17 (t, *J* = 7.6 Hz, 1H), 6.53 (t, *J* = 7.6 Hz, 1H), 6.43 - 6.30 (m, 1H), 5.53 - 5.39 (m, 1H), 4.95 - 4.66 (m, 4H), 4.36 - 4.13 (m, 2H), 3.92 - 3.73 (m, 2H), 1.58 (d, *J =* 6.4 Hz, 3H). | 509.14 |
| C23 | 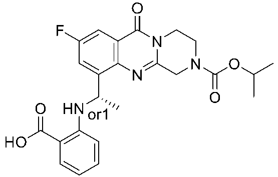 | ¹H NMR (400 MHz, DMSO-*d*₆) δ ppm 12.77 (br, 1H), 8.47 (s, 1H), 7.80 (dd, *J =* 8.0, 1.6 Hz, 1H), 7.70 (dd, *J =* 8.4, 3.2 Hz, 1H), 7.60 - 7.48 (m, 1H), 7.23 - 7.12 (m, 1H), 6.53 (t, *J =* 7.6 Hz, 1H), 6.36 (d, *J =* 8.8 Hz, 1H), 5.52 - 5.38 (m, 1H), 4.94 - 4.80 (m, 1H), 4.76 - 4.60 (m, 2H), 4.25 - 4.08 (m, 2H), 3.83 - 3.68 (m, 2H), 1.58 (d, *J* = 6.8 Hz, 3H), 1.58 (d, *J* = 6.0 Hz, 6H). | 469.18 |
| C24 | 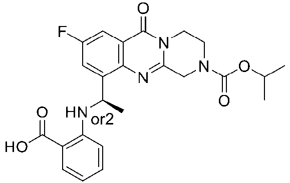 | ¹H NMR (400 MHz, DMSO-*d*₆) δ ppm 12.77 (br, 1H), 8.47 (s, 1H), 7.80 (dd, *J* = 8.0, 1.6 Hz, 1H), 7.70 (dd, *J* = 8.4, 3.2 Hz, 1H), 7.60 - 7.48 (m, 1H), 7.25 - 7.09 (m, 1H), 6.53 (t, *J* = 8.0 Hz, 1H), 6.36 (d, *J* = 8.4 Hz, 1H), 5.53 - 5.40 (m, 1H), 4.94 - 4.80 (m, 1H), 4.76 - 4.61 (m, 2H), 4.25 - 4.08 (m, 2H), 3.85 - 3.68 (m, 2H), 1.58 (d, *J =* 6.4 Hz, 3H), 1.58 (d, *J =* 6.4 Hz, 6H). | 469.18 |
| C25 | 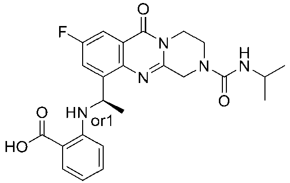 | ¹H NMR (400 MHz, DMSO-*d*₆) δ ppm 12.78 (br, 1H), 8.51 - 8.38 (m, 1H), 7.81 (dd, *J* = 8.0, 1.6 Hz, 1H), 7.70 (dd, *J* = 8.4, 2.8 Hz, 1H), 7.53 (dd, *J* = 9.6, 3.2 Hz, 1H), 7.23 - 7.13 (m, 1H), 6.59 - 6.44 (m, 2H), 6.33 (d, *J* = 8.4 Hz, 1H), 5.54 - 5.41 (m, 1H), 4.78 - 4.61 (m, 2H), 4.11 (t, *J* = 5.6 Hz, 2H), 3.89 - 3.65 (m, 3H), 1.58 (d, *J* = 6.8 Hz, 3H), 1.11 (d, *J =* 6.4 Hz, 6H). | 468.20 |
| C26 | 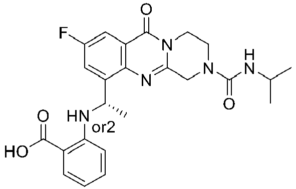 | ¹H NMR (400 MHz, DMSO-*d*₆) δ ppm 12.79 (br, 1H), 8.46 (d, *J* = 7.6 Hz, 1H), 7.81 (dd, *J* = 8.0*,* 1.6 Hz, 1H), 7.70 (dd, *J* = 8.0, 2.8 Hz, 1H), 7.53 (dd, *J =* 8.4, 3.6 Hz, 1H), 7.22 - 7.13 (m, 1H), 6.57 - 6.46 (m, 2H), 6.33 (d, *J* = 8.4 Hz, 1H), 5.51 - 5.43 (m, 1H), 4.76 - 4.60 (m, 2H), 4.11 (t, *J* = 5.6 Hz, 2H), 3.88 - 3.64 (m, 3H), 1.58 (d, *J* = 6.8 Hz, 3H), 1.11 (d, *J* = 6.4 Hz, 6H). | 468.20 |
| C27 | 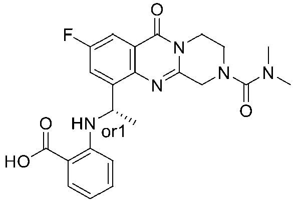 | ¹H NMR (400 MHz, CDCl₃) δ ppm 8.30 (br, 1H), 7.99 (dd, *J* = 8.0*,* 1.6 Hz, 1H), 7.79 (dd, *J =* 8.0, 2.8 Hz, 1H), 7.50 (dd, *J* = 9.6, 3.2 Hz, 1H), 7.25 - 7.12 (m , 1H), 6.64 - 6.54 (m, 1H), 6.38 (d, *J=* 8.8 Hz, 1H), 5.56 - 5.45(q, *J =* 6.8 Hz, 1H), 4.65 - 4.52 (m, 2H), 4.22 (t, *J =* 6.0 Hz, 2H), 3.78 - 3.65 (m, 2H), 2.99 (s, 6H), 1.66 (d, *J =* 6.8 Hz, 3H). | 454.18 |
| C28 | 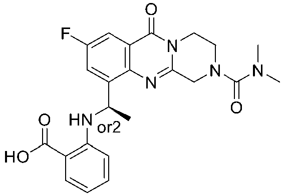 | ¹H NMR (400 MHz, CDCl₃) δ ppm 8.30 (br, 1H), 8.00 (dd, *J =* 8.0, 1.6 Hz, 1H), 7.79 (dd, *J =* 8.0, 2.8 Hz, 1H), 7.50 (dd, *J* = 9.6, 3.2 Hz, 1H), 7.23 - 7.15 (m , 1H), 6.59 (t, *J =* 8.0 Hz, 1H), 6.39 (d, *J* = 8.4 Hz, 1H), 5.58 - 5.45(m, 1H), 4.66 - 4.50 (m, 2H), 4.22 (t, *J* = 6.0 Hz, 2H), 3.78 - 3.65 (m, 2H), 2.98 (s, 6H), 1.66 (d, *J =* 6.8 Hz, 3H). | 454.18 |
| C29 | 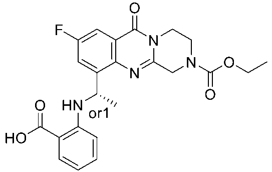 | ¹H NMR (400 MHz, DMSO-*d*₆) δ ppm 12.73 (br, 1H), 8.51 (s, 1H), 7.81 (dd, *J =* 8.0, 1.6 Hz, 1H), 7.70 (dd, *J =* 8.4, 3.2 Hz, 1H), 7.55 (dd, *J =* 9.6, 3.2 Hz, 1H), 7.20 - 7.17 (m, 1H), 6.57 - 6.47 (m, 1H), 6.36 (d, *J =* 8.4 Hz, 1H), 5.51 - 5.38 (m, 1H), 4.70 (s, 2H), 4.25 - 4.04 (m, 4H), 3.83 -3.68 (m, 2H), 1.58 (d, *J =* 6.8 Hz, 3H), 1.24 (t, *J =* 7.2 Hz, 3H). | 455.17 |
| C30 | 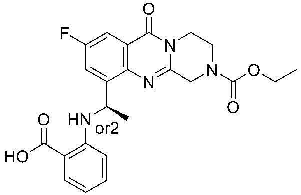 | ¹H NMR (400 MHz, DMSO-*d*₆) δ ppm 12.65 (br, 1H), 8.52 (s, 1H), 7.81 (dd, *J* = 8.0, 1.6 Hz, 1H), 7.70 (dd, *J =* 8.0, 2.8 Hz, 1H), 7.55 (dd, *J* = 9.2, 3.2 Hz, 1H), 7.23 - 7.07 (m, 1H), 6.58 - 6.47 (m, 1H), 6.36 (d, *J* = 8.4 Hz, 1H), 5.51 - 5.39 (m, 1H), 4.70 (s, 2H), 4.24 - 4.07 (m, 4H), 3.83 -3.69 (m, 2H), 1.58 (d, *J =* 6.8 Hz, 3H), 1.25 (t, *J =* 7.2 Hz, 3H). | 455.17 |
| C31 | 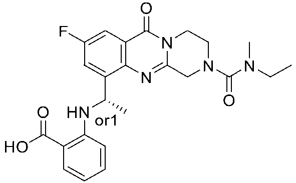 | ¹H NMR (400 MHz, CDCl₃) δ ppm 8.32 (br, 1H), 8.01 (dd, *J =* 8.4, 2.0 Hz, 1H), 7.79 (dd, *J =* 8.0, 2.8 Hz, 1H), 7.50 (dd, *J =* 9.2, 3.2 Hz, 1H), 7.24 - 7.14 (m, 1H), 6.59 (t, *J =* 7.6 Hz, 1H), 6.37 (d, *J =* 8.4 Hz, 1H), 5.53 (q, *J =* 6.4 Hz, 1H), 4.65 - 4.84 (m, 2H), 4.22 (t, *J =* 6.0 Hz, 2H), 3.77 - 3.65 (m, 2H), 3.35 (q, *J* = 7.2 Hz, 2H), 2.97 (s, 3H), 1.65 (d, *J =* 6.8 Hz, 3H), 1.24 (t, *J* = 7.2 Hz, 3H). | 468.20 |
| C32 | 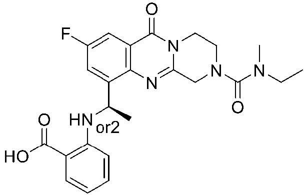 | ¹H NMR (400 MHz, DMSO-*d*₆) δ ppm 12.71 (br, 1H), 8.57 (s, 1H), 7.80 (dd, *J* = 7.6, 1.6 Hz, 1H), 7.69 (dd, *J* = 8.4, 3.2 Hz, 1H), 7.55 (dd, *J* = 9.6, 3.2 Hz, 1H), 7.22 - 7.13 (m, 1H), 6.52 (t, *J* = 7.6 Hz, 1H), 6.38 (d, *J* = 8.4 Hz, 1H), 5.50 - 5.39 (m, 1H), 4.45 (s, 2H), 4.10 - 3.94 (m, 2H), 3.64 (t, *J* = 6.0 Hz, 2H), 3.22 (q, *J* = 6.8 Hz, 2H), 2.85 (s, 3H), 1.58 (d, *J* = 6.8 Hz, 3H), 1.11 (t, *J* = 7.2 Hz, 3H). | 468.20 |
| C33 | 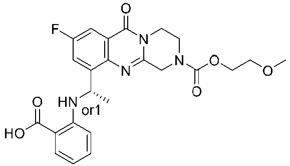 | ¹H NMR (400 MHz, DMSO-*d*₆) δ ppm 12.77 (s, 1H), 8.50 (s, 1H), 7.81 (dd, *J* = 8.0*,* 1.6 Hz, 1H), 7.71 (dd, *J =* 8.4, 3.2 Hz, 1H), 7.55 (dd, *J* = 9.6, 3.2 Hz, 1H), 7.22 - 7.09 (m, 1H), 6.53 (t, *J =* 7.6 Hz, 1H), 6.36 (d, *J* = 8.4 Hz, 1H), 5.56 - 5.38 (m, 1H), 4.71 (s, 2H), 4.39 - 4.06 (m, 4H), 3.85 - 3.69 (m, 2H), 3.64 - 3.51 (m, 2H), 3.29 (s, 3H), 1.58 (d, *J =* 6.8 Hz, 3H). | 485.18 |
| C34 | 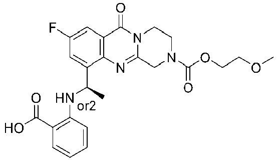 | ¹H NMR (400 MHz, DMSO-*d*₆) δ ppm 12.75 (s, 1H), 8.50 (s, 1H), 7.81 (dd, *J =* 8.0, 1.6 Hz, 1H), 7.71 (dd, *J =* 8.4, 3.2 Hz, 1H), 7.55 (dd, *J =* 9.6, 3.2 Hz, 1H), 7.23 - 7.09 (m, 1H), 6.53 (t, *J =* 7.6 Hz, 1H), 6.36 (d, *J* = 8.4 Hz, 1H), 5.56 - 5.38 (m, 1H), 4.71 (s, 2H), 4.39 - 4.06 (m, 4H), 3.85 - 3.69 (m, 2H), 3.66 - 3.51 (m, 2H), 3.29 (s, 3H), 1.58 (d, *J* = 6.8 Hz, 3H). | 485.18 |
| C35 | 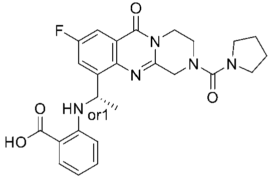 | ¹H NMR (400 MHz, DMSO-*d*₆) δ ppm 8.71 (br, 1H), 7.80 (dd, *J* = 8.4, 2.8 Hz, 1H), 7.69 (dd, *J* = 8.4, 3.2 Hz, 1H), 7.55 (dd, *J =* 9.6, 3.2 Hz, 1H), 7.19 - 7.07 (m, 1H), 6.51 (t, *J* = 7.6 Hz, 1H), 6.35 (d, *J* = 8.4 Hz, 1H), 5.50 - 5.39 (m, 1H), 4.52 (s, 2H), 4.14 - 4.00 (m, 2H), 3.69 (t, *J =* 5.6 Hz, 2H), 3.42 - 3.37 (m, 4H), 1.86 - 1.74 (m, 4H), 1.57 (d, *J* = 6.8 Hz, 3H). | 480.20 |
| C36 | 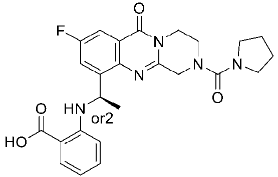 | ¹H NMR (400 MHz, DMSO-*d*₆) δ ppm 8.71 (br, 1H), 7.80 (dd, *J =* 8.4, 2.8 Hz, 1H), 7.70 (dd, *J* = 8.0, 3.6 Hz, 1H), 7.55 (dd, *J* = 9.2, 3.6 Hz, 1H), 7.20 - 7.11 (m, 1H), 6.52 (t, *J* = 7.6 Hz, 1H), 6.37 (d, *J* = 8.4 Hz, 1H), 5.47 - 5.41 (m, 1H), 4.52 (s, 2H), 4.15 - 3.99 (m, 2H), 3.69 (t, *J =* 6.0 Hz, 2H), 3.42 - 3.37 (m, 4H), 1.85 - 1.74 (m, 4H), 1.57 (d, *J* = 6.8 Hz, 3H). | 480.20 |
| C37 | 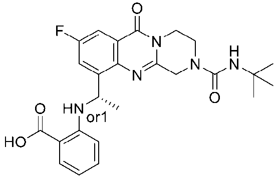 | ¹H NMR (400 MHz, DMSO-*d*₆) δ ppm 8.48 (d, *J* = 5.6 Hz, 1H), 7.81 (dd, *J =* 8.0, 1.6 Hz, 1H), 7.70 (dd, *J* = 8.4, 3.2 Hz, 1H), 7.53 (dd, *J* = 9.6, 3.2 Hz, 1H), 7.21 - 7.14 (m, 1H), 6.53 (t, *J* = 7.6 Hz, 1H), 6.34 (d, *J* = 8.4 Hz, 1H), 6.07 (s, 1H), 5.55 - 5.40 (m, 1H), 4.76 - 4.57 (m, 2H), 4.10 (t, *J* = 5.6 Hz, 2H), 3.82 - 3.62 (m, 2H), 3.17 (s, 1H), 1.58 (d, *J* = 6.8 Hz, 3H), 1.30 (s, 9H). | 482.21 |
| C38 | 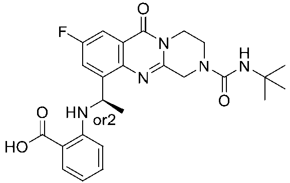 | ¹H NMR (400 MHz, DMSO-*d*₆) δ ppm 8.48 (d, *J* = 5.6 Hz, 1H), 7.81 (dd, *J=* 8.0, 2.0 Hz, 1H), 7.70 (dd, *J* = 8.4, 3.2 Hz, 1H), 7.53 (dd, *J =* 9.6, 3.2 Hz, 1H), 7.22 - 7.12 (m, 1H), 6.56 - 6.50 (m, 1H), 6.34 (d, *J* = 8.4 Hz, 1H), 6.07 (s, 1H), 5.53 - 5.42 (m, 1H), 4.74 - 4.58 (m, 2H), 4.10 (t, *J* = 6.0 Hz, 2H), 3.79 - 3.65 (m, 2H), 1.58 (d, *J* = 6.8 Hz, 3H), 1.30 (s, 9H). | 482.21 |
| C39 | 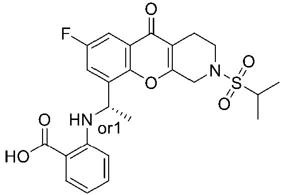 | ¹H NMR (400 MHz, DMSO-*d*₆) δ ppm 8.63 (br, 1H), 7.81 (dd, *J* = 8.0 1.6 Hz, 1H), 7.61 (dd, *J* = 8.0, 3.2 Hz, 1H), 7.55 (dd, *J* = 9.2, 3.2 Hz, 1H), 7.23 - 7.15 (m, 1H), 6.55 (t, *J =* 7.6 Hz, 1H), 6.48 (d, *J =* 8.8 Hz, 1H), 5.24 - 5.12 (m, 1H), 4.59 - 4.45 (m, 2H), 3.60 - 3.49 (m, 3H), 2.59 (t, *J =* 4.8 Hz, 2H), 1.62 (d, *J =* 6.8 Hz, 3H), 1.28 (d, *J = 6.8* Hz, 6H). | 489.14 |
| C40 | 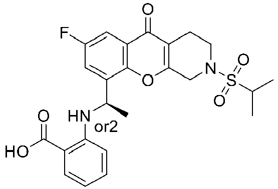 | ¹H NMR (400 MHz, DMSO-*d*₆) δ ppm 8.51 (br, 1H), 7.81 (dd, *J* = 8.0 1.6 Hz, 1H), 7.61 (dd, *J =* 8.0, 3.2 Hz, 1H), 7.55 (dd, *J =* 8.8, 3.2 Hz, 1H), 7.25 - 7.17 (m, 1H), 6.56 (t, *J =* 7.6 Hz, 1H), 6.50 (d, *J =* 8.8 Hz, 1H), 5.24 - 5.16 (m, 1H), 4.60 - 4.44 (m, 2H), 3.60 - 3.49 (m, 3H), 2.59 (t, *J =* 4.8 Hz, 2H), 1.63 (d, *J =* 6.8 Hz, 3H), 1.28 (d, *J =* 6.8 Hz, 6H). | 489.14 |
| C41 | 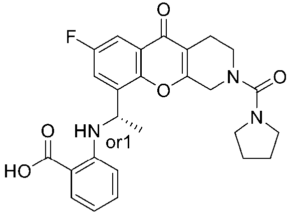 | ¹H NMR (400 MHz, CDCl₃) δ ppm 8.28 (d, *J* = 4.8 Hz, 1H), 8.02 (dd, *J =* 8.0, 1.6 Hz, 1H), 7.74 (dd, *J* = 8.0, 3.2 Hz, 1H), 7.41 (dd, *J =* 8.8, 3.2 Hz, 1H), 7.24 - 7.17 (m, 1H), 6.66 - 6.58 (m, 1H), 6.31 (d, *J =* 8.4 Hz, 1H), 5.15 - 5.05 (m, 1H), 4.45 (s, 2H), 3.57 (t, *J* = 6.0 Hz, 2H), 3.52 - 3.45 (m, 4H), 2.81 - 2.73 (m, 2H), 1.96 - 1.86 (m, 4H), 1.68 (d, *J =* 6.8 Hz, 3H). | 480.19 |
| C42 | 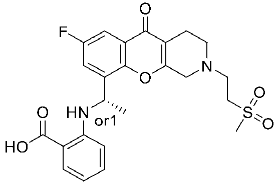 | ¹H NMR (400 MHz, CDCl₃) δ ppm 8.38 (d, *J* = 7.6 Hz, 1H), 8.01 (dd, *J* = 8.0, 1.6 Hz, 1H), 7.75 (dd, *J* = 8.0, 3.2 Hz, 1H), 7.41 (dd, *J* = 8.4, 3.2 Hz, 1H), 7.32 - 7.26 (m, 1H), 6.68 - 6.61 (m, 1H), 6.46 (d, *J* = 8.4 Hz, 1H), 5.10 - 5.02 (m, 1H), 3.92 (d, *J =* 16.4 Hz, 1H), 3.63 (d, *J =* 16.8 Hz, 1H), 3.46 - 3.37 (m, 1H), 3.34 - 3.11 (m, 3H), 3.08 (s, 3H), 3.00 - 2.91 (m, 1H), 2.87 - 2.78 (m, 1H), 2.77 - 2.66 (m, 2H), 1.73 (d, *J =* 6.8 Hz, 3H). | 489.14 |
| C43 | 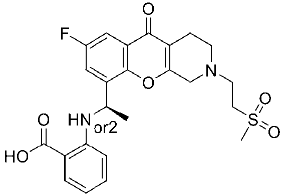 | ¹H NMR (400 MHz, CDCl3) δ ppm 8.38 (d, *J* = 7.2 Hz, 1H), 8.01 (dd, *J =* 8.0, 1.6 Hz, 1H), 7.75 (dd, *J* = 7.6, 3.2 Hz, 1H), 7.41 (dd, *J =* 8.8, 3.6 Hz, 1H), 7.31 - 7.26 (m, 1H), 6.69 - 6.60 (m, 1H), 6.46 (d, *J =* 8.4 Hz, 1H), 5.10 - 5.02 (m, 1H), 3.91 (d, *J =* 16.8 Hz, 1H), 3.63 (d, *J =* 16.4 Hz, 1H), 3.47 - 3.35 (m, 1H), 3.35 - 3.10 (m, 3H), 3.08 (s, 3H), 3.00 - 2.89 (m, 1H), 2.88 - 2.77 (m, 1H), 2.77 - 2.64 (m, 2H), 1.73 (d, *J* = 6.8 Hz, 3H). | 489.14 |
| C46 | 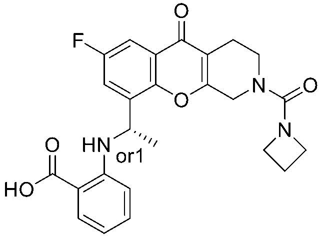 | ¹H NMR (400 MHz, CDCl₃) δ ppm 8.30 (s, 1H), 8.02 (dd, *J =* 8.0, 1.6 Hz, 1H), 7.74 (dd, *J =* 8.0, 3.2 Hz, 1H), 7.42 (dd, *J =* 8.4, 3.2 Hz, 1H), 7.26 - 7.17 (m, 1H), 6.63 (t, *J =* 7.6 Hz, 1H), 6.33 (d, *J =* 8.4 Hz, 1H), 5.14 - 5.09 (m, 1H), 4.64 - 4.43 (m, 2H), 4.14 (t, *J =* 7.2 Hz, 4H), 3.56 (q, *J =* 5.6 Hz, 2H), 2.81 - 3.65 (m, 2H), 2.64 - 3.46 (m, 2H), 2.40 - 2.25 (m, 2H), 1.69 (d, *J =* 6.8 Hz, 3H). | 466.17 |
| C47 | 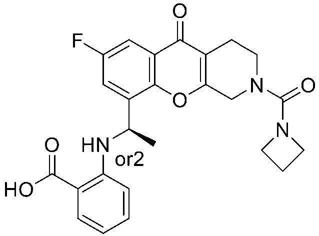 | ¹H NMR (400 MHz, CDCl₃) δ ppm 8.32 (br, 1H), 8.01 (dd, J =8.0, 1.6Hz, 1H), 7.73 (dd, J = 8.0, 3.2 Hz, 1H), 7.41 (dd, J =8.4, 2.8 Hz, 1H), 7.26-7.19 (m, 1H), 6.66-6.60 (m, 1H), 6.34 (d, J = 8.4 Hz, 1H), 5.13-5.04 (m, 1H), 4.66-4.44 (m, 2H), 4.20-4.08 (m, 4H), 3.63-3.48 (m, 2H), 2.79-2.69 (m, 2H), 2.39-2.29 (m, 2H), 1.69 (d, J = 6.8 Hz, 3H) | 466.17 |
| C48 | 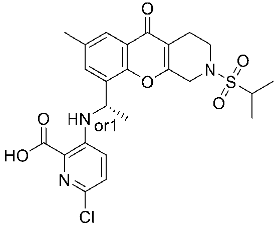 | ¹H NMR (400 MHz, DMSO-*d*₆) δ ppm 8.84 (br, 1H), 7.73 (s, 1H), 7.55 (s, 1H), 7.24 (d, J = 8.8 Hz, 1H), 7.04 (d, J = 8.8 Hz, 1H), 5.22 - 5.09 (m, 1H), 4.55 - 4.44 (m, 2H), 3.60-3.52 (m, 4H), 2.62 - 2.55 (m, 2H), 2.35 (s, 3H), 1.60 (d, J = 6.8 Hz, 3H), 1.28 (dd, J = 6.4, 1.2 Hz, 6H). | 520.12 |
| C49 | 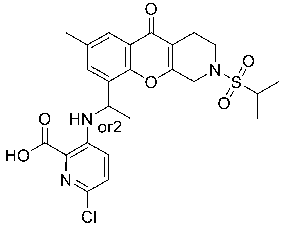 | ¹H NMR (400 MHz, DMSO-*d*₆) δ ppm 8.93 (br, 1H), 7.73 (s, 1H), 7.54 (s, 1H), 7.22 (d, J = 8.8 Hz, 1H), 7.02 (d, J = 8.8 Hz, 1H), 5.19 - 5.09 (m, 1H), 4.56 - 4.44 (m, 2H), 3.64 - 3.54 (m, 4H), 2.62 - 2.54 (m, 2H), 2.35 (s, 3H), 1.60 (d, J = 6.4 Hz, 3H), 1.28 (dd, J = 6.8, 1.2 Hz, 6H). | 520.12 |
| C50 | 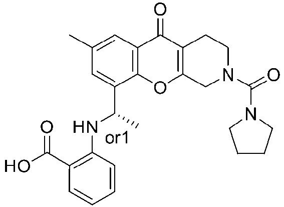 | ¹H NMR (400 MHz, DMSO-*d*₆) δ ppm 12.80 (br, 1H), 8.52 (br, 1H), 7.80 (dd, J = 8.4, 1.6 Hz, 1H), 7.74 - 7.69 (m, 1H), 7.57 - 7.51 (m, 1H), 7.27 - 7.18 (m, 1H), 6.57 - 6.49 (m, 2H), 5.20 - 5.10 (m, 1H), 4.40-4.28 (m, 2H), 3.57 - 3.37 (m, 6H), 2.59 - 2.52 (m, 2H), 2.35 (s, 3H), 1.85 - 1.73 (m, 4H), 1.60 (d, J = 6.8 Hz, 3H). | 476.21 |
| C51 | 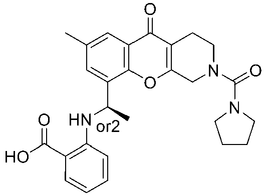 | ¹H NMR (400 MHz, DMSO-*d*₆) δ ppm 8.61 (br, 1H), 7.80 (dd, J = 8.4, 1.6 Hz, 1H), 7.74 - 7.69 (m, 1H), 7.57 - 7.51 (m, 1H), 7.24 - 7.16 (m, 1H), 6.57 - 6.48 (m, 2H), 5.21 - 5.09 (m, 1H), 4.41 - 4.28 (m, 2H), 3.55 - 3.45 (m, 6H), 2.62 - 2.53 (m, 2H), 2.35 (s, 3H), 1.85 - 1.74 (m, 4H), 1.60 (d, J = 6.8 Hz, 3H). | 476.21 |
| C52 | 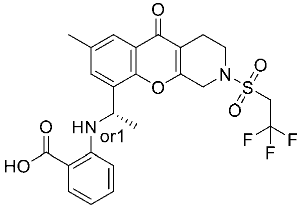 | ¹H NMR (400 MHz, DMSO-*d*₆) δ ppm 12.80 (br, 1H), 8.48 (d, J = 6.0 Hz, 1H), 7.81 (dd, J =8.0, 1.6 Hz, 1H), 7.76 - 7.71 (m, 1H), 7.56 - 7.50 (m, 1H), 7.26 - 7.18 (m, 1H), 6.58 - 6.51 (m, 1H), 6.49 (d, J = 8.4 Hz, 1H), 5.22 - 5.11 (m, 1H), 4.80-4.67 (m, 2H), 4.64 - 4.47 (m, 2H), 3.67 - 3.53 (m, 2H), 2.69-2.59 (m, 2H), 2.35 (s, 3H), 1.61 (d, J = 6.8 Hz, 3H). | 525.12 |
| C53 | 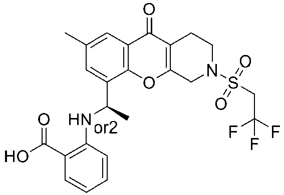 | ¹H NMR (400 MHz, DMSO-*d*₆) δ ppm 12.80 (br, 1H), 8.49 (d, J = 5.2 Hz, 1H), 7.81 (dd, J =8.0, 1.6 Hz, 1H), 7.76 - 7.71 (m, 1H), 7.56 - 7.50 (m, 1H), 7.25 - 7.18 (m, 1H), 6.58 - 6.51 (m, 1H), 6.48 (d, J = 8.4 Hz, 1H), 5.21 - 5.10 (m, 1H), 4.81-4.68 (m, 2H), 4.64 - 4.46 (m, 2H), 3.65 - 3.56 (m, 2H), 2.69 - 2.59 (m, 2H), 2.35 (s, 3H), 1.61 (d, J = 6.8 Hz, 3H). | 525.12 |
| C56 | 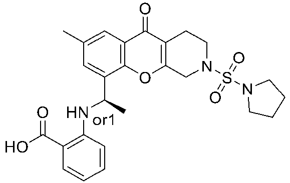 | ¹H NMR (400 MHz, DMSO-*d*₆) δ ppm 12.80 (br, 1H), 8.46 (d, J = 5.6 Hz, 1H), 7.80 (dd, J = 8.0, 1.6 Hz, 1H), 7.75 - 7.69 (m, 1H), 7.57 - 7.49 (m, 1H), 7.27 - 7.17(m, 1H), 6.58 - 6.48 (m, 2H), 5.23 - 5.12 (m, 1H), 4.48 - 4.29 (m, 2H), 3.56 - 3.41 (m, 2H), 3.31 - 3.20 (m, 4H), 2.63 - 2.55 (m, 2H), 2.35 (s, 3H), 1.92 - 1.79 (m, 4H), 1.60 (d, J = 6.8 Hz, 3H). | 512.18 |
| C57 | 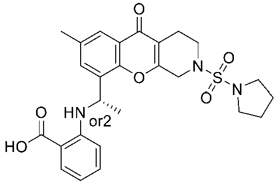 | ¹H NMR (400 MHz, DMSO-*d*₆) δ ppm 12.80 (br, 1H), 8.47 (d, J = 5.6 Hz, 1H), 7.80 (dd, J =8.0, 1.6 Hz, 1H), 7.75 - 7.70 (m, 1H), 7.56 - 7.52 (m, 1H), 7.26 - 7.18(m, 1H), 6.58 - 6.48 (m, 2H), 5.24 - 5.12 (m, 1H), 4.46 - 4.30 (m, 2H), 3.56 - 3.43 (m, 2H), 3.31 - 3.22 (m, 4H), 2.63 - 2.55 (m, 2H), 2.35 (s, 3H), 1.91 - 1.81 (m, 4H), 1.61 (d, J = 6.8 Hz, 3H). | 512.18 |
| C58 | 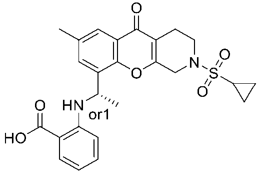 | ¹H NMR (400 MHz, DMSO-*d*₆) δ ppm 12.83 (br, 1H), 8.50 (d, J = 5.6 Hz, 1H), 7.80 (dd, J = 8.0, 1.6 Hz, 1H), 7.76 - 7.68 (m, 1H), 7.59 - 7.51 (m, 1H), 7.27 - 7.17(m, 1H), 6.59 - 6.47 (m, 2H), 5.23 - 5.10 (m, 1H), 4.57 - 4.40 (m, 2H), 3.62 - 3.49 (m, 2H), 2.84 - 2.72 (m, 1H), 2.69 - 2.57 (m, 2H), 2.35 (s, 3H), 1.61 (d, J = 6.8 Hz, 3H), 1.09 - 0.93 (m, 4H). | 483.15 |
| C59 | 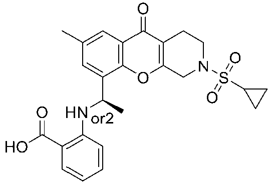 | ¹H NMR (400 MHz, DMSO-*d*₆) δ ppm 12.82 (br, 1H), 8.50 (d, J = 5.2 Hz, 1H), 7.81 (dd, J =8.0, 1.6 Hz, 1H), 7.75 - 7.71 (m, 1H), 7.57 - 7.51 (m, 1H), 7.25 - 7.18(m, 1H), 6.58 - 6.48 (m, 2H), 5.23 - 5.13 (m, 1H), 4.58 - 4.41 (m, 2H), 3.62 - 3.47 (m, 2H), 2.84 - 2.73 (m, 1H), 2.66 - 2.57 (m, 2H), 2.35 (s, 3H), 1.61 (d, J = 6.8 Hz, 3H), 1.07 - 0.94 (m, 4H). | 483.15 |
| C60 | 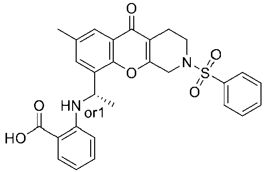 | ¹H NMR (400 MHz, DMSO-*d*₆) δ ppm 12.82 (br, 1H), 8.45 (d, J = 6.8 Hz, 1H), 7.92-7.85 (m, 2H), 7.81 (dd, J = 8.0, 1.2 Hz, 1H), 7.73 - 7.64 (m, 2H), 7.63 - 7.55 (m, 2H), 7.54 - 7.48 (m, 1H), 7.26 - 7.18 (m, 1H), 6.58 - 6.47 (m, 2H), 5.23 - 5.11 (m, 1H), 4.39 - 4.23 (m, 2H), 3.45 - 3.37 (m, 2H), 2.50 - 2.44 (m, 2H), 2.33 (s, 3H), 1.59 (d, J = 6.4 Hz, 3H), | 519.15 |
| C61 | 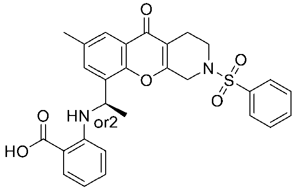 | ¹H NMR (400 MHz, DMSO-*d*₆) δ ppm 12.82 (br, 1H), 8.46 (d, J = 6.0 Hz, 1H), 7.91 - 7.86 (m, 2H), 7.81 (dd, J = 8.0, 1.2 Hz, 1H), 7.72 - 7.64 (m, 2H), 7.62 - 7.55 (m, 2H), 7.54 - 7.50 (m, 1H), 7.26 - 7.19 (m, 1H), 6.57 - 6.48 (m, 2H), 5.21 - 5.10 (m, 1H), 4.40 - 4.23 (m, 2H), 3.43 - 3.36 (m, 2H), 2.50 - 2.43 (m, 2H), 2.33 (s, 3H), 1.59 (d, J = 6.4 Hz, 3H), | 519.15 |
| C67 | 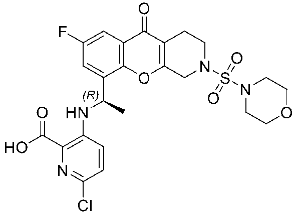 | 1H NMR (400 MHz, CDCl₃) δ ppm 10.73 (br, 1 H), 8.43 (d, J = 6.8 Hz, 1H), 7.78 (dd, J = 7.6, 3.2 Hz, 1H), 7.36 (dd, J = 8.0, 3.2 Hz, 1H), 7.24 (d, J = 9.2 Hz, 1H), 6.84 (d, J = 8.8 Hz, 1H), 5.07 - 4.97 (m, 1H), 4.48 - 4.34 (m, 2H), 3.86 - 3.76 (m, 4H), 3.62 - 3.54 (m, 2H), 3.38 - 3.28 (m, 4H), 2.87 - 2.77 (m, 2H), 1.75 (d, J = 6.8 Hz, 3H). | 567.10 |
| C68 | 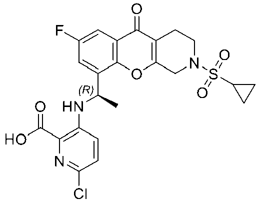 | 1H NMR (400 MHz, CDCl₃) δ ppm 8.40 (d, J = 5.2 Hz, 1H), 7.77 (dd, J = 7.6, 3.2 Hz, 1H), 7.37 (dd, J = 8.0, 3.2 Hz, 1H), 7.24 (d, J = 8.8 Hz, 1H), 6.82 (d, J = 8.8 Hz, 1H), 5.11 - 4.99 (m, 1H), 4.55 - 4.40 (m, 2H), 3.79 - 3.66 (m, 1H), 3.79 - 3.66 (m, 1H), 3.61-3.49 (m, 1H), 2.89 - 2.79 (m, 2H), 2.47 - 2.38 (m, 1H), 1.74 (d, J = 6.4 Hz, 3H), 1.33 - 1.29 (m, 2H), 1.13 - 1.08 (m, 2H). | 522.08 |
| C69 | 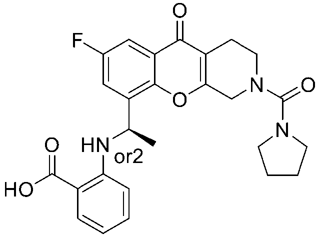 | ¹H NMR (400 MHz, CDCl3) δ 8.28 (m, 1H), 8.02 (dd, J = 8.0, 1.6 Hz, 1H), 7.74 (dd, J = 7.6, 3.2 Hz, 1H), 7.41 (dd, J = 8.4, 3.2 Hz, 1H), 7.25 - 7.17 (m, 1H), 6.62 (t, J = 7.2 Hz, 1H), 6.30 (d, J = 8.4 Hz, 1H), 5.16 - 5.04 (m, 1H), 4.45 (s, 2H), 3.57 (t, J = 5.6 Hz, 2H), 3.49 (t, J = 6.4 Hz, 4H), 2.78 (t, J = 5.2 Hz, 2H), 1.92 (t, J = 6.4 Hz, 4H), 1.68 (d, J = 6.8 Hz, 3H). | 480.19 |
| C70 | 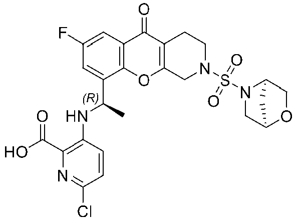 | ¹H NMR (400 MHz, CDCl₃) δ ppm 8.83 (br, 1H), 7.76 (dd, J = 7.6, 3.2 Hz, 1H), 7.39 (dd, J = 8.0, 2.8 Hz, 1H), 7.15 (d, J = 8.0 Hz, 1H), 6.76 (d, J = 8.4 Hz, 1H), 5.08 - 4.95 (m, 1H), 4.72 - 4.65 (m, 1H), 4.52 - 4.45 (m, 1H), 4.42 - 4.35 (m, 2H), 4.12 (d, J = 8.0 Hz, 1H), 3.82 (d, J = 8.0 Hz, 1H), 3.60 - 3.47 (m, 2H), 3.46 - 3.37 (m, 2H), 3.31 - 3.17 (m, 1H), 2.87 - 2.75 (m, 2H), 2.08 - 1.93 (m, 2H), 1.72 (d, J *=* 6.0 Hz, 3H) | 579.10 |
| C71 | 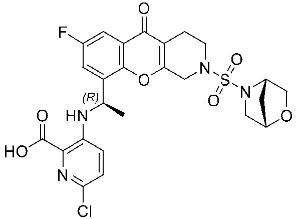 | 1H NMR (400 MHz, CDCl₃) δ ppm 8.48 (br, 1H), 7.78 (dd, J = 8.0, 3.2 Hz, 1H), 7.36 (dd, J = 8.4, 3.2 Hz, 1H), 7.23 (d, J = 8.4 Hz, 1H), 6.86 (d, J = 8.8 Hz, 1H), 5.06 - 4.95 (m, 1H), 4.76 - 4.65 (m, 1H), 4.56 - 4.50 (m, 1H), 4.47 (d, J = 17.6 Hz, 1H), 4.35 (d, J = 17.2 Hz, 1H), 4.20 (d, J = 7.6 Hz, 1H), 3.83 (dd, J = 1.2, 8.0 Hz, 1H), 3.65 - 3.47 (m, 2H), 3.46 - 3.36 (m, 2H), 2.90 - 2.71 (m, 2H), 2.12 - 1.95 (m, 3H), 1.76 (d, J = 6.8 Hz, 3H) | 579.10 |
| C72 | 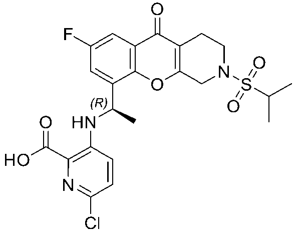 | 1H NMR (400 MHz, CDCl₃) δ 10.58 (br, 1H), 8.37 (br, 1H), 7.78 (dd, J = 3.2, 8.0 Hz, 1H), 7.36 (dd, J = 3.2, 8.4 Hz, 1H), 7.24 (d, J = 8.8 Hz, 1H), 6.82 (d, J = 8.8 Hz, 1H), 5.09 - 4.99 (m, 1 H), 4.50 (dd, J = 17.6, 30.4 Hz, 2H), 3.77 - 3.68 (m, 1H), 3.63 - 3.55 (m, 1H), 3.39 - 3.30 (m, 1H), 2.85 - 2.79 (m, 2H), 1.74 (d, J = 6.8 Hz, 3H), 1.45(dd, J = 6.8, 1.6 Hz, 6H). | 524.10 |
| C73 | 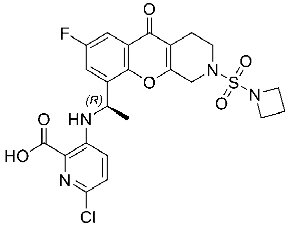 | ¹H NMR (400 MHz, CDCl₃) δ ppm 10.71 (br, 1H), 8.36 (d, J = 6.0 Hz, 1H), 7.78 (dd, J = 7.6, 3.2, Hz 1H), 7.36 (dd, J = 8.0, 2.8 Hz, 1H), 7.24 (d, J = 8.8 Hz, 1H), 6.80 (d, J = 8.8 Hz, 1H), 5.12 - 5.01 (m, 1H), 4.41 - 4.35 (m, 2H), 4.04 - 3.97 (m, 4H), 3.66 - 3.57 (m, 1H), 3.54 - 3.46 (m, 1H), 2.84 - 2.76 (m, 2H), 2.36 - 2.28 (m, 2H), 1.74 (d, J = 6.8 Hz, 3H) | 537.09 |
| C74 | 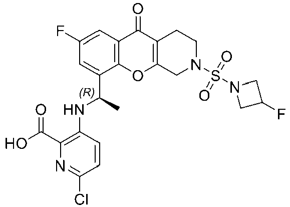 | ¹H NMR (400 MHz, CDCl₃) δ ppm 8.39 (d, J = 4.4 Hz, 1H), 7.78 (dd, J = 7.6, 2.8 Hz, 1H), 7.36 (dd, J = 8.4, 3.2 Hz, 1H), 7.24 (d, J = 9.2 Hz, 1H), 6.82 (d, J = 8.8 Hz, 1H), 5.44 - 5.19 (m, 1H), 5.10 - 4.99 (m, 1H), 4.44 - 4.34 (m, 2H), 4.29 - 4.10 (m, 4H), 3.68 - 3.47 (m, 2H), 2.85 - 2.75 (m, 2H), 1.74 (d, J = 6.8 Hz, 3H) | 555.08 |
| C75 | 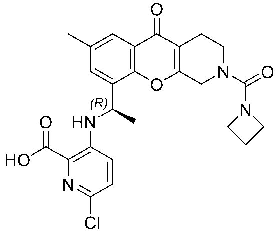 | ¹H NMR (400 MHz, CDCl₃) δ ppm 8.52 - 8.24 (br, 1H), 7.96 - 7.88 (m, 1H), 7.50 - 7.31 (m, 2H), 7.20 (d, J = 9.2 Hz, 1H), 6.86 (d, J = 8.8 Hz, 1H), 5.09 - 4.95 (m, 1H), 4.55 - 4.39 (m, 2H), 4.19 - 4.04 (m, 4H), 3.59 - 3.48 (m, 2H), 2.81 - 2.68 (m, 2H), 2.41 (s, 3H), 2.37 - 2.29 (m, 2H), 1.71 (d, J = 6.8 Hz, 3H). | 497.15 |
| C76 | 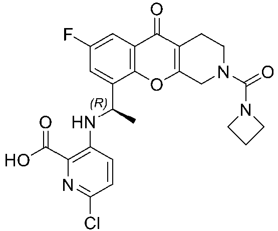 | 1H NMR (400 MHz, DMSO-*d*₆) δ ppm 9.43 (br, 1H), 7.64 - 7.51 (m, 2H), 7.14 (d, J = 8.4 Hz, 1H), 6.99 (d, J = 8.8 Hz, 1H), 5.22 - 5.09(m, 1H), 4.56 - 4.37 (m, 2H), 4.09 - 3.91 (m, 4H), 3.51 - 3.46 (m, 2H), 2.26 - 2.14 (m, 2H), 2.38 - 2.27 (m, 2H), 1.61 (d, J = 6.4 Hz, 3H). | 501.13 |
| C77 | 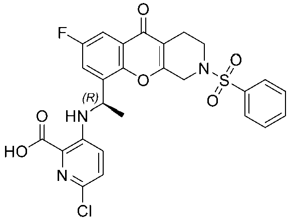 | ¹H NMR (400 MHz, CDCl₃) δ ppm 8.42(br, 1 H), 7.96 - 7.89 (m, 2H), 7.74 (dd, J = 7.6, 3.2 Hz, 1H), 7.72 - 7.61(m, 3H), 7.35 (dd, J = 8.4, 3.2 Hz, 1H), 7.21 (d, J = 8.8 Hz, 1H), 6.79 (d, J = 8.8 Hz, 1H), 5.08 - 4.97 (m, 1H), 4.32 (d, J = 16.8 Hz, 1H), 4.15 (d, J = 16.8 Hz, 1H), 3.60 - 3.48 (m, 1H), 3.29 - 3.16 (m, 1H), 2.88 - 2.73 (m, 2H), 1.73 (d, J = 6.8 Hz, 3H). | 558.08 |
| C78 | 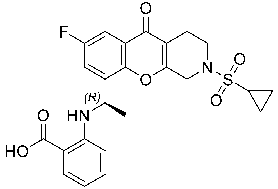 | 1H NMR (400 MHz, DMSO-*d*₆) δ ppm 12.90(br, 1 H), 8.52 (br, 1H), 7.72 (dd, J = 8.0, 1.2 Hz, 2H), 7.61 (dd, J = 8.0, 2.8 Hz, 1H), 7.56 (dd, J = 9.2, 3.2 Hz, 1H), 7.22 (td, J = 8.4, 1.2 Hz, 1H), 6.56 (t, J = 7.6 Hz, 1H), 6.51 (d J = 8.4 Hz, 1H), 5.27 - 5.17 (m, 1H), 4.52 (dd, J = 31.2, 17.6 Hz, 2H), 3.62 - 3.49 (m, 2H), 2.84 - 2.75 (m, 1H), 2.69 - 2.61 (m, 2H), 1.64 (d, J = 6.4 Hz, 3H), 1.09 - 0.96 (m, 4H). | 487.13 |
| C79 | 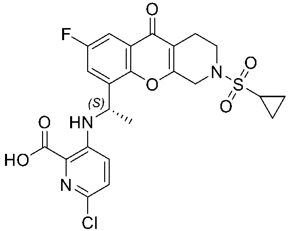 | 1H NMR (400 MHz, DMSO-*d*₆) δ ppm 8.75 (br, 1H), 7.67 - 7.58 (m, 2H), m), 7.27 (d, J *=* 8.8 Hz, 1H), 7.09 (d, J = 8.8 Hz, 1H), 5.28 - 5.16 (m, 1H), 4.59 - 4.43 (m, 2H), 3.62 - 3.52 (m, 2H), 2.85 - 2.75 (m, 1H), 2.66 - 2.61 (m, 2H), 1.64 (d, J = 6.4 Hz, 3H), 1.09 - 0.97 (m, 4H). | 522.08 |
| C80 | 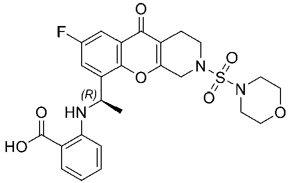 | ¹H NMR (400 MHz, DMSO-*d*₆) δ ppm 12.82 (br, 1 H), 8.51 (br, 1H), 7.82 (dd, J = 8.0, 1.2 Hz, 1H), 7.62 (dd, J = 8.0, 2.8 Hz, 1H), 7.58 (dd, J = 8.8, 2.8 Hz, 1H), 7.22 (t, J = 7.2 Hz, 1H), 6.56 (t, J = 7.6 Hz, 1H), 6.51 (d, J = 8.4 Hz, 1H), 5.26 - 5.17 (m, 1H), 4.47 (dd, J = 26.4, 17.6 Hz, 2H), 3.68 - 3.61 (m, 4H), 3.58 - 3.48 (m, 2H), 3.23 - 3.14 (m, 4H), 2.65 - 2.58 (m, 2H), 1.63 (d, J = 6.8 Hz, 3H). | 532.15 |
| C81 | 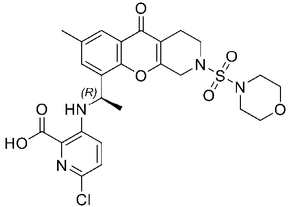 | ¹H NMR (400 MHz, CDCl₃) δ ppm 8.67 (br, 1H), 7.96 - 7.82 (m, 1H), 7.49 - 7.40 (m, 1H), 7.19 - 7.06 (m, 1H), 6.86 - 6.71 (m, 1H), 5.04 - 4.88 (m, 1H), 4.49 - 4.31 (m, 2H), 3.86 - 3.72 (m, 4H), 3.63 - 3.50 (m, 2H), 3.40 - 3.25 (m, 4H), 2.88 - 2.72 (m, 2H), 2.38 (s, 3H), 1.72 - 1.65 (m, 3H), | 563.13 |
| C82 | 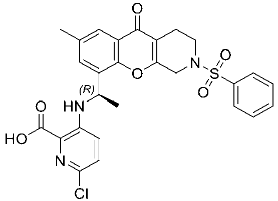 | ¹H NMR (400 MHz, CDCl₃) δ ppm 10.71(br, 1 H), 8.37 (d, J = 6.4 Hz, 1H), 7.97 - 7.87 (m, 3H), 7.72 - 7.58 (m, 3H), 7.43 - 7.38 (m, 1H), 7.20 (d, J = 9.2 Hz, 1H), 6.84 (d, J = 9.2 Hz, 1H), 5.06 - 4.95 (m, 1H), 4.32 (d, J = 16.8 Hz, 1H), 4.12 (d, J = 16.8 Hz, 1H), 3.61 - 3.50 (m, 1H), 3.24 - 3.13 (m, 1H), 2.84 - 2.74 (m, 2H), 2.39 (s, 3H), 1.71 (d, J = 6.8 Hz, 3H). | 554.11 |
| C83 | 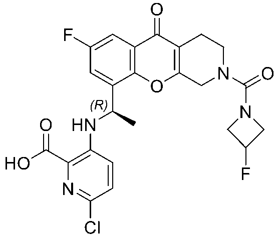 | ¹H NMR (400 MHz, CDCl₃) δ ppm 10.65 (br, 1H), 8.39 (d, J = 7.0 Hz, 1H), 7.78 (dd, J = 7.6, 3.2 Hz, 1H), 7.36 (dd, J = 8.4, 3.2 Hz, 1H), 7.24 (d, J = 9.2 Hz, 1H), 6.82 (d, J = 9.2 Hz, 1H), 5.46 - 5.41 (m, 0.5 H), 5.32 - 5.27 (m, 0.5 H), 5.09 - 5.02 (m, 1H), 4.53 - 4.16 (m, 6H), 3.64 - 3.50 (m, 2H), 2.77 - 2.68 (m, 2H), 1.75 (d, J = 6.8 Hz, 3H). | 519.12 |
| C84 | 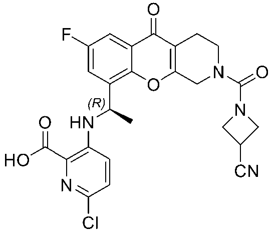 | ¹H NMR (400 MHz, DMSO-*d*₆) δ ppm 13.15 (br, 1H), 8.53 (d, J = 6.4 Hz, 1H), 7.66 (dd, J = 8.8, 2.8 Hz, 1H), 7.61 (dd, J = 8.0, 3.2 Hz, 1H), 7.34 (d, J = 9.2 Hz, 1H), 7.16 (d, J = 9.2 Hz, 1H), 5.29 - 5.20 (m, 1H), 4.45 (dd, J = 18.4, 28.8 Hz, 2H), 4.33 - 4.24 (m, 2H), 4.22 - 4.13 (m, 2H), 3.85 - 3.76 (m, 1H), 3.55 - 3.46 (m, 2H), 2.50 - 2.44 (m, 2H), 1.65 (d, J = 6.8 Hz, 3H). | 526.12 |
| C85 | 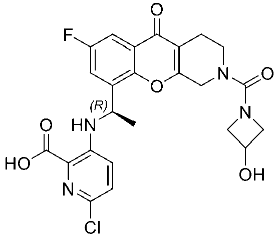 | 1H NMR (400 MHz, DMSO-*d*₆) δ ppm 8.57 (br, 1H),7.67 (dd, J = 8.8, 3.2 Hz, 1H), 7.61 (dd, J = 8.0, 3.2 Hz, 1H), 7.34 (d, J = 8.8 Hz, 1H), 7.16 (d, J = 9.2 Hz, 1H), 5.28 - 5.18 (m, 1H), 4.51 - 4.38 (m, 3H), 4.21 - 4.12 (m, 2H), 3.85 - 3.71 (m, 2H), 3.52 - 3.44 (m, 2H), 2.61 - 2.52 (m, 2H), 1.66 (d, J = 6.8 Hz, 3H). | 517.12 |
| C86 | 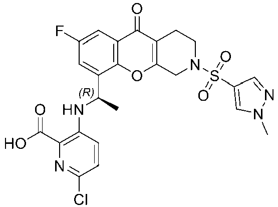 | 1H NMR (400 MHz, DMSO-*d*₆) δ ppm 13.15 (br, 1H), 8.49 (s, 1H), 8.47 (d, J = 6.4 Hz, 1H), 7.93 (s, 1H), 7.65 - 7.56 (m, 2H), m), 7.32 (d, J = 9.2 Hz, 1H), 7.12 (d, J = 9.2 Hz, 1H), 5.27 - 5.18 (m, 1H), 4.22 (dd, J = 42.8, 16.8 Hz, 2H), 3.90 (s, 3H), 3.34 - 3.31 (m, 1H), 3.20 - 3.14 (m, 1H), 2.64 - 2.56 (m, 2H), 1.64 (d, J = 6.8 Hz, 3H). | 562.09 |
| C87 | 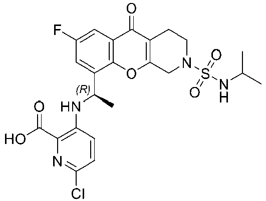 | 1H NMR (400 MHz, DMSO-*d*₆) δ ppm 13.14(br, 1 H), 8.58 (br, 1H), 7.69 - 7.54 (m, 3H), 7.31 (d, J = 8.8 Hz, 1H), 7.13 (d, J = 9.2 Hz, 1H), 5.28 - 5.17 (m, 1H), 4.38 - 4.26 (m, 2H), 3.48 - 3.37 (m, 3H), 2.64 - 2.56 (m, 2H), 1.65 (d, J = 6.8 Hz, 3H), 1.13 (d, J = 6.4 Hz, 6H). | 539.11 |
| C88 | 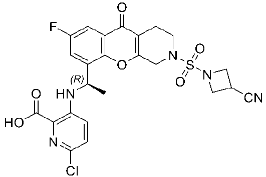 | ¹H NMR (400 MHz, CDCl₃) δ ppm 8.41 (d, J = 6.4 Hz, 1H), 7.79 (dd, J = 7.6, 3.2 Hz, 1H), 7.38 (dd, J = 8.4, 3.2 Hz, 1H), 7.25 (d, J = 8.8 Hz, 1H), 6.86 (d, J = 8.8 Hz, 1H), 5.08 - 4.98 (m, 1H), 4.40 (dd, J = 24.4, 17.6 Hz, 2H), 4.32-4.17 (m, 4H), 3.66 - 3.49 (m, 3H), 2.86 - 2.76 (m, 2H), 1.76 (d, J = 6.8 Hz, 3H), | 562.09 |
| C89 | 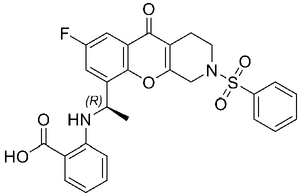 | ¹H NMR (400 MHz, DMSO-*d*₆) δ ppm 12.85(br, 1 H), 8.42 (d, J = 6.8 Hz, 1H), 7.94 (d, J = 7.2, Hz, 2H), 7.82 (dd, J = 8.0, 1.2 Hz, 1H), 7.73 - 7.66 (m, 1H), 7.64 - 7.57 (m, 2H), 7.57 - 7.51 (m, 2H), 7.23 (td, J = 8.4, 1.2 Hz, 1H), 6.57 (t, J = 7.6 Hz, 1H), 6.52 (t, J = 8.4 Hz, 1H), 5.27-5.16 (m, 1H), 4.34 (dd, J = 35.2, 17.6 Hz, 2H), 3.44 - 3.36 (m, 2H), 2.50 - 2.45 (m, 2H), 1.62 (d, J = 6.8 Hz, 3H). | 523.13 |
| C90 | 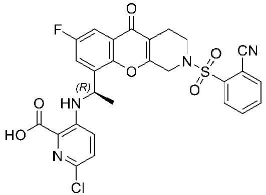 | ¹H NMR (400 MHz, DMSO-*d*₆) δ ppm 13.14(br, 1 H), 8.72 (br, 1H), 8.23(d, J = 7.6 Hz, 1H), 8.16(d, J = 7.2 Hz, 1H), 8.02 - 7.87 (m, 2H), 7.65 - 7.54 (m, 2H), 7.27 (d, J = 8.8 Hz, 1H), 7.05 (d, J = 8.8 Hz, 1H), 5.25 - 5.15 (m, 1H), 4.58 (dd, J = 25.6, 17.6 Hz, 2H), 3.70 - 3.52 (m, 2H), 2.48 - 2.40 (m, 2H), 1.63 (d, J = 6.4 Hz, 3H). | 583.08 |
| C91 | 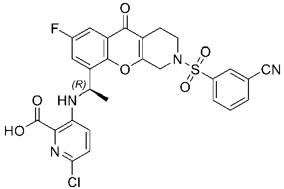 | ¹H NMR (400 MHz, DMSO-*d*₆) δ ppm 13.09 (br, 1 H), 8.47 (d, J = 6.4 Hz, 1H), 8.43 (s, 1H), 8.29 - 8.16 (m, 2H), 7.85(d, J = 8.0 Hz, 1H), 7.65 - 7.54 (m, 2H), 7.32 (d, J = 8.8 Hz, 1H), 7.11 (d, J = 9.2 Hz, 1H), 5.29 - 5.21 (m, 1H), 4.42 (dd, J = 38.8, 17.6 Hz, 2H), 3.57 - 3.47 (m, 1H), 3.45 - 3.38 (m, 1H), 2.62 - 2.53 (m, 2H), 1.64 (d, J = 6.8 Hz, 3H). | 583.08 |
| C92 | 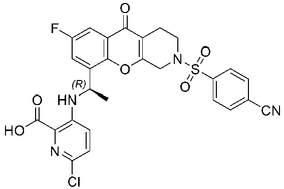 | ¹H NMR (400 MHz, DMSO-*d*₆) δ ppm 13.07(br, 1 H), 8.46 (d, J = 6.4 Hz, 1H), 8.14 (d, J = 8.4 Hz, 2H), 8.10 (d, J = 8.8 Hz, 2H), 7.64 (dd, J = 8.8, 3.2 Hz, 1H), 7.58 (d, J = 8.0, 3.2 Hz, 1H), 7.34 (d, J = 8.8 Hz, 1H), 7.14 (d, J = 9.2 Hz, 1H), 5.29 - 5.16 (m, 1H), 4.37 (dd, J = 47.2, 17.2 Hz, 2H), 3.53 - 3.45 (m, 1H), 3.37 - 3.31 (m, 1H), 2.60 - 2.53 (m, 2H), 1.64 (d, J = 6.8 Hz, 3H). | 583.08 |
| C93 | 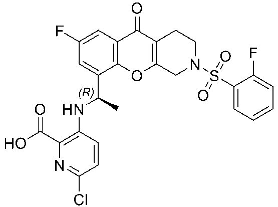 | ¹H NMR (400 MHz, DMSO-*d*₆) δ ppm 13.12(br, 1 H), 8.47 (d, J = 6.8 Hz, 1H), 7.94 (td, J = 7.6, 1.2 Hz, 1H), 7.82 - 7.72 (m, 1H), 7.66 - 7.54 (m, 2H), 7.51 - 7.40 (m, 2H), 7.33 (d, J = 8.8 Hz, 1H), 7.12 (d, J = 9.2 Hz, 1H), 5.30 - 5.20 (m, 1H), 4.47 (dd, J = 27.2, 17.2 Hz, 2H), 3.62 - 3.46 (m, 2H), 2.50 - 2.42 (m, 2H), 1.63 (d, J = 6.4 Hz, 3H). | 576.07 |
| C94 | 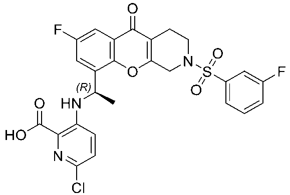 | 1H NMR (400 MHz, DMSO-*d*₆) δ ppm 8.57 (br, 1H), 7.83 - 7.74 (m, 2H), 7.73 - 7.66 (m, 1H), 7.64 - 7.39 (m, 3H), 7.36 - 7.26 (m, 1H), 7.10 (d, J = 8.8 Hz, 1H), 5.29 - 5.17 (m, 1H), 4.39 (dd, J = 32.4, 17.6 Hz, 2H), 3.49 - 3.42 (m, 2H), 2.56 - 2.51 (m, 2H), 1.63 (d, J = 6.8 Hz, 3H). | 576.07 |
| C95 | 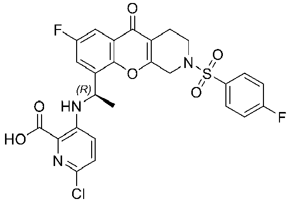 | 1H NMR (400 MHz, DMSO-*d*₆) δ ppm 13.13(br, 1 H), 8.45 (d, J = 6.8 Hz, 1H), 8.04 - 7.95 (m, 2H), 7.64 (dd, J = 8.8, 3.2 Hz, 1H), 7.58 (d, J = 8.0, 3.2 Hz, 1H), 7.53 - 7.45 (m, 2H), 7.34 (d, J = 8.8 Hz, 1H), 7.14 (d, J = 9.2 Hz, 1H), 5.29 - 5.18 (m, 1H), 4.31 (d, J = 46.8, 17.2 Hz, 2H), 3.49 - 3.25 (m, 2H), 2.63 - 2.53 (m, 2H), 1.64 (d, J = 6.8 Hz, 3H). | 576.07 |
| C96 | 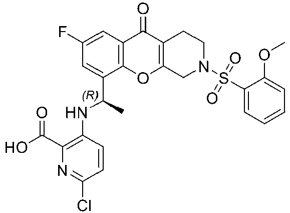 | 1H NMR (400 MHz, DMSO-*d*₆) δ ppm 13.15(br, 1 H), 8.50 (br, 1H), 7.88 (dd, J = 7.6, 1.2 Hz, 1H), 7.70 - 7.57 (m, 3H), 7.34 (d, J = 8.8 Hz, 1H), 7.21 - 7.10 (m, 3H), 5.35 - 5.24 (m, 1H), 4.56 (dd, J = 27.2, 18.0 Hz, 2H), 3.68 (s, 3 H), 3.61 - 3.45 (m, 2H), 2.32 - 2.18 (m, 2H), 1.65 (d, J = 6.8 Hz, 3H). | 588.09 |
| C97 | 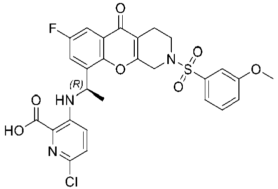 | ¹H NMR (400 MHz, DMSO-*d*₆) δ ppm 13.10(br, 1 H), 8.43 (br, 1H), 7.65 - 7.50 (m, 3H), 7.49 - 7.43 (m, 1H), 7.37 - 7.23 (m, 3H), 7.13 (d, J = 9.2 Hz, 1H), 5.30 - 5.19 (m, 1H), 4.35 (dd, J = 33.2, 17.2 Hz, 1H), 3.78 (s, 3 H), 3.52 - 3.22 (m, 2H), 2.50 - 2.46 (m, 2H), 1.64 (d, J = 6.8 Hz, 3H), | 588.09 |
| C98 | 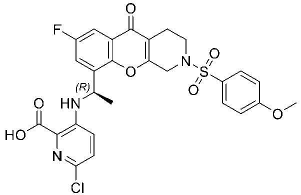 | ¹H NMR (400 MHz, DMSO-*d*₆) δ ppm 13.14(br, 1 H), 8.46 (d, J = 6.4 Hz, 1H), 7.87 - 7.76 (m, 2H), 7.63 (dd, J = 8.8, 2.8 Hz, 1H), 7.57 (d, J = 3.2, 8.0 Hz, 1H), 7.34 (d, J = 8.8 Hz, 1H), 7.19 - 7.07 (m, 3H), 5.28 - 5.18 (m, 1H), 4.25 (d, J = 43.6, 17.2 Hz, 2H), 3.81 (s, 3H), 3.43 - 3.20 (m, 2H), 2.58 - 2.52 (m, 2H), 1.64 (d, J = 6.8 Hz, 3H). | 588.09 |
| C99 | 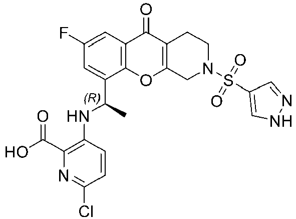 | ¹H NMR (400 MHz, DMSO-*d*₆) δ ppm 10.10 (d, J = 8.0 Hz, 2 H), 8.43 - 8.22 (br, 2H), 7.57 - 7.50 (m, 2H), 6.99 (d, J = 8.4 Hz, 1H), 6.87 (d, J = 8.8 Hz, 1H), 5.14 - 5.02 (m, 1H), 4.37 - 4.24 (m, 2H), 3.40 - 3.04 (m, 2H), 2.62 - 2.53 (m, 2H), 1.58 (d, J = 6.8 Hz, 3H), | 548.07 |
| C100 | 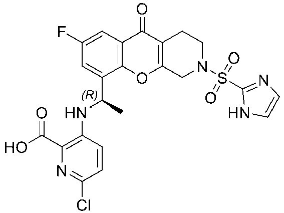 | ¹H NMR (400 MHz, DMSO-*d*₆) δ ppm 13.11(br, 2 H), 8.39 (d, J = 6.8 Hz, 1H), 7.63 - 7.54 (m, 2H), 7.34 (d, J = 8.8 Hz, 1H), 7.29 - 7.20 (br, 2H), 7.12 (d, J = 9.2 Hz, 1H), 5.31 - 5.20 (m, 1H), 4.59 - 4.56 (m, 2H), 3.56 - 3.42 (m, 2H), 2.50 - 2.47 (m, 2H), 1.65 (d, J = 6.4 Hz, 3H), | 548.07 |
| C101 | 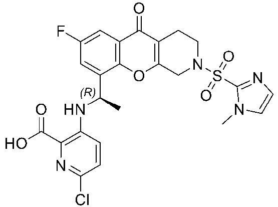 | ¹H NMR (400 MHz, DMSO-*d*₆) δ ppm 9.31(br, 1 H), 7.62-7.48 (m, 3H), 7.15 - 7.04 (m, 2H), 6.94 (d, J = 8.8 Hz, 1H), 5.24 - 5.14 (m, 1H), 4.69 - 4.56 (m, 2H), 3.93 (s, 3H), 3.68 - 3.56(m, 2H), 2.63 - 2.53(m, 2H), 1.58 (d, J = 6.8 Hz, 3H) | 562.09 |
| C102 | 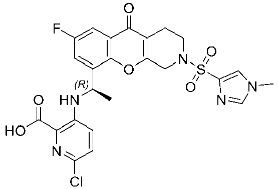 | ¹H NMR (400 MHz, DMSO-*d*₆) δ 13.17(br, 1 H), 8.51 (br, 1H), 7.97 (s, 1H), 7.82 (s, 1H), 7.63 - 7.56(m, 2H), 7.31 (d, J = 8.8 Hz, 1H), 7.11 (d, J = 8.8 Hz, 1H), 5.29 - 5.19 (m, 1H), 4.40 (dd, J *=* 17.6, 31.6 Hz, 2H), 3.70 (s, 3H), 3.46 - 3.36 (m, 2H), 2.61 - 2.53 (m, 2H), 1.63 (d, J = 6.4 Hz, 3H), | 562.09 |
| C103 | 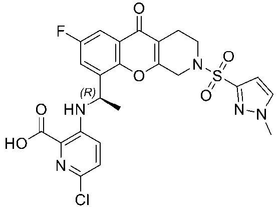 | ¹H NMR (400 MHz, DMSO-*d*₆) δ ppm 13.13(br, 1 H), 8.47 (br, 1H), 7.92 (d, J = 2.4 Hz, 1H), 7.66 - 7.55 (m, 2H), 7.32 (d, J = 8.8 Hz, 1H), 7.11 (d, J = 8.8 Hz, 1H), 6.79 (d, J = 2.0 Hz, 1H), 5.30 - 5.19 (m, 1H), 4.38 (dd, J = 30.0, 17.2 Hz, 1H), 3.84 (s, 3H), 3.52 - 3.38 (m, 2H), 2.61 - 2.54 (m, 2H), 1.64 (d, J = 6.8 Hz, 3H), | 562.09 |
| C104 | 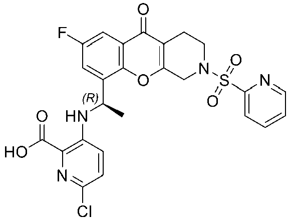 | ¹H NMR (400 MHz, DMSO-*d*₆) δ ppm 13.11(br, 1 H), 8.88 (br, 1H), 8.65 (d, J = 4.4 Hz, 1H), 8.16 - 8.03 (m, 2H), 7.72-7.53 (m, 3H), 7.23 (d, J = 8.8 Hz, 1H), 7.03 (d, J = 8.8 Hz, 1H), 5.25 - 5.15 (m, 1H), 4.65 - 4.52 (m, 2H), 3.63 - 3.52 (m, 2H), 2.48 - 2.40 (m, 2H), 1.62 (d, J = 6.4 Hz, 3H), | 559.08 |
| C105 | 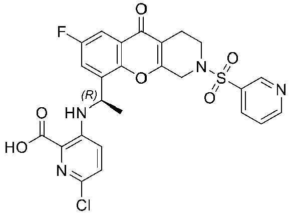 | ¹H NMR (400 MHz, DMSO-*d*₆) δ ppm 13.13(br, 1 H), 9.12 - 9.02 (m, 1H), 8.93 - 8.81 (m, 1H), 8.52 - 8.41 (m, 1H), 8.39 - 8.28 (m,1H), 7.72 - 7.48 (m, 3H), 7.33 (d, J = 8.8 Hz, 1H), 7.18 (d, J = 9.2 Hz, 1H), 5.29 - 5.16 (m, 1H), 4.42 (dd, J = 40.4, 17.2 Hz, 1H), 3.55 - 3.36 (m, 2H), 2.59 - 2.51 (m, 2H), 1.64 (d, J = 6.4 Hz, 3H), | 559.08 |
| C106 | 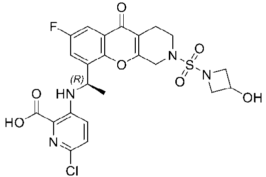 | / | 553.09 |
| C107 | 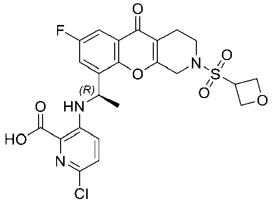 | ¹H NMR (400 MHz, CDCl₃) δ ppm 10.67 (br, 1H), 8.50-8.30 (br, 1H), 7.77 (dd, J = 7.6, 2.8 Hz, 1H), 7.37 (dd, J = 8.0, 2.8 Hz, 1H), 7.25 (d, J = 8.8 Hz, 1H), 6.85 (d, J = 9.2 Hz, 1H), 5.11 - 4.85 (m, 5H), 4.67 - 4.41 (m, 3H), 3.78 - 3.53 (m, 2H), 2.86 - 2.71 (m, 2H), 1.75 (d, J = 6.8 Hz, 3H), | 538.08 |
| C108 | 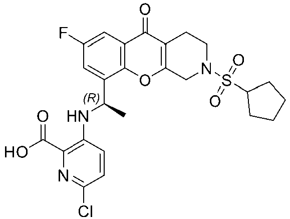 | ¹H NMR (400 MHz, DMSO-*d*₆) δ ppm 10.05 (d, J = 8.0 Hz, 1 H), 7.58 (dd, J = 8.0, 3.2 Hz, 1H), 7.50 (dd, J = 8.8, 2.8 Hz, 1H), 7.00 (d, J = 8.8 Hz, 1 H), 6.84 (d, J = 8.4 Hz, 1 H), 5.19 - 5.06 (m, 1H), 4.56 (dd, J = 45.2, 17.6 Hz, 1H), 3.97 - 3.85 (m, 1H), 3.61 - 3.48 (m, 2H), 2.64 - 2.55 (m, 2H), 2.06 - 1.93 (m, 2H), 1.92 - 1.81 (m, 2H), 1.76 - 1.53 (m, 7H) | 550.11 |
| C109 | 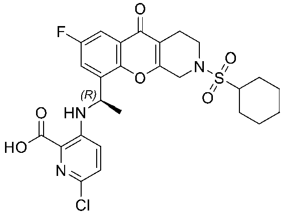 | ¹H NMR (400 MHz, DMSO-*d*₆) δ ppm 10.06 (d, J = 8.0 Hz, 1 H), 7.59 (dd, J = 8.0, 3.2 Hz, 1H), 7.49 (dd, J = 8.8, 3.2 Hz, 1H), 6.99 (d, J = 8.4 Hz, 1 H), 6.83 (d, J = 8.4 Hz, 1 H), 5.17 - 5.06 (m, 1H), 4.62 (dd, J = 43.2, 17.6 Hz, 1H), 3.63 - 3.48 (m, 2H), 3.44 - 3.36 (m, 1H), 2.63 - 2.55 (m, 2H), 2.09 - 1.99 (m, 2H), 1.85 - 1.75 (m, 2H), 1.68 - 1.59 (m, 2H), 1.58 (d, J = 6.4 Hz, 3H), 1.49 - 1.05 (m, 4H) | 564.13 |
| C110 | 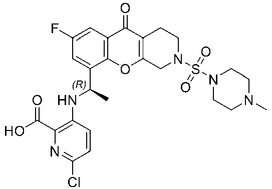 | ¹H NMR (400 MHz, DMSO-*d*₆) δ ppm 9.56 (d, J = 9.2 Hz, 1 H), 7.92 - 7.81 (m, 1H), 7.64 - 7.56 (m, 1H), 7.32 (d, J = 8.8 Hz, 1H), 7.23 (d, J = 8.8 Hz, 1H), 5.18 - 5.06 (m, 1H), 4.57 (d, J = 16.8 Hz, 1H), 4.23 (d, J = 16.4 Hz, 1H), 4.37 - 4.24 (m, 2H), 3.91 - 3.86 (m, 2H), 3.55 - 3.48 (m, 2H), 3.24 - 3.16 (m, 1H), 3.06 - 2.87 (m, 4H), 2.70 - 2.52 (m, 6H), 1.67 (d, J = 6.4 Hz, 3H), | 580.14 |
| C113 | 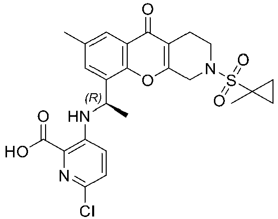 | ¹H NMR (400 MHz, DMSO-*d*₆) δ ppm 13.04(br, 1 H), 8.51 (br, 1H), 7.75 (s, 1H), 7.58 (s, 1H), 7.34 (d, J = 8.8 Hz, 1H), 7.10 (d, J = 9.2 Hz, 1H), 5.27 - 5.16 (m, 1H), 4.57 - 4.43 (m, 2H), 3.67 - 3.53 (m, 2H), 2.63 - 2.55 (m, 2H), 2.37 (s, 3H), 1.62 (d, J = 6.4 Hz, 3H), 1.44 (s, 3H), 1.32 - 1.26(m, 2H), 0.96 - 0.89(m, 2H) | 532.12 |
| C114 | 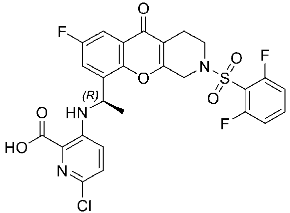 | ¹H NMR (400 MHz, DMSO-*d*₆) δ ppm 13.12(br, 1 H), 8.47 (br, 1H), 7.85 - 7.73 (m, 1H), 7.66 - 7.55 (m, 2H), 7.41 - 7.26 (m, 3H), 7.10 (d, J = 9.2 Hz, 1H), 5.35 - 5.21 (m, 1H), 4.61-4.45 (m, 2H), 3.69 - 3.50 (m, 2H), 2.60 - 2.53 (m, 2H), 1.63 (d, J = 6.4 Hz, 3H). | 594.06 |
| C115 | 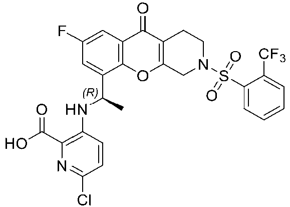 | ¹H NMR (400 MHz, DMSO-*d*₆) δ ppm 13.18(br, 1 H), 8.53 (br, 1H), 8.24(d, J = 6.8 Hz, 1H), 8.07(d, J = 6.8 Hz, 1H), 7.99 - 7.89 (m, 2H), 7.66 - 7.58 (m, 2H), 7.31 (d, J = 8.8 Hz, 1H), 7.13 (d, J = 9.2 Hz, 1H), 5.30 - 5.20 (m, 1H), 4.63 - 4.47 (m, 2H), 3.70 - 3.54 (m, 2H), 2.60 - 2.53 (m, 2H), 1.63 (d, J = 6.4 Hz, 3H). | 626.07 |
| C116 | 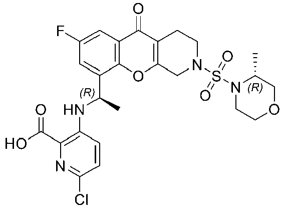 | ¹H NMR (400 MHz, DMSO-*d*₆) δ ppm 13.11(br, 1 H), 8.65 (br, 1H), 7.69 - 7.61 (m, 2H), 7.30 (d, J = 8.4 Hz, 1H), 7.13 (d, J = 9.2 Hz, 1H), 5.26 - 5.21 (m, 1H), 4.37(s, 2H), 3.85 - 3.72 (m, 2H), 3.65 - 3.57 (m, 2H), 3.48 - 3.42 (m, 2H), 3.28 - 3.24 (m, 2H), 2.63 - 2.58 (m, 2H), 1.44 (d, J = 6.4 Hz, 3H), 1.29 (d, J = 6.8 Hz, 3H). | 581.12 |
| C117 | 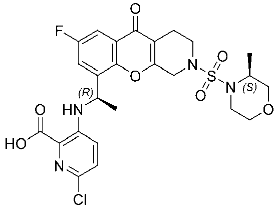 | / | 581.12 |
| C118 | 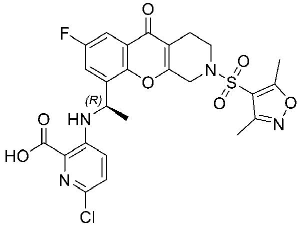 | 1H NMR (400 MHz, DMSO-*d*₆) δ ppm 8.70 (br, 1H), 7.65 - 7.59 (m, 2H), 7.28 (d, J = 8.8 Hz, 1H), 7.09 (d, J = 9.2 Hz, 1H), 5.26 - 5.20 (m, 1H), 4.51-4.32 (m, 2H), 3.60 - 3.42 (m, 2H), 2.72 (s, 3H), 2.62 - 2.51 (m, 2H), 2.39 (s, 3H), 1.63 (d, J = 6.4 Hz, 3H). | 577.09 |
| C119 | 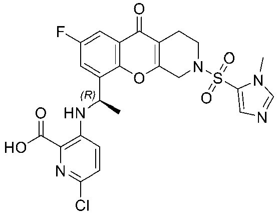 | ¹H NMR (400 MHz, DMSO-*d*₆) δ ppm 8.69 (br, 1H), 8.01 (s, 1H), 7.69 (s, 1H), 7.60 (d, J = 8.0 Hz, 2H), 7.27 (d, J = 8.4 Hz, 1H), 7.08 (d, J *=* 8.8 Hz, 1H), 5.23 (s, 1H), 4.53 - 4.32 (m, 2H), 3.82 (s, 3H), 3.56 - 3.44 (m, 2H), 2.57 (t, J = 6.6 Hz, 2H), 1.62 (d, J = 6.4 Hz, 3H). | 562.09 |
| C120 | 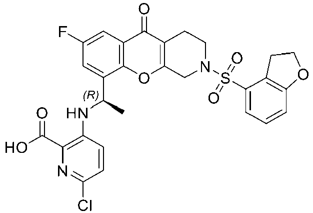 | ¹H NMR (400 MHz, DMSO-*d*₆) δ ppm 9.26 (br, 1H), 7.56 (d, *J* = 8.4 Hz, 2H), 7.39 - 7.27 (m, 2H), 7.11 (dd, *J* = 23.2, 8.8 Hz, 2H), 7.05 - 6.91 (m, 1H), 5.24 - 5.11 (m, 1H), 4.70 - 4.54 (m, 2H), 4.41 (s, 2H), 3.61 - 3.45 (m, 4H), 2.56 - 2.52 (m, 2H), 1.60 (d, *J =* 6.4 Hz, 3H). | 600.09 |
| C121 | 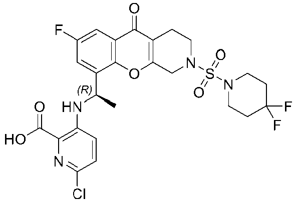 | ¹H NMR (400 MHz, DMSO-*d*₆) δ 9.52 (br, 1H), 7.68 - 7.45 (m, 2H), 7.10 (d, *J* = 8.4 Hz, 1H), 6.98 (s, 1H), 5.24 - 5.08 (m, 1H), 4.59 - 4.35 (m, 2H), 3.57 - 3.45 (m, 3H), 3.44 - 3.38 (m, 4H), 2.64 - 2.54 (m, 2H), 2.18 - 2.01 (m, 3H), 1.61 (d, *J =* 6.4 Hz, 3H). | 601.11 |
| C122 | 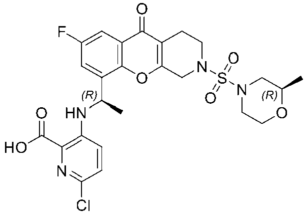 | ¹H NMR (400 MHz, DMSO-*d*₆) δ ppm 9.33 (br, 1H), 7.68 - 7.47 (m, 2H), 7.13 (d, *J* = 8.4 Hz, 1H), 7.05 - 6.90 (m, 1H), 5.23 - 5.10(s, 1H), 4.58 - 4.36 (m, 2H), 3.86 (d, *J* = 10.8 Hz, 1H), 3.60 - 3.40 (m, 6H), 2.97 - 2.86 (m, 1H), 2.63 - 2.55 (m, 3H), 1.61 (d, *J* = 6.8 Hz, 3H), 1.11 (d, *J* = 6.4 Hz, 3H). | 581.12 |
| C123 | 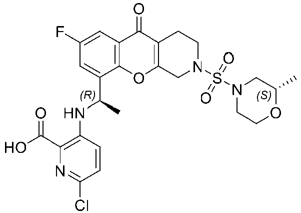 | ¹H NMR (400 MHz, DMSO-*d*₆) δ ppm 9.62 (br, 1H), 7.59 (dd, *J* = 8.0, 3.2 Hz, 1H), 7.54 (d, *J* = 8.4 Hz, 1H), 7.09 (d, *J* = 8.8 Hz, 1H), 6.95 (s, 1H), 5.26 - 5.10 (m, 1H), 4.61 - 4.38 (m, 2H), 3.85 (d, *J* = 11.2 Hz, 1H), 3.61 - 3.39 (m, 6H), 2.97 - 2.87 (m, 1H), 2.66 - 2.55 (m, 3H), 1.60 (d, *J* = 6.8 Hz, 3H), 1.10 (d, *J* = 6.4 Hz, 3H). | 581.12 |
| C124 | 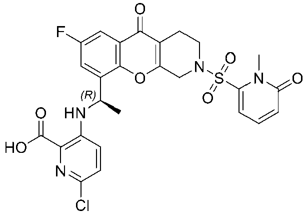 | ¹H NMR (400 MHz, DMSO-*d*₆) δ ppm 8.89 (br, 1H), 7.67 - 7.53 (m, 3H), 7.23 (d, *J* = 9.2 Hz, 1H), 7.12 - 7.00 (m, 2H), 6.77 (d, *J =* 8.8 Hz, 1H), 5.25 - 5.14 (m, 1H), 4.64 (s, 2H), 3.76 - 3.67 (m, 2H), 3.65 (s, 3H), 2.70 - 2.51 (m, 2H), 1.62 (d, *J =* 6.4 Hz, 3H). | 589.09 |
| C125 | 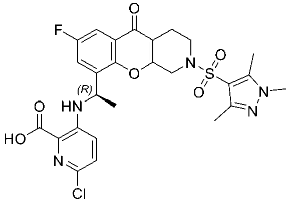 | ¹H NMR (400 MHz, DMSO-*d*₆) δ ppm 9.29 (br, 1H), 7.63 - 7.51 (m, 2H), 7.14 (d, *J =* 8.8 Hz, 1H), 7.07 - 6.94 (m, 1H), 5.19 (d, *J = 8.0* Hz, 1H), 4.34 - 4.15 (m, 2H), 3.73 (s, 3H), 3.28 - 3.13 (m, 2H), 2.60 - 2.53 (m, 2H), 2.52 - 2.50 (m, 3H), 2.31 (s, 3H), 1.60 (d, *J* = 6.4 Hz, 3H). | 590.12 |
| C126 | 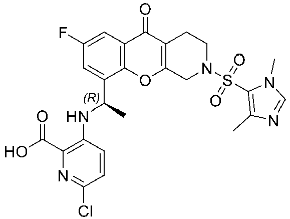 | ¹H NMR (400 MHz, DMSO-*d*₆) δ ppm 8.58 (br, 1H), 7.88 (s, 1H), 7.68 - 7.55 (m, 2H), 7.30 (d, *J =* 9.2 Hz, 1H), 7.12 (d, *J = 9.2* Hz, 1H), 5.25 (s, 1H), 4.49 - 4.33 (m, 2H), 3.76 (s, 3H), 3.54 - 3.39 (m, 2H), 2.56 - 2.52 (m, 1H), 2.38 (s, 3H), 1.63 (d, *J =* 6.4 Hz, 3H). | 576.10 |
| C127 | 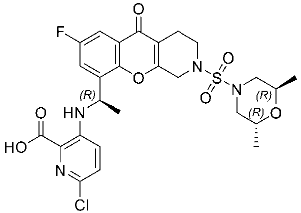 | ¹H NMR (400 MHz, DMSO-*d*₆) δ ppm 8.97 (br, 1H), 7.62 (d, *J* = 8.4 Hz, 2H), 7.22 (d, *J* = 8.8 Hz, 1H), 7.05 (d, *J* = 8.8 Hz, 1H), 5.20 (d, *J* = 4.0 Hz, 1H), 4.54 - 4.38 (d, *J* = 2.8 Hz, 2H), 4.07 - 3.89 (m, 2H), 3.59 - 3.45 (m, 2H), 3.29 - 3.23 (m, 2H), 2.95 - 2.86 (m, 2H), 2.64 - 2.55 (m, 2H), 1.63 (d, *J = 6.8* Hz, 3H), 1.15 (d, *J =* 6.4 Hz, 6H). | 595.14 |
| C128 | 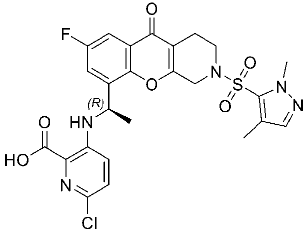 | ¹H NMR (400 MHz, DMSO-*d*₆) δ ppm 10.15 (dd, *J =* 34.8, 7.2 Hz, 1H), 8.49 (s, 1H), 7.66 - 7.48 (m, 2H), 7.00 - 6.90 (m, 1H), 6.84 - 6.62 (m, 1H), 5.35 - 5.08 (m, 2H), 4.60 - 4.41 (m, 1H), 3.86 (s, 3H), 2.69 - 2.57 (m, 2H), 2.05 - 1.96 (m, 3H), 1.63 - 1.51 (m, 3H), 1.28 - 1.88 (m, 3H). | 576.10 |
| C129 | 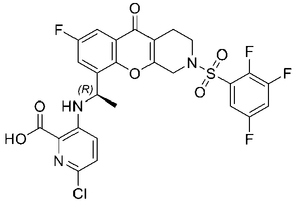 | ¹H NMR (400 MHz, DMSO-*d*₆) δ ppm 13.06 (br, 1H), 8.50 (s, 1H), 8.05 - 7.94 (m, 1H), 7.70 - 7.55 (m, 3H), 7.30 (d, *J* = 9.6 Hz, 1H), 7.09 (d, *J* = 9.2 Hz, 1H), 5.31 - 5.20 (m, 1H), 4.63 - 4.37 (m, 2H), 3.68 - 3.49 (m, 2H), 2.56 (t, *J* = 6.4 Hz, 2H), 1.63 (d, *J* = 6.8 Hz, 3H). | 612.05 |
| C130 | 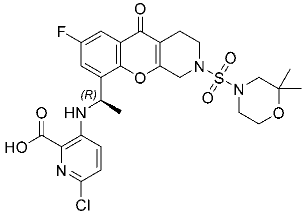 | ¹H NMR (400 MHz, DMSO-*d*₆) δ ppm 8.45 (d, *J* = 6.8 Hz, 1H), 7.70 - 7.56 (m, 2H), 7.34 (d, *J* = 8.4 Hz, 1H), 7.17 (d, *J* = 9.6 Hz, 1H), 5.29 - 5.19 (m, 1H), 4.50 - 4.36 (m, 2H), 3.72 - 3.63 (m, 2H), 3.58 - 3.44 (m, 3H), 3.19 - 3.10 (m, 3H), 3.00 (s, 2H), 2.59 (t, *J =* 5.6 Hz, 2H), 1.65 (d, *J = 6.8* Hz, 3H), 1.19 (s, 6H). | 597.15 |
| C131 | 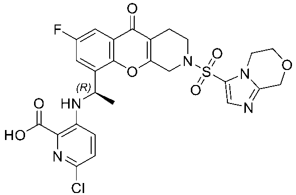 | ¹H NMR (400 MHz, DMSO-*d*₆) δ ppm 12.93 (br, 1H), 8.41 (d, *J* = 6.8 Hz, 1H), 7.70 (s, 1H), 7.66 - 7.55 (m, 2H), 7.33 (d, *J* = 8.8 Hz, 1H), 7.14 (d, *J* = 9.2 Hz, 1H), 5.37 - 5.13 (m, 1H), 4.81 (s, 2H), 4.50 - 4.32 (m, 2H), 4.24 - 4.12 (m, 2H), 4.11 - 3.98 (m, 2H), 3.53 - 3.41 (m, 2H), 2.59 (t, *J =* 6.0 Hz, 2H), 1.64 (d, *J =* 6.8 Hz, 3H). | 604.10 |
| C132 | 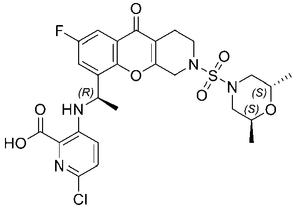 | ¹H NMR (400 MHz, DMSO-*d*₆) δ ppm 9.24 (br, 1H), 7.67 - 7.46 (m, 2H), 7.15 (d, *J* = 8.4 Hz, 1H), 7.01 (d, *J* = 8.4 Hz, 1H), 5.18 (s, 1H), 4.64 - 4.35 (m, 2H), 4.10 - 3.85 (m, 2H), 3.54 - 3.47 (m, 2H), 3.29 - 3.24 (m, 4H), 2.96 - 2.85 (m, 2H), 2.59 (t, *J* = 5.6 Hz, 2H), 1.61 (d, *J* = 6.8 Hz, 2H), 1.16 (d, *J* = 6.4 Hz, 6H). | 595.14 |
| C133 | 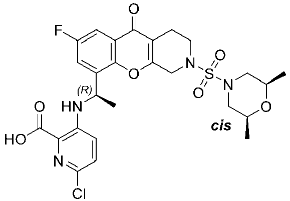 | ¹H NMR (400 MHz, DMSO-*d*₆) δ ppm 9.42 (br, 1H), 7.63 - 7.50 (m, 2H), 7.12 (d, *J =* 8.4 Hz, 1H), 6.96 (d, *J =* 8.8 Hz, 1H), 5.17 (d, *J =* 6.4 Hz, 1H), 4.45 - 4.38 (m, 2H), 4.10 - 3.85 (m, 2H), 3.66 - 3.44 (m, 6H), 2.62-2.52 (m, 4H), 1.60 (d, *J =* 6.8 Hz, 2H), 1.16 (d, *J =* 6.4 Hz, 6H). | 595.14 |
| C134 | 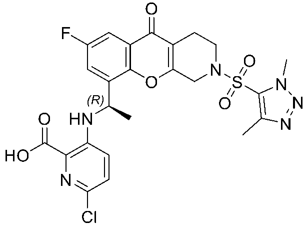 | ¹H NMR (400 MHz, DMSO-*d*₆) δ ppm 9.19 (br, 1H), 7.66 - 7.54 (m, 2H), 7.16 (d, *J =* 8.8 Hz, 1H), 7.00 (d, *J =* 8.8 Hz, 1H), 5.19 (d, *J =* 7.6 Hz, 1H), 4.56 (s, 2H), 4.24 (s, 3H), 3.66 - 3.58 (m, 2H), 3.55 - 3.50 (m, 3H), 2.57 (t, *J =* 6.4 Hz, 2H), 2.49 (s, 3H), 1.60 (d, *J =* 6.6 Hz, 3H). | 577.10 |
| C135 | 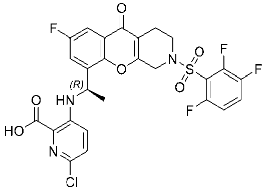 | ¹H NMR (400 MHz, DMSO-*d*₆) δ ppm 9.46 (br, 1H), 7.97 - 7.81 (m, 1H), 7.60 - 7.53 (m, 1H), 7.53 - 7.39 (m, 2H), 7.09 (d, *J* = 8.8 Hz, 1H), 6.97 - 6.87 (m, 1H), 5.21 (s, 1H), 4.64 - 4.49 (m, 2H), 3.71 - 3.50 (m, 3H), 2.64 - 2.54 (m, 2H), 1.58 (d, *J* = 6.8 Hz, 2H). | 612.05 |
| C136 | 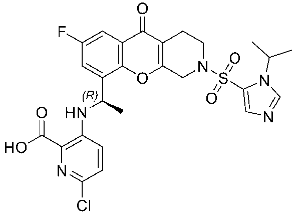 | ¹H NMR (400 MHz, DMSO-*d*₆) δ ppm 8.48 (br, 1H), 8.28 (s, 1H), 7.68 - 7.58 (m, 3H), 7.32 (d, *J* = 8.8 Hz, 1H), 7.13 (d, *J* = 9.2 Hz, 1H), 5.30 - 5.20 (m, 1H), 4.75 - 4.68 (m, 1H), 4.53 - 4.39 (m, 2H), 3.59 - 3.44(m, 3H), 2.55 - 2.51 (m, 1H), 1.63 (d, *J =* 6.4 Hz, 3H), 1.43 (dd, *J* = 10.4, 6.4 Hz, 6H). | 590.12 |
| C137 | 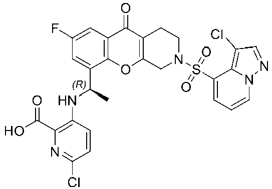 | ¹H NMR (400 MHz, DMSO-*d*₆) δ ppm 12.94 (br, 1H), 9.03 (dd, *J =* 6.8, 0.8 Hz, 1H), 8.45 (s, 1H), 8.34 (s, 1H), 8.05 (dd, *J =* 8.0, 0.8 Hz, 1H), 7.68 - 7.56 (m, 2H), 7.32 (d, *J =* 8.8 Hz, 1H), 7.19 - 7.09 (m, 2H), 5.29 - 5.17 (m, 1H), 4.66 - 4.48 (m, 2H), 3.70 - 3.55 (m, 2H), 2.58 - 2.52 (m, 2H), 1.63 (d, *J =* 6.8 Hz, 3H). | 632.05 |
| C138 | 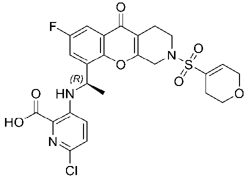 | ¹H NMR (400 MHz, DMSO-*d*₆) δ ppm 13.06 (br, 1H), 8.45 (s, 1H), 7.68 - 7.58 (m, 2H), 7.33 (d, *J* = 8.8 Hz, 1H), 7.14 (d, *J* = 8.8 Hz, 1H), 6.92 - 6.80 (m, 1H), 5.29 - 5.14 (m, 1H), 4.39 (d, *J =* 7.2 Hz, 2H), 4.34 - 4.20 (m, 2H), 3.74 (t, *J* = 5.2 Hz, 2H), 3.54 - 3.41 (m, 2H), 2.62 - 2.55 (m, 2H), 2.38 - 2.30 (m, 2H), 1.64 (d, *J =* 6.8 Hz, 3H). | 564.09 |
| C139 | 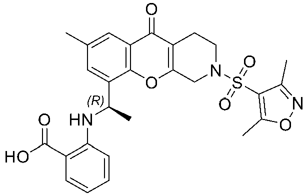 | ¹H NMR (400 MHz, DMSO-*d*₆) δ ppm 12.74 (br, 1H), 8.42 (d, *J* = 7.2 Hz, 1H), 7.81 (dd, *J =* 8.0, 1.6 Hz, 1H), 7.73 - 7.68 (m, 1H), 7.54 (d, *J* = 2.4 Hz, 1H), 7.26 - 7.15 (m, 1H), 6.58 - 6.48 (m, 2H), 5.26 - 5.11 (m, 1H), 4.50 - 4.33 (m, 2H), 3.52 - 3.46 (m, 2H), 2.69 (s, 3H), 2.62 - 2.54 (m, 2H), 2.37 (s, 3H), 2.35 (s, 3H), 1.59 (d, *J =* 6.8 Hz, 3H). | 538.16 |
| C140 | 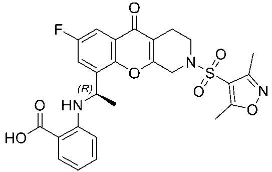 | ¹H NMR (400 MHz, DMSO-*d*₆) δ ppm 12.77 (br, 1H), 8.42 (d, *J* = 7.2 Hz, 1H), 7.82 (dd, *J* = 8.0, 1.6 Hz, 1H), 7.65 - 7.47 (m, 2H), 7.28 - 7.14 (m, 1H), 6.57 (t, *J* = 7.2 Hz, 1H), 6.50 (d, *J =* 8.8 Hz, 1H), 5.29 - 5.16(m, 1H), 4.53 - 4.34 (m, 2H), 3.58 - 3.45 (m, 2H), 2.70 (s, 3H), 2.64 - 2.55 (m, 2H), 2.38 (s, 3H), 1.62 (d, *J =* 6.8 Hz, 3H). | 542.13 |
| C141 | 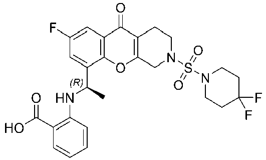 | ¹H NMR (400 MHz, DMSO-*d*₆) δ ppm 12.79 (br, 1H), 8.45 (s, 1H), 7.81 (dd, *J* = 8.0*,* 1.6 Hz, 1H), 7.66 - 7.49 (m, 2H), 7.28 - 7.15 (m, 1H), 6.66 - 6.40 (m, 2H), 5.28 - 5.13 (m, 1H), 4.55 - 4.32 (m, 2H), 3.57 - 3.43 m, 2H), 3.39 (t, *J* = 5.6 Hz, 4H), 2.64 - 2.54 (m, 2H), 2.15 - 2.99 (m, 4H), 1.62 (d, *J =* 6.8 Hz, 3H). | 566.15 |
| C142 | 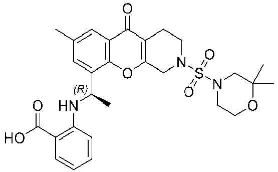 | ¹H NMR (400 MHz, DMSO-*d*₆) δ ppm 12.73 (br, 1H), 8.45 (d, *J =* 6.4 Hz, 1H), 7.80 (dd, *J* = 8.0, 1.6 Hz, 1H), 7.75 - 7.70 (m, 1H), 7.55 (d, *J* = 2.0 Hz, 1H), 7.26 - 7.16 (m, 1H), 6.61 - 6.46 (m, 2H), 5.23 - 5.11 (m, 1H), 4.50 - 4.34 (m, 2H), 3.79 - 3.59 (m, 2H), 3.60 - 3.44 (m, 2H), 3.19 - 3.10 (m, 2H), 2.99 (s, 2H), 2.64 - 2.52 (m, 2H), 2.36 (s, 3H), 1.60 (d, *J =* 6.8 Hz, 3H), 1.18 (s, 6H). | 556.20 |
| C143 | 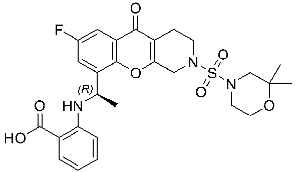 | ¹H NMR (400 MHz, DMSO-*d*₆) δ ppm 12.80 (br, 1H), 8.44 (d, *J* = 8.4 Hz, 1H), 7.82 (dd, *J* = 8.0, 1.6 Hz, 1H), 7.61 (dd, *J =* 8.0, 3.2 Hz, 1H), 7.57 (dd, *J =* 9.2, 3.2 Hz, 1H), 7.26 - 7.17 (m, 1H), 6.61 - 6.49 (m, 2H), 5.27 - 5.16 (q, *J =* 6.7 Hz, 1H), 4.53 - 4.36 (m, 2H), 3.74 - 3.64 (m, 2H), 3.61 - 3.45 (m, 2H), 3.19 - 3.09 (m, 2H), 3.00 (s, 2H), 2.60 (t, *J =* 5.2 Hz, 2H), 1.63 (d, *J* = 6.8 Hz, 3H), 1.19 (s, 6H). | 560.18 |
| C144 | 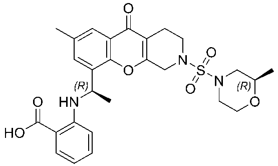 | ¹H NMR (400 MHz, DMSO-*d*₆) δ ppm 12.74 (br, 1H), 8.42 (s, 1H), 7.80 (dd, *J* = 8.0, 1.6 Hz, 1H), 7.75 - 7.71 (m, 1H), 7.55 (d, *J* = 2.4 Hz, 1H), 7.27 - 7.17 (m, 1H), 6.59 - 6.48 (m, 2H), 5.25 - 5.09(m, 1H), 4.53 - 4.33 (m, 2H), 3.90 - 3.81 (m, 1H), 3.56 - 3.39 (m, 6H), 2.95 - 2.85 (m, 1H), 2.63 - 2.55 (m, 3H), 2.36 (s, 3H), 1.61 (d, *J =* 6.8 Hz, 3H), 1.10 (d, *J =* 6.4 Hz, 3H). | 542.19 |
| C145 | 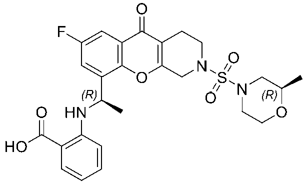 | ¹H NMR (400 MHz, DMSO-*d*₆) δ ppm 12.80 (br, 1H), 8.42 (d, *J =* 7.2 Hz, 1H), 7.82 (dd, *J =* 8.0, 1.6 Hz, 1H), 7.61 (dd, *J =* 8.0, 3.2 Hz, 1H), 7.56 (d, *J* = 2.4 Hz, 1H), 7.27 - 7.19 (m, 1H), 6.61 - 6.49 (m, 2H), 5.28 - 5.16 m, 1H), 4.45 (d, *J =* 6.8 Hz, 1H), 3.90 - 3.81 (m, 1H), 3.60 - 3.49 (m, 3H), 3.52 - 3.38 (m, 2H), 2.97 - 2.85 (m, 1H), 2.64 - 2.55 (m, 3H), 1.63 (d, *J =* 6.8 Hz, 3H), 1.10 (d, *J* = 6.4 Hz, 3H). | 546.16 |
| C146 | 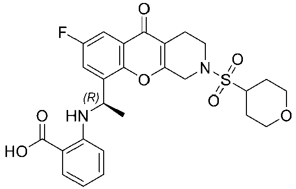 | ¹H NMR (400 MHz, DMSO-*d*₆) δ ppm 12.75 (br, 1H), 8.47 (s, 1H), 7.82 (dd, *J* = 8.0*,* 1.6 Hz, 1H), 7.61 (dd, *J =* 8.0, 3.2 Hz, 1H), 7.55 (dd, *J =* 8.8, 3.2 Hz, 1H), 7.27 - 7.18 (m, 1H), 6.57 (t, *J =* 7.6 Hz, 1H), 6.50 (d, *J =* 8.4 Hz, 1H), 5.27 - 5.13 (m, 1H), 4.63 - 4.43 (m, 2H), 4.03 - 3.86 (m, 2H), 3.72 - 3.49 (m, 2H), 3.40 - 3.34 (m, 3H), 2.65 - 2.55 (m, 2H), 1.95 - 1.85 (m, 2H), 1.77 - 1.66 (m, 2H), 1.63 (d, *J =* 6.8 Hz, 3H). | 531.15 |
| C147 | 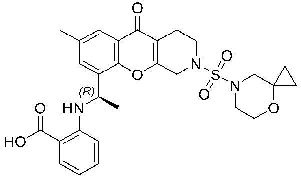 | ¹H NMR (400 MHz, DMSO-*d*₆) δ ppm 8.66 (br, 1H), 7.81 (dd, *J =* 8.0, 1.6 Hz, 1H), 7.74 - 7.71 (m, 1H), 7.55 (d, *J =* 2.4 Hz, 1H), 7.21 - 7.14 (m, 1H), 6.59 - 6.43 (m, 2H), 5.21 - 5.09 (m, 1H), 4.50 - 4.34 (m, 2H), 3.72 - 3.64 (m, 2H), 3.57 - 3.47 (m, 2H), 3.33 - 3.25 (m, 2H), 3.20 (s, 2H), 2.63 - 2.58 (m, 2H), 2.35 (s, 3H), 1.59 (d, *J =* 6.8 Hz, 3H), 0.75 - 0.69 (m, 2H), 0.66 - 0.60 (m, 2H). | 554.19 |
| C148 | 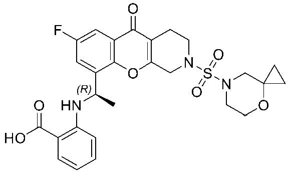 | ¹H NMR (400 MHz, DMSO-*d*₆) δ ppm 12.96 (br, 1H), 8.48 (s, 1H), 7.82 (dd, *J* = 8.0*,* 1.6 Hz, 1H), 7.61 (dd, *J* = 8.0, 3.2 Hz, 1H), 7.57 (dd, *J =* 8.8, 3.2 Hz, 1H), 7.29 - 7.15 (m, 1H), 6.65 - 6.47 (m, 2H), 5.30 - 5.11 (m, 1H), 4.59 - 4.31 m, 2H), 3.76 - 3.64 (m, 2H), 3.60 - 3.46 (m, 2H), 3.31 - 3.24 (m, 2H), 3.21 (s, 2H), 2.61 (t, *J =* 5.6 Hz, 2H), 1.63 (d, *J =* 6.8 Hz, 3H), 0.74 - 0.67 (m, 2H), 0.66 - 0.60 (m, 2H). | 558.16 |
| C149 | 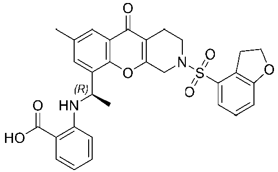 | ¹H NMR (400 MHz, DMSO-*d*₆) δ ppm 8.50 (s, 1H), 7.81 (dd, *J =* 8.0, 1.6 Hz, 1H), 7.69 (d, *J* = 2.4 Hz, 1H), 7.54 (d, *J* = 2.4 Hz, 1H), 7.38 - 7.26 (m, 2H), 7.24 - 7.15 m, 1H), 7.12 - 7.00 (m, 1H), 6.61 - 6.41 (m, 2H), 5.25 - 5.07 (m, 1H), 4.56 (t, *J =* 8.8 Hz, 2H), 4.45 - 4.29 (m, 2H), 3.53 - 3.43 (m, 4H), 2.52 (s, 2H), 2.34 (s, 3H), 1.59 (d, *J* = 6.8 Hz, 3H). | 561.16 |
| C150 | 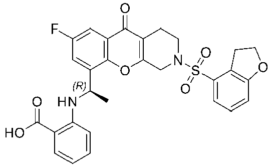 | ¹H NMR (400 MHz, DMSO-*d*₆) δ ppm 12.74 (br, 1H), 8.48 (s, 1H), 7.82 (dd, *J* = 8.0*,* 1.8 Hz, 1H), 7.59 - 7.52 (m, 2H), 7.34 - 7.28 (m, 2H), 7.25 - 7.17 (m, 1H), 7.13 - 7.02 (m, 1H), 6.56 (t, *J* = 7.6 Hz, 1H), 6.51 (d, *J* = 8.4 Hz, 1H), 5.29 - 5.13 (m, 1H), 4.57 (t, *J* = 8.8 Hz, 2H), 4.47 - 4.35 (m, 2H), 3.52 - 3.45 (m, 4H), 2.54 - 2.51 (m, 2H), 1.62 (d, *J =* 6.8 Hz, 3H). | 565.14 |
| C151 | 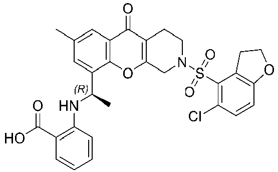 | ¹H NMR (400 MHz, DMSO-*d*₆) δ ppm 8.49 (s, 1H), 7.80 (dd, *J =* 8.0, 1.6 Hz, 1H), 7.70 (d, *J* = 2.4 Hz, 1H), 7.55 (d, *J* = 2.4 Hz, 1H), 7.36 (d, *J* = 8.4 Hz, 1H), 7.26 - 7.15 (m, 1H), 7.05 (d, *J* = 8.4 Hz, 1H), 6.58 - 6.45 (m, 2H), 5.26 - 5.07 (m, 1H), 4.71 - 4.51 (m, 4H), 3.69 - 3.55 (m, 4H), 2.43 - 2.37 (m, 2H), 2.35 (s, 3H), 1.59 (d, *J* = 6.8 Hz, 3H). | 595.12 |
| C152 | 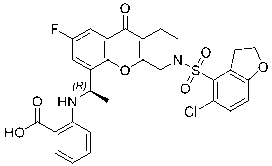 | ¹H NMR (400 MHz, DMSO-*d*₆) δ ppm 8.52 (s, 1H), 7.82 (dd, *J=* 8.0, 1.6 Hz, 1H), 7.63 - 7.49 (m, 2H), 7.37 (d, *J =* 8.4 Hz, 1H), 7.25 - 7.12 (m, 1H), 7.04 (d, *J* = 8.4 Hz, 1H), 6.60 - 6.52 (m, 1H), 6.49 (d, *J =* 8.4 Hz, 1H), 5.28 - 5.14 (m, 1H), 4.72 - 4.55 (m, 4H), 3.69 - 3.54 (m, 4H), 2.46 - 2.36 (m, 2H), 1.61 (d, *J* = 6.8 Hz, 3H). | 599.10 |
| C153 | 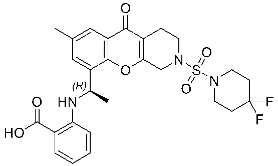 | ¹H NMR (400 MHz, DMSO-*d*₆) δ ppm 12.75 (br, 1H), 8.45 (d, *J* = 7.2 Hz, 1H), 7.81 (dd, *J =* 8.0, 1.6 Hz, 1H), 7.73 (d, *J* = 2.4 Hz, 1H), 7.55 (d, *J* = 2.4 Hz, 1H), 7.27 - 7.16 (m, 1H), 6.58 - 6.48 (m, 2H), 5.28 - 5.03 (m, 1H), 4.49 - 4.22 (m, 2H), 3.59 - 3.45 (m, 2H), 3.43 - 3.37 (m, 4H), 2.63 - 2.55 (m, 2H), 2.36 (s, 3H), 2.16 - 1.97 (m, 4H), 1.61 (d, *J =* 6.8 Hz, 3H). | 562.17 |
| C154 | 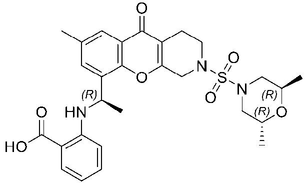 | ¹H NMR (400 MHz, DMSO-*d*₆) δ ppm 8.66 (br, 1H), 7.80 (d, *J* = 8.0 Hz, 1H), 7.72 (d, *J* = 2.0 Hz, 1H), 7.55 (d, *J* = 2.4 Hz, 1H), 7.27 - 7.10 (m, 1H), 6.58 - 6.43 (m, 2H), 5.21 - 5.07 (m, 1H), 4.51 - 4.31 (m, 2H), 4.06 - 3.89 (m, 2H), 3.55 - 3.46 (m, 2H), 3.24 (dd, *J* = 12.0, 3.2 Hz, 2H), 2.92 - 2.84 (m, 2H), 2.65 - 2.54 m, 2H), 2.34 (s, 3H), 1.58 (d, *J* = 6.8 Hz, 3H), 1.13 (d, *J* = 6.4 Hz, 6H). | 556.20 |
| C155 | 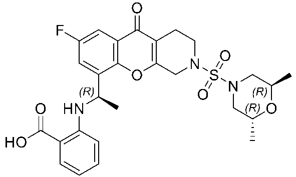 | ¹H NMR (400 MHz, DMSO-*d*₆) δ ppm 8.56 (br, 1H), 7.82 (dd, *J =* 8.0, 1.6 Hz, 1H), 7.61 (dd, *J =* 8.0, 3.2 Hz, 1H), 7.57 (dd, *J =* 9.2, 3.2 Hz, 1H), 7.25 - 7.16 (m, 1H), 6.61 - 6.44 (m, 2H), 5.27 - 5.16 (m, 1H), 4.54 - 4.36 (m, 2H), 4.04 - 3.92 m, 2H), 3.58 - 3.46 (m, 2H), 3.25 (dd, *J =* 12.0, 3.2 Hz, 2H), 2.94 - 2.86 (m, 2H), 2.64 - 2.57 (m, 2H), 1.63 (d, *J =* 6.8 Hz, 3H), 1.15 (d, *J =* 6.4 Hz, 6H). | 560.18 |
| C156 | 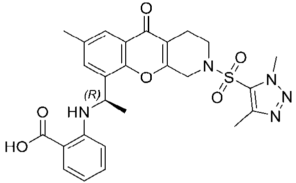 | ¹H NMR (400 MHz, DMSO-*d*₆) δ ppm 8.89 (br, 1H), 7.80 (d, *J =* 7.6 Hz, 1H), 7.70 (d, *J =* 2.4 Hz, 1H), 7.55 (d, *J =* 2.4 Hz, 1H), 7.12 (t, *J =* 7.6 Hz, 1H), 6.53 - 6.38 (m, 2H), 5.22 - 5.08 (m, 1H), 4.54 (s, 2H), 4.22 (s, 3H), 3.64 - 3.55 (m, 2H), 2.58 - 2.53 (m, 2H), 2.47 (s, 3H), 2.34 (s, 3H), 1.57 (d, *J =* 6.8 Hz, 3H). | 538.17 |
| C157 | 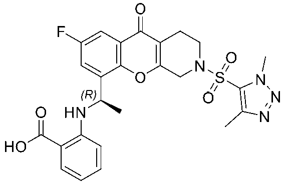 | ¹H NMR (400 MHz, DMSO-*d*₆) δ ppm 9.80 (br, 1H), 8.47 (t, *J =* 2.4 Hz, 1H), 7.81 (dd, *J = 7.6,* 2.0 Hz, 1H), 7.65 - 7.48 (m, 3H), 7.05 - 6.91 (m, 1H), 6.49 - 6.26 (m, 3H), 5.26-5.12 (m, 1H), 4.01 (s, 3H), 3.93 - 3.82 (m, 2H), 2.65-2.55 (m, 2H), 2.23 (s, 3H), 1.59 (d, *J =* 6.8 Hz, 3H). | 542.14 |
| C158 | 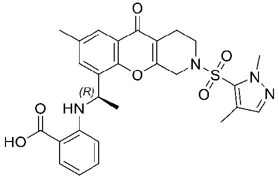 | ¹H NMR (400 MHz, DMSO-*d*₆) δ ppm 8.46 (s, 1H), 7.81 (dd, *J =* 8.0, 1.6 Hz, 1H), 7.72 - 7.67 (m, 1H), 7.55 (d, *J =* 2.0 Hz, 1H), 7.49 (s, 1H), 7.26 - 7.15 (m, 1H), 6.60 - 6.45 (m, 2H), 5.25 - 5.08 (m, 1H), 4.55 - 4.40 (m, 2H), 4.00 (s, 3H), 3.58 - 3.52 (m, 2H), 2.50 - 2.46 (m, 2H), 2.35 (s, 3H), 2.21 (s, 3H), 1.59 (d, *J =* 6.8 Hz, 3H). | 537.17 |
| C159 | 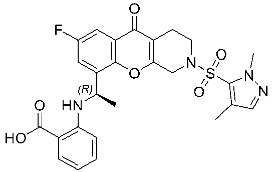 | ¹H NMR (400 MHz, DMSO-*d*₆) δ ppm 12.50 (br, 1H), 8.45 (s, 1H), 7.82 (dd, *J* = 8.0*,* 1.6 Hz, 1H), 7.62 - 7.53 (m, 2H), 7.50 (s, 1H), 7.26 - 7.16 (m, 1H), 6.61 - 6.53 (m, 1H), 6.51 (d, *J =* 8.8 Hz, 1H), 5.29 - 5.18 (m, 1H), 4.58 - 4.42 (m, 2H), 4.02 (s, 3H), 3.59 - 3.49 (m, 2H), 2.54 - 2.51 (m, 2H), 2.22 (s, 3H), 1.62 (d, *J =* 6.8 Hz, 3H). | 541.15 |
| C160 | 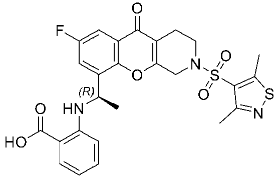 | ¹H NMR (400 MHz, DMSO-d6) δ ppm 8.46 (s, 1H), 7.82 (d, J = 8.0 Hz, 1H), 7.65 - 7.50(m, 2H), 7.22 (t, J = 7.6 Hz, 1H), 6.56 (t, J = 7.6 Hz, 1H), 6.50 (d, J = 8.4 Hz, 1H), 5.36 - 5.17 (m, 1H), 4.59 - 4.36 (m, 2H), 3.62 - 3.48 (m, 2H), 2.79 (s, 3H), 2.58 (s, 3H), 2.56 - 2.53 (m, 2H), 1.61 (d, J = 6.8 Hz, 3H). | 558.11 |
| C161 | 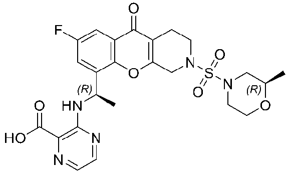 | ¹H NMR (400 MHz, DMSO-*d*₆) ppm δ 10.44 - 10.08 (m, 1H), 8.21 - 7.70 (m, 2H), 7.65 - 7.48 (m, 2H), 7.40 - 7.08 (m, 1H), 5.73 - 5.55 (m, 1H), 4.68 - 4.49 (m, 1H), 4.45- 4.29 (m, 1H), 3.90 - 3.71 (m, 1H), 3.66 - 3.41 (m, 6H), 2.98 - 2.80 (m, 1H), 2.59 (s, 3H), 1.61 (d, J = 3.2 Hz, 3H), 1.14 - 0.95 (m, 3H). | 548.15 |
| C162 | 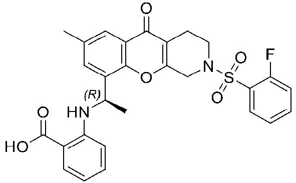 | ¹H NMR (400 MHz, DMSO-*d*₆) δ ppm 12.71 (br, 1H), 8.45 (s, 1H), 7.98 - 7.87 (m, 1H), 7.81 (dd, *J =* 8.0, 1.6 Hz, 1H), 7.78 - 7.70 (m, 1H), 7.69 - 7.64 (m, 1H), 7.53 (d, *J =* 2.4 Hz, 1H), 7.48 - 7.38 (m, 2H), 7.25 - 7.16 (m, 1H), 6.63 - 6.42 (m, 2H), 5.26 - 5.06 (m, 1H), 4.61 - 4.38 (m, 2H), 3.64 - 3.49 (m, 2H), 2.47 - 2.40 (m, 2H), 2.33 (s, 3H), 1.59 (d, *J =* 6.8 Hz, 3H). | 537.14 |
| C165 | 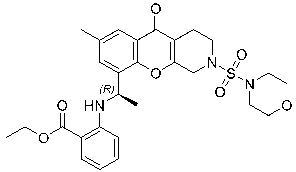 | ¹H NMR (400 MHz, DMSO-*d*₆) δ ppm 8.33 (d, *J* = 7.6 Hz, 1H), 7.82 (dd, *J =* 8.0, 1.6 Hz, 1H), 7.77 - 7.70 (m, 1H), 7.60 (d, *J =* 2.4 Hz, 1H), 7.32 - 7.20(m, 1H), 6.69 - 6.51 (m, 2H), 5.23 - 5.11 (m, 1H), 4.46 (s, 2H), 4.38 - 4.26 (m, 2H), 3.70 - 3.60 (m, 4H), 3.58 - 3.47 (m, 2H), 3.23 - 3.11 (m, 4H), 2.63 - 2.55 m, 2H), 2.37 (s, 3H), 1.62 (d, *J* = 6.8 Hz, 3H), 1.34 (t, *J* = 7.2 Hz, 3H). | 556.20 |
| C168 | 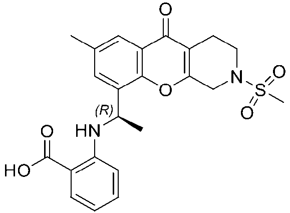 | ¹H NMR (400 MHz, DMSO-*d*₆) δ ppm 12.75 (br, 1H), 8.46 (d, *J* = 6.8 Hz, 1H), 7.81 (dd, *J =* 8.0, 1.6 Hz, 1H), 7.74 (d, *J* = 2.0 Hz, 1H), 7.54 (d, *J* = 2.4 Hz, 1H), 7.27 - 7.17 (m, 1H), 6.59 - 6.47 (m, 2H), 5.22 - 5.09 (m, 1H), 4.58 - 4.34 (m, 2H), 3.55 - 3.40 (m, 2H), 3.07 (s, 3H), 2.67 - 2.57 (m, 2H), 2.35 (s, 3H), 1.61 (d, *J =* 6.8 Hz, 3H). | 457.14 |
| C169 | 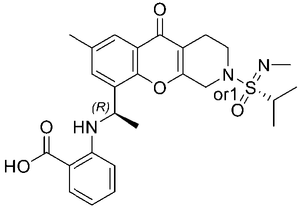 | ¹H NMR (400 MHz, DMSO-*d*₆) δ ppm 12.68 (br, 1H), 8.46 (s, 1H), 7.82 (d, *J* = 8.0 Hz, 1H), 7.76 - 7.67 (m, 1H), 7.54 (s, 1H), 7.26 - 7.13 (m, 1H), 6.61 - 6.43 (m, 2H), 5.22 - 5.07 (m, 1H), 4.55 - 4.32 (m, 2H), 3.64 - 3.46 (m, 2H), 3.43 - 3.38 (m, 1H), 2.63 - 2.54 (m, 2H), 2.35 (s, 3H), 1.59 (d, *J =* 6.8 Hz, 3H), 1.34 - 1.21(m, 9H). | 498.20 |
| C170 | 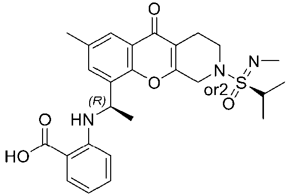 | ¹H NMR (400 MHz, DMSO-*d*₆) δ ppm 12.74 (br, 1H), 8.47 (s, 1H), 7.86 - 7.76 (m, 1H), 7.73 (d, *J* = 2.0 Hz, 1H), 7.54 (d, *J* = 2.4 Hz, 1H), 7.21 (t, *J* = 7.6 Hz, 1H), 6.60 - 6.44 (m, 2H), 5.22 - 5.11 (m, 1H), 4.43 (s, 2H), 3.57 - 3.48 (m, 2H), 3.43 - 3.37 (m, 1H), 2.61 - 2.53 (m, 2H), 2.35 (s, 3H), 1.60 (d, *J =* 6.8 Hz, 3H), 1.32 - 1.19(m, 9H). | 498.20 |
| C171 | 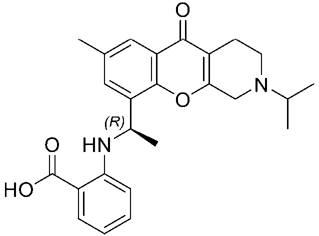 | ¹H NMR (400 MHz, DMSO-*d*₆) δ ppm 8.46 (d, *J* = 6.8 Hz, 1H), 7.80 (dd, *J =* 8.0, 1.6 Hz, 1H), 7.71 (d, *J* = 2.0 Hz, 1H), 7.52 (d, *J* = 2.4 Hz, 1H), 7.28 - 7.17 (m, 1H), 6.58 - 6.47 (m, 2H), 5.18 - 5.08 (m, 1H), 3.64 (s, 2H), 2.98 - 2.88 (m, 1H), 2.70 (t, *J =* 5.6 Hz, 2H), 2.48 - 2.43 (m, 2H), 2.35 (s, 3H), 1.60 (d, *J =* 6.8 Hz, 3H), 1.09 (d, *J =* 6.4 Hz, 6H). | 421.20 |
| C172 | 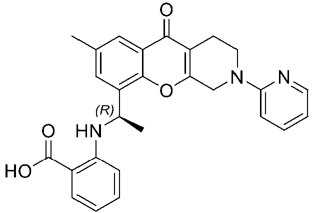 | ¹H NMR (400 MHz, DMSO-*d*₆) δ ppm 12.73 (br, 1H), 8.53 (d, *J* = 7.2 Hz, 1H), 8.19 (dd, *J* = 5.2, 2.0 Hz, 1H), 7.81 (dd, *J* = 8.0, 1.6 Hz, 1H), 7.73 (d, *J* = 2.4 Hz, 1H), 7.66 - 7.58 (m, 1H), 7.56 (d, *J* = 2.4 Hz, 1H), 7.28 - 7.19 (m, 1H), 7.03 (d, *J* = 8.4 Hz, 1H), 6.76 - 6.68 (m, 1H), 6.62 - 6.50 (m, 2H), 5.26 - 5.12 (m, 1H), 4.83 - 4.56 (m, 2H), 3.92 - 3.75 (m, 2H), 2.64 - 2.56 (t, *J* = 5.7 Hz, 2H), 2.36 (s, 3H), 1.65 (d, *J* = 6.8 Hz, 3H). | 456.18 |
| C173 | 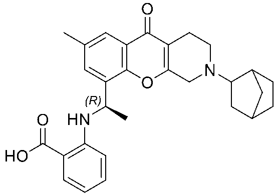 | ¹H NMR (400 MHz, DMSO-*d*₆) δ ppm 8.43 (d, *J* = 7.2 Hz, 1H), 7.83 - 7.75 (m, 1H), 7.71 (d, *J* = 2.4 Hz, 1H), 7.53 (s, 1H), 7.27 - 7.16 (m, 1H), 6.58 - 6.47 (m, 2H), 5.19 - 5.08 (m, 1H), 3.61 - 3.45 (m, 2H), 2.67 - 2.57 (m, 2H), 2.48 - 2.39 (m, 3H), 2.35 (s, 3H), 2.19 (s, 1H), 1.84 - 1.67 (m, 2H), 1.60 (d, *J =* 6.8 Hz, 3H), 1.56 - 1.11 (m, 6H), 1.04 - 0.93 (m, 1H). | 473.24 |
| C174 | 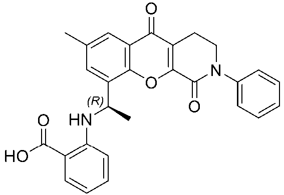 | ¹H NMR (400 MHz, DMSO-*d*₆) δ ppm 8.66 (br, 1H), 7.82 (dd, *J* = 8.0, 1.6 Hz, 1H), 7.79 - 7.76 (m, 1H), 7.58 (d, *J* = 24 Hz, 1H), 7.52 - 7.44 (m, 4H), 7.37 - 7.30 (m, 1H), 7.19 - 7.12 (m, 1H), 6.58 - 6.49 (m, 1H), 6.37 (d, *J* = 8.4 Hz, 1H), 5.24 - 5.15 (m, 1H), 4.05 (t, *J =* 6.8 Hz, 2H), 2.98 (t, *J =* 6.8 Hz, 2H), 2.37 (s, 3H), 1.62 (d, *J* = 6.8 Hz, 3H). | 469.17 |
| C175 | 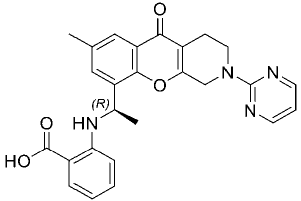 | ¹H NMR (400 MHz, DMSO-*d*₆) δ ppm 8.74 (s, 1H), 8.46 (d, *J* = 4.8 Hz, 2H), 7.81 (d, *J* = 8.0 Hz, 1H), 7.72 (s, 1H), 7.54 (s, 1H), 7.18 (t, *J* = 7.6 Hz, 1H), 6.74 (t, *J* = 4.8 Hz, 1H), 6.59 - 6.44 (m, 2H), 5.26 - 5.12 (m, 1H), 5.00 - 4.76 (m, 2H), 4.17 - 4.05 (m, 1H), 4.04 - 3.93 (m, 1H), 2.65 - 2.55 (m, 2H), 2.34 (s, 3H), 1.62 (d, *J* = 6.8 Hz, 3H). | 457.18 |
| C176 | 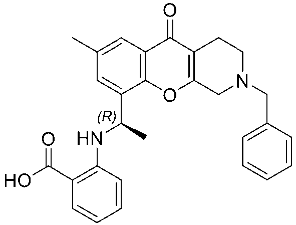 | ¹H NMR (400 MHz, DMSO-*d*₆) δ ppm 8.41 (d, *J* = 7.2 Hz, 1H), 7.79 (dd, *J* = 8.0, 1.6 Hz, 1H), 7.71 (d, *J* = 2.0 Hz, 1H), 7.50 (d, *J* = 2.4 Hz, 1H), 7.42 - 7.34 (m, 4H), 7.33 - 7.26 (m, 1H), 7.23 - 7.16 (m, 1H), 6.57 - 6.45 (m, 2H), 5.18 - 5.03 (m, 1H), 3.74 (s, 2H), 3.61 (s, 2H), 2.77 - 2.63 (m, 2H), 2.49 - 2.47 (m, 2H), 2.34 (s, 3H), 1.56 (d, *J =* 6.8 Hz, 3H). | 469.20 |
| C177 | 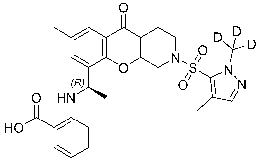 | ¹H NMR (400 MHz, DMSO-*d*₆) δ ppm 8.63 (s, 1H), 7.81 (dd, *J =* 8.0, 1.6 Hz, 1H), 7.70 (d, *J =* 2.0 Hz, 1H), 7.55 (d, *J* = 2.4 Hz, 1H), 7.49 (s, 1H), 7.27 - 7.09 (m, 1H), 6.62 - 6.40 (m, 2H), 5.23 - 5.12 (m, 1H), 4.60 - 4.41 (m, 2H), 3.58 - 3.52 (m, 2H), 2.50 - 2.46 (m, 2H), 2.34 (s, 3H), 2.21 (s, 3H), 1.58 (d, *J* = 6.8 Hz, 3H). | 540.19 |
| C178 | 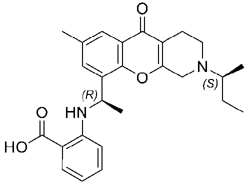 | ¹H NMR (400 MHz, DMSO-*d*₆) δ ppm 8.44 (d, *J* = 7.2 Hz, 1H), 7.80 (dd, *J* = 8.0, 1.6 Hz, 1H), 7.71 (d, *J* = 2.0 Hz, 1H), 7.51 (d, *J* = 2.4 Hz, 1H), 7.26 - 7.17 (m, 1H), 6.60 - 6.47 (m, 2H), 5.19 - 5.02 (m, 1H), 3.71 - 3.58 (m, 2H), 2.79 - 2.60 (m, 4H), 2.49 - 2.43 (m, 2H), 2.34 (s, 3H), 1.60 (d, *J* = 6.8 Hz, 3H), 1.43 - 1.33 (m, 1H), 1.02 (d, *J* = 6.4 Hz, 3H), 0.90 (t, *J* = 7.2 Hz, 3H). | 435.22 |
| C179 | 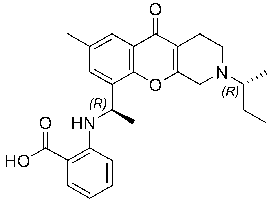 | ¹H NMR (400 MHz, DMSO-*d*₆) δ ppm 8.44 (d, *J* = 7.2 Hz, 1H), 7.80 (dd, *J* = 8.0, 1.6 Hz, 1H), 7.71 (d, *J* = 2.0 Hz, 1H), 7.52 (d, *J* = 2.4 Hz, 1H), 7.30 - 7.17 (m, 1H), 6.59 - 6.45 (m, 2H), 5.19 - 5.06(m, 1H), 3.70 - 3.61 (m, 2H), 2.81 - 2.58 (m, 4H), 2.49 - 2.42 (m, 2H), 2.34 (s, 3H), 1.62 (d, *J* = 6.8 Hz, 3H), 1.43 - 4.31 (m, 1H), 1.03 (d, *J* = 6.4 Hz, 3H), 0.90 (t, *J* = 7.2 Hz, 3H). | 435.22 |
| C180 | 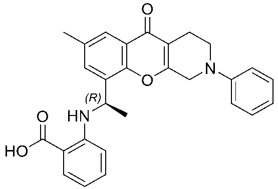 | ¹H NMR (400 MHz, DMSO-*d*₆) δ ppm 9.70 (br, 1H), 7.80 (dd, *J* = 7.6*,* 1.6 Hz, 1H), 7.72 - 7.67 (m, 1H), 7.56 (d, *J* = 2.4 Hz, 1H), 7.33 - 7.20 (m, 2H), 7.12 (d, *J =* 8.0 Hz, 2H), 7.03 - 6.95 (m, 1H), 6.83 (t, *J* = 7.2 Hz, 1H), 6.44 - 6.30 (m, 2H), 5.17 - 5.01 (m, 1H), 4.52 - 4.31 (m, 2H), 3.67 - 3.49 (m, 2H), 2.66 - 2.58 (m, 2H), 2.34 (s, 3H), 1.59 (d, *J =* 6.8 Hz, 3H). | 455.19 |
| C181 | 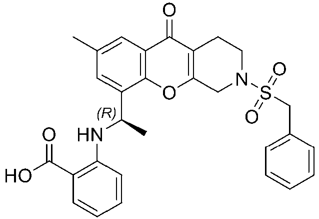 | ¹H NMR (400 MHz, DMSO-*d*₆) δ ppm 12.77 (br, 1H), 8.45 (d, *J* = 7.2 Hz, 1H), 7.81 (dd, *J* = 8.0, 1.6 Hz, 1H), 7.73 (d, *J* = 2.4 Hz, 1H), 7.53 (d, *J* = 2.4 Hz, 1H), 7.47 -7.38 (m, 2H), 7.37 - 7.27 (m, 3H), 7.26 - 7.19 (m, 1H), 6.61 - 6.43 (m, 2H), 5.21 - 5.05 (m, 1H), 4.61 (s, 2H), 4.46 - 4.32 (m, 2H), 3.50 - 3.39 m, 4H), 2.35 (s, 3H), 1.59 (d, *J* = 6.8 Hz, 3H). | 533.17 |
| C182 | 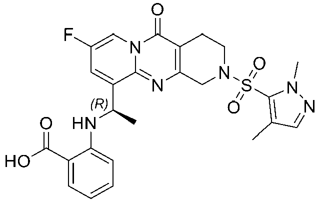 | ¹H NMR (400 MHz, DMSO-*d*₆) δ ppm 12.76 (br, 1H), 8.79 (dd, *J* = 4.8*,* 2.8 Hz, 1H), 8.49 (d, *J =* 6.4 Hz, 1H), 7.88 (dd, *J =* 8.0, 2.8 Hz, 1H), 7.82 (dd, *J =* 8.0, 1.6 Hz, 1H), 7.49 (s, 1H), 7.23 - 7.15 (m, 1H), 6.61 - 6.54 (m, 1H), 6.37 (d, *J* = 8.4 Hz, 1H), 5.39 - 5.26 (m, 1H), 4.50 - 4.39 (m, 2H), 4.01 (s, 3H), 3.63 (t, *J* = 6.0 Hz, 2H), 2.72 - 2.61 (m, 2H), 2.23 (s, 3H), 1.62 (d, *J =* 6.8 Hz, 3H). | 541.16 |
| C183 | 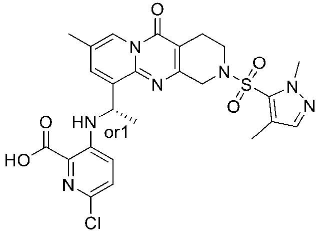 | / | 572.14 |
| C184 | 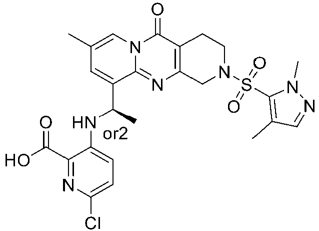 | ¹H NMR (400 MHz, DMSO-*d*₆) δ ppm 8.65 (br, 1H), 8.56 (d, *J =* 7.2 Hz, 1H), 7.77 (d, *J =* 2.0 Hz, 1H), 7.48 (s, 1H), 7.32 (d, *J* = 8.8 Hz, 1H), 7.07 (d, *J =* 9.2 Hz, 1H), 5.40-5.24 (m, 1H), 4.49 - 4.32 (m, 2H), 4.00 (s, 3H), 3.65 - 3.57 (m, 2H), 2.70 - 2.61 (m, 2H), 2.33 (s, 3H), 2.22 (s, 3H), 1.62 (d, *J =* 6.8 Hz, 3H). | 572.14 |
| C187 | 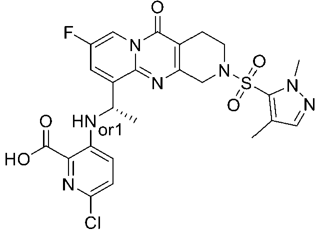 | / | 576.12 |
| C188 | 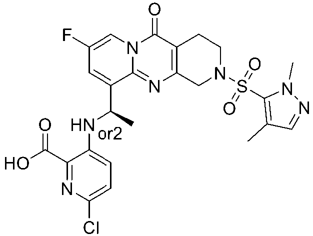 | / | 576.12 |
| C189 | 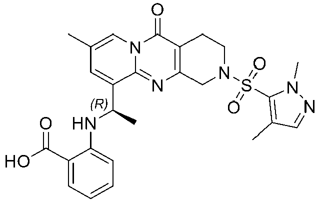 | ¹H NMR (400 MHz, DMSO-*d*₆) δ ppm 12.70 (br, 1H), 8.65 (s, 1H), 8.49 (s, 1H), 7.81 (dd, *J =* 8.0, 1.6 Hz, 1H), 7.70 (d, *J =* 2.0 Hz, 1H), 7.47 (s, 1H), 7.25 - 7.12 (m, 1H), 6.54 (t, *J =* 7.6 Hz, 1H), 6.36 (d, *J = 8.4* Hz, 1H), 5.40 - 5.26 (m, 1H), 4.51 - 4.34 (m, 2H), 4.00 (s, 3H), 3.64 (t, *J =* 6.0 Hz, 2H), 2.69 - 2.59 (m, 2H), 2.32 (s, 3H), 2.22 (s, 3H), 1.59 (d, *J =* 6.8 Hz, 3H). | 537.18 |
| C190 | 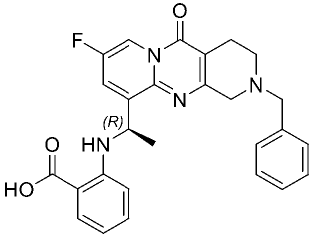 | ¹H NMR (400 MHz, DMSO-*d*₆) δ ppm 12.78 (br, 1H), 8.77 (dd, J = 4.8, 2.8 Hz, 1H), 8.48 (s, 1H), 7.86 - 7.72 (m, 2H), 7.42 - 7.35 (m, 4H), 7.32 - 7.27 (m, 1H), 7.20 - 7.12 (m, 1H), 6.56 (t, J = 7.6 Hz, 1H), 6.31 (d, J = 8.4 Hz, 1H), 5.38 - 5.21 (m, 1H), 3.82 - 3.68 (m, 2H), 3.60 (s, 2H), 2.84 - 2.72 (m, 2H), 2.71 - 2.61 (m, 2H), 1.58 (d, J = 6.4 Hz, 3H). | 473.2 |
| C191 | 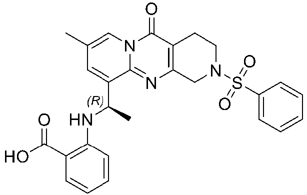 | ¹H NMR (400 MHz, DMSO-*d*₆) δ ppm 8.67 - 8.57 (m, 1H), 8.51 (s, 1H), 7.92 - 7.85 (m, 2H), 7.81 (dd, *J =* 8.0*,* 1.6 Hz, 1H), 7.75 - 7.58 (m, 3H), 7.23 - 7.13 (m, 1H), 6.54 (t, *J =* 7.6 Hz, 1H), 6.34 (d, *J* = 8.4 Hz, 1H), 5.40 - 5.22 (m, 1H), 4.32 - 4.07 (m, 2H), 3.46 - 3.39 (m, 2H), 2.73 - 2.65 (m, 2H), 2.30 (s, 3H), 2.06 - 1.92 (m, 1H), 1.58 (d, *J =* 6.8 Hz, 3H). | 519.2 |
| C192 | 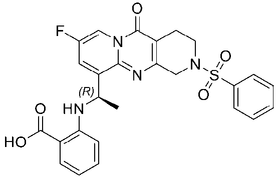 | ¹H NMR (400 MHz, DMSO-*d*₆) δ ppm 8.81 - 8.72 (m, 1H), 8.52 (s, 1H), 7.96 - 7.87 (m, 2H), 7.86 - 7.79 (m, 2H), 7.51 - 7.68 (m, 1H), 7.67 - 7.59 (m, 2H), 7.25 - 7.13 (m, 1H), 6.57 (t, *J* = 7.6 Hz, 1H), 6.34 (d, *J =* 8.4 Hz, 1H), 5.37 - 5.22 (m, 1H), 4.32 - 4.14 (m, 2H), 3.48 - 3.38 (m, 2H), 2.76 - 2.66 (m, 2H), 1.61 (d, *J =* 6.8 Hz, 3H). | 523.1 |
| C193 | 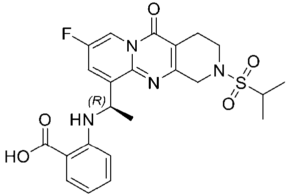 | ¹H NMR (400 MHz, DMSO-*d*₆) δ ppm 8.81 (dd, *J = 4.8,* 2.8 Hz, 1H), 8.60 (s, 1H), 7.91 - 7.75 (m, 2H), 7.30 - 7.07 (m, 1H), 6.56 (t, *J* = 7.6 Hz, 1H), 6.35 (d, *J =* 8.4 Hz, 1H), 5.40 - 5.23 (m, 1H), 4.49 (s, 2H), 3.72 - 3.61 (m, 2H), 3.58 - 3.46 (m, 1H), 2.81 - 2.67 (m, 2H), 1.62 (d, *J =* 6.8 Hz, 3H), 1.35 - 1.22 (m, 6H). | 489.2 |
| C194 | 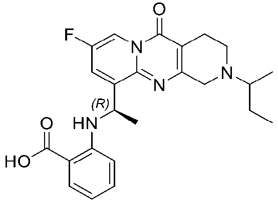 | ¹H NMR (400 MHz, DMSO-*d*₆) δ ppm 8.77 (dd, *J = 4.8,* 2.8 Hz, 1H), 8.46 (d, *J =* 6.8 Hz, 1H), 7.89 - 7.73 (m, 2H), 7.26 - 7.13 (m, 1H), 6.57 (t, *J =* 7.6 Hz, 1H), 6.35 (dd, *J =* 8.4, 2.4 Hz, 1H), 5.41 - 5.22 (m, 1H), 3.81 - 3.63 (m, 2H), 2.89 - 2.78 (m, 1H), 2.79 - 2.59 (m, 4H), 1.79 - 1.64 (m, 1H), 1.62 (d, *J =* 6.8 Hz, 3H), 1.46 - 1.32 (m, 1H), 1.05 (d, *J =* 6.4 Hz, 3H), 0.91 (t, *J =* 7.6 Hz, 3H). | 439.2 |
| C195 | 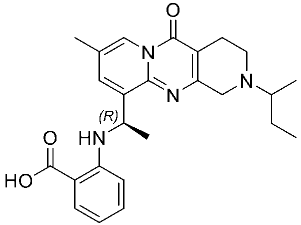 | ¹H NMR (400 MHz, DMSO-*d*₆) δ ppm 8.64 (s, 1H), 8.53 - 8.40 (m, 1H), 7.81 (d, *J =* 8.0 Hz, 1H), 7.63 (s, 1H), 7.20 (t, *J =* 8.0 Hz, 1H), 6.54 (t, *J =* 7.6 Hz, 1H), 6.34 (d, *J =* 8.4 Hz, 1H), 5.47 - 5.26 (m, 1H), 3.79 - 3.66 (m, 2H), 2.92 - 2.80 (m, 1H), 2.82 - 2.68 (m, 2H), 2.69 - 2.57 (m, 2H), 2.30 (s, 3H), 1.69 - 1.60 (m, 1H), 1.58 (d, *J =* 6.8 Hz, 3H), 1.47 - 1.33 (m, 1H), 1.06 (d, *J =* 6.6 Hz, 3H), 0.91 (t, *J =* 7.2 Hz, 3H). | 435.2 |
| C196 | 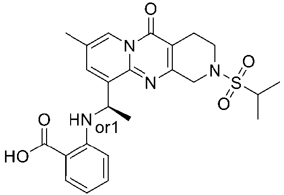 | ¹H NMR (400 MHz, DMSO-*d*₆) δ ppm 12.74 (br, 1H), 8.67 (s, 1H), 8.55 - 8.44 (m, 1H), 7.87 - 7.76 (m, 1H), 7.73 - 7.63 (m, 1H), 7.30 - 7.11 (m, 1H), 6.54 (t, *J =* 7.6 Hz, 1H), 6.36 (d, *J =* 8.4 Hz, 1H), 5.38 - 5.28 (m, 1H), 4.47 (s, 2H), 3.70 - 3.58 (m, 2H), 3.58 - 3.46 (m, 1H), 2.76 - 2.68 (m, 2H), 2.32 (s, 3H), 1.60 (d, *J =* 6.8 Hz, 3H), 1.35 - 1.19 (m, 6H). | 485.2 |
| C197 | 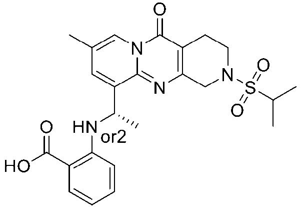 | / | 485.2 |
| C198 | 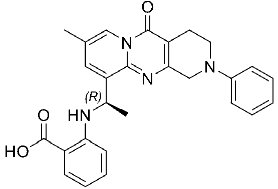 | / | 455.2 |
| C199 | 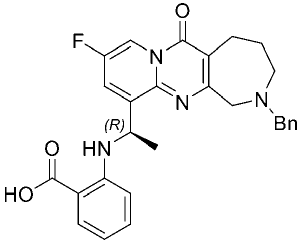 | ¹H NMR (400 MHz, DMSO-*d*₆) δ ppm 12.73 (br, 1H), 8.77 (dd, J = 4.8, 2.8 Hz, 1H), 8.48 (s, 1H), 7.86 - 7.72 (m, 2H), 7.45 - 7.33 (m, 4H), 7.31 - 7.26 (m, 1H), 7.22 - 7.10 (m, 1H), 6.56 (t, J = 7.6 Hz, 1H), 6.31 (d, J = 8.4 Hz, 1H), 5.38 - 5.21 (m, 1H), 3.82 - 3.68 (m, 2H), 3.60 (s, 2H), 2.84 - 2.72 (m, 2H), 2.71 - 2.61 (m, 2H), 1.61-1.41 (m, 5H). | 487.2 |
| C200 | 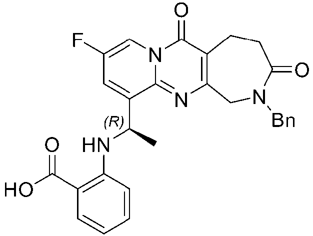 | ¹H NMR (400 MHz, DMSO-*d*₆) δ ppm 12.68 (br, 1H), 8.77 (dd, J = 4.8, 2.8 Hz, 1H), 8.48 (s, 1H), 7.86 - 7.72 (m, 2H), 7.39 - 7.30 (m, 4H), 7.29 - 7.24 (m, 1H), 7.28 - 7.13 (m, 1H), 6.53 (t, J = 7.6 Hz, 1H), 6.30 (d, J = 8.4 Hz, 1H), 5.36 - 5.20 (m, 1H), 3.82 - 3.68 (m, 2H), 3.67 (s, 2H) , 2.31 - 2.24 (m, 2H), 2.71 - 2.61 (m, 2H), 1.58 (d, J = 6.4 Hz, 3H). | 501.2 |
| C201 | 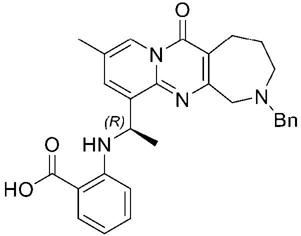 | ¹H NMR (400 MHz, DMSO-d6) δ ppm 8.73 - 8.67 (m, 1H), 8.49 (s, 1H), 7.80 (dd, *J =* 8.0, 1.6 Hz, 1H), 7.64 (d, *J =* 2.0 Hz, 1H), 7.36 - 7.30 (m, 2H), 7.29 - 7.12 (m, 4H), 6.53 (t, *J =* 7.6 Hz, 1H), 6.32 (d, *J =* 8.4 Hz, 1H), 5.34 - 5.23 (m, 1H), 4.04 - 3.92 (m, 2H), 3.64 (s, 2H), 3.11 - 3.04 (m, 2H), 303 - 2.92 (m, 2H), 2.33 (s, 3H), 1.80 - 1.65 (m, 2H), 1.51 (d, *J* = 6.6 Hz, 3H). | 483.2 |
| C202 | 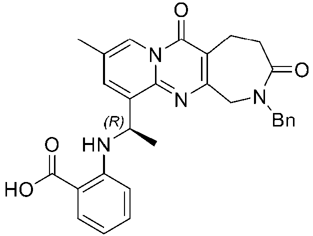 | ¹H NMR (400 MHz, DMSO-d6) δ ppm 8.86 - 8.82 (m, 1H), 8.56 (br 1H), 7.87 - 7.73 (m, 2H), 7.31 - 7.20 (m, 5H), 7.18 - 7.11 (m, 1H), 6.53 (t, J = 7.6 Hz, 1H), 6.41 (dd, J = 38.4, 8.4 Hz, 1H), 5.43 (d, J = 14.4 Hz, 1H), 5.39 - 5.25 (m, 1H), 4.68 - 4.54 (m, 1H), 2.61 - 2.53 (m, 1H), 2.49 - 2.42 (m, 2H), 2.37 (s, 3H), 2.34 - 2.23 (m, 1H), 2.21 - 2.03 (m, 2H), 1.58 (dd, J = 17.2, 6.8 Hz, 3H). | 497.2 |
| C203 | 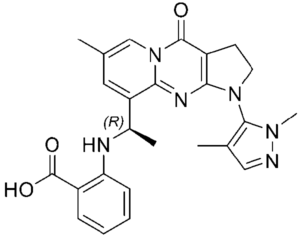 | ¹H NMR (400 MHz, DMSO-*d*₆) δ ppm 8.66 (s, 1H), 8.53 - 8.45 (m, 1H), 7.83 (dd, *J =* 8.0, 1.6 Hz, 1H), 7.72 - 7.67 (m, 1H), 7.28 - 7.15 (m, 1H), 6.63 - 6.42 (m, 2H), 6.36 (d, *J* = 8.4 Hz, 1H), 5.38 - 5.28 (m, 1H), 3.45 (s, 3H), 3.25 - 3.13 (m, 2H), 2.83 - 2.65 (m, 2H), 2.35 (s, 3H), 2.16 (s, 3H), 1.59 (d, *J =* 6.8 Hz, 3H). | 459.2 |
| C204 | 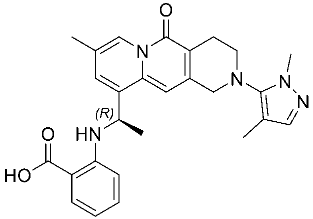 | ¹H NMR (400 MHz, DMSO-*d*₆) δ ppm 12.70 (br, 1H), 8.65 (s, 1H), 8.55 - 8.45 (m, 1H), 7.81 (dd, *J =* 8.0, 1.6 Hz, 1H), 7.70 (d, *J* = 2.0 Hz, 1H), 7.47 (s, 1H), 7.25 - 7.12 (m, 1H), 6.54 (t, *J* = 7.6 Hz, 1H), 6.36 (d, *J =* 8.4 Hz, 1H), 5.83 - 5.77 (m, 1H), 5.40 - 5.26 (m, 1H), 4.51 - 4.34 (m, 2H), 3.55 (s, 3H), 3.64 (t, *J =* 6.0 Hz, 2H), 2.69 - 2.59 (m, 2H), 2.37 (s, 3H), 2.18 (s, 3H), 1.60 (d, *J =* 6.8 Hz, 3H). | 472.2 |
| C205 | 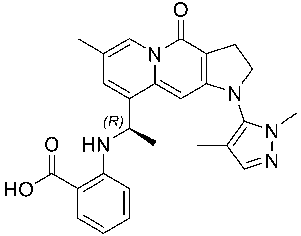 | ¹H NMR (400 MHz, DMSO-*d*₆) δ ppm 8.66 (s, 1H), 8.53 - 8.45 (m, 1H), 7.83 (dd, *J =* 8.0, 1.6 Hz, 1H), 7.72 - 7.67 (m, 1H), 7.28 - 7.15 (m, 1H), 6.63 - 6.42 (m, 2H), 6.36 (d, *J* = 8.4 Hz, 1H), 5.85 - 5.79 (m, 1H), 5.38 - 5.28 (m, 1H), 3.45 (s, 3H), 3.25 - 3.13 (m, 2H), 2.83 - 2.65 (m, 2H), 2.35 (s, 3H), 2.16 (s, 3H), 1.59 (d, *J =* 6.8 Hz, 3H). | 458.2 |
| C206 | 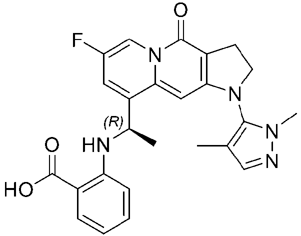 | / | 462.2 |
| C207 | 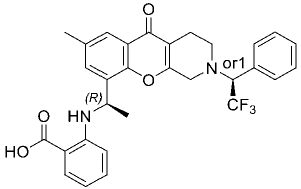 | ¹H NMR (400 MHz, DMSO-*d*₆) δ ppm 12.76 (br, 111), 8.40 (d, *J =* 6.8 Hz, 1H), 7.81 (dd, *J =* 8.0, 1.6 Hz, 1H), 7.68 (d, *J =* 2.0 Hz, 1H), 7.56 - 7.38 (m, 6H), 7.26 - 7.15 (m, 1H), 6.54 (t, *J =* 7.6 Hz, 1H), 6.46 (d, *J =* 8.4 Hz, 1H), 5.16 - 5.03 (m, 1H), 4.99 - 4.85 (m, 1H), 3.81 (s, 2H), 3.06 - 2.95 (m, 1H), 2.76 - 2.64 (m, 1H), 2.49 - 2.44 (m, 2H), 2.32 (s, 3H), 1.56 (d, *J =* 6.8 Hz, 3H). | 537.19 |
| C208 | 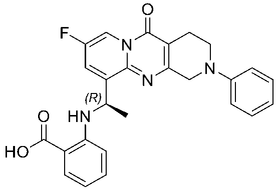 | ¹H NMR (400 MHz, DMSO-*d*₆) δ ppm 8.79 (dd, *J =* 4.8, 2.8 Hz, 1H), 8.61 (br, 1H), 7.88 (dd, *J* = 8.0, 2.8 Hz, 1H), 7.82 (dd, *J =* 8.0, 1.6 Hz, 1H), 7.31 - 7.24 (m, 2H), 7.23 - 7.18 (m, 1H), 7.10 (d, *J =* 8.0 Hz, 2H), 6.84 - 6.78 (m, 1H), 6.59 - 6.52 (m, 1H), 6.46 (d, *J =* 8.4 Hz, 1H), 5.43 - 5.37 (m, 1H), 4.48 - 4.31 (m, 2H), 3.69 - 3.56 (m, 2H), 2.86 - 2.73 (m, 2H), 1.67 (d, *J =* 6.8 Hz, 3H). | 459.18 |
| C209 | 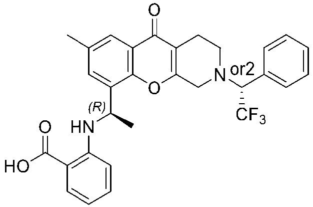 | ¹H NMR (400 MHz, DMSO-*d*₆) δ ppm 12.68 (br, 1H), 8.40 (d, *J =* 6.8 Hz, 1H), 7.81 (dd, *J =* 8.0, 1.6 Hz, 1H), 7.68 (d, *J =* 2.0 Hz, 1H), 7.56 - 7.39 (m, 6H), 7.24 - 7.15 (m, 1H), 6.54 (t, *J =* 7.6 Hz, 1H), 6.47 (d, *J =* 8.4 Hz, 1H), 5.14 - 5.05 (m, 1H), 4.98 - 4.87 (m, 1H), 3.80 (s, 2H), 3.07 - 2.96 (m, 1H), 2.75 - 2.65 (m, 1H), 2.49 - 2.44 (m, 2H), 2.33 (s, 3H), 1.56 (d, *J =* 6.8 Hz, 3H). | 537.19 |
| C210 | 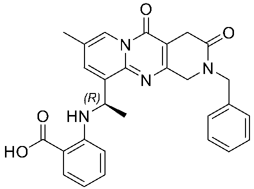 | ¹H NMR (400 MHz, DMSO-*d*₆) δ ppm 13.02 - 12.46 (br, 1H), 8.68 (s, 1H), 8.46 (s, 1H), 7.80 (dd, *J* = 8.0, 1.6 Hz, 1H), 7.68 (d, *J =* 2.0 Hz, 1H), 7.37 (d, *J =* 4.4 Hz, 4H), 7.33 - 7.25 (m, 1H), 7.21 - 7.10 (m, 1H), 6.53 (t, *J =* 7.6 Hz, 1H), 6.29 (d, *J =* 8.4 Hz, 1H), 5.35 - 5.24 (m, 1H), 4.83 - 4.63 (m, 2H), 4.53 (s, 2H), 3.54 (s, 2H), 2.32 (s, 3H), 1.54 (d, *J =* 6.8 Hz, 3H). | 483.20 |
| C211 | 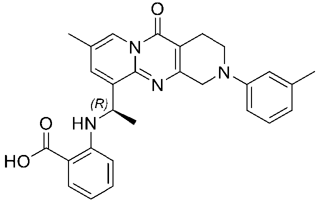 | ¹H NMR (400 MHz, DMSO-d6) δ ppm 8.70 - 8.50 (m, 1H), 8.62 (s, 1H), 7.81 (dd, *J =* 8.0, 1.6 Hz, 1H), 7.68 (d, *J =* 2.0 Hz, 1H), 7.27 - 7.17 (m, 1H), 7.14 (t, *J =* 7.6 Hz, 1H), 6.97 - 6.84 (m, 2H), 6.63 (d, *J* = 7.2 Hz, 1H), 6.54 (t, *J =* 7.6 Hz, 1H), 6.44 (d, *J =* 8.4 Hz, 1H), 5.44 - 5.33 (m, 1H), 4.45 - 4.22 (m, 2H), 3.58 (t, *J =* 5.6 Hz, 2H), 2.78 (t, *J =* 5.6 Hz, 2H), 2.31 (s, 3H), 2.28 (s, 3H), 1.63 (d, *J =* 6.8 Hz, 3H). | 469.22 |
| C212 | 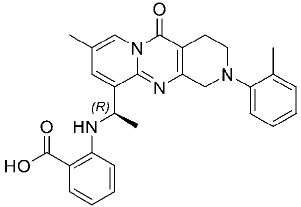 | ¹ H NMR (400 MHz, DMSO-d6) δ ppm 12.64 (br, 1H), 8.72 - 8.65 (m, 1H), 8.55 (d, J = 7.2 Hz, 1H), 7.80 (dd, J = 8.0, 1.6 Hz, 1H), 7.70 (d, J = 2.0 Hz, 1H), 7.27 - 7.16 (m, 4H), 7.06 - 6.97 (m, 1H), 6.59 - 6.49 (m, 1H), 6.45 (d, J = 8.4 Hz, 1H), 5.40 - 5.28 (m, 1H), 4.13 (s, 2H), 3.23 - 3.16 (m, 2H), 2.83 - 2.75 m, 2H), 2.36 - 2.33 (m, 3H), 2.32 (s, 3H), 1.61 (d, J = 6.8 Hz, 3H). | 469.22 |
| C213 | 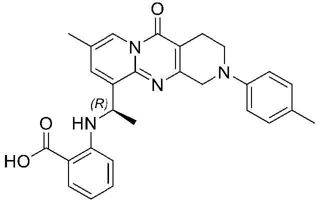 | ¹H NMR (400 MHz, DMSO-*d*₆) δ ppm 8.82 - 8.60 (m, 2H), 7.81 (dd, *J =* 8.0, 1.6 Hz, 1H), 7.68 (d, *J =* 2.0 Hz, 1H), 7.24 - 7.15 (m, 1H), 7.12 - 7.04 (m, 2H), 7.03 - 6.94 (m, 2H), 6.53 (t, *J =* 7.6 Hz, 1H), 6.42 (d, *J =* 8.4 Hz, 1H), 5.43 - 5.29 (m, 1H), 4.43 - 4.20 (m, 2H), 3.58 - 3.51 (m, 2H), 2.82 - 2.71 (m, 2H), 2.31 (s, 3H), 2.22 (s, 3H), 1.62 (d, *J =* 6.8 Hz, 3H). | 469.22 |
| C214 | 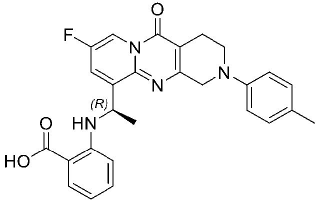 | ¹ H NMR (400 MHz, DMSO-d6) δ ppm 8.82 - 8.75 (m, 1H), 8.72 - 8.51 (m, 1H), 7.96 - 7.78 (m, 2H), 7.22 (t, *J =* 7.6 Hz, 1H), 7.14 - 7.04 (m, 2H), 7.03 - 6.94 (m, 2H), 6.57 (t, *J* = 7.2 Hz, 1H), 6.45 (d, *J =* 8.4 Hz, 1H), 5.43 - 5.26 (m, 1H), 4.43 - 4.25 (m, 2H), 3.60 - 3.50 m, 2H), 2.86 - 2.72 (m, 2H), 2.22 (s, 3H), 1.66 (d, *J =* 6.8 Hz, 3H). | 473.19 |
| C215 | 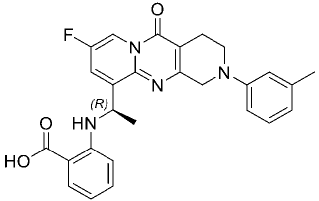 | ¹H NMR (400 MHz, DMSO-d6) δ ppm 8.86 - 8.73 (m, 2H), 7.90 - 7.78 (m, 2H), 7.22 - 7.10 (m, 2H), 6.96 - 6.85 (m, 2H), 6.64 (d, *J =* 7.2 Hz, 1H), 6.55 (t, *J = 7.6* Hz, 1H), 6.40 (d, *J =* 8.4 Hz, 1H), 5.48 - 5.24 (m, 1H), 4.38 (s, 2H), 3.60 (t, *J =* 5.6 Hz, 2H), 2.83 - 2.74 (m, 2H), 2.29 (s, 3H), 1.65 (d, *J =* 6.8 Hz, 3H). | 473.19 |
| C216 | 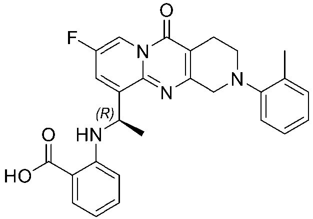 | ¹ H NMR (400 MHz, DMSO-*d*6) δ ppm 8.82 - 8.73 (m, 1H), 8.72 - 8.51 (m, 1H), 7.97 - 7.78 (m, 2H), 7.22 - 7.10 (m, 1H), 7.14 - 7.04 (m, 2H), 7.03 - 6.94 (m, 2H), 6.55 (t, *J =* 7.6 Hz, 1H), 6.40 (d, *J =* 8.4 Hz, 1H), 5.48 - 5.24 (m, 1H), 4.30 (s, 2H), 3.60 - 3.51 m, 2H), 2.85 - 2.74 (m, 2H), 2.32 (s, 3H), 1.61 (d, *J =* 6.8 Hz, 3H). | 473.19 |

### Cell Proliferation Inhibition Assay

### 1. Experimental Materials

| **Cell Line** | **Supplier** |
|---|---|
| Human breast cancer cells SK-BR-3 (WT) | ATCC |
| Human breast cancer cells T47D (H1047R) | ATCC |

### II. Experimental Protocol

### 1. Cell Seeding;

a) Cell suspension was prepared;
b) The medium was removed from the culture flask;
c) The cells were rinsed once with PBS;
d) Trypsin was added for digestion, and the cells were centrifuged and collected;
e) The cells were resuspended in medium, counted and adjusted to an appropriate concentration;
f) 100 µL of cell suspension was added to each well of a 96-well plate. One plate was used for each cell line, which was incubated overnight in a 37 °C, 5% CO₂ incubator.

### 2. Treatment of Compounds

### Compound Dilution

a) Serial dilutions of the test compounds were prepared: The compound was prepared as a 10 mM stock solution. 18 µL of the stock solution was dissolved in 982 µL of the culture medium containing DMSO, followed by 4-fold serial dilutions in culture medium containing 0.5% DMSO to give 9 concentrations. The concentrations of the diluted compound were as follows:
   180000 nM, 45000 nM, 11250 nM, 2812.5 nM, 703.13 nM, 175.78 nM, 43.95 nM, 10.99 nM, and 2.75 nM.
b) After thorough mixing, 20 µL of each compound solution was added to the cell plate containing 100 µL of cells per well. Each concentration was tested in triplicate. Final concentrations of the compound were as follows:
   30000 nM, 7500 nM, 1875 nM, 468.75 nM, 117.188 nM, 29.30 nM, 7.32 nM, 1.83 nM, and 0.46 nM.
c) The cells were transferred into the incubator and incubated for 3 days.

### 3. CTG Assay

The cell plate was removed, 25 µL of CTG reagent was added to each well of the 96-well plate, the mixture was shook on a plate shaker at room temperature for 10 minutes, and the plate was read.

### 4. Data Analysis

The cell survival rate was calculated using the following formula (% Cell Survival): $% Cell Survival = 100 % \times \left(OD_Sample-OD_LCave\right) / \left(OD_HC-OD_LCave\right)$
OD_HC: Reading of the 0.1‰ DMSO control group
OD_Sample: Reading of cells treated with compound
OD_LCave: Reading of blank medium

Analyzed by Prism: Dose-response-Inhibition - Log (inhibitor) vs response (three parameters for the best fit).

The results of the cell proliferation inhibition assay are shown in Table 1 below.

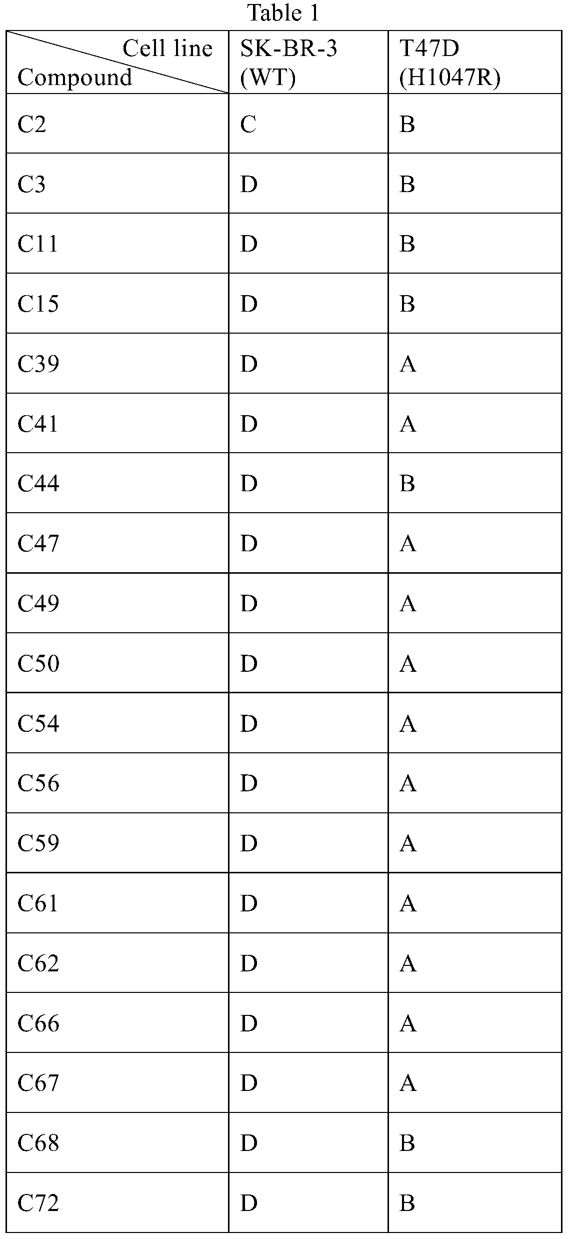

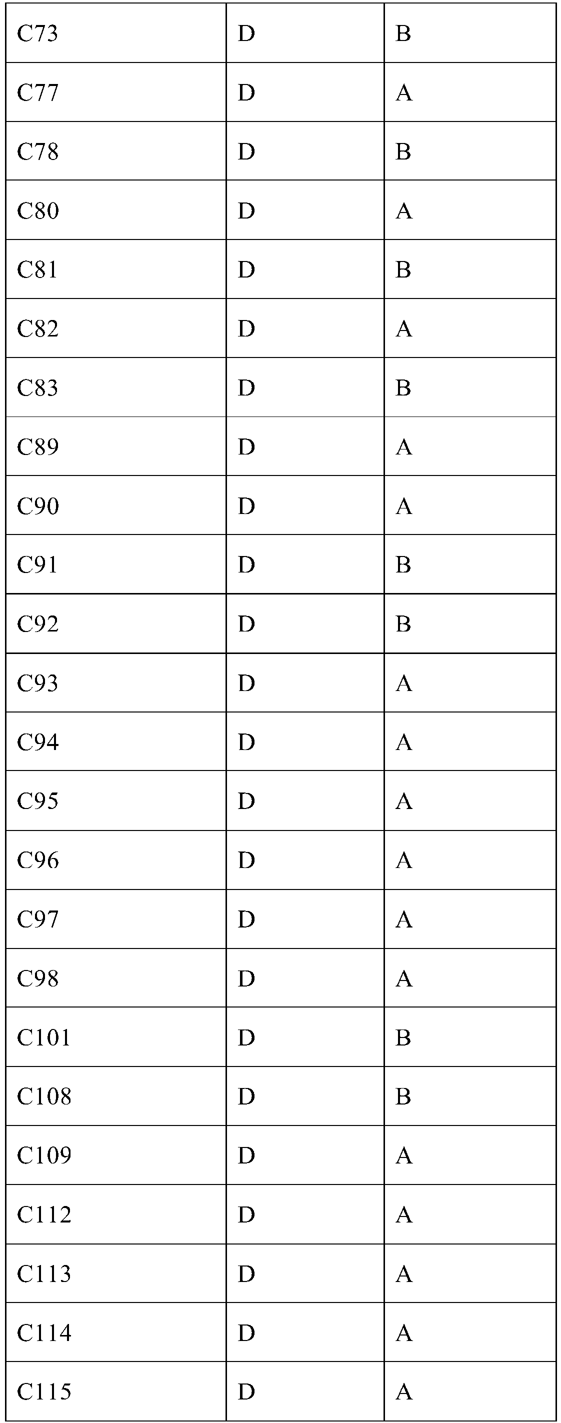

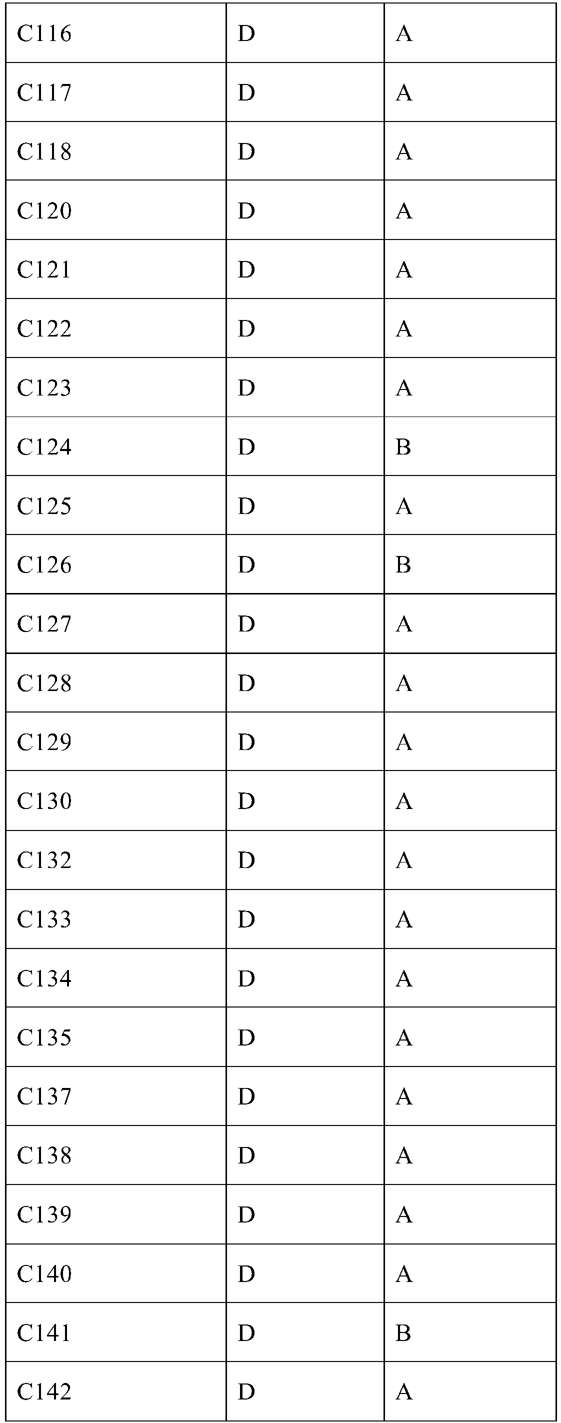

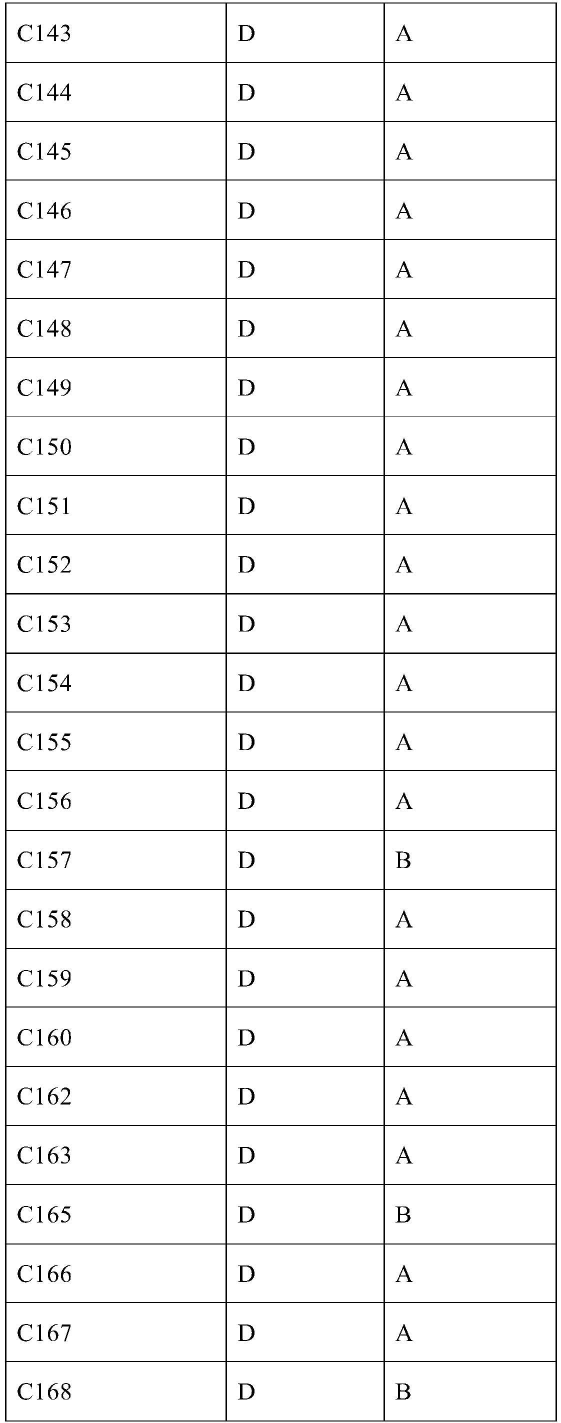

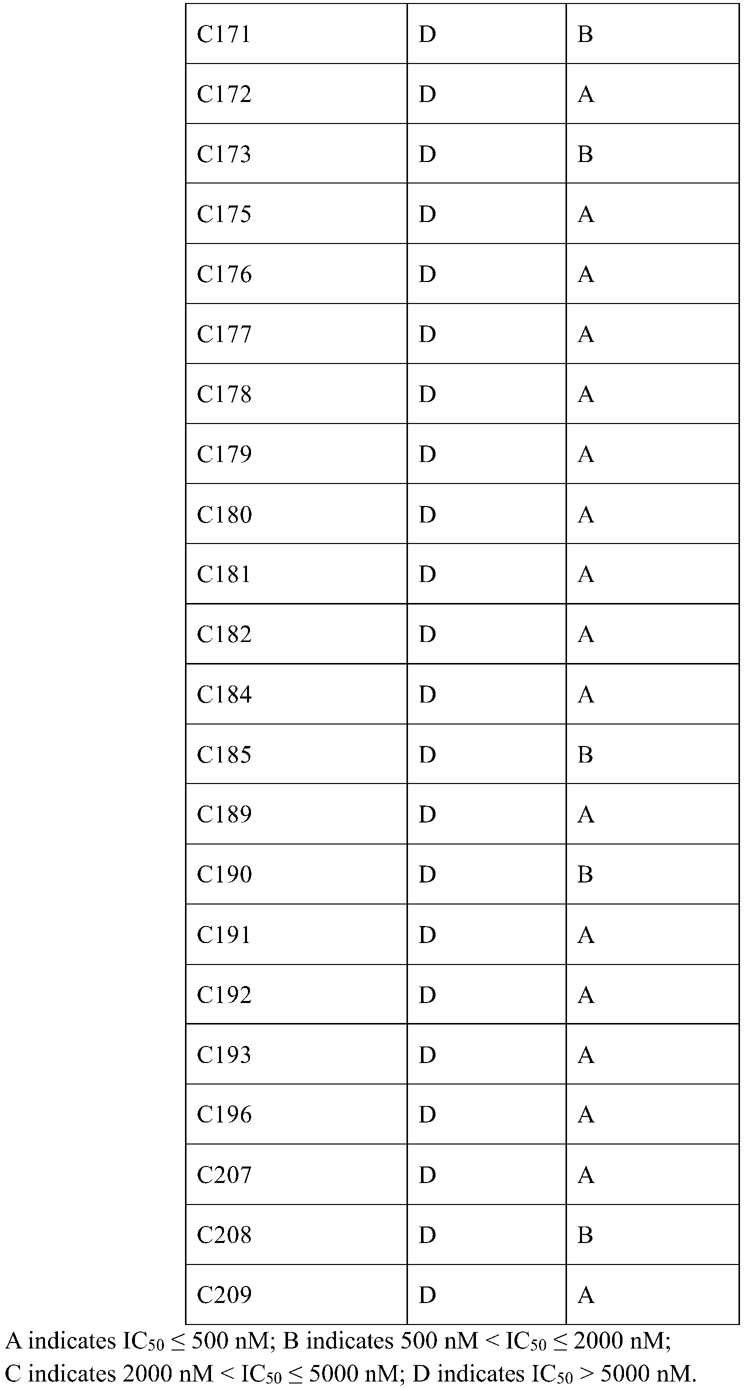

The present invention provides a series of compounds with selective inhibitory activity against the PI3Kα H1047R mutant activity, as well as methods for their synthesis, which exhibit broad application prospects.

All documents mentioned herein are incorporated by reference in this invention as if each document was individually incorporated by reference. In addition, it should be understood that after reading the above teaching content of the present invention, those skilled in the art can make various changes or modifications to the present invention, and these equivalent forms also fall within the scope defined by the appended claims of the present invention.

## Claims

1. A compound, wherein the compound is a compound of Formula (I), or a pharmaceutically acceptable salt, a stereoisomer, a deuterated compound, a solvate or a prodrug thereof, 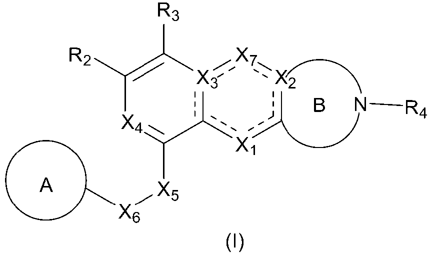 wherein,
X₁ is selected from the group consisting of: N, O, S, NR₁₁, and C(R₁₁)_{q};
X₂ and X₃ are each independently selected from the group consisting of: N and C;
X₄ is N or CR;
X₅ is selected from the group consisting of: absent, O, NR₅₁, C₁₋₆ alkylene, C₃₋₆ cycloalkylene, and 3-6 membered heterocycloalkylene containing 1-4 heteroatoms selected from N, O and S, wherein the C₁₋₆ alkylene, C₃₋₆ cycloalkylene, and 3-6 membered heterocycloalkylene are optionally substituted with 1, 2, 3, 4, 5 or 6 R₅₂ groups;
X₆ is selected from the group consisting of: absent, NR₆₁, and C₁₋₆ alkylene, wherein the C₁₋₆ alkylene is optionally substituted with 1, 2, 3, 4, 5 or 6 R₆₂ groups;
X₇ is selected from the group consisting of: C=O, C=S, and CR₇₁;
ring B is a 5-7 membered heterocycloalkyl containing one N atom and 0-3 additional heteroatoms selected from N, O and S, which is optionally substituted with one or more R₅ groups;
ring A is selected from the group consisting of: C₆₋₁₀ aryl, 5-10 membered heteroaryl containing 1-4 heteroatoms selected from N, O and S, and 5-10 membered heterocycloalkyl containing 1-4 heteroatoms selected from N, O and S; wherein the C₆₋₁₀ aryl and 5-10 membered heteroaryl are optionally substituted with m R₆ groups;
R, R₂, R₃, R₁₁, R₅₂ and R₆₂ are each independently selected from the group consisting of: H, deuterium, halogen, oxo (=O), -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, halogenated C₃₋₆ cycloalkyl, and deuterated C₃₋₆ cycloalkyl;
alternatively, R and R₂ together with the atoms to which they are attached form a C₄₋₆ cycloalkyl or a 4-6 membered heterocycloalkyl containing 1-4 heteroatoms selected from N, O and S; wherein the C₄₋₆ cycloalkyl and 4-6 membered heterocycloalkyl are optionally substituted with 1, 2, 3, 4, 5 or 6 R₆ groups;
alternatively, R₂ and R₃ together with the atoms to which they are attached form a C₄₋₆ cycloalkyl or a 4-6 membered heterocycloalkyl containing 1-4 heteroatoms selected from N, O and S; wherein the C₄₋₆ cycloalkyl and 4-6 membered heterocycloalkyl are optionally substituted with 1, 2, 3, 4, 5 or 6 R₆ groups;
R₅₁ and R₆₁ are each independently selected from the group consisting of: H, -CN, -NO₂, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl;
R₇₁ is selected from the group consisting of: H, halogen, -CN, -NO₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, and -C(O)R₈;
R₄ is selected from the group consisting of: -L-(C₁₋₆ alkyl), -L-C₆₋₁₀ aryl, -L-(5-10 membered heteroaryl containing 1-4 heteroatoms selected from N, O and S), -L-(saturated or partially saturated C₃₋₈ cycloalkyl), and -L-(saturated or partially saturated 4-8 membered heterocycloalkyl containing 1-4 heteroatoms selected from N, O and S); wherein the C₁₋₆ alkyl, C₆₋₁₀ aryl, 5-10 membered heteroaryl, C₃₋₈ cycloalkyl, and 4-8 membered heterocycloalkyl are optionally substituted with n R₇ groups;
each R₅ is independently selected from the group consisting of: H, deuterium, halogen, -CN, oxo (=O), C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, halogenated C₃₋₆ cycloalkyl, and 3-8 membered heterocycloalkyl containing 1-3 heteroatoms selected from N, O and S;
each R₆ is independently selected from the group consisting of: H, deuterium, halogen, -CN, - NO₂, C₁₋₆ alkyl, halogenated C₁₋₆ alkyl, deuterated C₁₋₆ alkyl, C₁₋₆ alkoxy, halogenated C₁₋₆ alkoxy, - C(O)R₈, -C(O)OR₈, -C(O)NR₈R₉, -SO₂R₈, -S(O)(NR₈)R₉, -SO₂NR₈R₉, and -P(O)R₈R₉;
each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -NR₈R₉, =NR₈, - C(O)R₈, -C(O)OR₈, -C(O)NR₈R₉, -NR₈C(O)R₉, -SR₈, -SO₂R₈, -NR₈SO₂R₉, -SO₂NR₈R₉, - NR₈SO₂NR₉R₁₀, -P(O)R₈R₉, -L₁-(C₁₋₆ alkyl), -L₁-(saturated or partially saturated C₃₋₁₀ cycloalkyl), - L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-12 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, 3-6 membered heterocycloalkyl containing 1-4 heteroatoms selected from N, O and S, - SO₂R₈, -C(O)R₈, -C(O)OR₈, -C(O)NR₈R₉, and -NR₈R₉;
alternatively, two R₇ groups together with the atoms to which they are attached form C₃₋₆ cycloalkyl, C₆₋₁₀ aryl, 3-6 membered heterocycloalkyl containing 1-4 heteroatoms selected from N, O and S, or 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NR₈R₉, =NR₈, -C(O)R₈, -C(O)OR₈, -C(O)NR₈R₉, -NR₈C(O)R₉, -SR₈, -SO₂R₈, - NR₈SO₂R₉, -SO₂NR₈R₉, -NR₈SO₂NR₉R₁₀, -P(O)R₈R₉, C₁₋₆ alkyl, C₁₋₆ alkoxy, C₁₋₆ heteroalkyl containing 1-4 heteroatoms selected from N, O and S, halogenated C₁₋₆ alkyl, halogenated C₁₋₆ alkoxy, halogenated C₁₋₆ heteroalkyl containing 1-4 heteroatoms selected from N, O and S, C₃₋₆ cycloalkyl, and 3-6 membered heterocycloalkyl containing 1-4 heteroatoms selected from N, O and S;
L and L₁ are each independently a bond or selected from the group consisting of: C₁₋₆ alkylene, C₂₋₆ alkenylene, C₂₋₆ alkynylene, 1-6 membered heteroalkylene containing 1-4 heteroatoms selected from N, O and S, -NR₈-, -O-, -C(O)-, -C(O)NR₈-, -C(O)O-, -NR₈C(O)-, -(SO₂)-, -SO₂NR₈-, -NR₈SO₂-, -NR₈SO₂NR₉-, and -S(O)(NR₈)-, wherein the C₁₋₆ alkylene, C₂₋₆ alkenylene, C₂₋₆ alkynylene, and 1-6 membered heteroalkylene are optionally substituted with 0-6 substituents selected from the group consisting of: deuterium, halogen, =O, -CN, -OH, -NR₈R₉, and =NR₈;
each of R₈, R₉ and R₁₀ is independently selected from the group consisting of: H, C₁₋₆ alkyl, halogenated C₁₋₆ alkyl, C₃₋₆ cycloalkyl, C₆₋₁₀ aryl, 3-6 membered heterocycloalkyl containing 1-4 heteroatoms selected from N, O and S, and 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ alkylamino, halogenated C₁₋₆ alkylamino, C₁₋₆ alkylthio, halogenated C₁₋₆ alkylthio, C₁₋₆ heteroalkyl containing 1-4 heteroatoms selected from N, O and S, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S, halogenated C₃₋₆ cycloalkyl, and halogenated 3-6 membered heterocycloalkyl containing 1-4 heteroatoms selected from N, O and S;
alternatively, R₈ and R₉ together with the heteroatom to which they are attached form a 3-6 membered heterocycloalkyl containing 1-4 heteroatoms selected from N, O and S, wherein the 3-6 membered heterocycloalkyl is optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ alkylamino, halogenated C₁₋₆ alkylamino, C₁₋₆ alkylthio, halogenated C₁₋₆ alkylthio, C₁₋₆ heteroalkyl containing 1-4 heteroatoms selected from N, O and S, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S, halogenated C₃₋₆ cycloalkyl, halogenated 3-6 membered heterocycloalkyl containing 1-4 heteroatoms selected from N, O and S;
alternatively, R₉ and R₁₀ together with the heteroatom to which they are attached form a 3-6 membered heterocycloalkyl containing 1-4 heteroatoms selected from N, O and S, wherein the 3-6 membered heterocycloalkyl is optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ alkylamino, halogenated C₁₋₆ alkylamino, C₁₋₆ alkylthio, halogenated C₁₋₆ alkylthio, C₁₋₆ heteroalkyl containing 1-4 heteroatoms selected from N, O and S, halogenated C₁₋₆ heteroalkyl containing 1-4 heteroatoms selected from N, O and S, C₃₋₆ cycloalkyl, 3-6 membered heterocycloalkyl containing 1-4 heteroatoms selected from N, O and S, halogenated C₃₋₆ cycloalkyl, halogenated 3-6 membered heterocycloalkyl containing 1-4 heteroatoms selected from N, O and S;
each "-̅ -̅ -̅" is independently a single bond or a double bond;
each of m, n and q is independently selected from the group consisting of: 0, 1, 2, 3, 4, and 5.

2. A compound, wherein the compound is a compound of Formula (I-a), or a pharmaceutically acceptable salt, a stereoisomer, a deuterated compound, a solvate or a prodrug thereof, 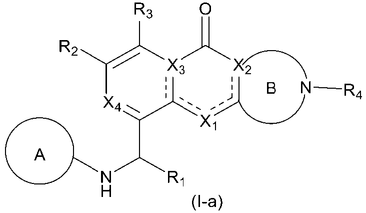 wherein,
X₁ is selected from the group consisting of: N, O, and C(R₁₁)_{q};
X₂ and X₃ are each independently selected from the group consisting of: N and C;
X₄ is N or CR;
ring B is a 5-7 membered heterocycloalkyl containing one N atom and 0-3 additional heteroatoms selected from N, O and S, which is optionally substituted with one or more R₅ groups;
ring A is selected from the group consisting of: C₆₋₁₀ aryl and 5-8 membered heteroaryl containing 1-4 heteroatoms selected from N, O and S; wherein the C₆₋₁₀ aryl and 5-8 membered heteroaryl are optionally substituted with m R₆ groups;
R₁ is selected from the group consisting of: H, deuterium, halogen, -CN, C₁₋₆ alkyl, halogenated C₁₋₆ alkyl, deuterated C₁₋₆ alkyl, C₁₋₆ alkoxy, and halogenated C₁₋₆ alkoxy;
R, R₂, R₃ and R₁₁ are each independently selected from the group consisting of: H, halogen, -CN, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, and halogenated C₃₋₆ cycloalkyl;
R₄ is selected from the group consisting of: -L-(C₁₋₆ alkyl), -L-C₆₋₁₀ aryl, -L-(5-8 membered heteroaryl containing 1-4 heteroatoms selected from N, O and S), -L-(saturated or partially saturated C₃₋₈ cycloalkyl), and -L-(saturated or partially saturated 4-8 membered heterocycloalkyl containing 1-4 heteroatoms selected from N, O and S); wherein the C₁₋₆ alkyl, C₆₋₁₀ aryl, 5-8 membered heteroaryl, C₃₋₈ cycloalkyl, and 4-8 membered heterocycloalkyl are optionally substituted with n R₇ groups;
each R₅ is independently selected from the group consisting of: H, deuterium, halogen, -CN, oxo (=O), C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, C₃₋₆ cycloalkyl, halogenated C₃₋₆ cycloalkyl, and 3-8 membered heterocycloalkyl containing 1-3 heteroatoms selected from N, O and S;
each R₆ is independently selected from the group consisting of: H, deuterium, halogen, -CN, - NO₂, C₁₋₆ alkyl, halogenated C₁₋₆ alkyl, deuterated C₁₋₆ alkyl, C₁₋₆ alkoxy, halogenated C₁₋₆ alkoxy, - C(O)R₈, -C(O)OR₈, -C(O)NR₈R₉, -SO₂R₈, -S(O)(NR₈)R₉, -SO₂NR₈R₉, and -P(O)R₈R₉;
each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -NR₈R₉, =NR₈, - C(O)R₈, -C(O)OR₈, -C(O)NR₈R₉, -NR₈C(O)R₉, -SR₈, -SO₂R₈, -NR₈SO₂R₉, -SO₂NR₈R₉, - NR₈SO₂NR₉R₁₀, -P(O)R₈R₉, -L₁-(C₁₋₆ alkyl), -L₁-(saturated or partially saturated C₃₋₁₀ cycloalkyl), - L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-10 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, 3-6 membered heterocycloalkyl containing 1-4 heteroatoms selected from N, O and S, - SO₂R₈, -C(O)R₈, -C(O)OR₈, -C(O)NR₈R₉, and -NR₈R₉;
alternatively, two R₇ groups together with the atoms to which they are attached form C₃₋₆ cycloalkyl, C₆₋₁₀ aryl, 3-6 membered heterocycloalkyl containing 1-4 heteroatoms selected from N, O and S, or 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NR₈R₉, =NR₈, -C(O)R₈, -C(O)OR₈, -C(O)NR₈R₉, -NR₈C(O)R₉, -SR₈, -SO₂R₈, - NR₈SO₂R₉, -SO₂NR₈R₉, -NR₈SO₂NR₉R₁₀, -P(O)R₈R₉, C₁₋₆ alkyl, C₁₋₆ alkoxy, C₁₋₆ heteroalkyl containing 1-4 heteroatoms selected from N, O and S, halogenated C₁₋₆ alkyl, halogenated C₁₋₆ alkoxy, halogenated C₁₋₆ heteroalkyl containing 1-4 heteroatoms selected from N, O and S, C₃₋₆ cycloalkyl, and 3-6 membered heterocycloalkyl containing 1-4 heteroatoms selected from N, O and S;
L and L₁ are each independently a bond or selected from the group consisting of: C₁₋₆ alkylene, 1-6 membered heteroalkylene containing 1-4 heteroatoms selected from N, O and S, -NR₈-, -O-, - C(O)-, -C(O)NR₈-, -C(O)O-, -NR₈C(O)-, -(SO₂)-, -SO₂NR₈-, -NR₈SO₂-, -NR₈SO₂NR₉-, and - S(O)(NR₈)-;
each of R₈, R₉ and R₁₀ is independently selected from the group consisting of: H, C₁₋₆ alkyl, halogenated C₁₋₆ alkyl, C₃₋₆ cycloalkyl, C₆₋₁₀ aryl, 3-6 membered heterocycloalkyl containing 1-4 heteroatoms selected from N, O and S, and 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ heteroalkyl containing 1-4 heteroatoms selected from N, O and S, and halogenated C₁₋₆ heteroalkyl containing 1-4 heteroatoms selected from N, O and S;
alternatively, R₈ and R₉ together with the heteroatom to which they are attached form a 3-6 membered heterocycloalkyl containing 1-4 heteroatoms selected from N, O and S;
alternatively, R₉ and R₁₀ together with the heteroatom to which they are attached form a 3-6 membered heterocycloalkyl containing 1-4 heteroatoms selected from N, O and S;
"-̅ -̅ -̅" is a single bond or a double bond;
each of m, n and q is independently selected from the group consisting of: 0, 1, 2, 3, 4, and 5.

3. The compound according to claim 2, or the pharmaceutically acceptable salt, the stereoisomer, the deuterated compound, the solvate or the prodrug thereof, wherein, the compound has a structure selected from the group consisting of: 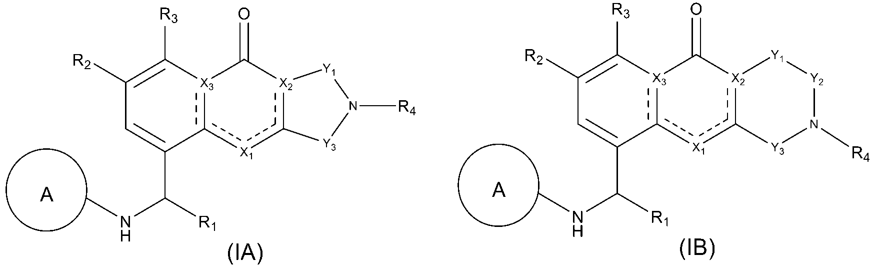 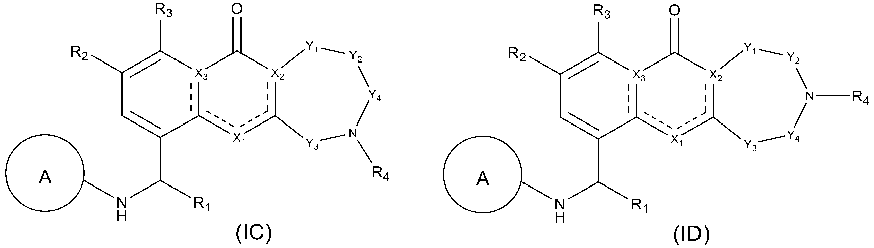 wherein,
X₁ is selected from the group consisting of: N, O, and C(R₁₁)_{q};
X₂ and X₃ are each independently selected from the group consisting of: N and C;
Y₁, Y₂, Y₃ and Y₄ are each independently selected from the group consisting of: O, C(R₅)₂, CHR₅, C(=O), S(=O), S(=O)₂, and NR₅;
ring A is selected from the group consisting of: phenyl and 5-6 membered heteroaryl containing 1-4 heteroatoms selected from N, O and S; wherein the phenyl and 5-6 membered heteroaryl are optionally substituted with m R₆ groups;
R₁ is selected from the group consisting of: H, deuterium, halogen, -CN, C₁₋₃ alkyl, halogenated C₁₋₃ alkyl, deuterated C₁₋₃ alkyl, C₁₋₃ alkoxy, and halogenated C₁₋₃ alkoxy;
R₂, R₃ and R₁₁ are each independently selected from the group consisting of: H, halogen, -CN, C₁₋₃ alkyl, halogenated C₁₋₃ alkyl, deuterated C₁₋₃ alkyl, C₁₋₃ alkoxy, halogenated C₁₋₃ alkoxy, deuterated C₁₋₃ alkoxy, C₃₋₅ cycloalkyl, and halogenated C₃₋₅ cycloalkyl;
R₄ is selected from the group consisting of: -L-(C₁₋₆ alkyl), -L-phenyl, -L-(5-6 membered heteroaryl containing 1-4 heteroatoms selected from N, O and S), -L-(saturated or partially saturated C₃₋₆ cycloalkyl), and -L-(saturated or partially saturated 4-6 membered heterocycloalkyl containing 1-4 heteroatoms selected from N, O and S); wherein the C₁₋₆ alkyl, phenyl, 5-6 membered heteroaryl, C₃₋₆ cycloalkyl, and 4-6 membered heterocycloalkyl are optionally substituted with n R₇ groups;
each R₅ is independently selected from the group consisting of: H, deuterium, halogen, -CN, oxo (=O), C₁₋₃ alkyl, halogenated C₁₋₃ alkyl, deuterated C₁₋₃ alkyl, C₁₋₃ alkoxy, halogenated C₁₋₃ alkoxy, deuterated C₁₋₃ alkoxy, C₃₋₅ cycloalkyl, halogenated C₃₋₅ cycloalkyl, and 3-5 membered heterocycloalkyl containing 1-3 heteroatoms selected from N, O and S;
each R₆ is independently selected from the group consisting of: H, deuterium, halogen, -CN, - NO₂, C₁₋₆ alkyl, halogenated C₁₋₆ alkyl, deuterated C₁₋₃ alkyl, C₁₋₆ alkoxy, halogenated C₁₋₆ alkoxy, - C(O)R₈, -C(O)OR₈, -C(O)NR₈R₉, -SO₂R₈, -S(O)(NR₈)R₉, -SO₂NR₈R₉, and -P(O)R₈R₉;
each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -NR₈R₉, =NR₈, - C(O)R₈, -C(O)OR₈, -C(O)NR₈R₉, -NR₈C(O)R₉, -SR₈, -SO₂R₈, -NR₈SO₂R₉, -SO₂NR₈R₉, - NR₈SO₂NR₉R₁₀, -P(O)R₈R₉, -L₁-(C₁₋₆ alkyl), -L₁-(saturated or partially saturated C₃₋₁₀ cycloalkyl), - L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-10 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, 3-6 membered heterocycloalkyl containing 1-4 heteroatoms selected from N, O and S, - SO₂R₈, -C(O)R₈, -C(O)OR₈, -C(O)NR₈R₉, and -NR₈R₉;
alternatively, two R₇ groups together with the atoms to which they are attached form C₃₋₆ cycloalkyl, C₆₋₁₀ aryl, 3-6 membered heterocycloalkyl containing 1-4 heteroatoms selected from N, O and S, or 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NR₈R₉, =NR₈, -C(O)R₈, -C(O)OR₈, -C(O)NR₈R₉, -NR₈C(O)R₉, -SR₈, -SO₂R₈, - NR₈SO₂R₉, -SO₂NR₈R₉, -NR₈SO₂NR₉R₁₀, -P(O)R₈R₉, C₁₋₆ alkyl, C₁₋₆ alkoxy, C₁₋₆ heteroalkyl containing 1-4 heteroatoms selected from N, O and S, halogenated C₁₋₆ alkyl, halogenated C₁₋₆ alkoxy, halogenated C₁₋₆ heteroalkyl containing 1-4 heteroatoms selected from N, O and S, C₃₋₆ cycloalkyl, and 3-6 membered heterocycloalkyl containing 1-4 heteroatoms selected from N, O and S;
L and L₁ are each independently a bond or selected from the group consisting of: C₁₋₆ alkylene, 1-6 membered heteroalkylene containing 1-4 heteroatoms selected from N, O and S, -NR₈-, -O-, - C(O)-, -C(O)NR₈, -C(O)O-, -NR₈C(O)-, -(SO₂)-, -SO₂NR₈-, -NR₈SO₂-, and -NR₈SO₂NR₉-;
each of R₈, R₉ and R₁₀ is independently selected from the group consisting of: H, C₁₋₆ alkyl, halogenated C₁₋₆ alkyl, C₃₋₆ cycloalkyl, C₆₋₁₀ aryl, 3-6 membered heterocycloalkyl containing 1-4 heteroatoms selected from N, O and S, and 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₁₋₆ heteroalkyl containing 1-4 heteroatoms selected from N, O and S, and halogenated C₁₋₆ heteroalkyl containing 1-4 heteroatoms selected from N, O and S;
alternatively, R₈ and R₉ together with the heteroatom to which they are attached form a 3-6 membered heterocycloalkyl containing 1-4 heteroatoms selected from N, O and S;
alternatively, R₉ and R₁₀ together with the heteroatom to which they are attached form a 3-6 membered heterocycloalkyl containing 1-4 heteroatoms selected from N, O and S;
"-̅ -̅ -̅" is a single bond or a double bond;
each of m, n and q is independently selected from the group consisting of: 0, 1, 2, 3, 4, and 5.

4. The compound according to claim 3, or the pharmaceutically acceptable salt, the stereoisomer, the deuterated compound, the solvate or the prodrug thereof, wherein, the compound has a structure selected from the group consisting of: 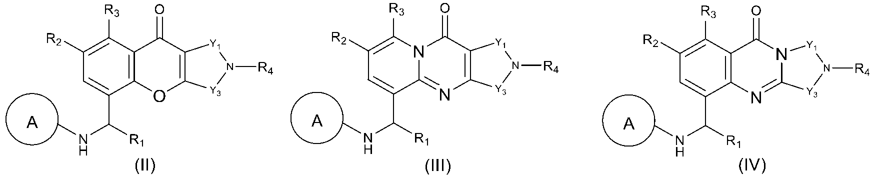 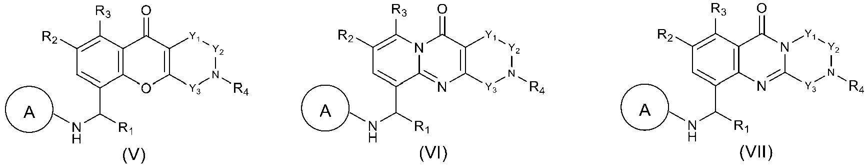 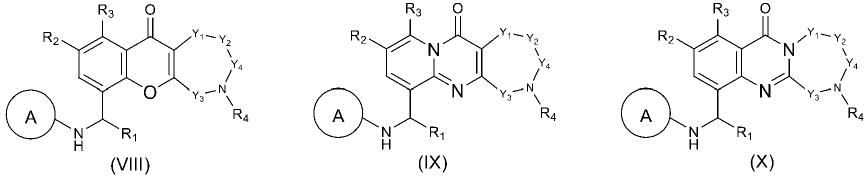 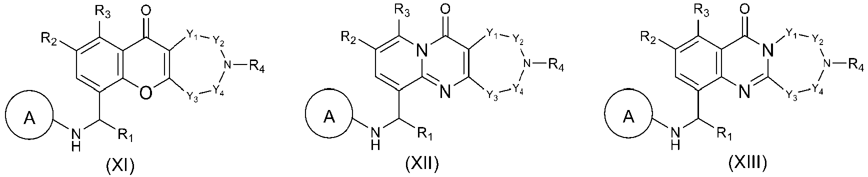 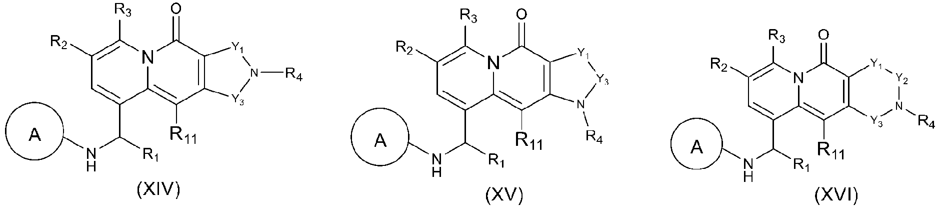 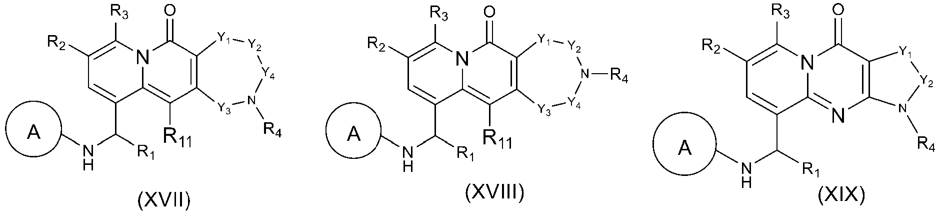
wherein, R₁, R₂, R₃, R₄, R₁₁, Y₁, Y₂, Y₃, Y₄, and ring A are as defined in claim 1 or 2;
the structure 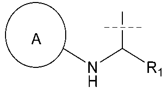 includes the following configurations: 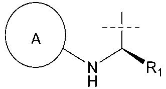 and

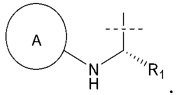

5. The compound according to claim 1 or 2, or the pharmaceutically acceptable salt, the stereoisomer, the deuterated compound, the solvate or the prodrug thereof, wherein, ring A has a structure selected from the group consisting of: 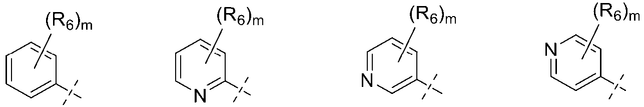 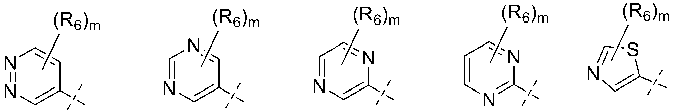
wherein each R₆ is independently selected from the group consisting of: H, deuterium, halogen, - CN, -NO₂, C₁₋₆ alkyl, halogenated C₁₋₆ alkyl, deuterated C₁₋₃ alkyl, C₁₋₆ alkoxy, halogenated C₁₋₆ alkoxy, -C(O)R₈, -C(O)OR₈, -C(O)NR₈R₉, -SO₂R₈, -S(O)(NR₈)R₉, -SO₂NR₈R₉, and -P(O)R₈R₉;
each R₈ and R₉ are as defined in claim 1 or 2;
m is selected from the group consisting of: 0, 1, 2, 3, 4, and 5.

6. The compound according to claim 1 or 2, or the pharmaceutically acceptable salt, the stereoisomer, the deuterated compound, the solvate or the prodrug thereof, wherein, R₄ has a structure selected from the group consisting of: 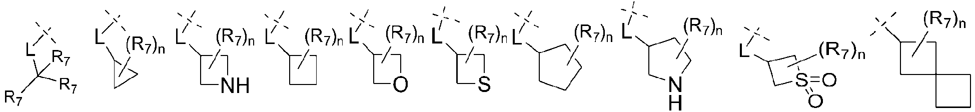 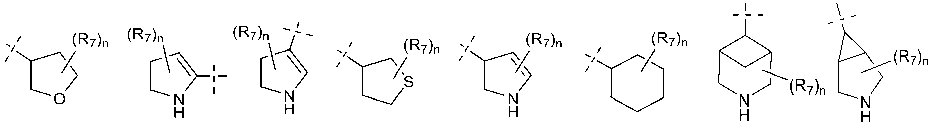 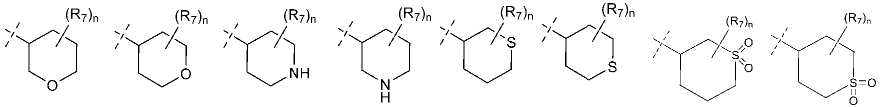 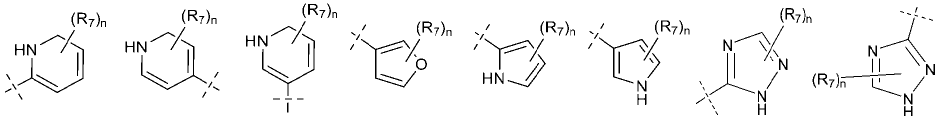 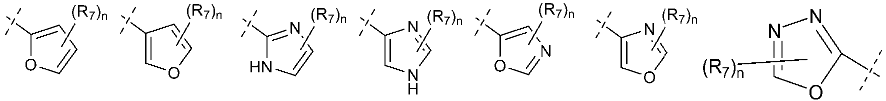 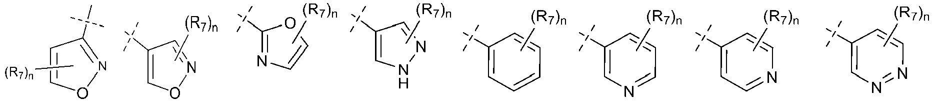 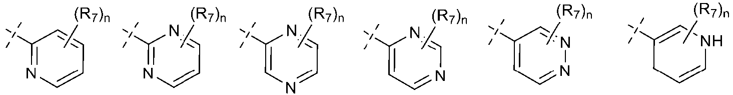 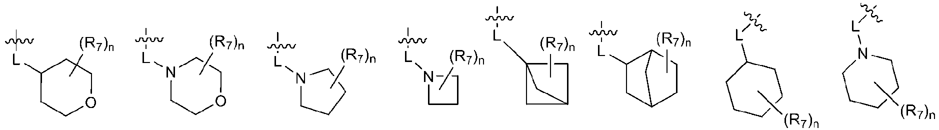 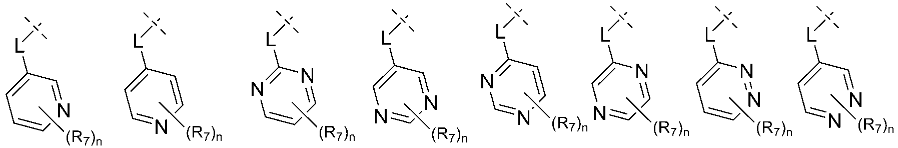 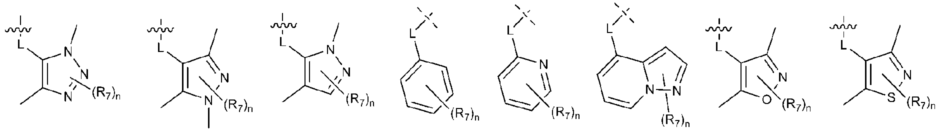
wherein, L and L₁ are each independently a bond or selected from the group consisting of: C₁₋₆ alkylene, -C(O)-, -C(O)NH-, -C(O)O-, -(SO₂)-, and -SO₂NH-;
each R₇ is independently selected from the group consisting of: hydrogen, deuterium, halogen, oxo (=O), -CN, -OH, -NO₂, C₁₋₆ alkyl, C₁₋₆ alkoxy, halogenated C₁₋₆ alkyl, halogenated C₁₋₆ alkoxy, deuterated C₁₋₆ alkyl, C₁₋₆ heteroalkyl containing 1-4 heteroatoms selected from N, O and S, halogenated C₁₋₆ heteroalkyl containing 1-4 heteroatoms selected from N, O and S, -NR₈R₉, =NR₈, - C(O)R₈, -C(O)OR₈, -C(O)NR₈R₉, -NR₈C(O)R₉, -SR₈, -SO₂R₈, -NR₈SO₂R₉, -SO₂NR₈R₉, - NR₈SO₂NR₉R₁₀, -P(O)R₈R₉, -L₁-(C₁₋₆ alkyl), -L₁-(saturated or partially saturated C₃₋₁₀ cycloalkyl), - L₁-(C₆₋₁₀ aryl), -L₁-(saturated or partially saturated 3-12 membered heterocycloalkyl containing 1-4 heteroatoms selected from N, O and S), and -L₁-(5-10 membered heteroaryl containing 1-4 heteroatoms selected from N, O and S), wherein the C₁₋₆ alkyl, C₁₋₆ heteroalkyl, C₃₋₁₀ cycloalkyl, C₆₋₁₀ aryl, 3-12 membered heterocycloalkyl, and 5-10 membered heteroaryl are optionally substituted with 0-6 substituents selected from the group consisting of: deuterium, halogen, =O, -CN, -OH, -NH₂, C₁₋₆ alkyl, halogenated C₁₋₆ alkyl, C₁₋₆ alkoxy, halogenated C₁₋₆ alkoxy, C₃₋₆ cycloalkyl, halogenated C₃₋₆ cycloalkyl, 3-6 membered heterocycloalkyl containing 1-4 heteroatoms selected from N, O and S, - SO₂R₈, -C(O)R₈, -C(O)OR₈, -C(O)NR₈R₉, and -NR₈R₉;
alternatively, two R₇ groups together with the atoms to which they are attached form C₃₋₆ cycloalkyl, C₆₋₁₀ aryl, 3-6 membered heterocycloalkyl containing 1-4 heteroatoms selected from N, O and S, or 5-6 membered heteroaryl containing 1-4 heteroatoms selected from N, O and S, wherein the C₃₋₆ cycloalkyl, C₆₋₁₀ aryl, 3-6 membered heterocycloalkyl, and 5-6 membered heteroaryl are optionally substituted with 0-6 substituents selected from the group consisting of: deuterium, halogen, =O, -CN, -OH, -NR₈R₉, =NR₈, -C(O)R₈, -C(O)OR₈, -C(O)NR₈R₉, -NR₈C(O)R₉, -SR₈, -SO₂R₈, - NR₈SO₂R₉, -SO₂NR₈R₉, -NR₈SO₂NR₉R₁₀, -P(O)R₈R₉, C₁₋₆ alkyl, C₁₋₆ alkoxy, C₁₋₆ heteroalkyl containing 1-4 heteroatoms selected from N, O and S, halogenated C₁₋₆ alkyl, halogenated C₁₋₆ alkoxy, halogenated C₁₋₆ heteroalkyl containing 1-4 heteroatoms selected from N, O and S, C₃₋₆ cycloalkyl, and 3-6 membered heterocycloalkyl containing 1-4 heteroatoms selected from N, O and S;
each of R₈, R₉ and R₁₀ is as defined in claim 1 or 2;
n is selected from the group consisting of: 0, 1, 2, 3, 4, and 5.

7. The compound according to claim 3, or the pharmaceutically acceptable salt, the stereoisomer, the deuterated compound, the solvate or the prodrug thereof, wherein, the compound has a structure selected from the group consisting of: 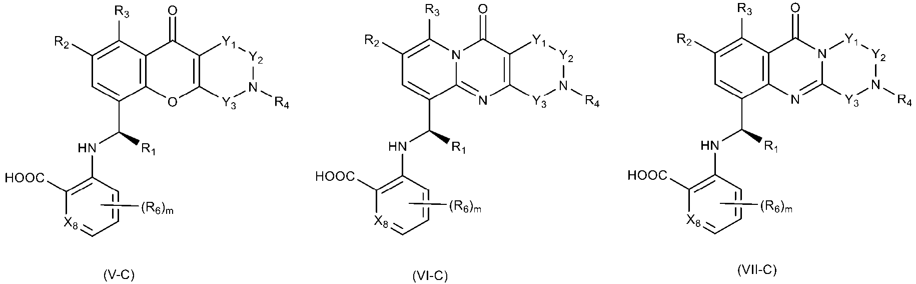 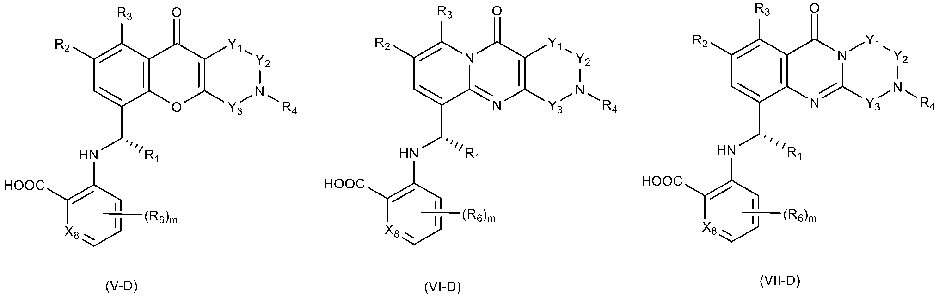
wherein, R₁, R₂, R₃, R₄, Y₁, Y₂ and Y₃ are as defined in claim 4;
X₈ is CR₆ or N;
each R₆ is independently selected from the group consisting of: H, deuterium, halogen, -CN, - NO₂, C₁₋₆ alkyl, halogenated C₁₋₆ alkyl, deuterated C₁₋₃ alkyl, C₁₋₆ alkoxy, halogenated C₁₋₆ alkoxy, - C(O)R₈, -C(O)OR₈, -C(O)NR₈R₉, -SO₂R₈, -S(O)(NR₈)R₉, -SO₂NR₈R₉, and -P(O)R₈R₉;
m is selected from the group consisting of: 0, 1, 2, 3 and 4.

8. The compound according to claim 1 or 2, or the pharmaceutically acceptable salt, the stereoisomer, the deuterated compound, the solvate or the prodrug thereof, wherein, the compound is selected from the group consisting of:

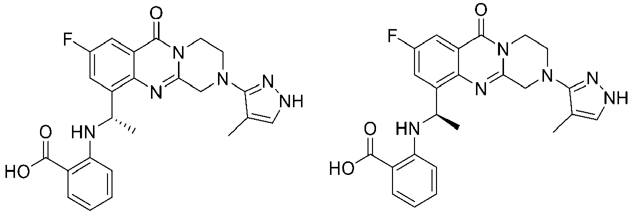

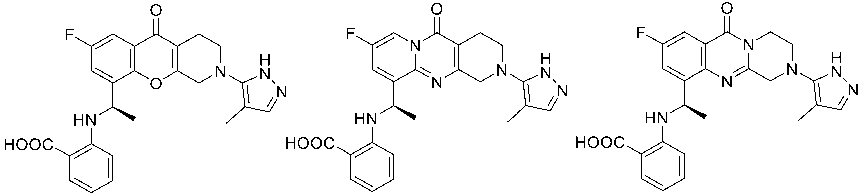

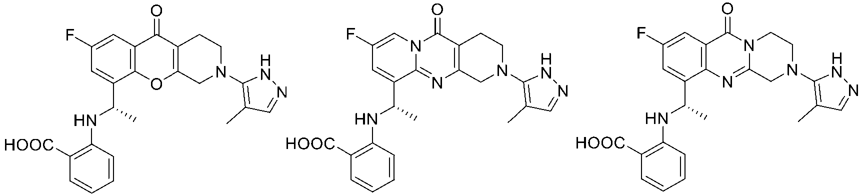

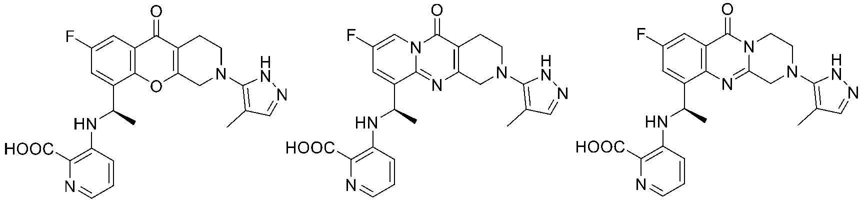

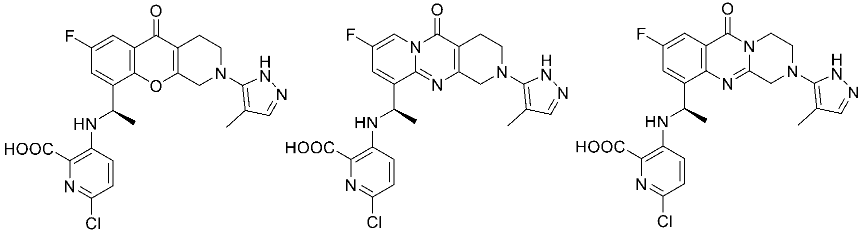

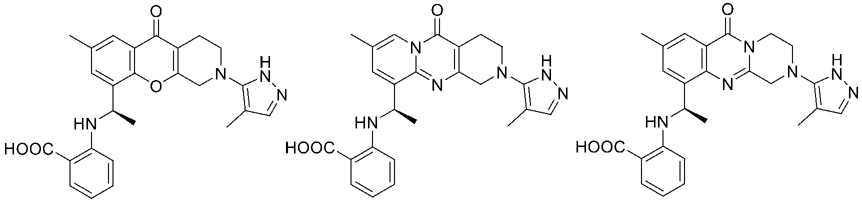

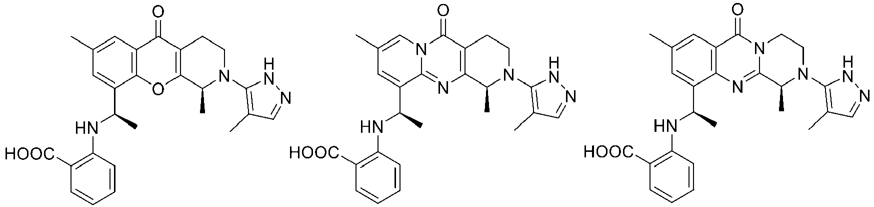

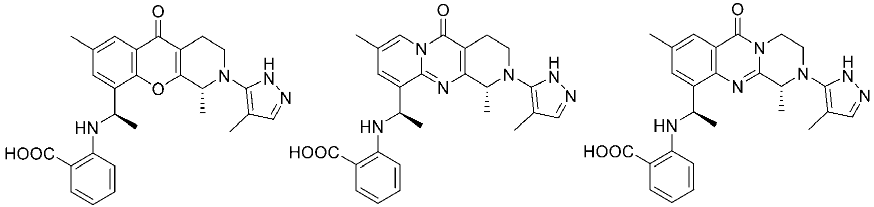

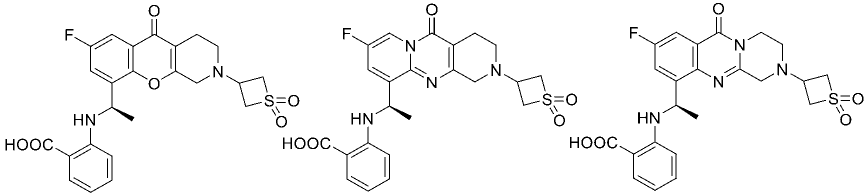

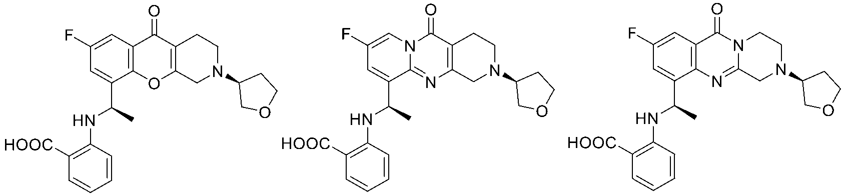

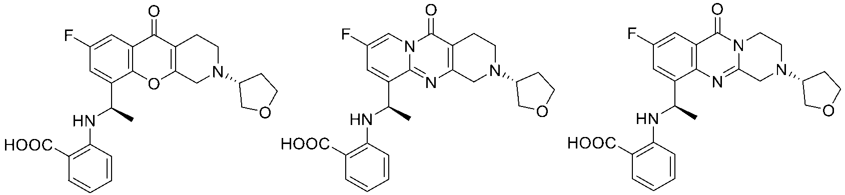

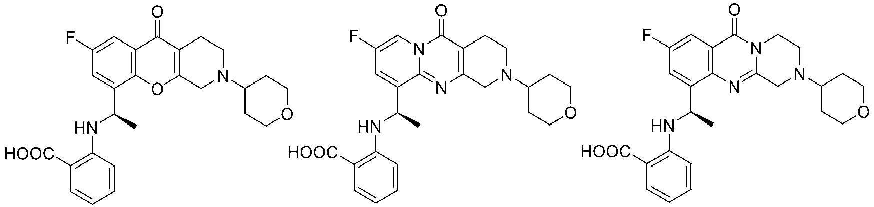

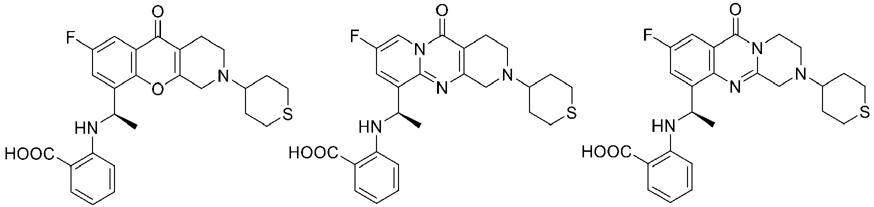

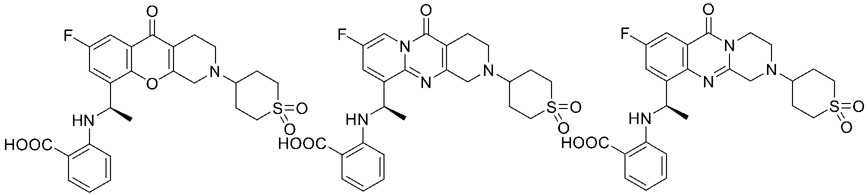

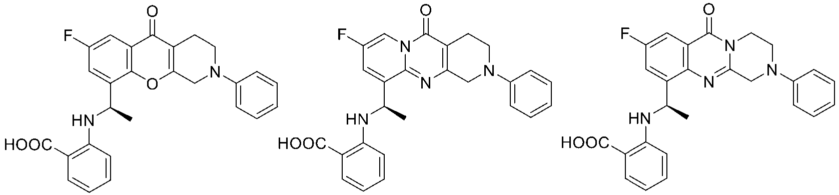

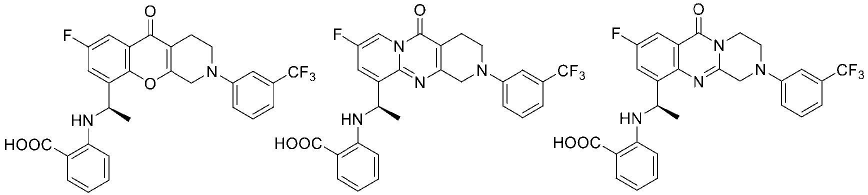

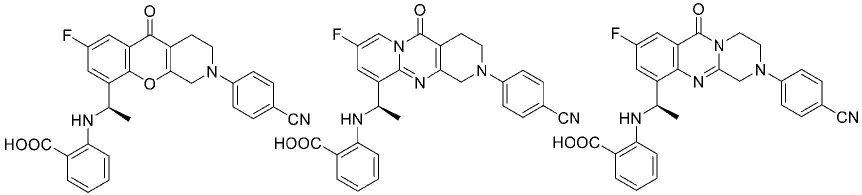

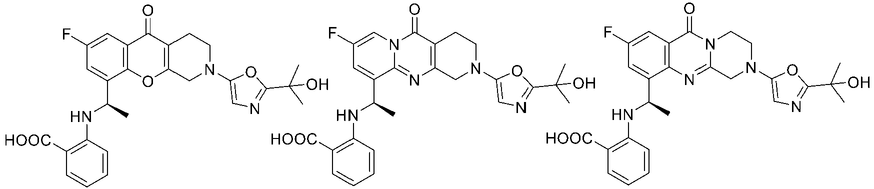

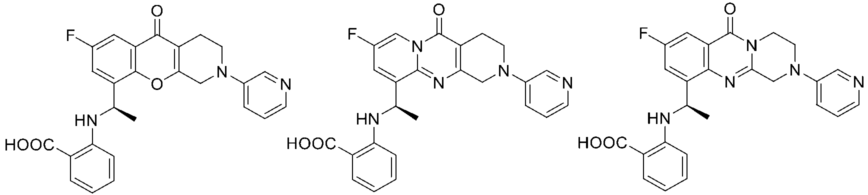

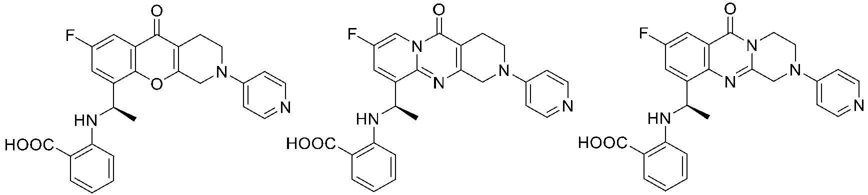

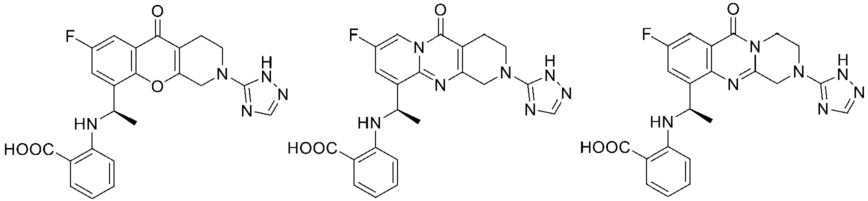

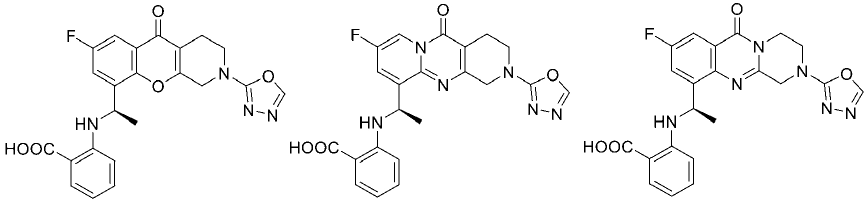

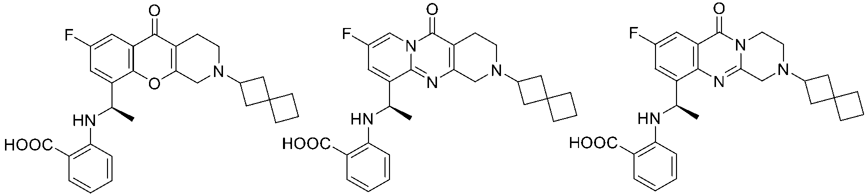

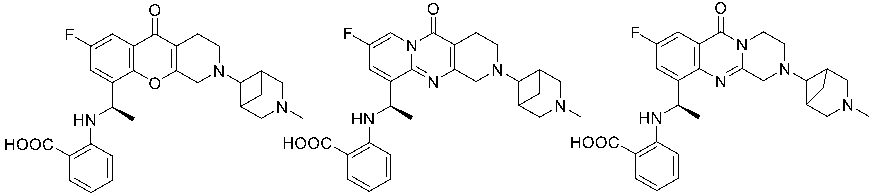

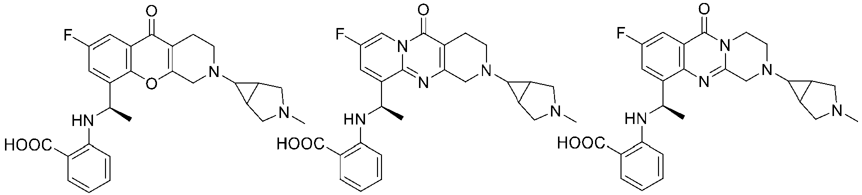

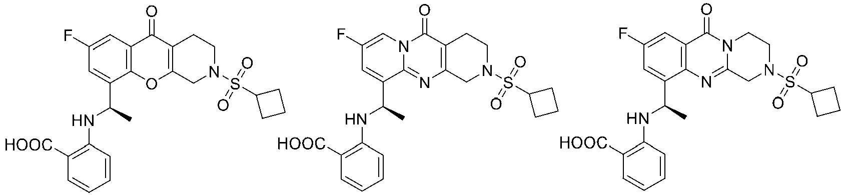

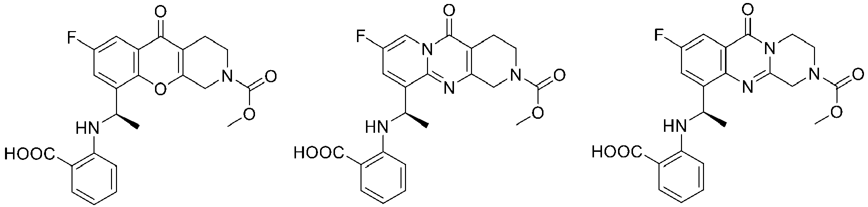

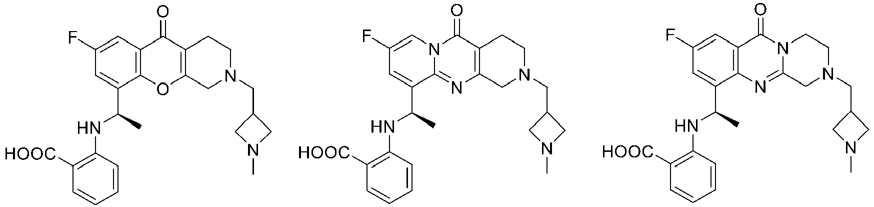

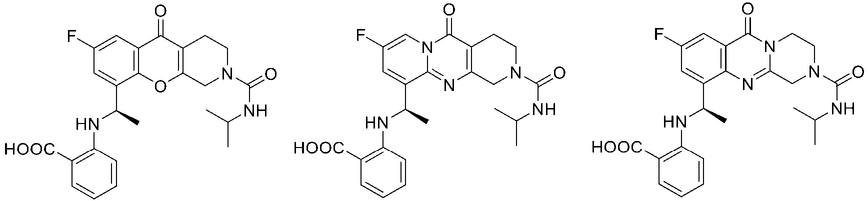

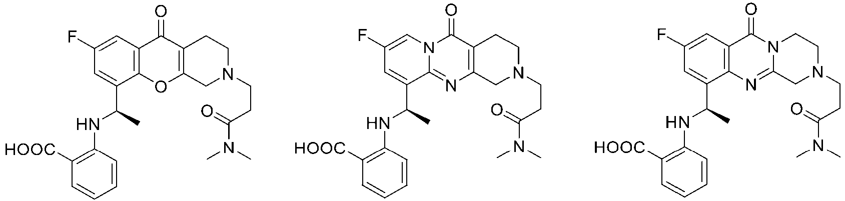

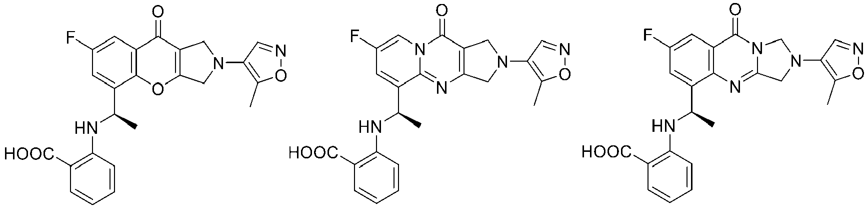

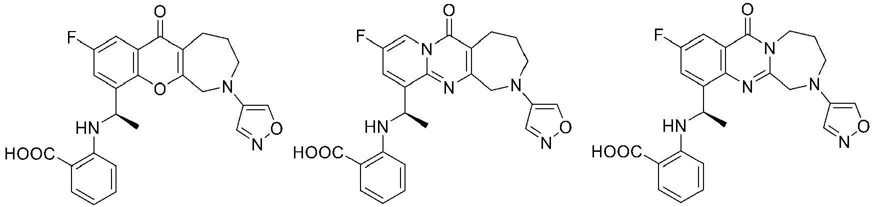

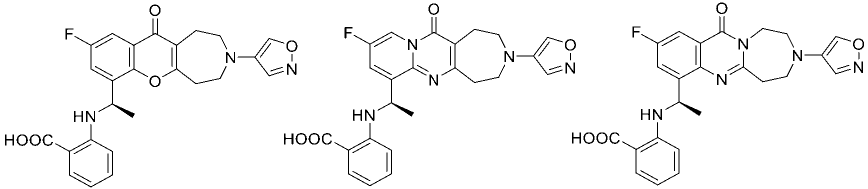

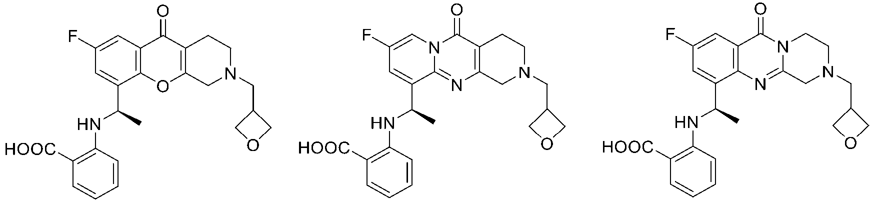

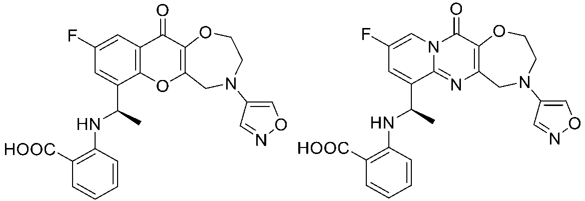

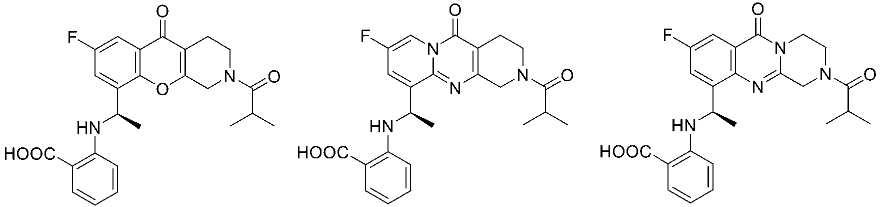

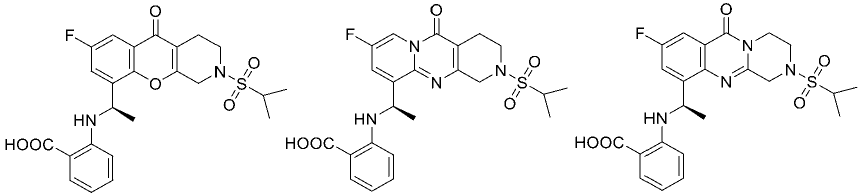

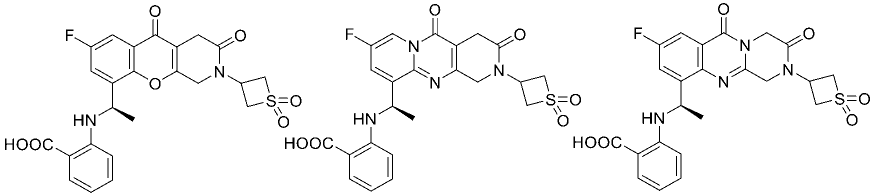

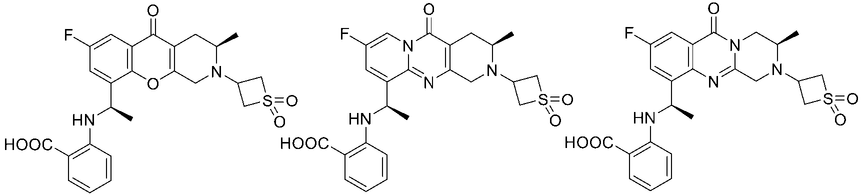

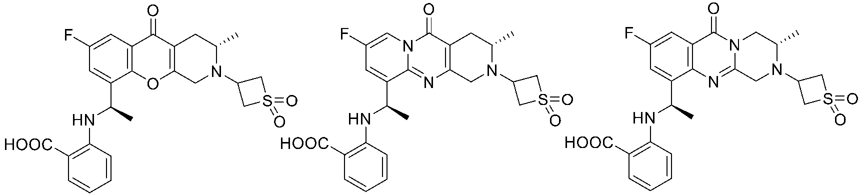

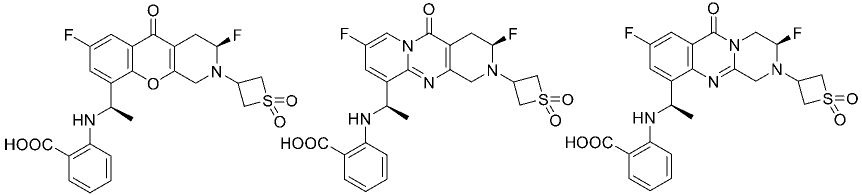

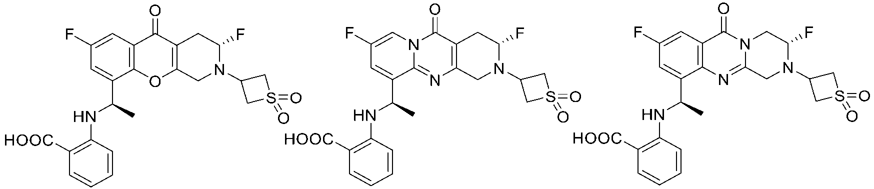

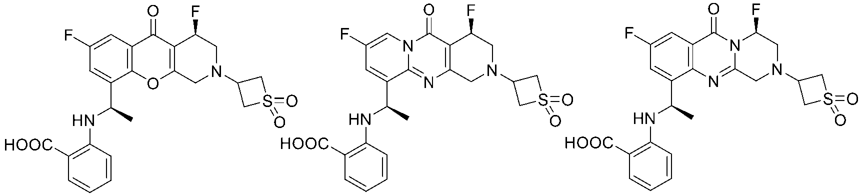

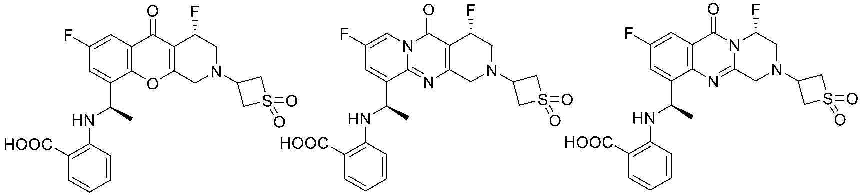

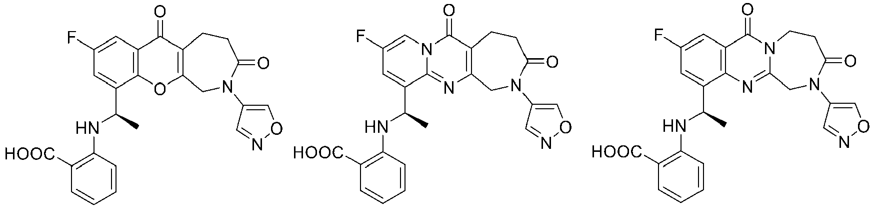

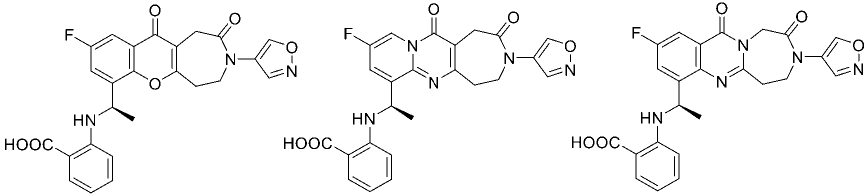

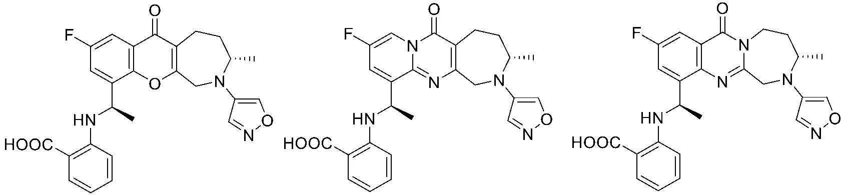

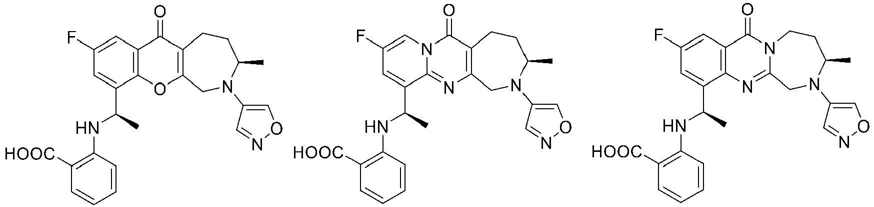

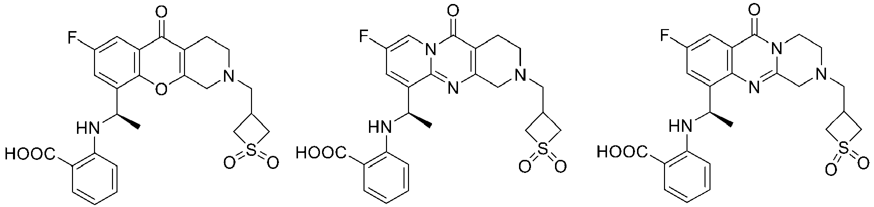

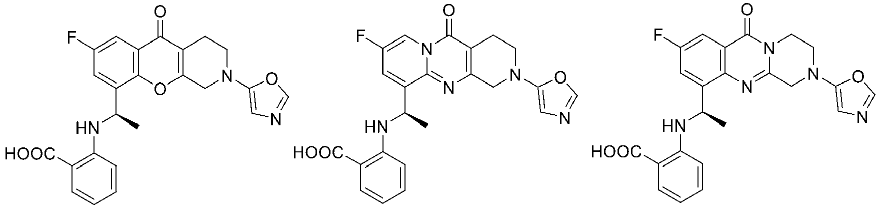

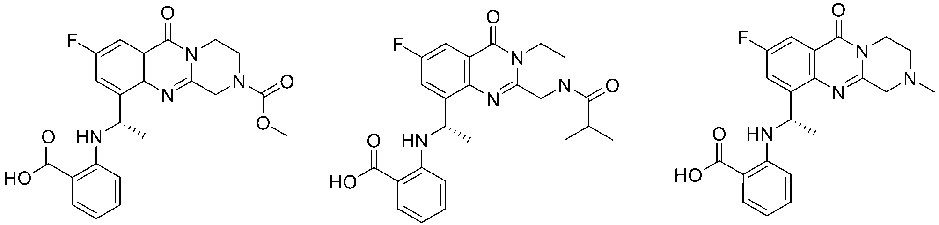

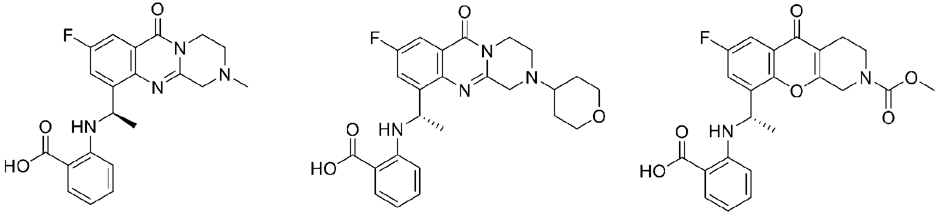

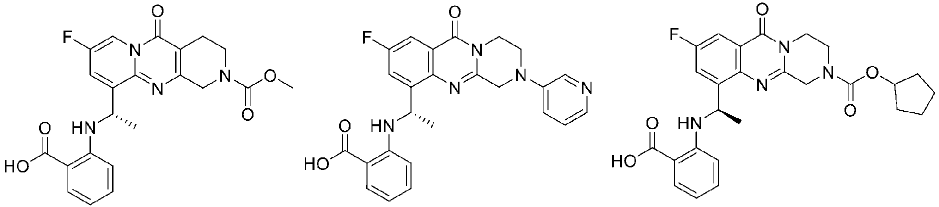

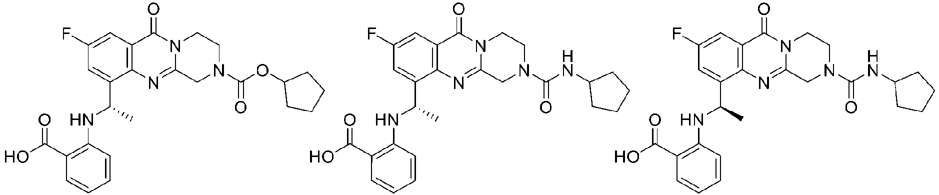

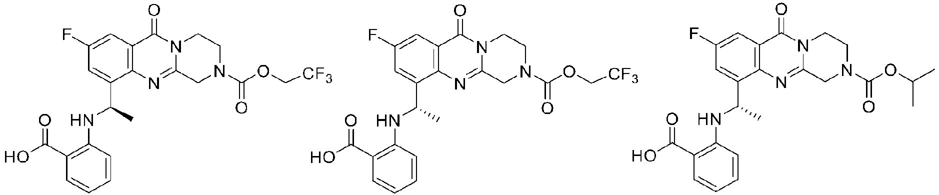

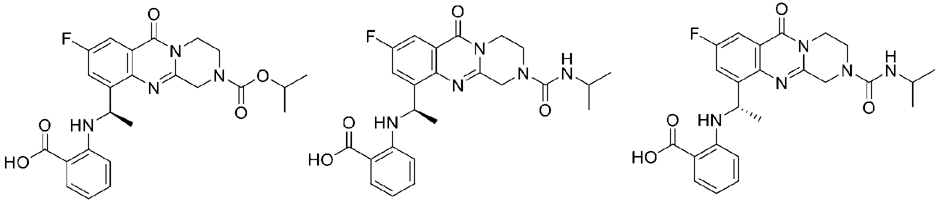

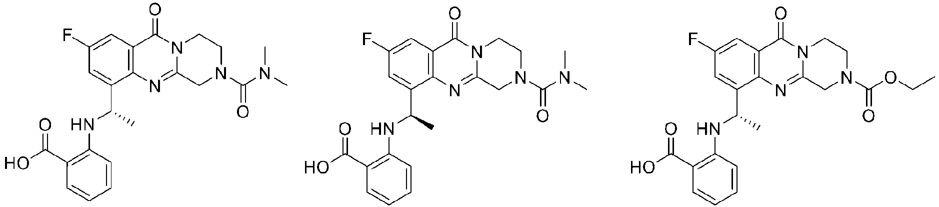

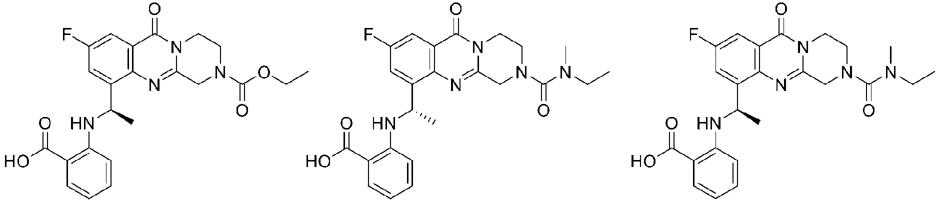

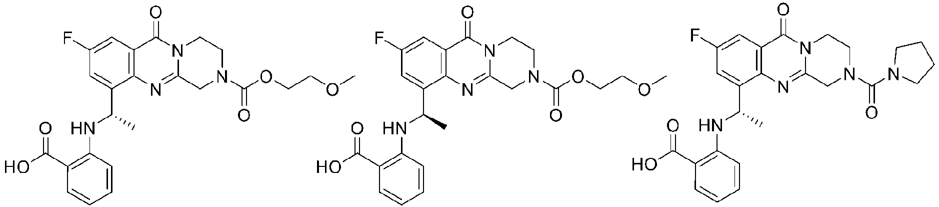

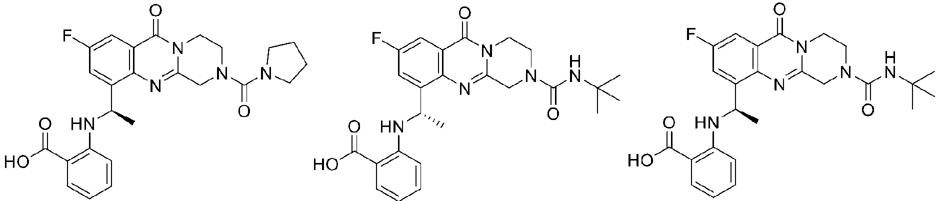

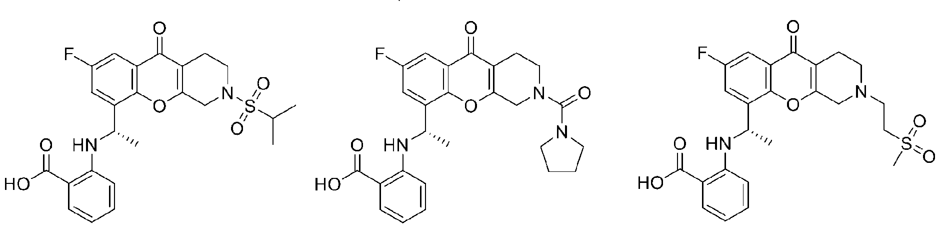

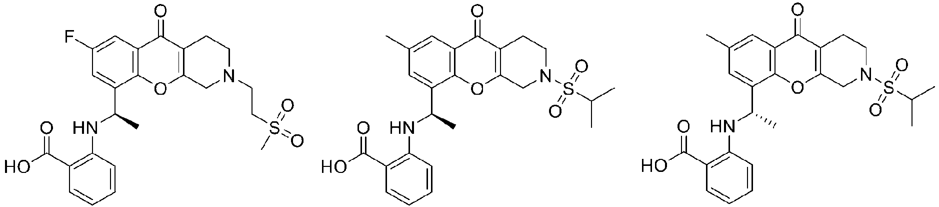

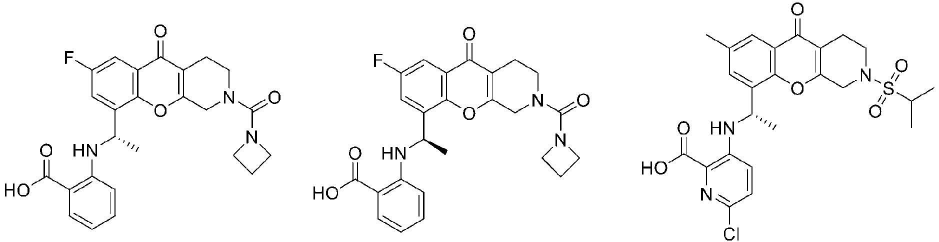

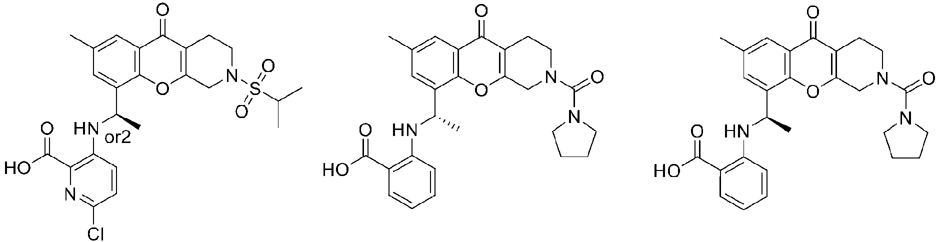

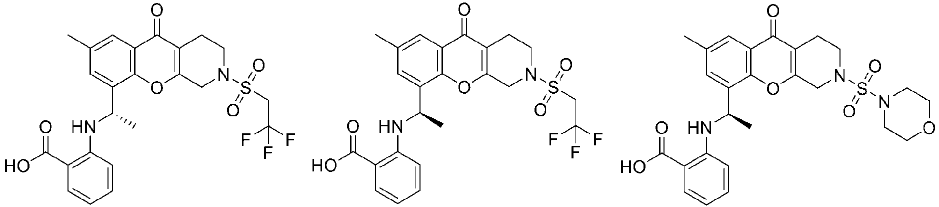

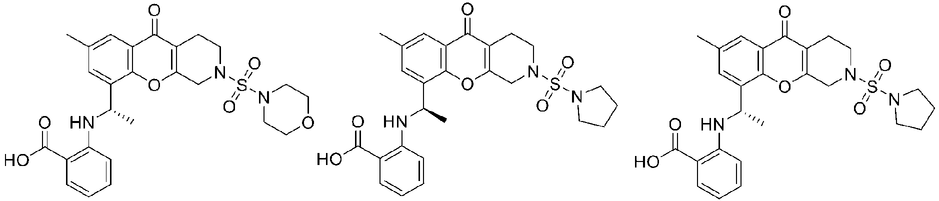

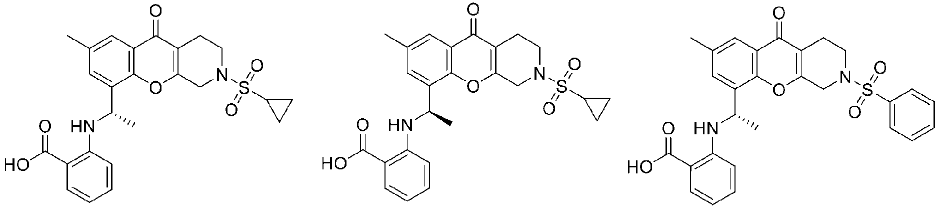

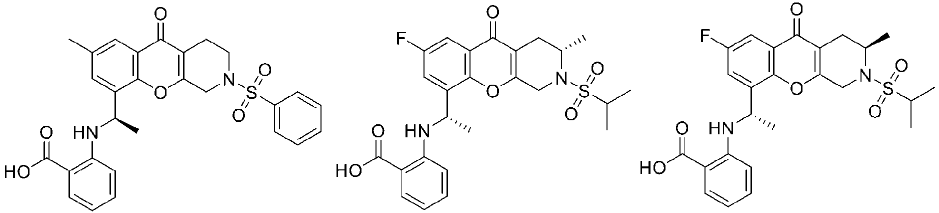

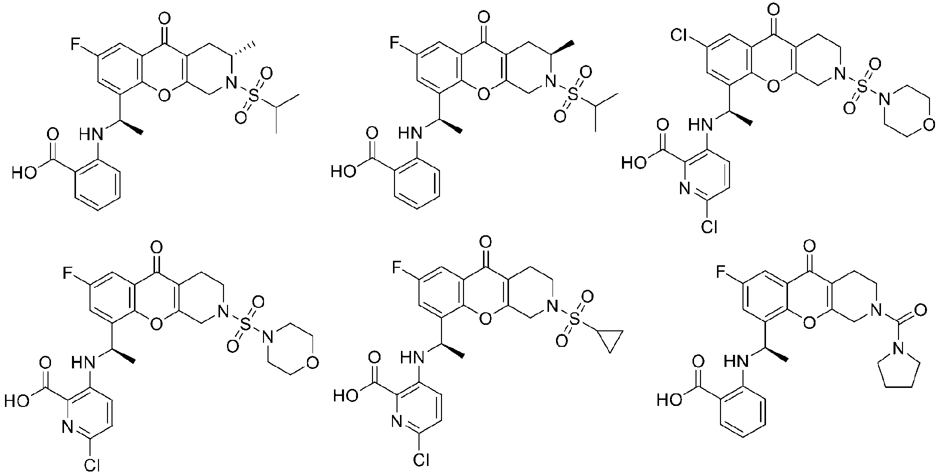

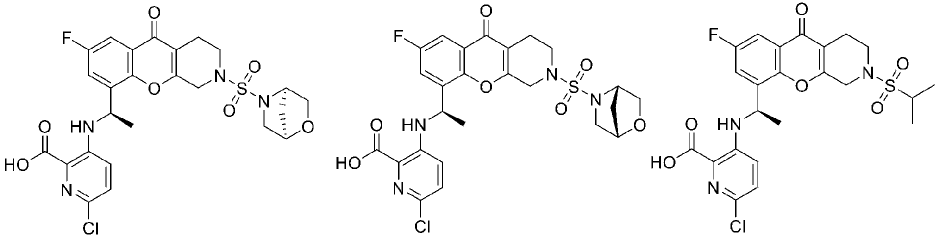

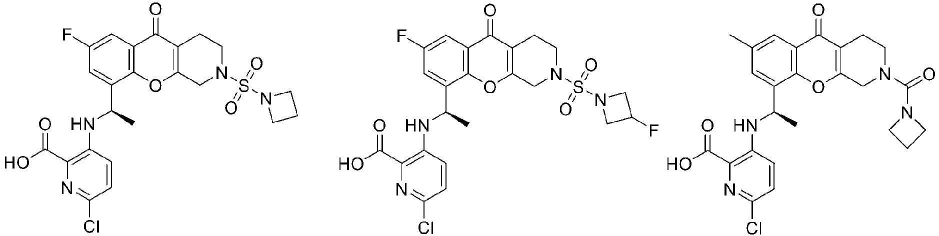

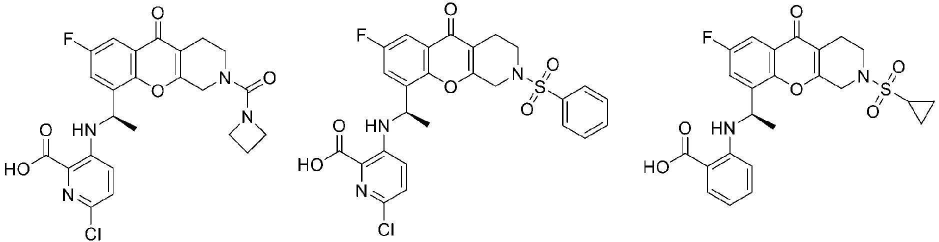

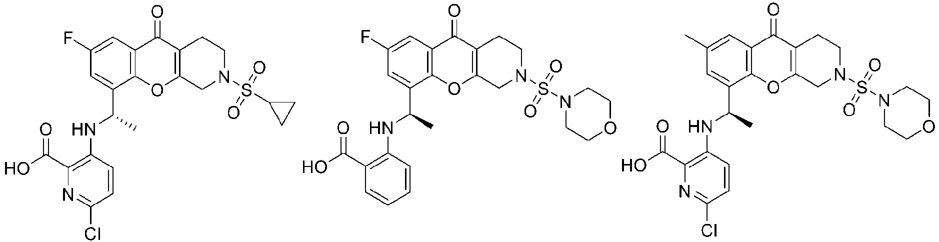

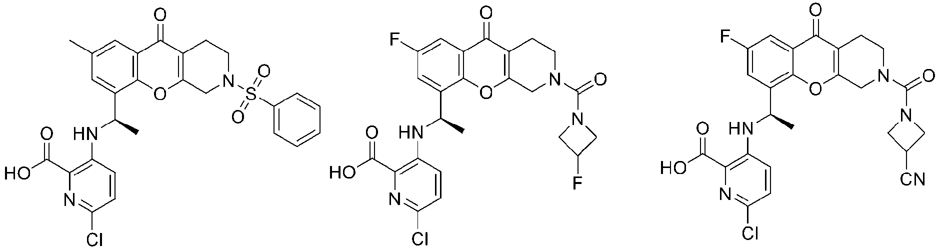

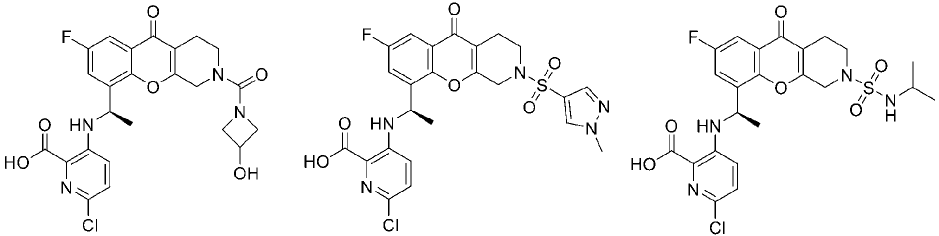

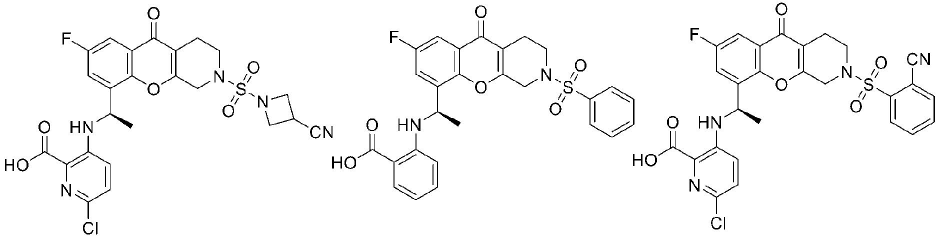

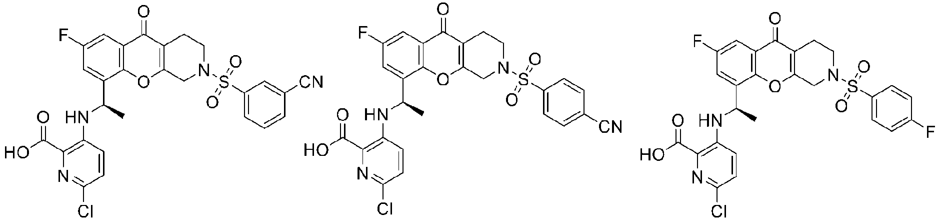

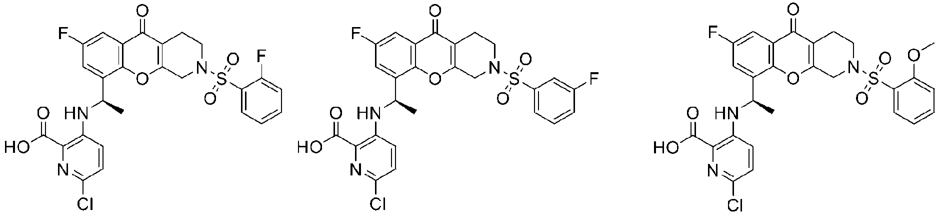

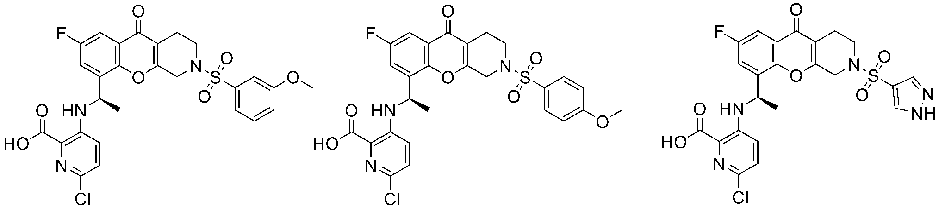

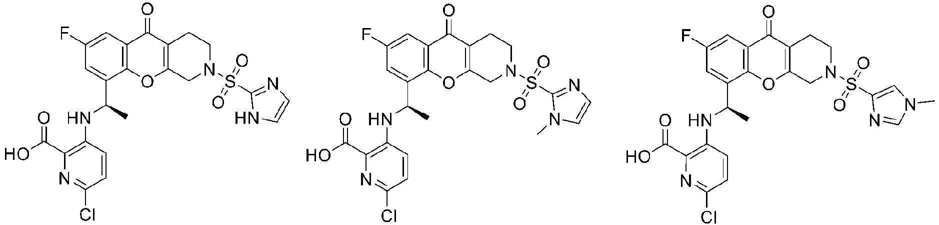

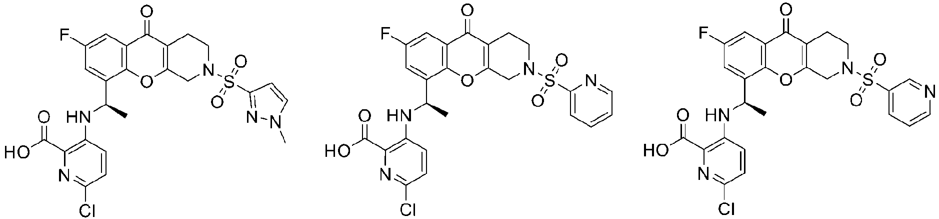

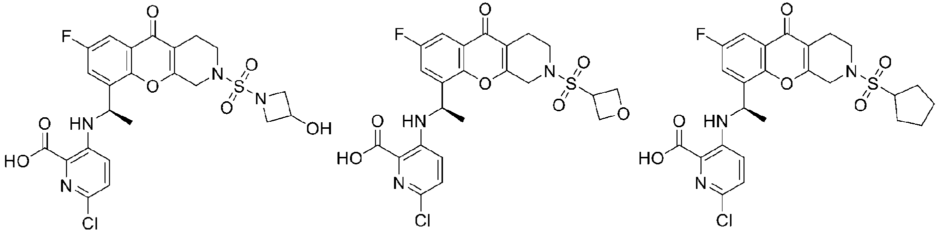

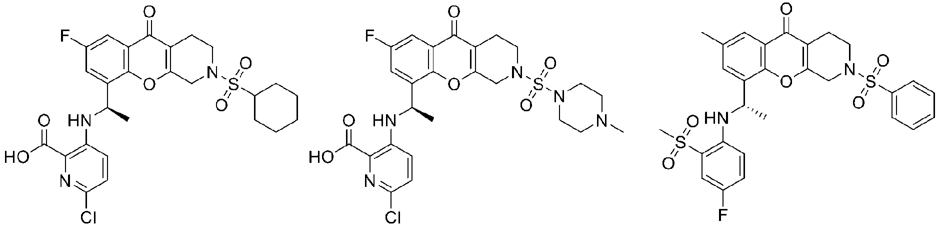

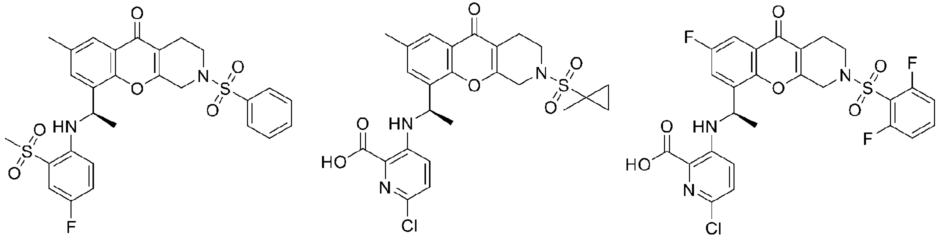

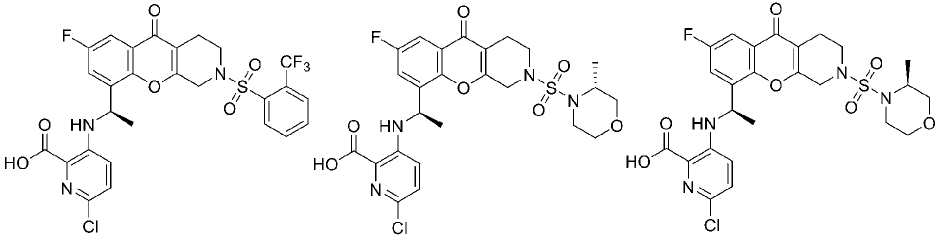

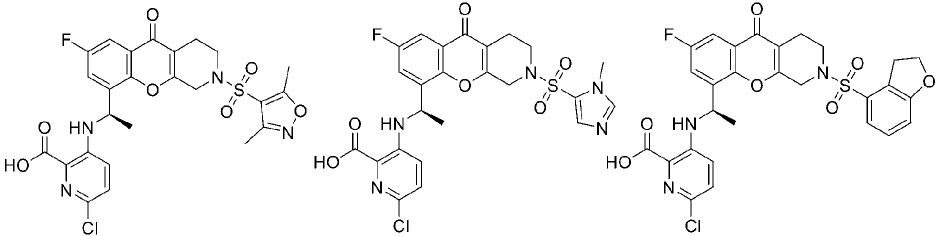

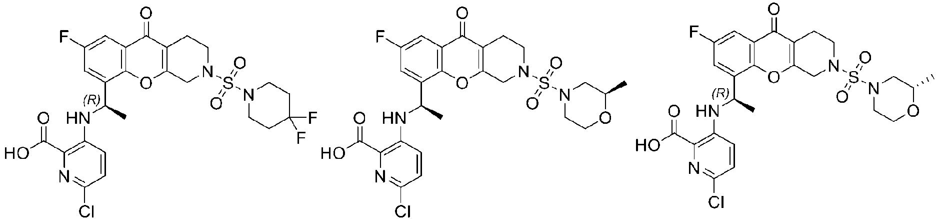

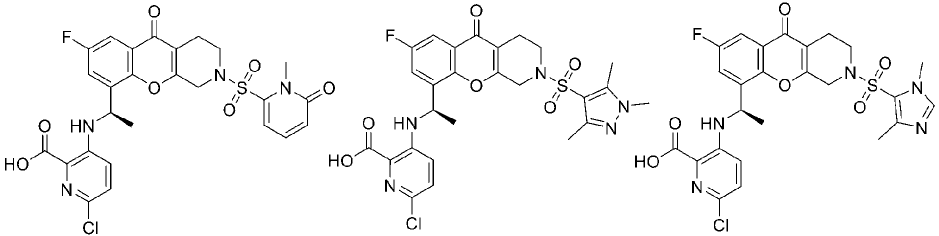

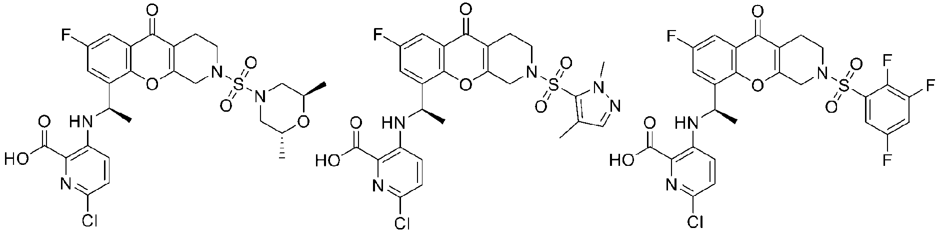

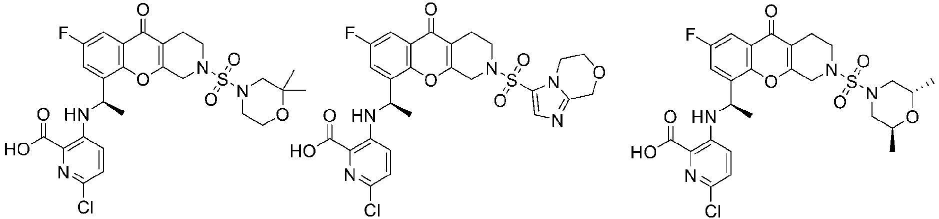

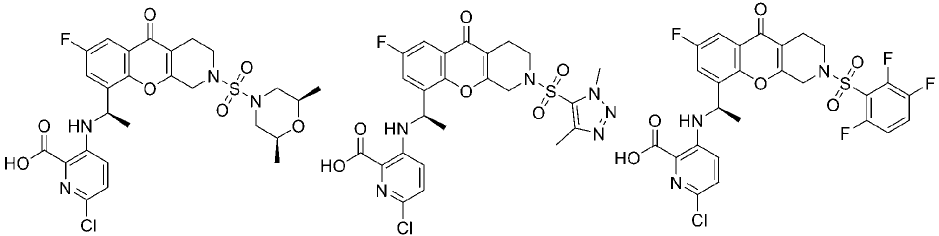

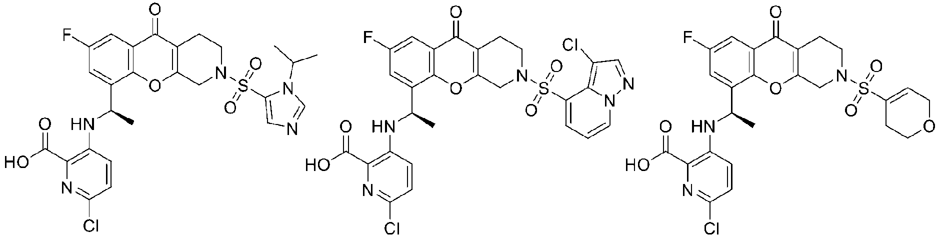

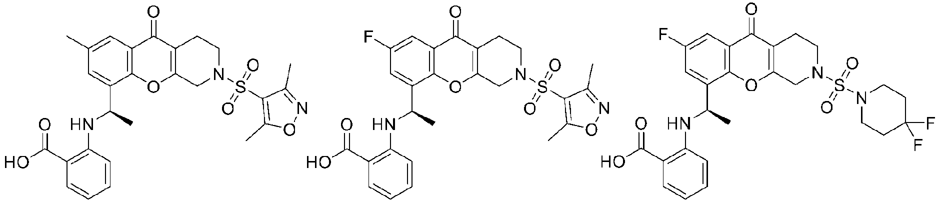

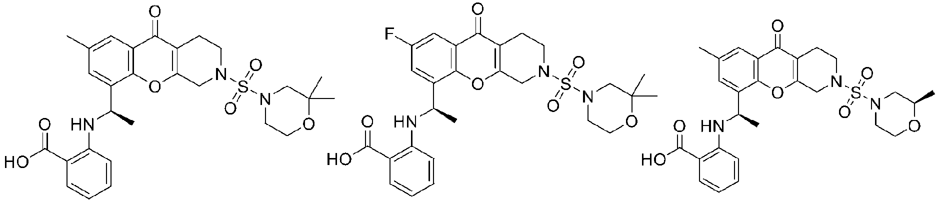

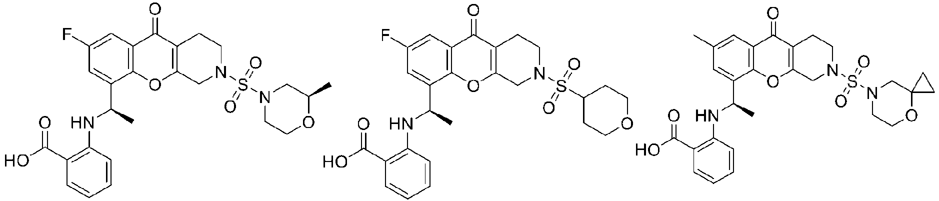

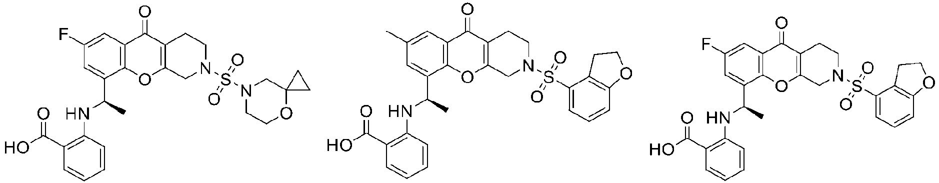

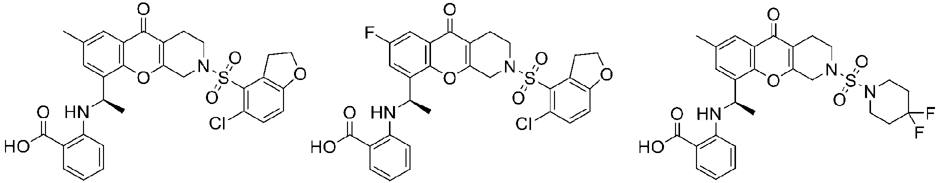

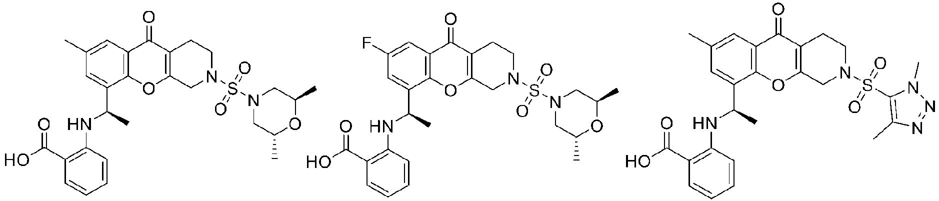

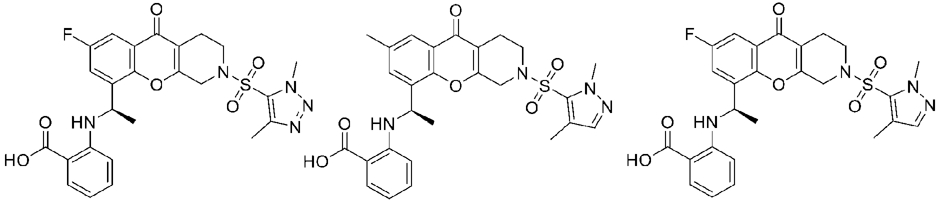

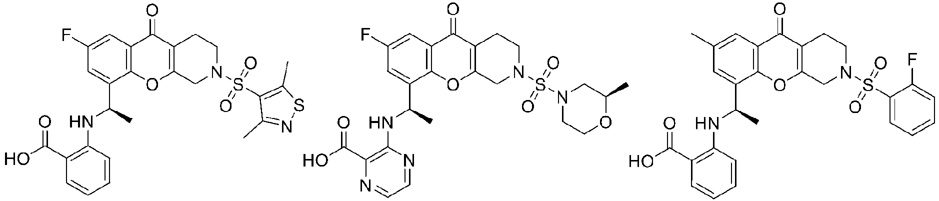

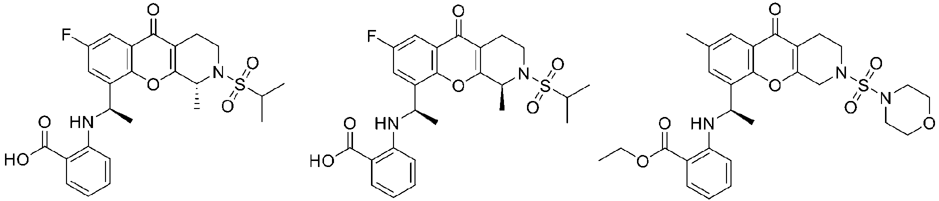

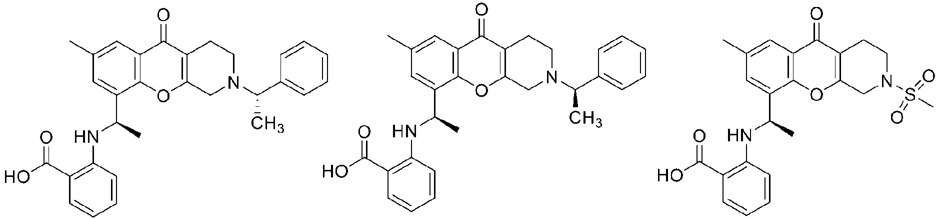

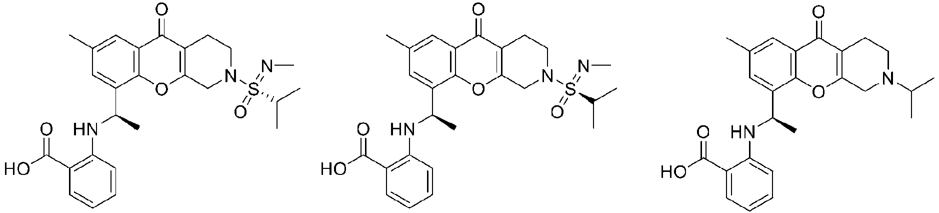

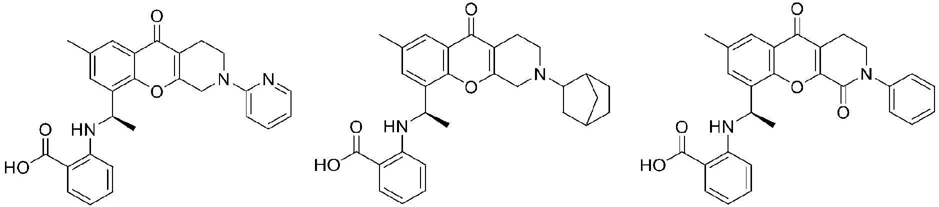

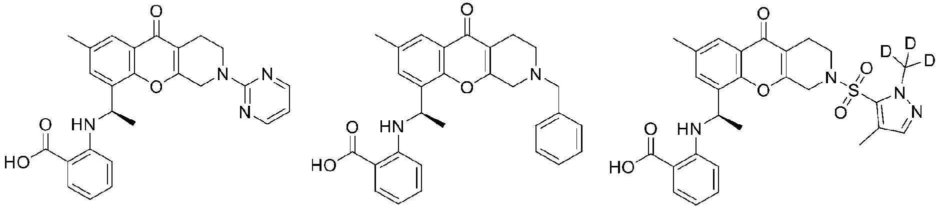

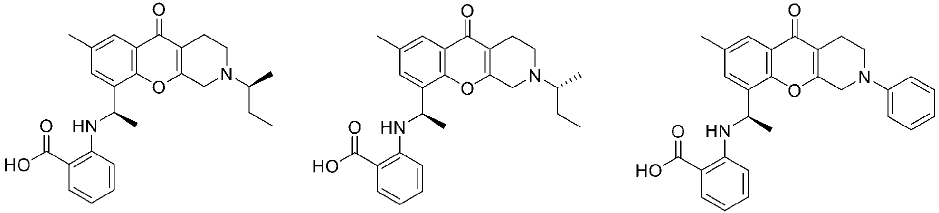

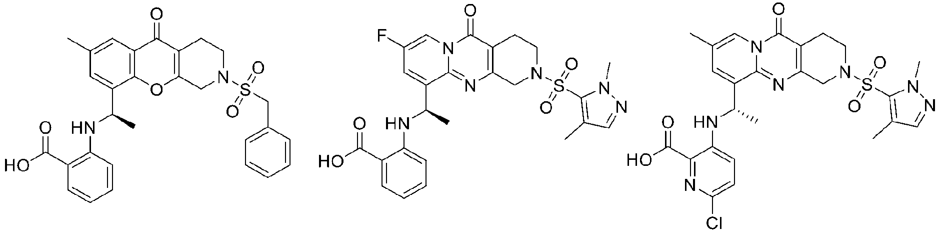

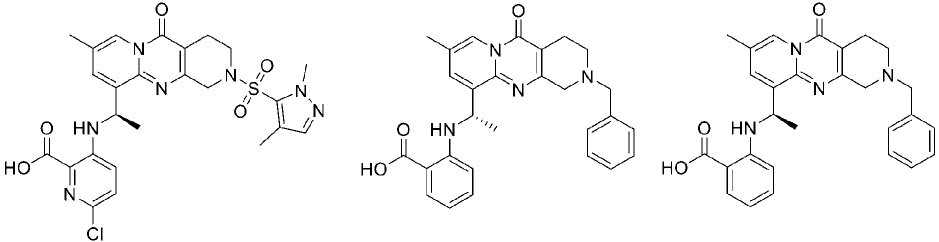

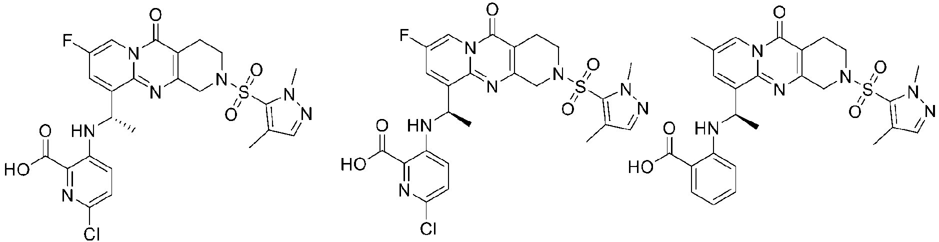

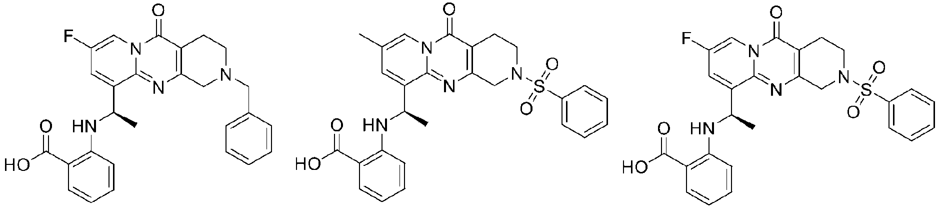

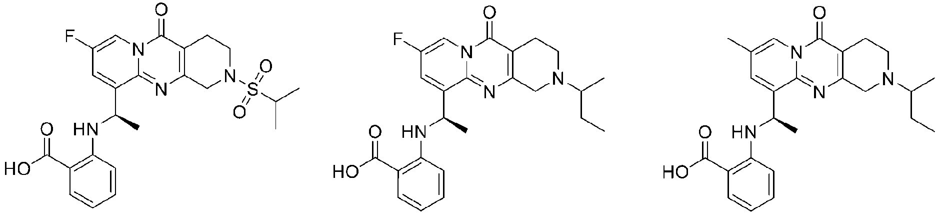

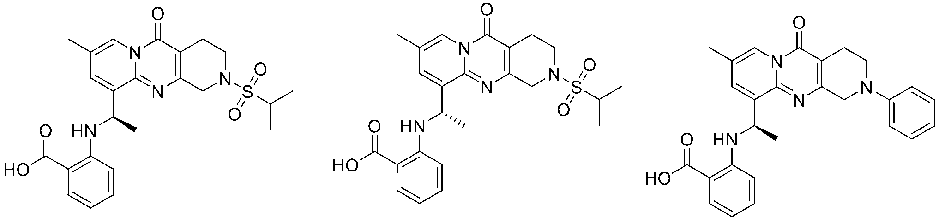

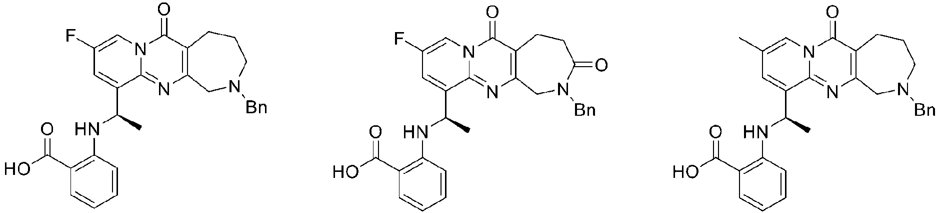

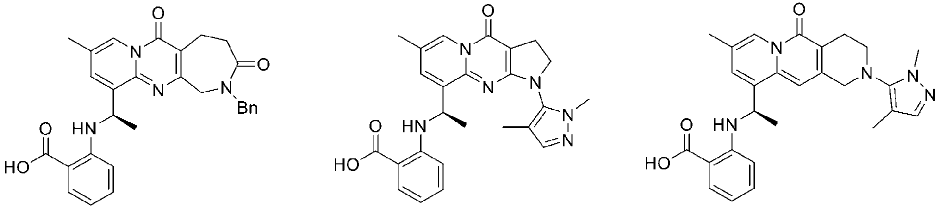

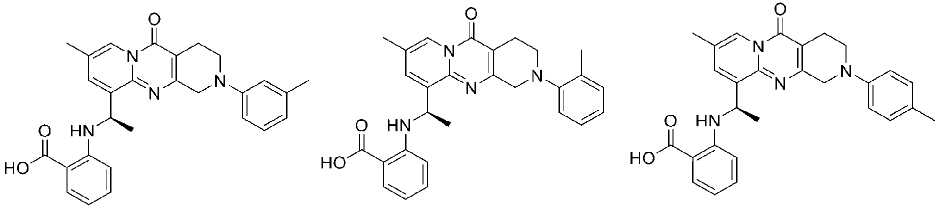

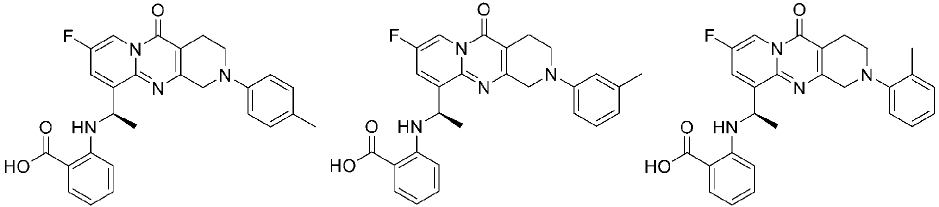

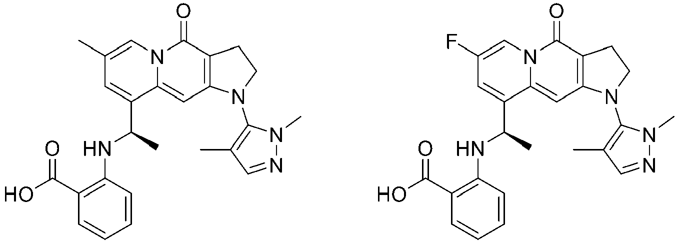

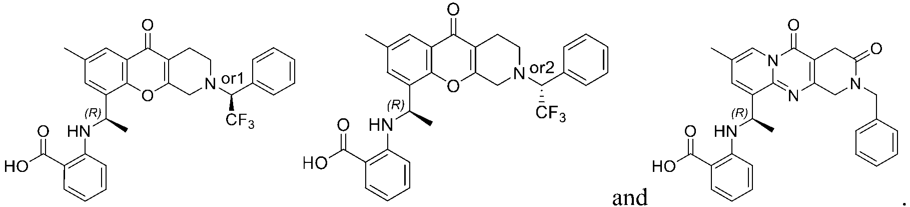

9. A pharmaceutical composition, comprising a safe and effective amount of the compound according to claim 1 or 2, or the pharmaceutically acceptable salt, the stereoisomer, the deuterated compound, the solvate, or the prodrug thereof, and pharmaceutically acceptable carriers.

10. Use of the compound according to claim 1 or 2, or the pharmaceutically acceptable salt, the stereoisomer, the deuterated compound, the solvate, or the prodrug thereof, for a use selected from the group consisting of:
1) preparation of selective PI3Kα inhibitors;
2) preparation of drugs for regulating PI3Kα activity or treating PI3Kα-related diseases.
